(19) Europäisches Patentamt
European Patent Office
Office européen des brevets

(11) EP 4 656 640 A1

(12) **EUROPEAN PATENT APPLICATION**
published in accordance with Art. 153(4) EPC

(43) Date of publication:
**03.12.2025 Bulletin 2025/49**

(21) Application number: **24766366.9**

(22) Date of filing: **01.03.2024**

(51) International Patent Classification (IPC):
***C07D 487/04*** (2006.01) ***C07D 401/14*** (2006.01)
***C07D 471/04*** (2006.01) ***C07D 471/08*** (2006.01)
***C07D 471/10*** (2006.01) ***A61K 31/513*** (2006.01)
***A61K 31/496*** (2006.01) ***A61K 31/40*** (2006.01)
***A61P 35/00*** (2006.01) ***A61P 37/00*** (2006.01)
***A61P 25/00*** (2006.01) ***A61P 29/00*** (2006.01)

(52) Cooperative Patent Classification (CPC):
**A61K 31/40; A61K 31/496; A61K 31/513; A61P 25/00; A61P 29/00; A61P 35/00; A61P 37/00; C07D 401/14; C07D 471/04; C07D 471/08; C07D 471/10; C07D 487/04**

(86) International application number:
**PCT/CN2024/079646**

(87) International publication number:
**WO 2024/183650 (12.09.2024 Gazette 2024/37)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **03.03.2023 CN 202310204306**
**11.05.2023 CN 202310532885**
**15.06.2023 CN 202310715434**
**09.08.2023 CN 202311004166**
**22.02.2024 CN 202410196769**

(71) Applicant: **Shanghai Qilu Pharmaceutical Research and Development Centre Ltd.**
**Shanghai 201203 (CN)**

(72) Inventors:
- **BI, Fangchao**
  **Shanghai 201203 (CN)**
- **QIN, Hua**
  **Shanghai 201203 (CN)**
- **FU, Jiasheng**
  **Shanghai 201203 (CN)**
- **SHI, Guqin**
  **Shanghai 201203 (CN)**
- **QU, Shulan**
  **Shanghai 201203 (CN)**
- **LIU, Xin**
  **Shanghai 201203 (CN)**
- **MA, Huimin**
  **Shanghai 201203 (CN)**
- **CHEN, Bo**
  **Shanghai 201203 (CN)**
- **SUN, Daqing**
  **Shanghai 201203 (CN)**
- **TAO, Weikang**
  **Shanghai 201203 (CN)**

(74) Representative: **Dai, Simin**
**Reyda IP**
**A073**
**157, Quai du Président Roosevelt**
**92130 Issy-les-Moulineaux (FR)**

# (54) IRAK4 DEGRADER AND USE THEREOF

(57) The present invention relates to an IRAK4 degrader and a use thereof, and specifically relates to a bifunctional compound as represented by formula (I), an isomer thereof, a deuterated product thereof, or a pharmaceutically acceptable salt thereof, and a use of the compound in treatment for IRAK4-mediated related diseases.

EP 4 656 640 A1

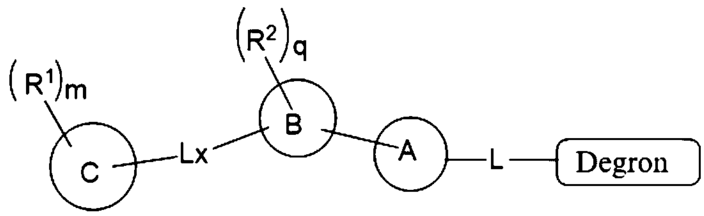

Target structure

Formula (I)

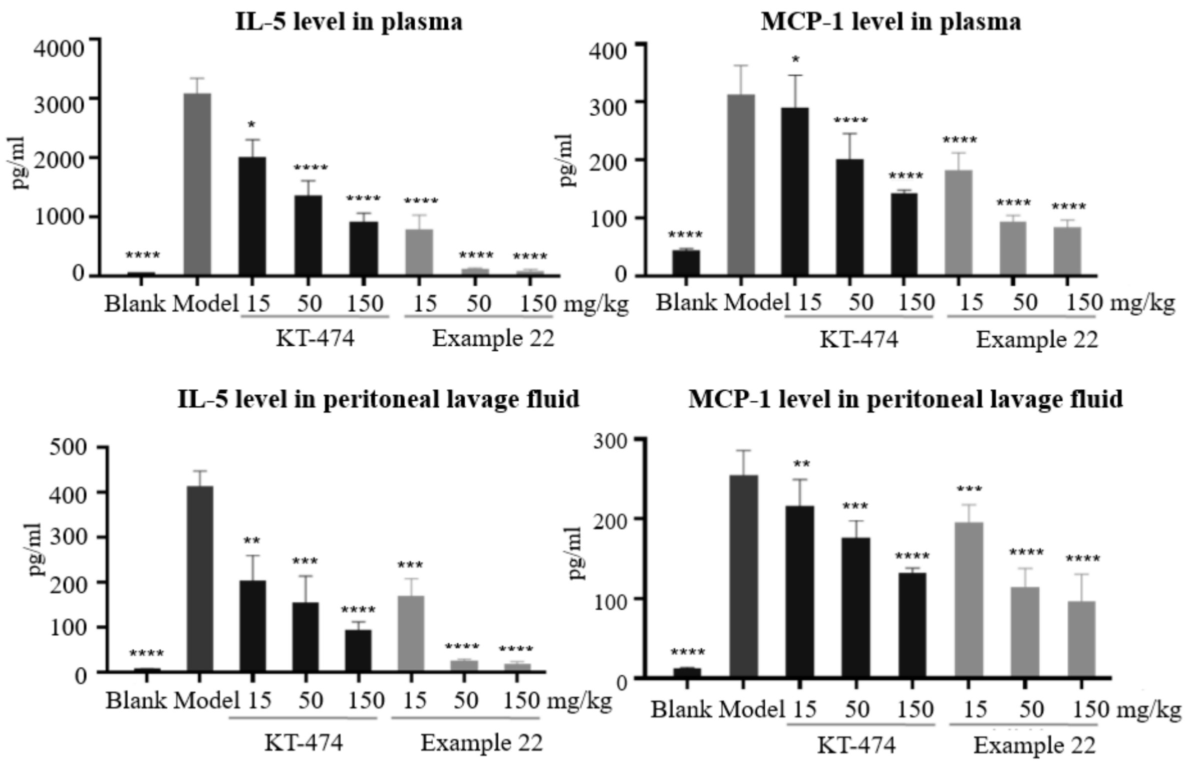


FIG. 1

## Description

The present application claims the priorities of Chinese patent application 2023102043068 filed on March 3, 2023, Chinese patent application 2023105328859 filed on May 11, 2023, Chinese patent application 2023107154349 filed on June 15, 2023, Chinese patent application 2023110041666 filed on August 09, 2023 and Chinese patent application 2024101967699 filed on February 22, 2024. The contents of the above Chinese patent applications are incorporated herein by reference in their entireties.

TECHNICAL FIELD

The present disclosure belongs to the field of medicine, and specifically relates to a compound that binds to interleukin-1 receptor-associated kinase 4 (IRAK4), an isomer thereof, a deuterated compound thereof, or a pharmaceutically acceptable salt thereof, a pharmaceutical composition thereof, a preparation method therefor, and a medical use thereof.

BACKGROUND

Proteolysis-targeting Chimaeras (abbreviated as PROTACs) technology is an emerging new drug development technology in recent years. Unlike the principle of traditional small molecules inhibiting protein function, PROTACs work by delivering these proteins to the proteasome for complete degradation, which breaks the definition of traditional drugs. Compared to traditional small-molecule inhibitor drugs, it offers multiple advantages, such as transforming targets from "undruggable" to "druggable", not relying on "occupancy-driven", and addressing drug resistance issues.

PROTAC is a bifunctional conjugate molecule comprising three parts: one end is a ligand structure targeting the target protein (TBM, a small-molecule inhibitor or common functional group capable of recognizing the target protein); the other end is a ligand structure capable of recruiting a protein degradation system, such as an E3 ligase; and the middle is a linker connecting these two ligands. The PROTAC molecule recruits the target protein (TBM) to the E3 ligase by binding to the target protein, enabling the E3 ligase to ubiquitinate the target protein (the E2 enzyme linked to the E3 ligase brings the TBM into sufficiently close proximity for polyubiquitination). The ubiquitinated target protein can be degraded via the proteasomal pathway. There are more than 600 types of E3 ligases in the human body, yet so far only approximately 10 types have been developed for use in protein degraders (CRBN, VHL, IAP, MDM2, DCAF15, DCAF16, RNF114, etc.), with the most widely used ones being CRBN and VHL. These two E3 ligases exhibit the highest functional domains and enable flexible and efficient degradation of a wide range of target proteins, with relatively wide ranges of expression to achieve high levels of systemic degradation.

Interleukin-1 receptor-associated kinase 4 (IRAK-4), as a member of the intracellular serine-threonine kinase IRAK family, mediates the inflammatory signaling pathways mediated by the activation of Toll-like receptors (TLRs) and IL-1 receptors (IL-1Rs), and plays a pivotal bridging role in the innate immune signaling pathways. IRAK4 can be activated by being recruited by MyD88 to form the Myddosome, thereby activating downstream IRAK1/2, leading to their subsequent hyperphosphorylation, dissociation from the complex, and binding to TRAF6. The activated TRAF6 complex triggers downstream signaling pathways to generate pro-inflammatory factors (NF-κB, CREB, AP-1, IRF, etc.). The inhibition of IRAK4 may play an important role in the onset and progression of inflammatory diseases. Currently, no inhibitors targeting IRAK4 have been approved. Therefore, knocking out IRAK4 using PROTAC technology may be another strategy for treating IRAK4-related diseases.

KT-474 developed by Kymera is a potent, highly selective, orally bioavailable IRAK4 degrader that primarily functions as a heterobifunctional molecule targeting IRAK4-binding to IRAK4 and recruiting E3 ubiquitin ligase to "tag" IRAK4, thereby utilizing the proteasome degradation system to degrade IRAK4. KT-474 is primarily used for the treatment of IL-1R/TLR-driven diseases, including atopic dermatitis (HS) and hidradenitis suppurativa (AD), as well as rheumatoid arthritis (RA) and other inflammatory diseases. In preclinical studies, KT-474 exhibits potent anti-inflammatory activity. Results from healthy patients in the Phase I clinical trial demonstrated that a single dose of KT-474 dose-dependently reduced the levels of IRAK4 and various pro-inflammatory cytokines, with good safety and tolerability.

Although there are IRAK4 degraders under development for the treatment of inflammatory diseases, there is still great uncertainty before the products reach the market. Clinically, there is still a lack of drugs with structural diversity, therapeutic differentiation, and good safety. Therefore, the continued development of novel IRAK4 degraders to expand the variety of clinical samples and improve patient accessibility remains of significant clinical importance.

SUMMARY

The present disclosure provides a bifunctional compound targeting IRAK4 (i.e., a PROTAC molecule targeting IRAK4), which can effectively recruit IRAK4 to an E3 ubiquitin ligase for degradation.

The bifunctional compound targeting IRAK4 described in the present disclosure, i.e., an IRAK4 degrader, is a tripartite compound comprising a target structure, a linker, and a degron, as shown in formula (X),

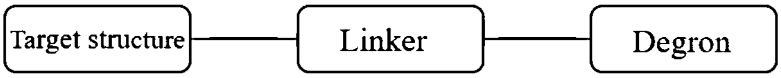


(X)

wherein the target structure may specifically bind to a target protein, such as an IRAK protein, and is linked to the linker in the tripartite compound via a covalent bond; the linker is a connecting group between the target structure and the degron, with one end covalently bonded to the target structure and the other end covalently bonded to the degron; the degron can bind to a ubiquitin ligase, such as an E3 ubiquitin ligase, and is covalently bonded to the linker.

Specifically, in a first aspect, the present disclosure provides a bifunctional compound represented by the following general formula (I), an isomer thereof, a deuterated compound thereof, or a pharmaceutically acceptable salt thereof,

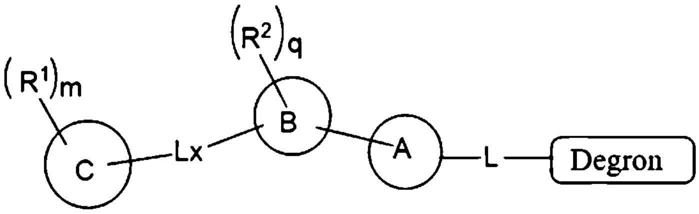

Target structure

Formula (I)

wherein ring C is phenyl, 5- to 6-membered heteroaryl, or 9- to 10-membered fused heteroaryl;

$R^1$ is selected from the group consisting of -CN, $-NR^{1a}R^{1b}$, -OH, halogen, $C_{1-6}$ alkyl, halo-$C_{1-6}$ alkyl, hydroxy-$C_{1-6}$ alkyl, $C_{1-6}$ alkoxy, halo-$C_{1-6}$ alkoxy, 3- to 6-membered cycloalkyl, 3- to 6-membered heterocyclyl, 7- to 9-membered bridged heterocyclyl, 6- to 10-membered fused heterocyclyl, 3- to 6-membered cycloalkyl-NH-, phenyl, 5- to 6-membered heteroaryl, and 9- to 10-membered fused heteroaryl, wherein the heterocyclyl, bridged heterocyclyl, fused heterocyclyl, cycloalkyl, heteroaryl, and fused heteroaryl are optionally substituted by one or more Rx, and the Rx is selected from the group consisting of $-NH_2$, $-NR^{1a}R^{1b}$, -CN, -OH, halogen, $C_{1-6}$ alkyl, and halo-$C_{1-6}$ alkyl;

$R^{1a}$ and $R^{1b}$ are each independently selected from the group consisting of H, $C_{1-6}$ alkyl, halo-$C_{1-6}$ alkyl, 3- to 6-membered cycloalkyl, 3- to 6-membered cycloalkyl-$C_{1-6}$ alkyl-, 3- to 6-membered heterocyclyl, and 3- to 6-membered heterocyclyl-$C_{1-6}$ alkyl-;

m is selected from the group consisting of 0, 1, 2, 3, and 4;

ring B is selected from the group consisting of 5- to 6-membered heteroaryl and 9- to 10-membered fused heteroaryl;

$R^2$ is selected from the group consisting of H, halogen, $C_{1-6}$ alkyl, halo-$C_{1-6}$ alkyl, hydroxy-$C_{1-6}$ alkyl, $C_{1-6}$ alkoxy, and halo-$C_{1-6}$ alkoxy;

q is selected from the group consisting of 0, 1, 2, 3, and 4;

Lx is $-C(O)-NR^{La}-$;

$R^{La}$ is selected from the group consisting of H, $C_{1-6}$ alkyl, halo-$C_{1-6}$ alkyl, and 3- to 6-membered cycloalkyl;

ring A is selected from the group consisting of divalent groups optionally substituted by one or more Rz: 7- to 11-membered spirocycloalkylene, 7- to 11-membered spiroheterocyclylene, 7- to 9-membered bridged cycloalkylene, and 7- to 9-membered bridged heterocyclylene, wherein the Rz is selected from the group consisting of halogen, -OH, $-NH_2$, $C_{1-6}$ alkyl, halo-$C_{1-6}$ alkyl, $C_{1-6}$ alkoxy, halo-$C_{1-6}$ alkoxy, 3- to 6-membered cycloalkyl, and 3-to 6-membered heterocyclyl.

L is $-(B^1)n_1-(B^2)n_2-(B^3)n_3-$;

$B^1$ is a $C_{1-6}$ alkylene chain, any one methylene unit of which is optionally replaced by -O-, -C(O)-, $-C(R^a)(R^b)-$, $-N(R^c)-$, or -S-; $n_1$ is selected from the group consisting of 0 and 1;

$B^2$ is selected from the group consisting of the following divalent rings optionally substituted by one or more Ry: $C_{3-6}$ cycloalkylene, $C_{5-8}$ bridged cycloalkylene, 4- to 6-membered heterocyclylene, 6- to 8-membered bridged heterocyclylene, phenylene, and 6- to 8-membered heterocycloalkenylene, wherein the Ry is selected from the group consisting of oxo, halogen, $C_{1-6}$ alkyl, and halo-$C_{1-6}$ alkyl; $n_2$ is selected from the group consisting of 0 and 1;

$B^3$ is a saturated or unsaturated $C_{1-6}$ hydrocarbylene chain, any one methylene unit of which is optionally replaced by -O-, -C(O)-, -C($R^a$)($R^b$)-, -N($R^c$)-, or -S-; $n_3$ is selected from the group consisting of 0 and 1;

$R^a$ and $R^b$ are each independently selected from the group consisting of H, deuterium, halogen, $C_{1-6}$ alkyl, and halo-$C_{1-6}$ alkyl, and $R^a$ and $R^b$ are not simultaneously H;

alternatively, $R^a$ and $R^b$ on the same carbon atom together with the carbon atom to which they are attached form a 3- to 6-membered cycloalkyl ring or a 3- to 6-membered heterocyclyl ring;

$R^c$ is selected from the group consisting of H and $C_{1-6}$ alkyl;

the degron is a ligand that binds to an E3 enzyme.

In another preferred embodiment, the bifunctional compound, the isomer thereof, the deuterated compound thereof, or the pharmaceutically acceptable salt thereof has a structure represented by the following formula (IA),

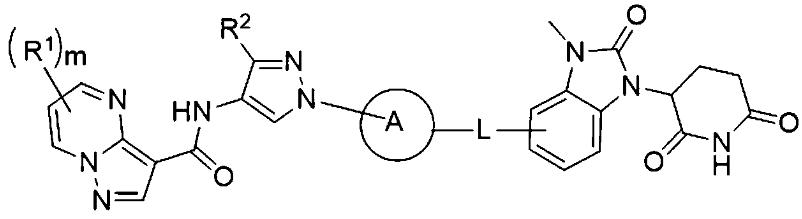

Target structure Degron

Formula (IA)

wherein $R^1$ is selected from the group consisting of -CN, halogen, $C_{1-4}$ alkyl, halo-$C_{1-4}$ alkyl, $C_{1-4}$ alkoxy, 5- to 6-membered heterocyclyl, 7- to 9-membered bridged heterocyclyl, 6-to 10-membered fused heterocyclyl, and 3- to 6-membered cycloalkyl-NH-, wherein the heterocyclyl, bridged heterocyclyl, fused heterocyclyl, and cycloalkyl are optionally substituted by one or more Rx, and the Rx is selected from the group consisting of -$NH_2$, -OH, halogen, $C_{1-4}$ alkyl, and halo-$C_{1-4}$ alkyl;

m is selected from the group consisting of 0, 1, 2, 3, and 4;

$R^2$ is selected from the group consisting of $C_{1-4}$ alkyl and halo-$C_{1-4}$ alkyl;

ring A is selected from the group consisting of the following divalent groups: 7- to 11-membered spirocycloalkylene, 7- to 11-membered spiroheterocyclylene, 7- to 9-membered bridged cycloalkylene, and 7- to 9-membered bridged heterocyclylene;

L is -$(B^1)n_1$-$(B^2)n_2$-$(B^3)n_3$-;

$B^1$ is a $C_{1-3}$ alkylene chain, any one methylene unit of which is optionally replaced by -O-, -C(O)-, -C($R^a$)($R^b$)-, -N($R^c$)-, or -S-; $n_1$ is selected from the group consisting of 0 and 1;

$B^2$ is selected from the group consisting of the following divalent rings optionally substituted by one or more Ry: $C_{3-6}$ cycloalkylene, $C_{5-8}$ bridged cycloalkylene, 4- to 6-membered heterocyclylene, 6- to 8-membered bridged heterocyclylene, phenylene, and 6- to 8-membered heterocycloalkenylene, wherein the Ry is selected from the group consisting of oxo, halogen, $C_{1-4}$ alkyl, and halo-$C_{1-4}$ alkyl; $n_2$ is selected from the group consisting of 0 and 1;

$B^3$ is a saturated or unsaturated $C_{1-6}$ hydrocarbylene chain, any one methylene unit of which is optionally replaced by -O-, -C(O)-, -C($R^a$)($R^b$)-, -N($R^c$)-, or -S-; $n_3$ is selected from the group consisting of 0 and 1;

$R^a$ and $R^b$ are each independently selected from the group consisting of H, deuterium, halogen, $C_{1-4}$ alkyl, and halo-$C_{1-4}$ alkyl, and $R^a$ and $R^b$ are not simultaneously H;

alternatively, $R^a$ and $R^b$ on the same carbon atom together with the carbon atom to which they are attached form a 3- to 6-membered cycloalkyl ring;

$R^c$ is selected from the group consisting of H and $C_{1-4}$ alkyl.

In another preferred embodiment, the bifunctional compound, the isomer thereof, the deuterated compound thereof, or the pharmaceutically acceptable salt thereof,

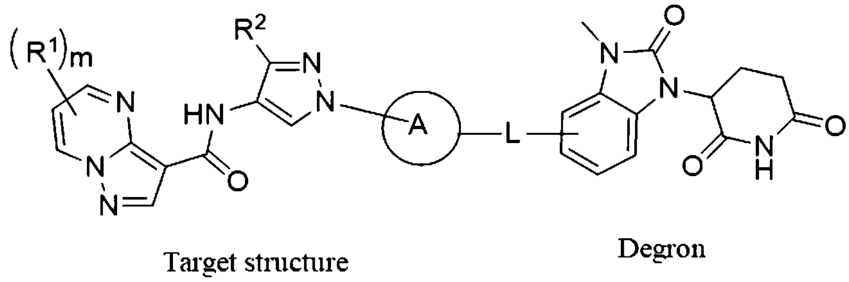


Formula (IA)

wherein $R^1$ is selected from the group consisting of -CN, halogen, $C_{1-4}$ alkyl, halo-$C_{1-4}$ alkyl, $C_{1-4}$ alkoxy, 5- to 6-membered heterocyclyl, 7- to 9-membered bridged heterocyclyl, 6-to 10-membered fused heterocyclyl, and 3- to 6-membered cycloalkyl-NH-, wherein the heterocyclyl, bridged heterocyclyl, fused heterocyclyl, and cycloalkyl are optionally substituted by one or more Rx, and the Rx is selected from the group consisting of -$NH_2$, -OH, halogen, $C_{1-4}$ alkyl, and halo-$C_{1-4}$ alkyl;

m is selected from the group consisting of 0, 1, 2, 3, and 4;

$R^2$ is selected from the group consisting of $C_{1-4}$ alkyl and halo-$C_{1-4}$ alkyl;

ring A is selected from the group consisting of the following divalent groups: 7- to 11-membered spirocycloalkylene, 7- to 11-membered spiroheterocyclylene, 7- to 9-membered bridged cycloalkylene, and 7- to 9-membered bridged heterocyclylene;

L is -$(B^1)n_1$-$(B^2)n_2$-$(B^3)n_3$-;

$B^1$ is a $C_{1-3}$ alkylene chain, any one methylene unit of which is optionally replaced by -O-, -C(O)-, -C($R^a$)($R^b$)-, -N($R^c$)-, or -S-; $n_1$ is selected from the group consisting of 0 and 1;

$B^2$ is selected from the group consisting of the following divalent rings optionally substituted by one or more Ry: $C_{3-6}$ cycloalkylene, $C_{6-8}$ bridged cycloalkylene, 4- to 6-membered heterocyclylene, 6- to 8-membered bridged heterocyclylene, phenylene, and 6- to 8-membered heterocycloalkenylene, wherein the Ry is selected from the group consisting of oxo, halogen, $C_{1-4}$ alkyl, and halo-$C_{1-4}$ alkyl; $n_2$ is selected from the group consisting of 0 and 1;

$B^3$ is a saturated or unsaturated $C_{1-6}$ hydrocarbylene chain, any one methylene unit of which is optionally replaced by -O-, -C(O)-, -C($R^a$)($R^b$)-, -N($R^c$)-, or -S-; $n_3$ is selected from the group consisting of 0 and 1;

$R^a$ and $R^b$ are each independently selected from the group consisting of H, deuterium, halogen, $C_{1-4}$ alkyl, and halo-$C_{1-4}$ alkyl, and $R^a$ and $R^b$ are not simultaneously H;

alternatively, $R^a$ and $R^b$ on the same carbon atom together with the carbon atom to which they are attached form a 3- to 6-membered cycloalkyl ring;

$R^c$ is selected from the group consisting of H and $C_{1-4}$ alkyl.

In another preferred embodiment, the bifunctional compound, the isomer thereof, the deuterated compound thereof, or the pharmaceutically acceptable salt thereof,

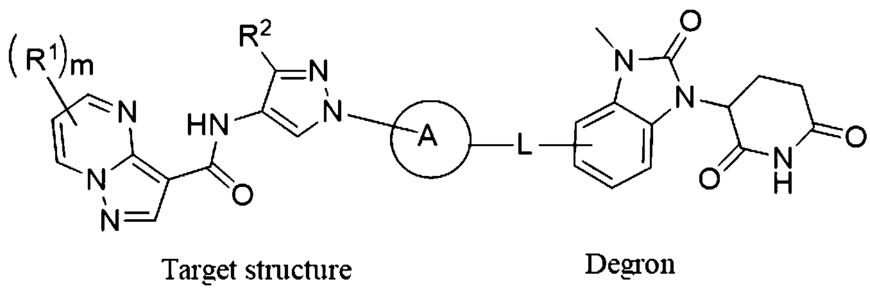


Formula (IA)

wherein $R^1$ is selected from the group consisting of -CN, halogen, $C_{1-4}$ alkyl, halo-$C_{1-4}$ alkyl, $C_{1-4}$ alkoxy, 5- to 6-membered heterocyclyl, 7- to 9-membered bridged heterocyclyl, 6-to 10-membered fused heterocyclyl, and 3- to 6-membered cycloalkyl-NH-, wherein the heterocyclyl, bridged heterocyclyl, fused heterocyclyl, and cycloalkyl are optionally substituted by one or more Rx, and the Rx is selected from the group consisting of -$NH_2$, -OH, halogen, $C_{1-4}$

alkyl, and halo-$C_{1-4}$ alkyl;

m is selected from the group consisting of 0, 1, 2, 3, and 4;

$R^2$ is selected from the group consisting of $C_{1-4}$ alkyl and halo-$C_{1-4}$ alkyl;

ring A is selected from the group consisting of the following divalent groups: 7- to 11-membered spirocycloalkylene, 7- to 11-membered spiroheterocyclylene, 7- to 9-membered bridged cycloalkylene, and 7- to 9-membered bridged heterocyclylene;

L is $-(B^1)n_1-(B^2)n_2-(B^3)n_3-$;

$B^1$ is a $C_{1-3}$ alkylene chain, any one methylene unit of which is optionally replaced by -O-, -C(O)-, $-C(R^a)(R^b)-$, $-N(R^c)-$, or -S-; $n_1$ is selected from the group consisting of 0 and 1;

$B^2$ is selected from the group consisting of the following divalent rings optionally substituted by one or more Ry: $C_{3-6}$ cycloalkylene, $C_{6-8}$ bridged cycloalkylene, 4- to 6-membered heterocyclylene, and 6- to 8-membered bridged heterocyclylene, wherein the Ry is selected from the group consisting of oxo, halogen, $C_{1-4}$ alkyl, and halo-$C_{1-4}$ alkyl; $n_2$ is selected from the group consisting of 0 and 1;

$B^3$ is a saturated or unsaturated $C_{1-6}$ hydrocarbylene chain, any one methylene unit of which is optionally replaced by -O-, -C(O)-, $-C(R^a)(R^b)-$, $-N(R^c)-$, or -S-; $n_3$ is selected from the group consisting of 0 and 1;

$R^a$ and $R^b$ are each independently selected from the group consisting of H, halogen, $C_{1-4}$ alkyl, and halo-$C_{1-4}$ alkyl, and $R^a$ and $R^b$ are not simultaneously H;

alternatively, $R^a$ and $R^b$ on the same carbon atom together with the carbon atom to which they are attached form a 3- to 6-membered cycloalkyl ring;

$R^c$ is selected from the group consisting of H and $C_{1-4}$ alkyl.

In another preferred embodiment, the heteroatom in the heterocyclyl, bridged heterocyclyl, fused heterocyclyl, heteroaryl, fused heteroaryl, or spiroheterocyclyl is independently selected from the group consisting of one, two, or three types of N, O, and S, and the number of heteroatoms is independently 1, 2, 3, or 4.

In another preferred embodiment, $R^1$ is selected from the group consisting of -CN, halogen, $C_{1-6}$ alkyl, halo-$C_{1-6}$ alkyl, $C_{1-6}$ alkoxy, 3- to 6-membered heterocyclyl, 7- to 9-membered bridged heterocyclyl, 6- to 10-membered fused heterocyclyl, and 3- to 6-membered cycloalkyl-NH-.

In another preferred embodiment, $R^1$ is 7- to 9-membered bridged heterocyclyl.

In another preferred embodiment, $R^1$ is selected from the group consisting of -F, $-CH_3$, -CN, $-CHF_2$, $-CF_3$, $-OCH_3$,

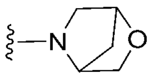, 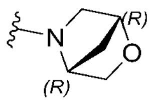, 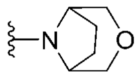, 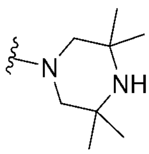,

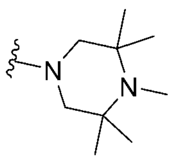, 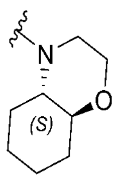, and 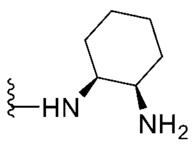;

m is selected from the group consisting of 1 and 2.

In another preferred embodiment, $R^1$ is selected from the group consisting of

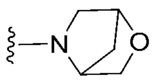 and 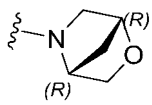.

In another preferred embodiment, the Rx is selected from the group consisting of - $NH_2$ and $C_{1-6}$ alkyl.

In another preferred embodiment, m is selected from the group consisting of 1 and 2, preferably 1.

In another preferred embodiment, ring B is 5- to 6-membered heteroaryl.

In another preferred embodiment, $R^2$ is $C_{1-6}$ alkyl or halo-$C_{1-6}$ alkyl, preferably halo-$C_{1-6}$ alkyl.

In another preferred embodiment, $R^2$ is selected from the group consisting of $-CH_3$, - $CH_2F$, $-CHF_2$, and $-CF_3$.

In another preferred embodiment, $R^2$ is $-CHF_2$.

In another preferred embodiment, q is 1.

In another preferred embodiment, Lx is -C(O)-$NR^{La}$-*, wherein the * terminus is connected to ring B.

In another preferred embodiment, ring A is selected from the group consisting of the following unsubstituted divalent groups: 7- to 11-membered spirocycloalkylene, 7- to 11-membered spiroheterocyclylene, 7- to 9-membered bridged cycloalkylene, and 7- to 9-membered bridged heterocyclylene.

In another preferred embodiment, ring A is 7- to 9-membered nitrogen-containing spiroheterocyclylene.

In another preferred embodiment, ring A is selected from the group consisting of

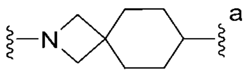 , 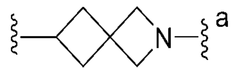 , 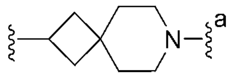 , 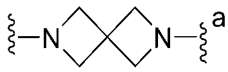 ,

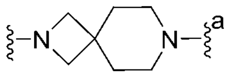 , 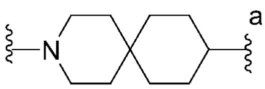 , 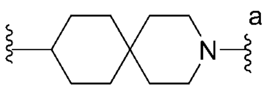 , 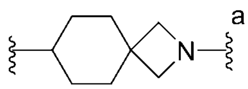 ,

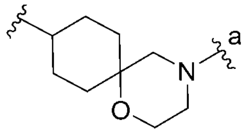 , 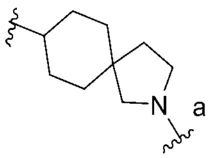 , 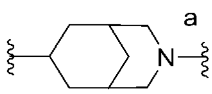 , 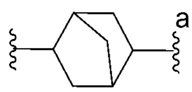 , 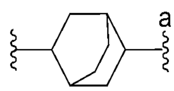 ,

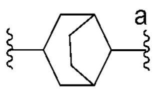 , 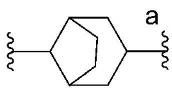 , 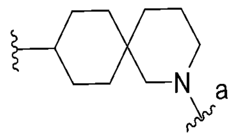 , 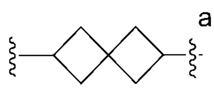 , 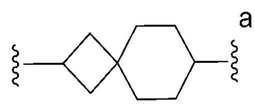 ,

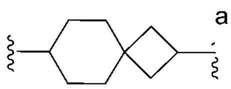 , and 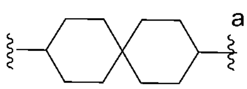 ,

wherein the "a" terminus denotes the end connected to L in general formula (IA).

In another preferred embodiment, ring A is

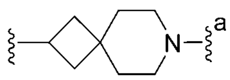 .

In another preferred embodiment, in L, -$(B^3)_{n_3}$- terminus is connected to the degron.

In another preferred embodiment, L is selected from the group consisting of -$B^1$-$B^2$-$B^3$-, -$B^1$-$B^3$-, and -$B^2$-$B^3$-; preferably, the -$B^3$- terminus is connected to the degron.

In another preferred embodiment, L is -$B^1$-$B^2$-$B^3$-; preferably, the -$B^3$- terminus is connected to the degron.

In another preferred embodiment, L is -$B^3$-.

In another preferred embodiment, $B^1$ is a $C_{1-3}$ alkylene chain, wherein 1 to 2 methylene units are optionally replaced by -O-, -C(O)-, -C($R^a$)($R^b$)-, or -N($R^c$)-; preferably, 1 methylene unit is optionally replaced by -C(O)-.

In another preferred embodiment, $B^3$ is an unsaturated $C_{2-6}$ hydrocarbylene chain, wherein 1 to 2 methylene units are optionally replaced by -O-, -C(O)-, or -C($R^a$)($R^b$)-; the hydrocarbylene chain is connected to the degron via the alkyne terminus; preferably, 1 methylene unit is optionally replaced by -O-.

In another preferred embodiment, $R^a$ and $R^b$ are each independently selected from the group consisting of H, deuterium, and -$CH_3$; and $R^a$ and $R^b$ are not simultaneously H;

alternatively, $R^a$ and $R^b$ on the same carbon atom together with the carbon atom to which they are attached form a cyclopropyl ring or a cyclobutyl ring.

In another preferred embodiment, $R^c$ is selected from the group consisting of H and - $CH_3$; preferably H.

In another preferred embodiment, $B^2$ is selected from the group consisting of the following groups:

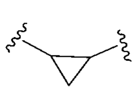, 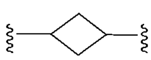, 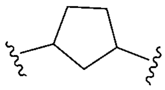, 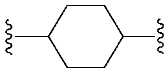, 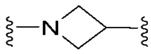,

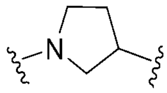, 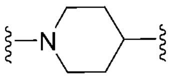, 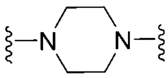, 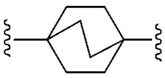, 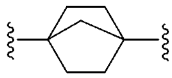, 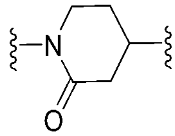,

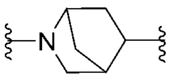, 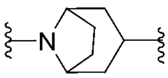, 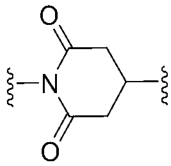, 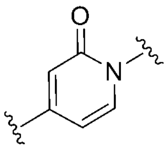, 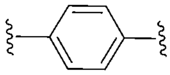, 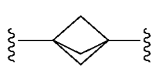,

and

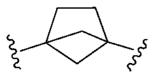;

preferably

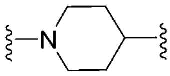, 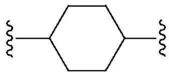, or 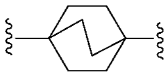.

In another preferred embodiment, $B^2$ is selected from the group consisting of the following groups:

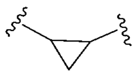, 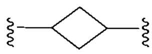, 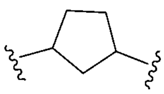, 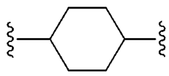, 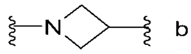,

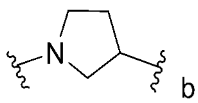, 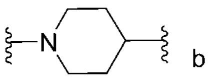, 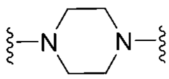, 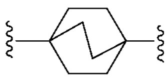, 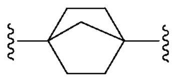,

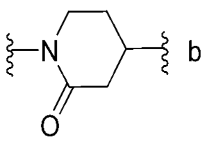, 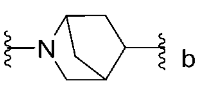, 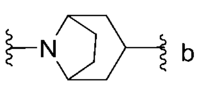, 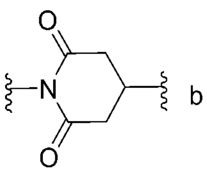, 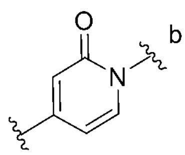,

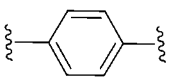, 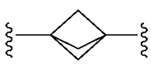, and 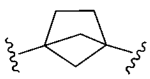,

wherein the b terminus is connected to $-(B^3)n_3-$; preferably

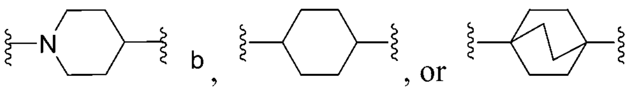

, , or .

In another preferred embodiment, $B^1$ is a $C_{1-3}$ alkylene chain, wherein 1 to 2 methylene units are optionally replaced by -O-, -C(O)-, -C($R^a$)($R^b$)-, or -N($R^c$)-;

$B^3$ is an unsaturated $C_{2-6}$ hydrocarbylene chain, wherein 1 to 2 methylene units are optionally replaced by -O-, -C(O)-, or -C($R^a$)($R^b$)-; the hydrocarbylene chain is connected to the degron via the alkyne terminus;
$R^a$ and $R^b$ are each independently selected from the group consisting of H, deuterium, and $-CH_3$; and $R^a$ and $R^b$ are not simultaneously H;
alternatively, $R^a$ and $R^b$ on the same carbon atom together with the carbon atom to which they are attached form a cyclopropyl ring or a cyclobutyl ring;
$R^c$ is selected from the group consisting of H and $-CH_3$;
$B^2$ is selected from the group consisting of the following groups:

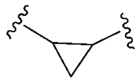,

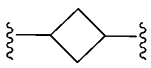, 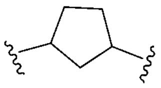, 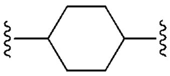, 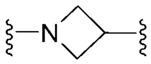, 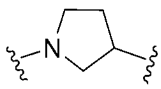, 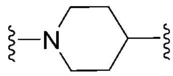,

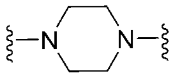, 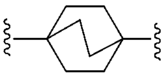, 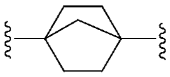, 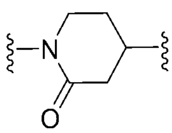, 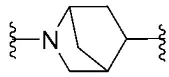, 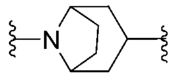,

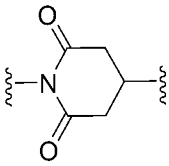, 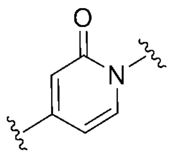, 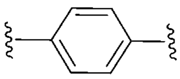, 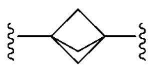, and 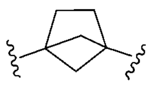.

In another preferred embodiment, $B^1$ is a $C_{1-3}$ alkylene chain, wherein 1 to 2 methylene units are optionally replaced by -O-, -C(O)-, -C($R^a$)($R^b$)-, or -N($R^c$)-;

$B^3$ is an unsaturated $C_{2-6}$ hydrocarbylene chain, wherein 1 to 2 methylene units are optionally replaced by -O-, -C(O)-, or -C($R^a$)($R^b$)-; the hydrocarbylene chain is connected to the degron via the alkyne terminus;
$R^a$ and $R^b$ are each independently selected from the group consisting of H and $-CH_3$; and $R^a$ and $R^b$ are not simultaneously H;
alternatively, $R^a$ and $R^b$ on the same carbon atom together with the carbon atom to which they are attached form a cyclopropyl ring or a cyclobutyl ring;
$R^c$ is selected from the group consisting of H and $-CH_3$;
$B^2$ is selected from the group consisting of the following groups:

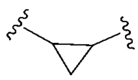,

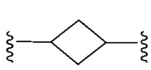, 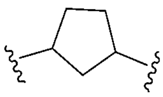, 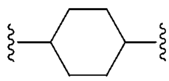, 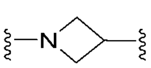, 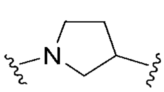, 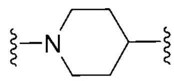,

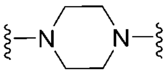, 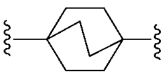, 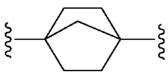, 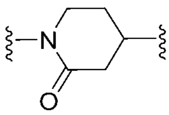, 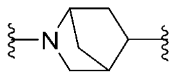, 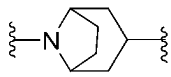,

and

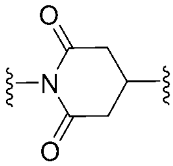;

wherein the description of the $B^2$ group does not limit the connection sequence with the adjacent groups on both sides.

In another preferred embodiment, $B^2$ is selected from the group consisting of the following groups:

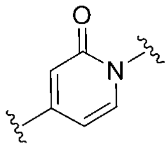 and 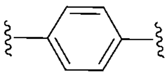.

In another preferred embodiment, $B^2$ is selected from the group consisting of the following groups:

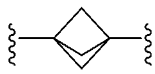 and 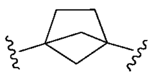.

In another preferred embodiment, $R^a$ and $R^b$ are each independently deuterium.

In another preferred embodiment, $B^3$ is a $C_{2-6}$ alkynylene chain, wherein 1 to 2 methylene units are optionally replaced by -O- or -C(O)-; the alkynylene chain is connected to the degron via the alkyne terminus.

In another preferred embodiment, $B^3$ is a $C_{2-6}$ alkynylene chain containing only one triple bond at the terminal, wherein 1 to 2 methylene units are optionally replaced by -O- or - C(O)-; the alkynylene chain is connected to the degron via the alkyne terminus.

In another preferred embodiment, $B^3$ is a $C_{2-4}$ alkynylene chain containing only one triple bond at the terminal, wherein 1 to 2 methylene units are optionally replaced by -O- or - C(O)-; the alkynylene chain is connected to the degron via the alkyne terminus.

In another preferred embodiment, $B^1$ is selected from the group consisting of $-CH_2-$, $-CD_2-$, and -C(O)-;

$B^2$ is selected from the group consisting of the following divalent rings: $C_{5-6}$ cycloalkylene, $C_{6-8}$ bridged cycloalkylene, 6-membered heterocyclylene, and 6- to 8-membered bridged heterocyclylene;

$B^3$ is selected from the group consisting of

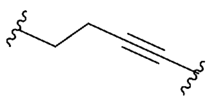, 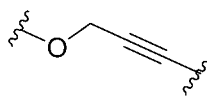,

and

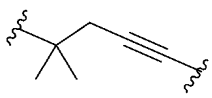.

In another preferred embodiment, L is selected from the group consisting of

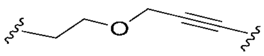 , 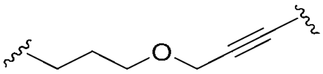 , 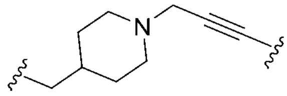 ,

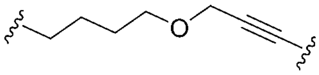 , 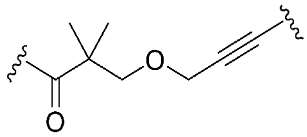 , 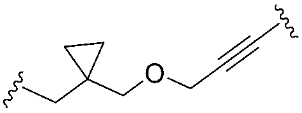 ,

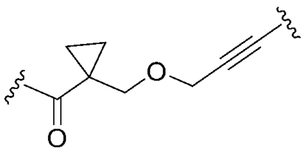 , 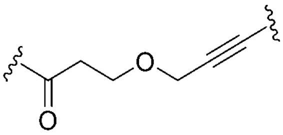 , 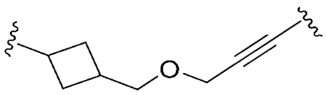 ,

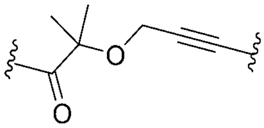 , 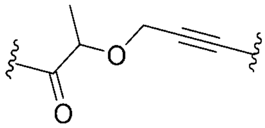 , 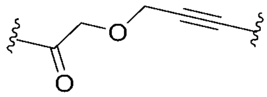 ,

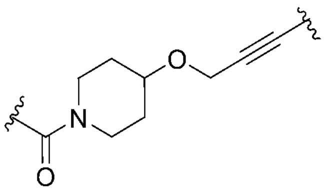 , 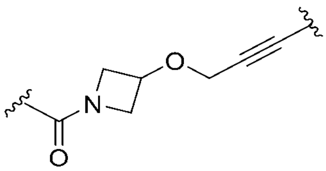 , 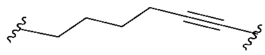 ,

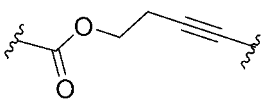 , 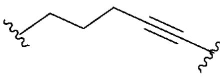 , 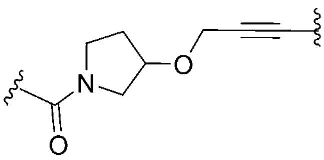 ,

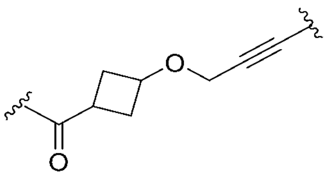 , 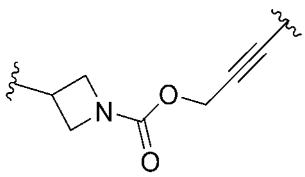 , 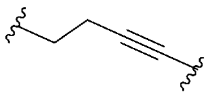 , 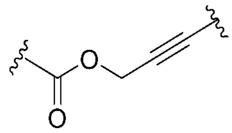 ,

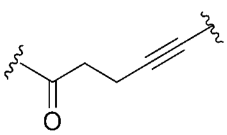 , 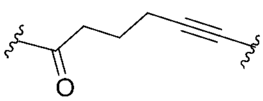 , 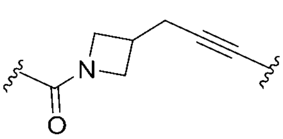 , 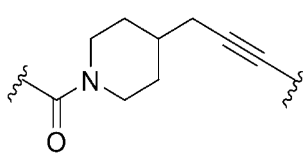 ,

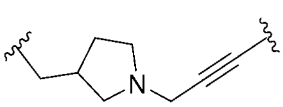 , 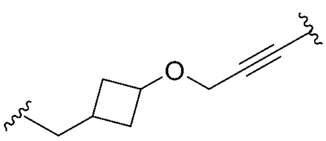 , 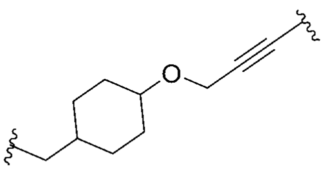 ,

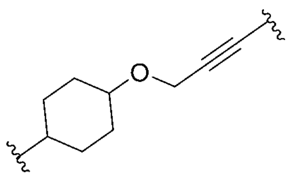 ,
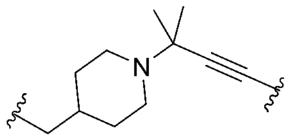 ,
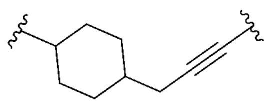 ,

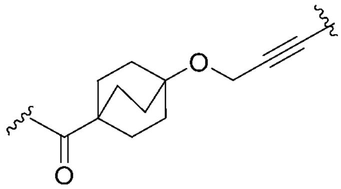 ,
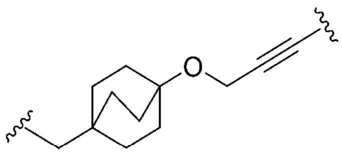 ,
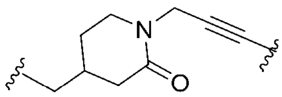 ,

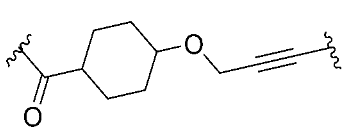 ,
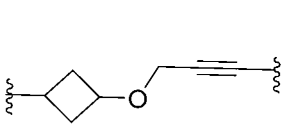 ,
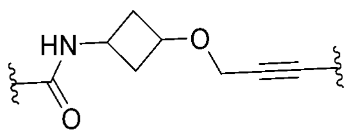 ,

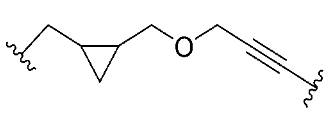 ,
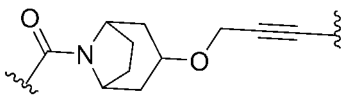 ,
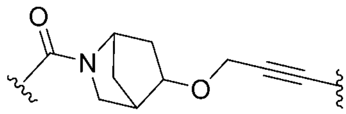 ,

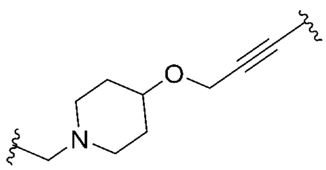 ,
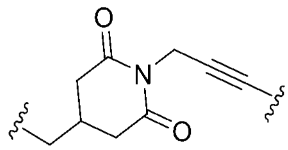 ,
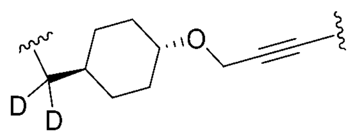 ,

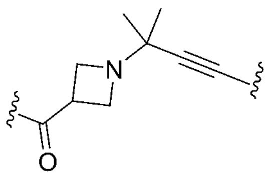 ,
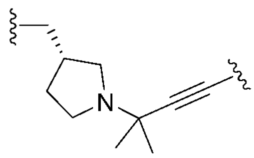 ,
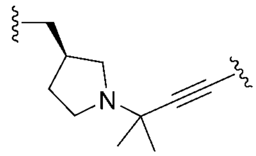 ,
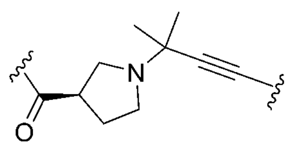 ,

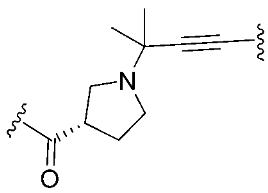 ,
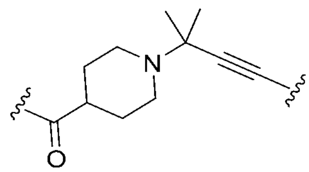 ,
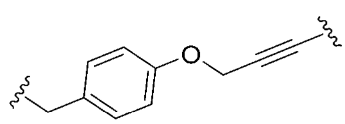 ,

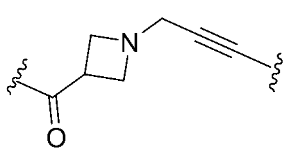 ,
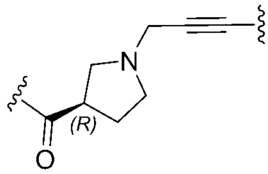 ,
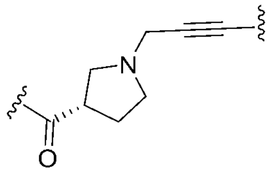 ,
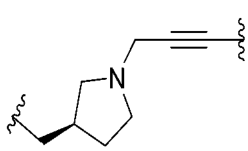 ,

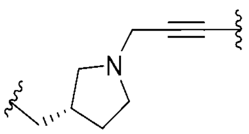, 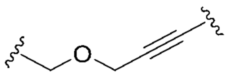, 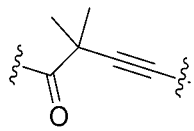, 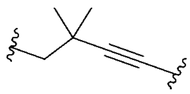,

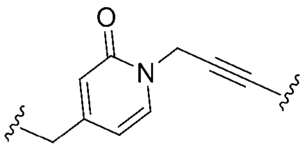, 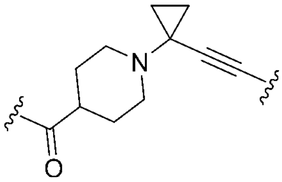, 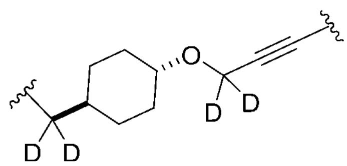,

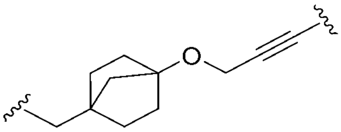, 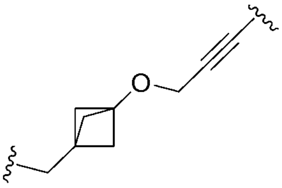, and 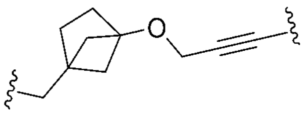;

preferably, the alkyne terminus of L is connected to the degron.

In another preferred embodiment, L is selected from the group consisting of

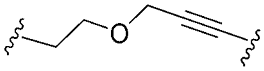, 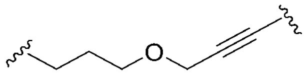, 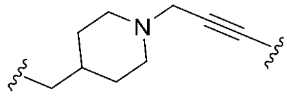,

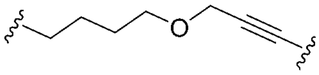, 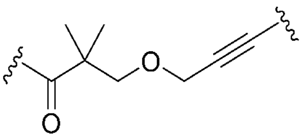, 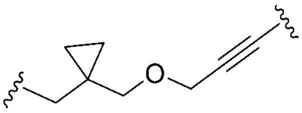,

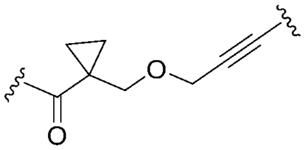, 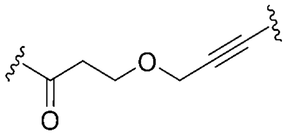, 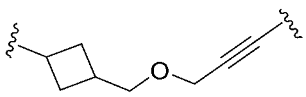,

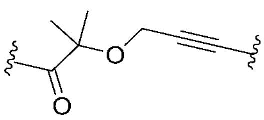, 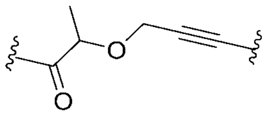, 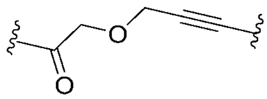,

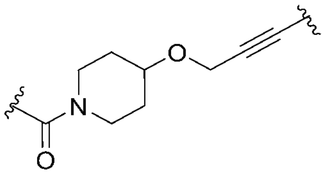, 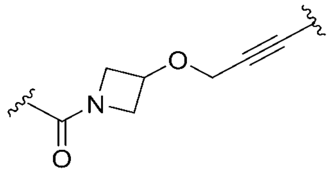, 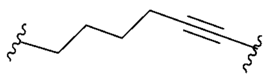,

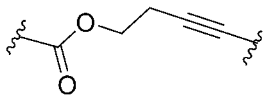, 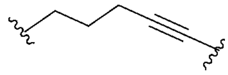, 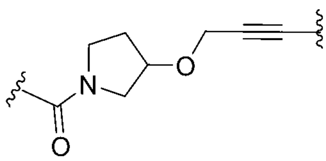,

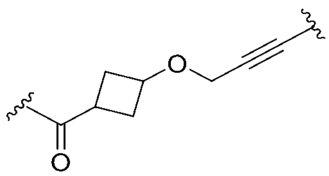, 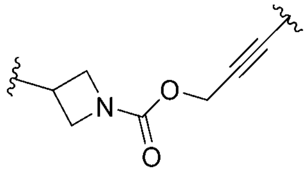, 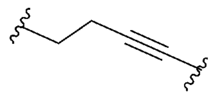, 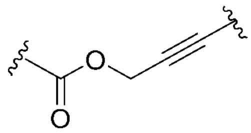,

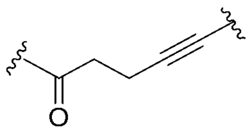, 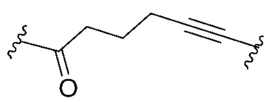, 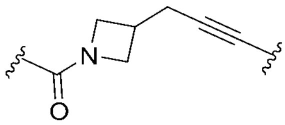, 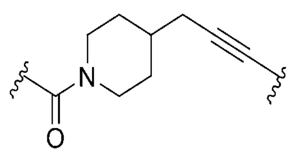,

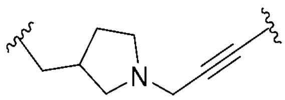, 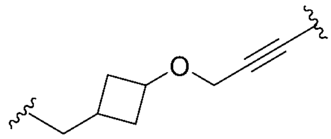, 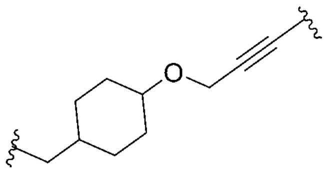,

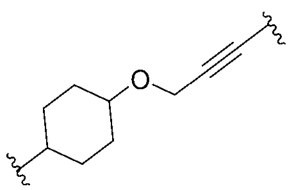, 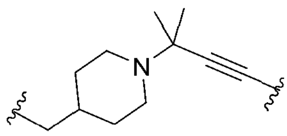, 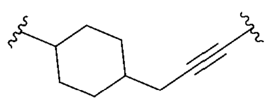,

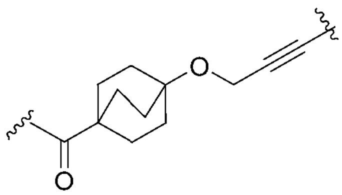, 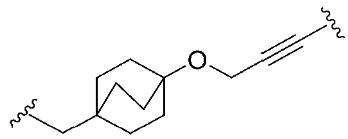, 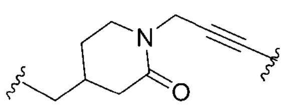,

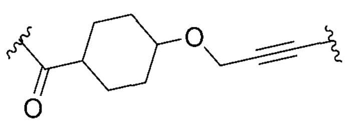, 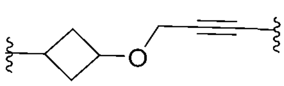, 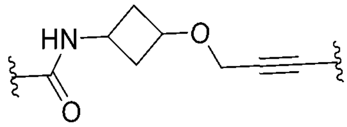,

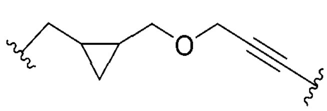, 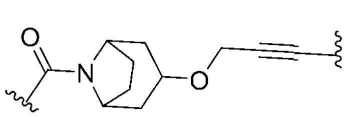, 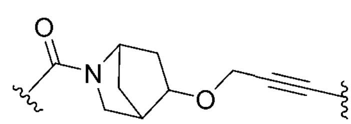,

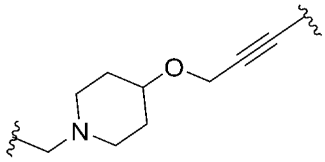, and 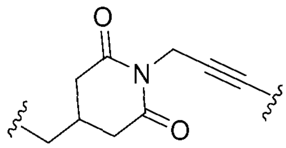;

preferably, the alkyne terminus of L is connected to the degron.

In another preferred embodiment, L is selected from the group consisting of

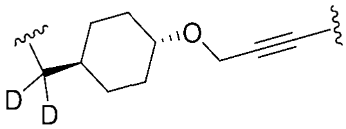, 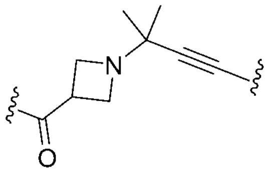, 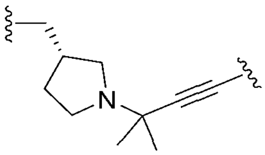, 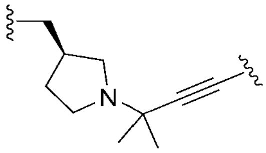, 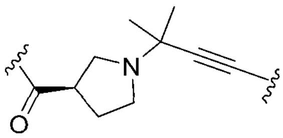, 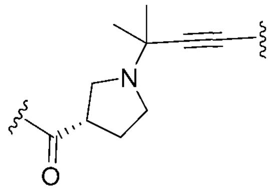, 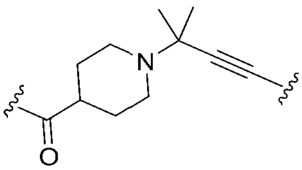, 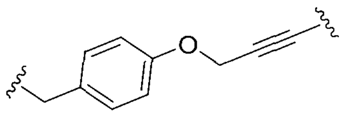, 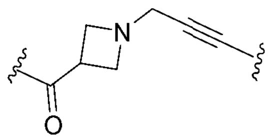, 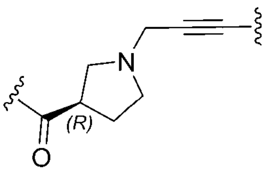, 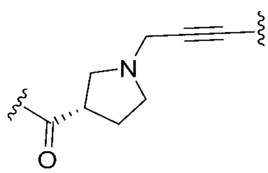, 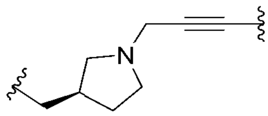, 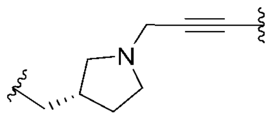, 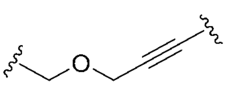, 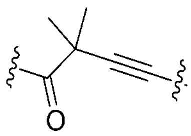, 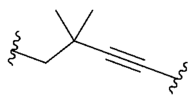, 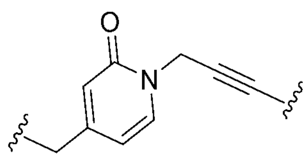, 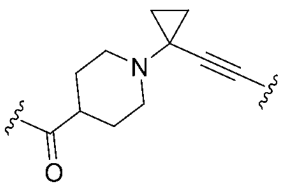, and 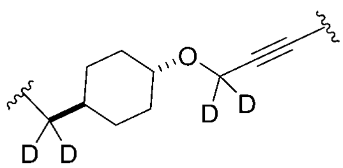;

preferably, the alkyne terminus of L is connected to the degron.

In another preferred embodiment, L is selected from the group consisting of

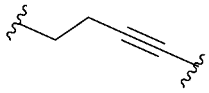, 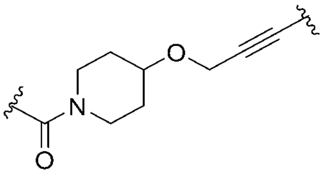, 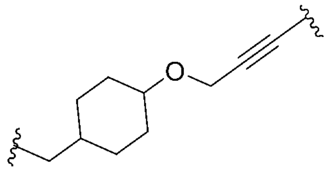, 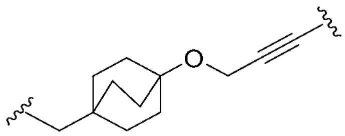, 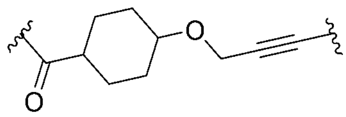, 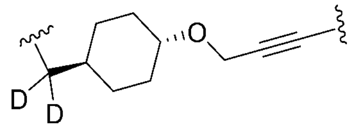, 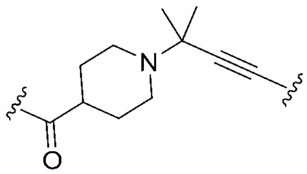, and 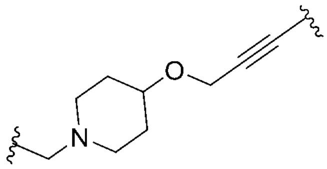;

preferably, the alkyne terminus of L is connected to the degron.

In another preferred embodiment, L is selected from the group consisting of

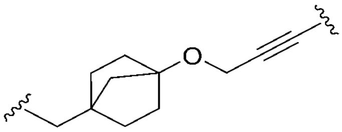, 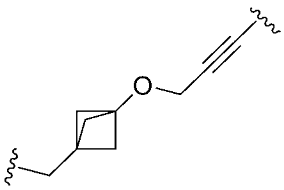, and 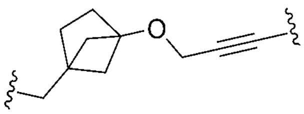;

preferably, the alkyne terminus of L is connected to the degron.

In another preferred embodiment, L is selected from the group consisting of

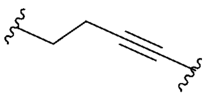, 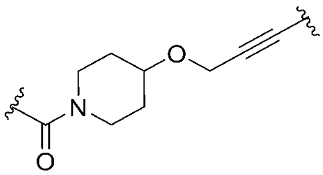, 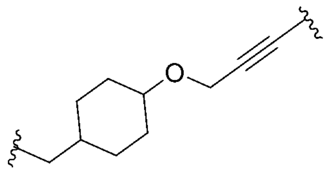,

and

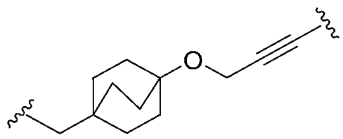;

preferably, the alkyne terminus of L is connected to the degron.

In another preferred embodiment, the bifunctional compound, the isomer thereof, the deuterated compound thereof, or the pharmaceutically acceptable salt thereof has a structure represented by the following general formula:

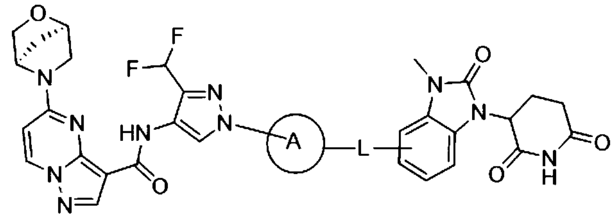

Formula (IA-1)

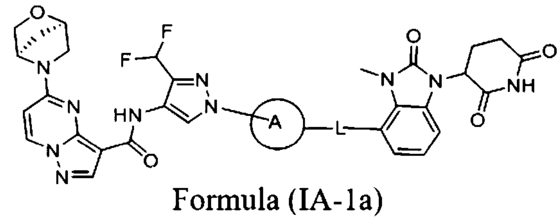

Formula (IA-1a)

or

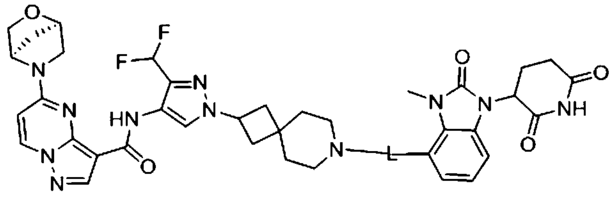

Formula (IA-1b)

ring A and L are as defined in any one of the preceding embodiments.

In another preferred embodiment, each 9- to 10-membered fused heteroaryl is pyrazolopyrimidinyl, for example,

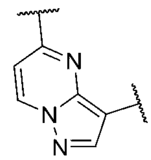

.

In another preferred embodiment, each halogen is fluorine, chlorine, bromine, or iodine, for example, fluorine.

In another preferred embodiment, each $C_{1-6}$ alkyl is $C_{1-4}$ alkyl, for example, methyl.

In another preferred embodiment, each $C_{1-6}$ alkoxy is $C_{1-4}$ alkoxy, for example, methoxy.

In another preferred embodiment, the heteroatom in each 3- to 6-membered heterocyclyl is independently selected from the group consisting of one, two, or three types of N, O, and S, and the number of heteroatoms is independently 1 or 2, for example,

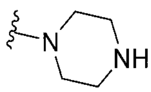

.

In another preferred embodiment, the heteroatom in each 7- to 9-membered bridged heterocyclyl is independently selected from the group consisting of one, two, or three types of N, O, and S, and the number of heteroatoms is independently 1 or 2, for example,

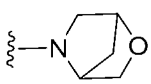

,

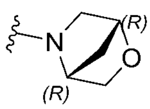

, or

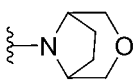

.

In another preferred embodiment, the heteroatom in each 6- to 10-membered fused heterocyclyl is independently selected from the group consisting of one, two, or three types of N, O, and S, and the number of heteroatoms is independently 1 or 2, for example,

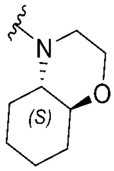.

In another preferred embodiment, each 3- to 6-membered cycloalkyl is cyclopropyl, cyclobutyl, cyclopentyl, or cyclohexyl, for example, cyclohexyl.

In another preferred embodiment, each 5- to 6-membered heteroaryl is pyrazolyl, for example,

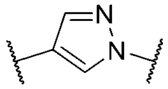.

In another preferred embodiment, each 7- to 11-membered spirocycloalkylene is

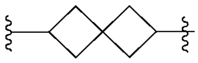, 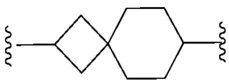, or 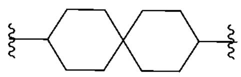.

In another preferred embodiment, each 7- to 11-membered spiroheterocyclylene is

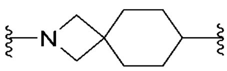, 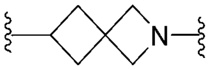, 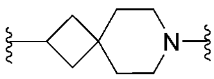, 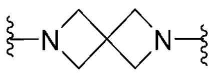, 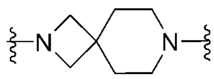,

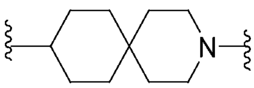, 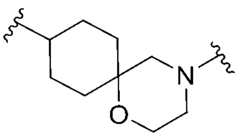, 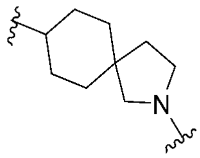, or 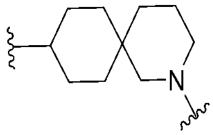.

In another preferred embodiment, each 7- to 9-membered bridged cycloalkylene is

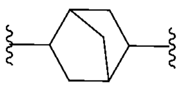, 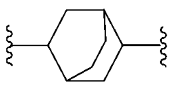, or 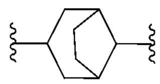.

In another preferred embodiment, each 7- to 9-membered bridged heterocyclylene is

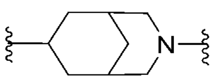.

In another preferred embodiment, each $C_{1-6}$ alkylene chain is a $C_{1-4}$ alkylene chain, for example, methylene or ethylene.

In another preferred embodiment, each $C_{3-6}$ cycloalkylene is:

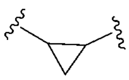,

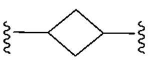, 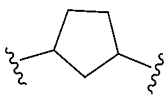, or 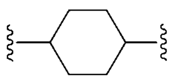.

In another preferred embodiment, each $C_{5\text{-}8}$ bridged cycloalkylene is

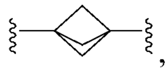,

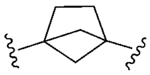, 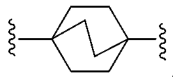, or 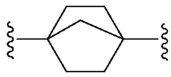.

In another preferred embodiment, each 4- to 6-membered heterocyclylene is

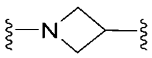, 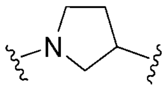, 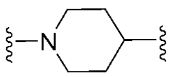, or 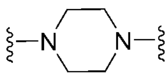.

In another preferred embodiment, each 6- to 8-membered bridged heterocyclylene is

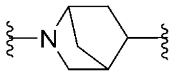 or 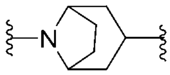.

In another preferred embodiment, each 6- to 8-membered heterocycloalkenylene is

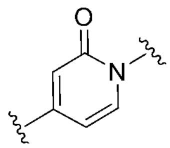.

In another preferred embodiment, each saturated or unsaturated $C_{1\text{-}6}$ hydrocarbylene chain is an unsaturated $C_{3\text{-}6}$ alkynylene chain, for example,

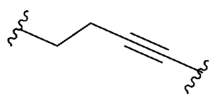.

Any one substituent and any one optional group thereof in the technical solutions described in the present disclosure may be combined with each other to form a new complete technical solution. The newly formed technical solution has the same or similar technical effects as those described in the present application, and all are included within the scope of the present disclosure.

The above technical solutions of the present disclosure may be combined with each other for limitation, and all newly formed solutions are included within the scope of the present disclosure.

In another preferred embodiment, the bifunctional compound, the isomer thereof, the deuterated compound thereof, or the pharmaceutically acceptable salt thereof is selected from the group consisting of the following compounds:

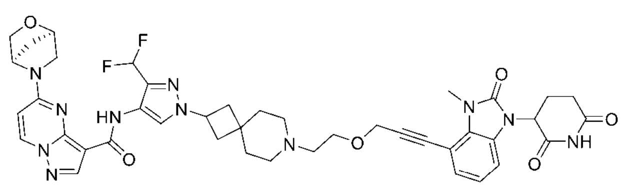 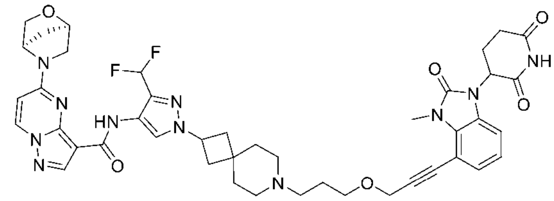

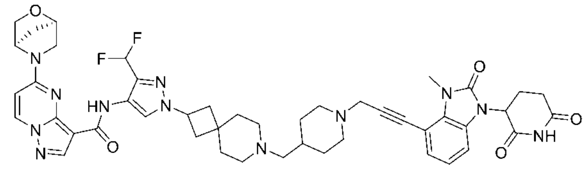

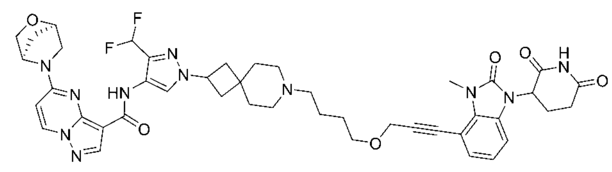

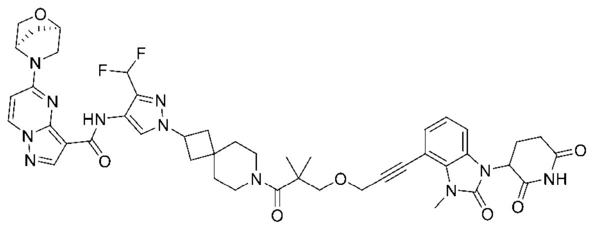

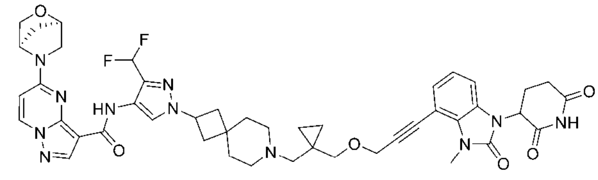

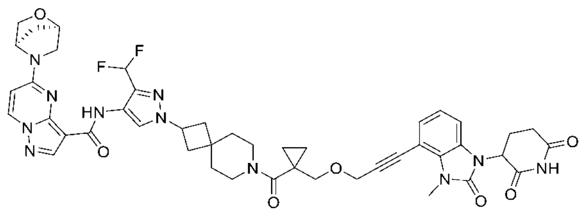

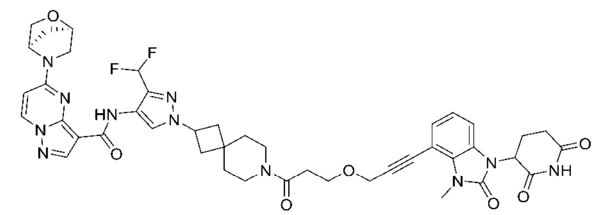

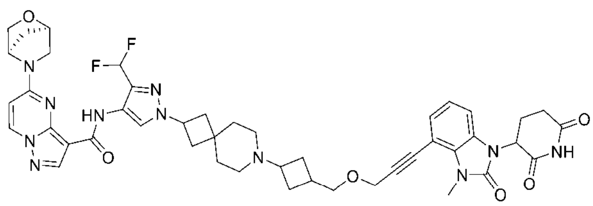

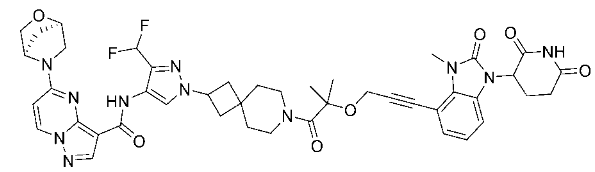

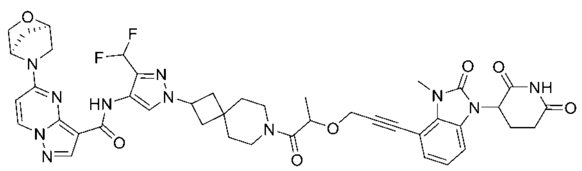

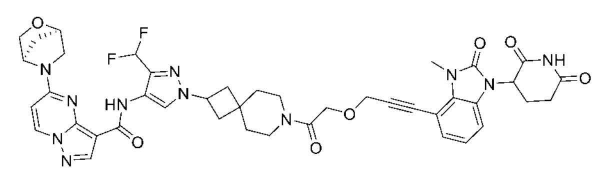

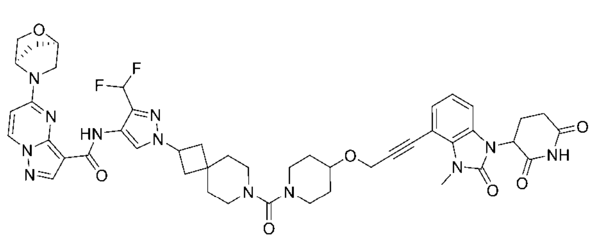

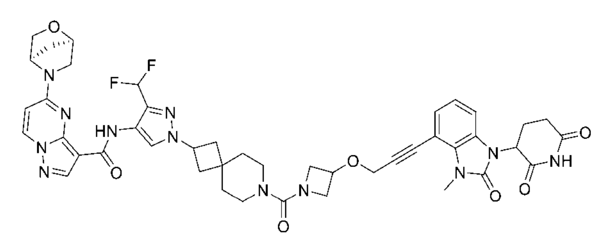

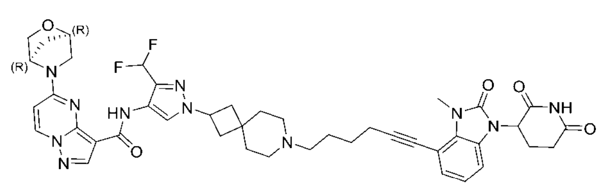

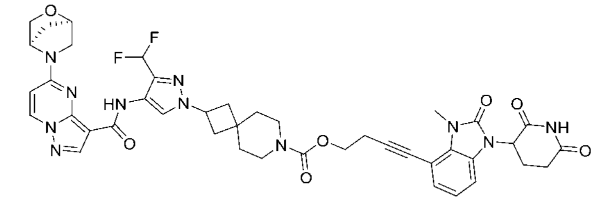

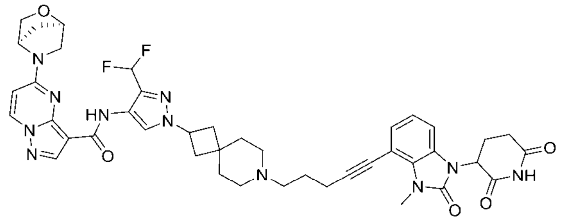

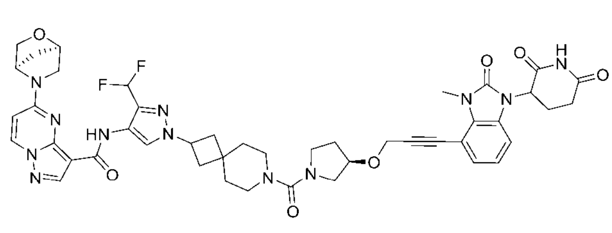

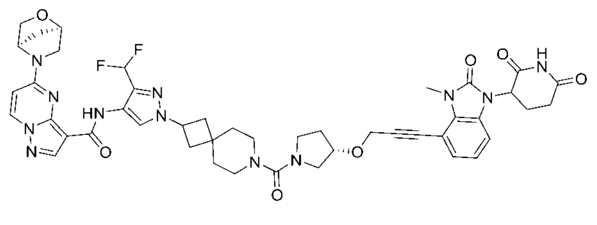

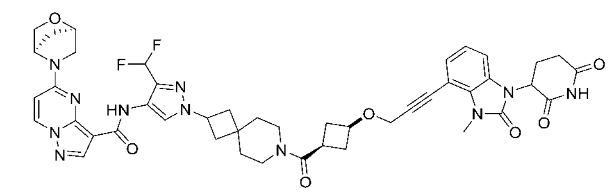

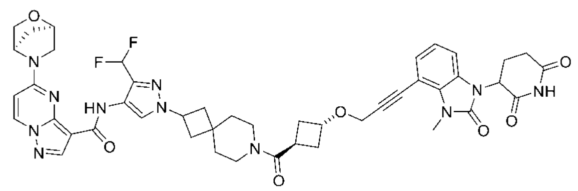

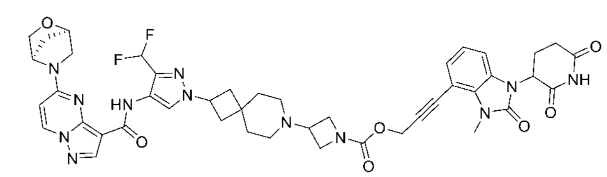

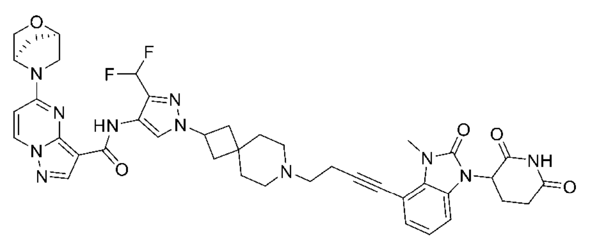

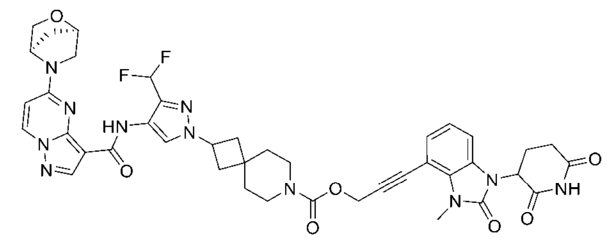

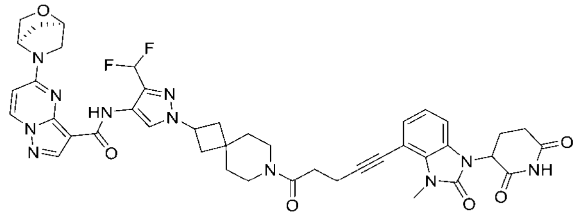

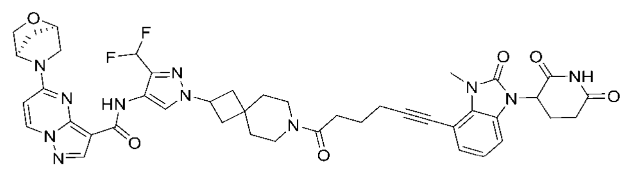

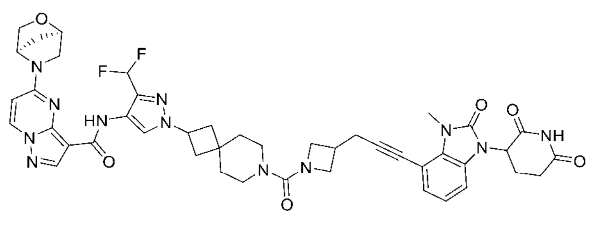

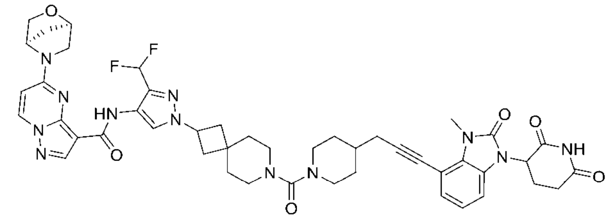

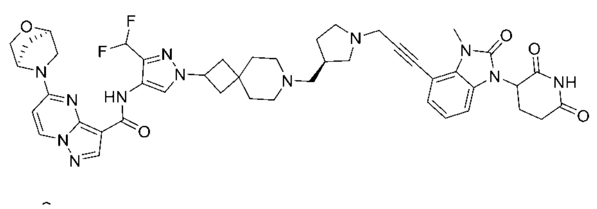

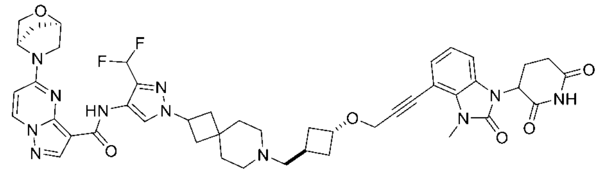

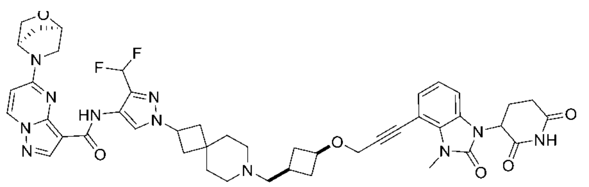

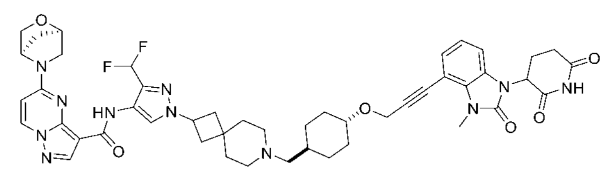

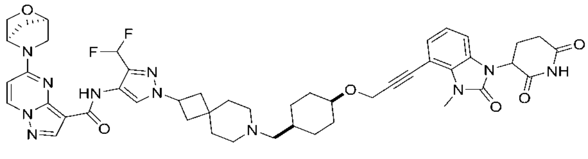

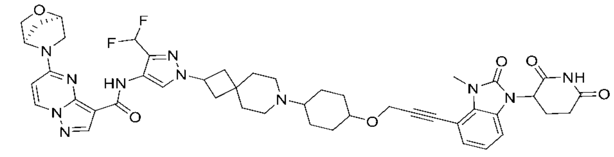

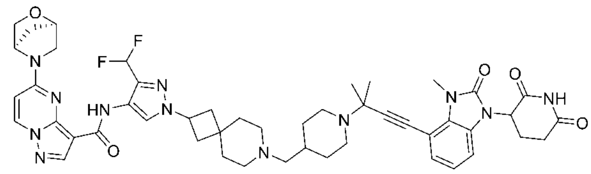

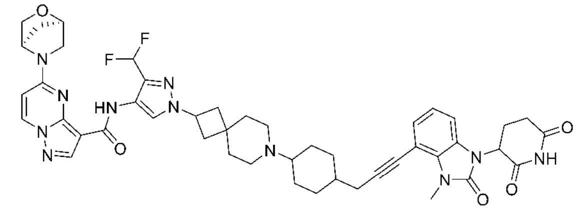

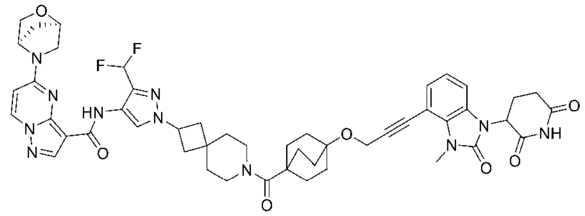

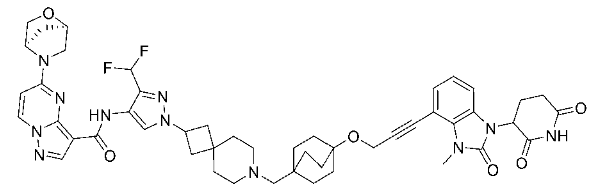

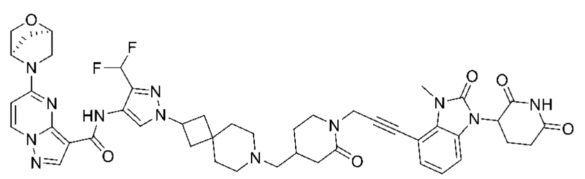

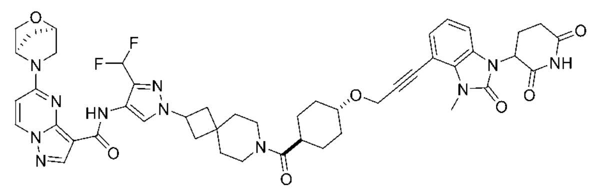

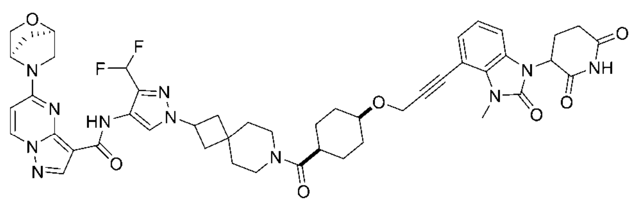

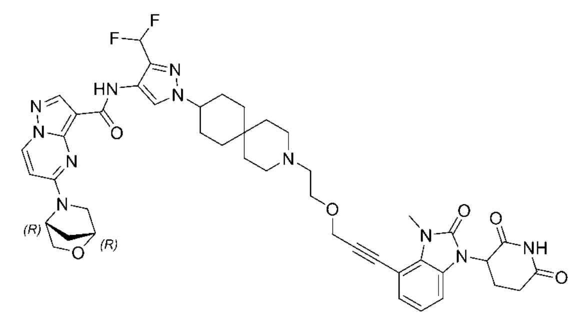

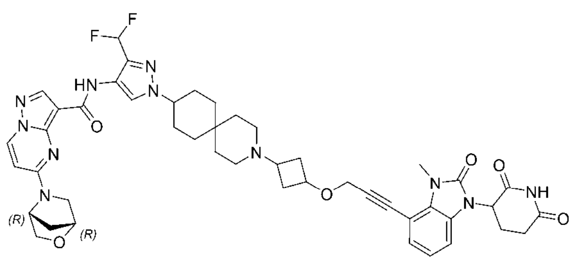

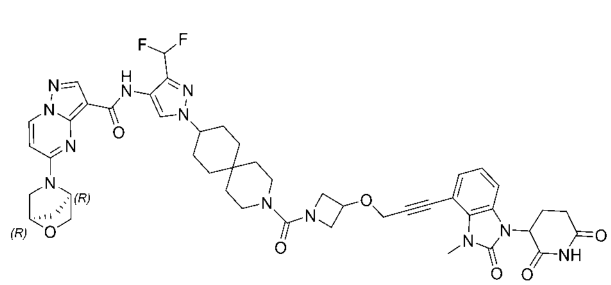

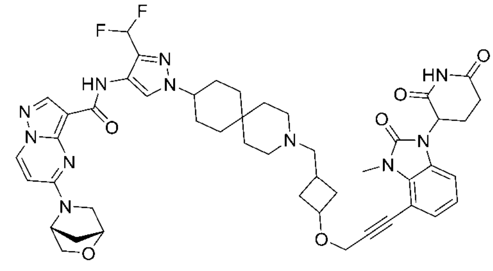

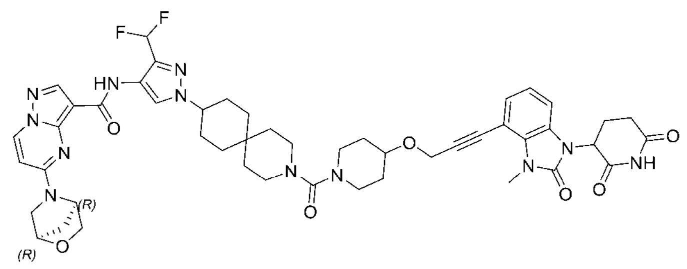

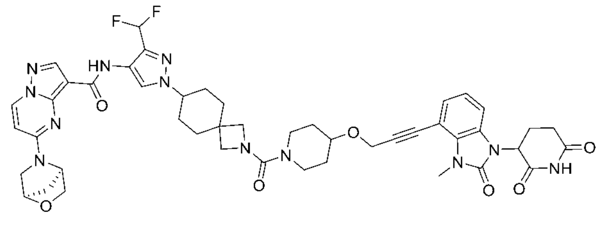

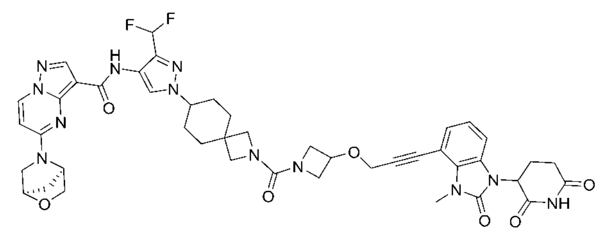

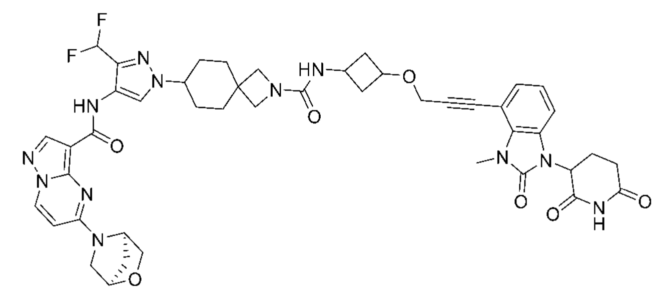

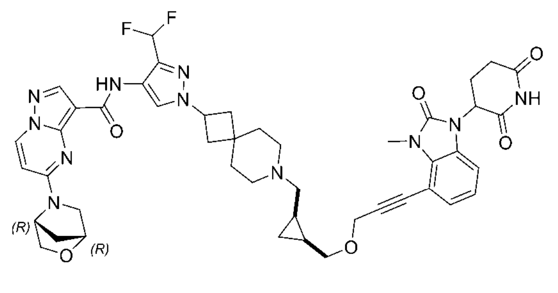

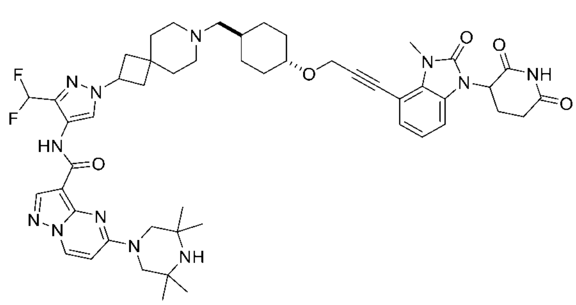

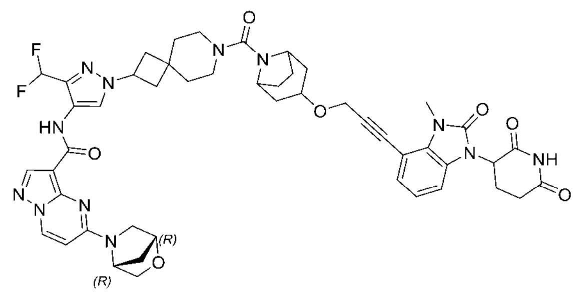

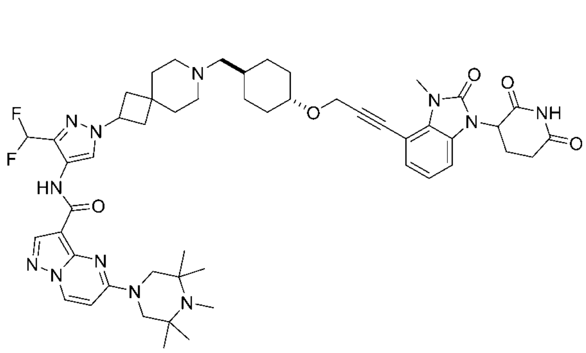

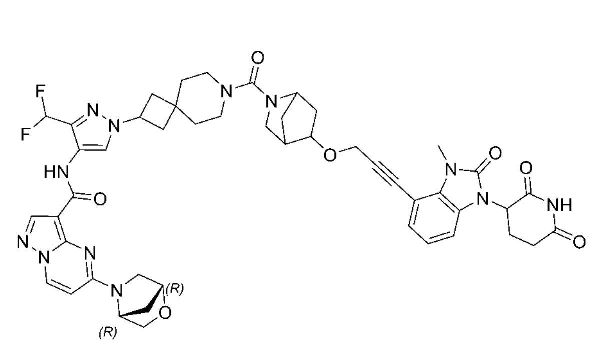

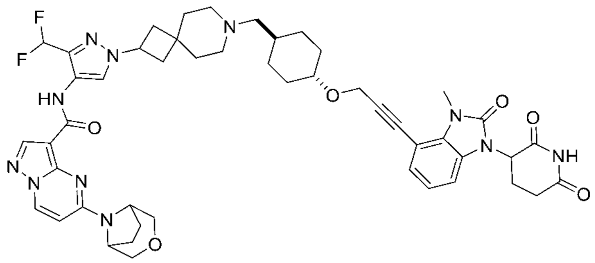

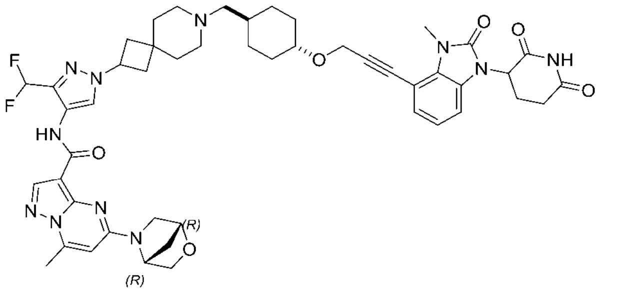

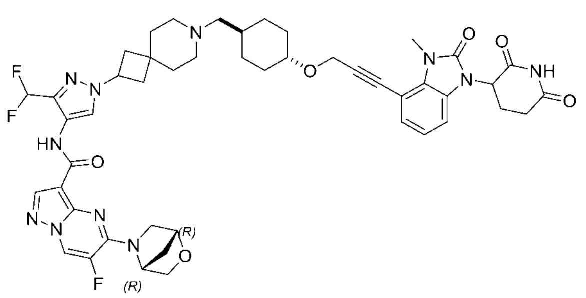

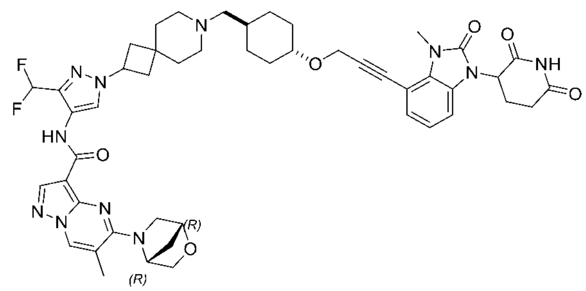

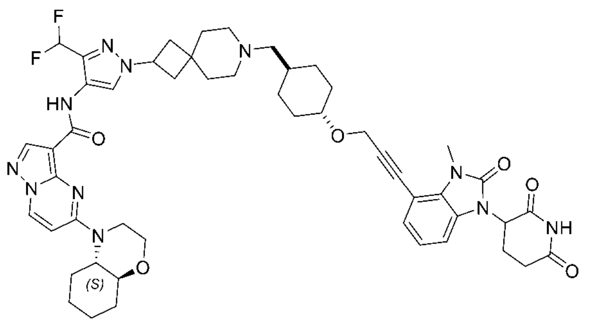

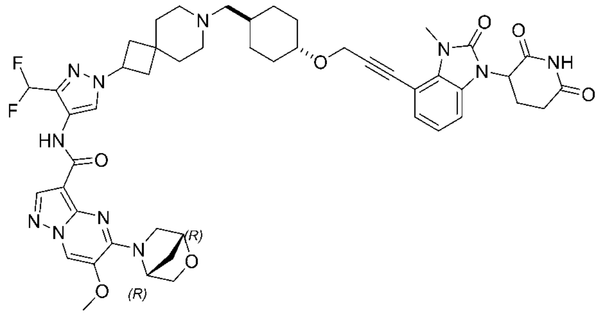

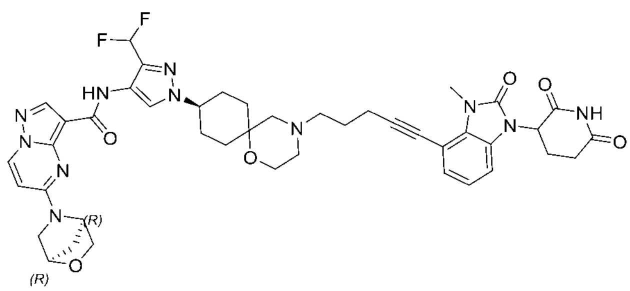

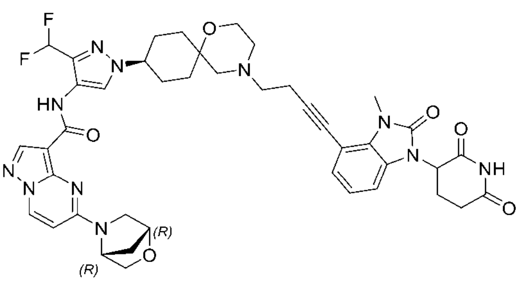

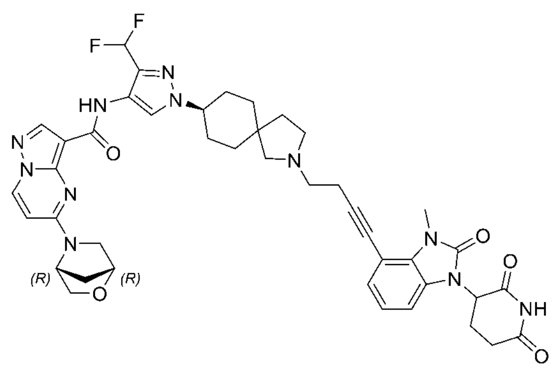

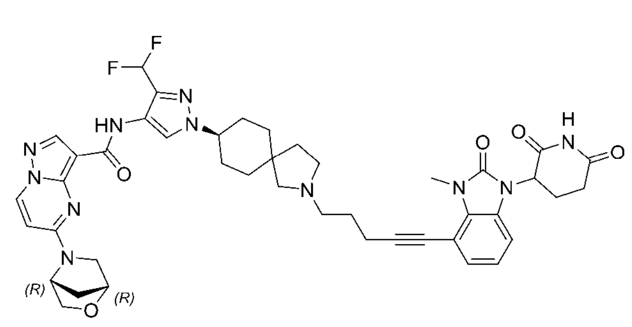

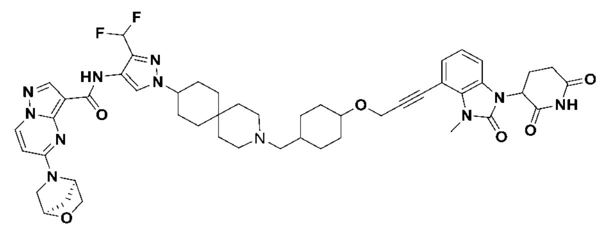

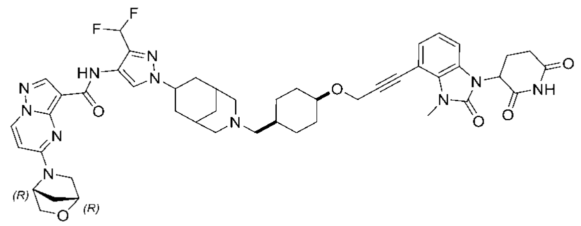

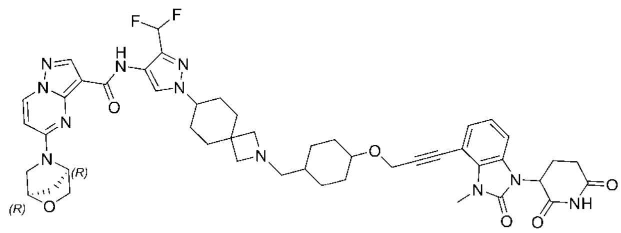

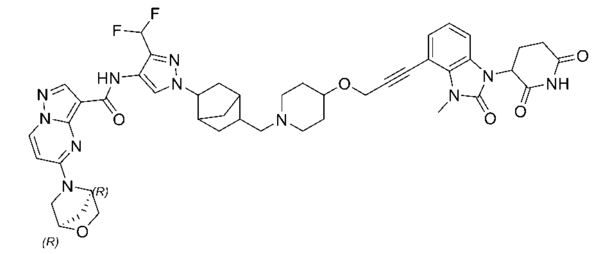

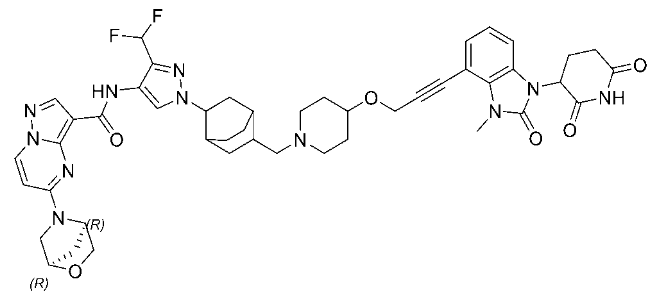

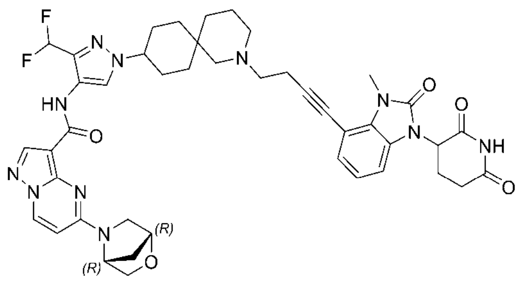

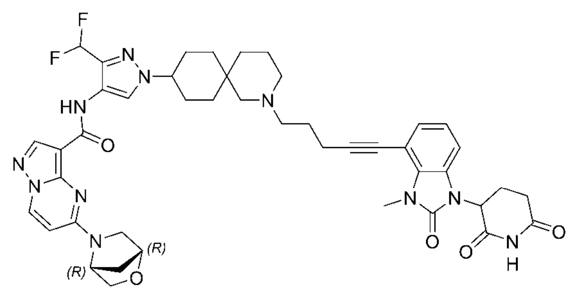

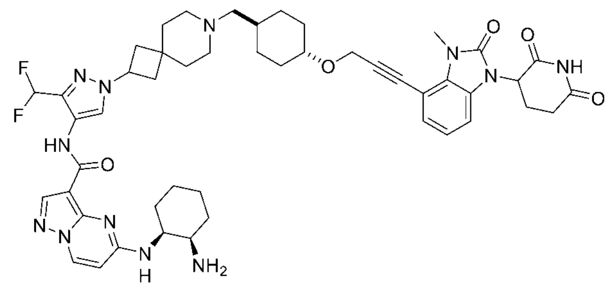

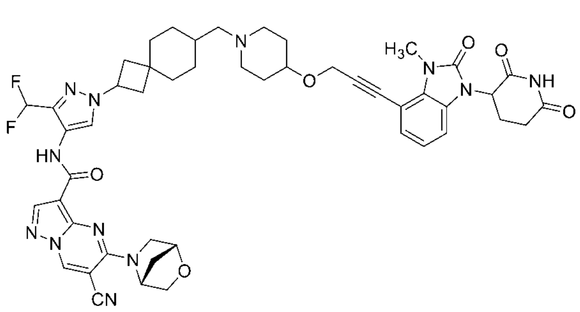

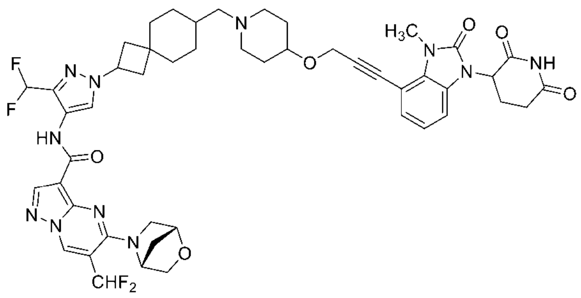

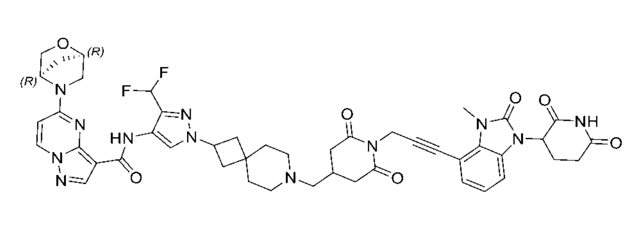

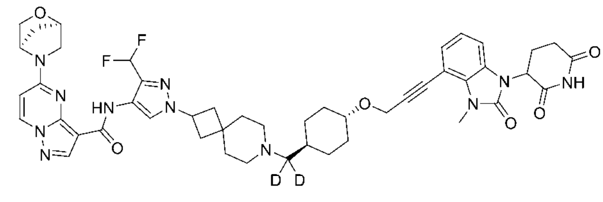

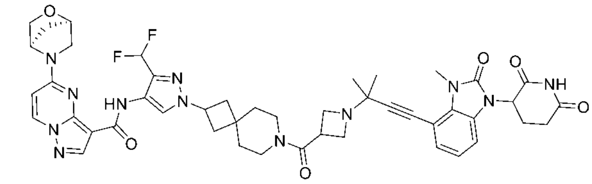

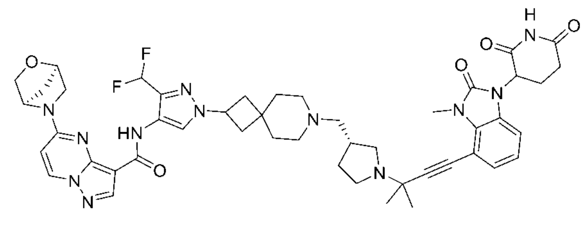

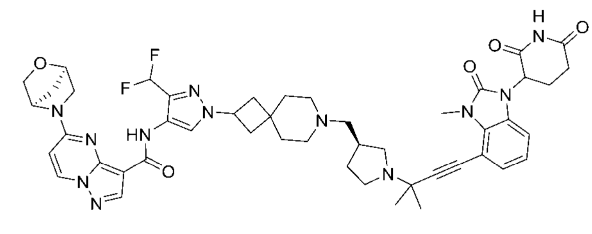

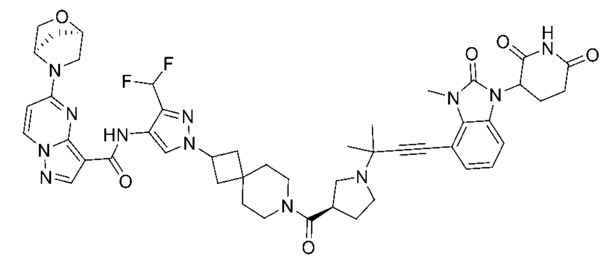

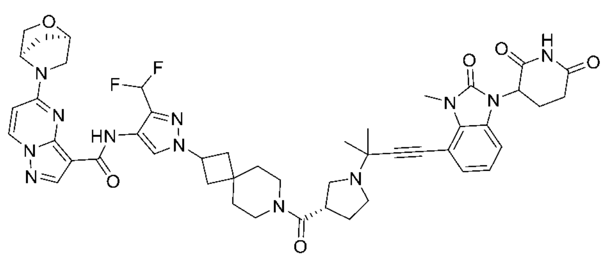

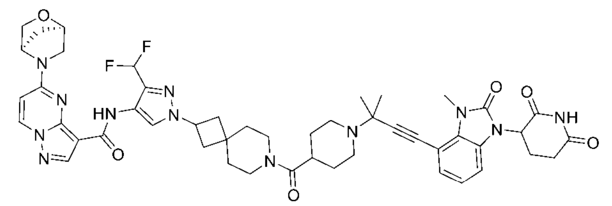

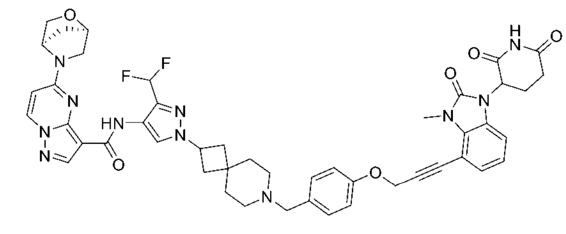

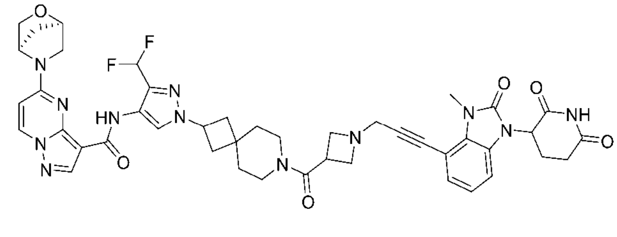

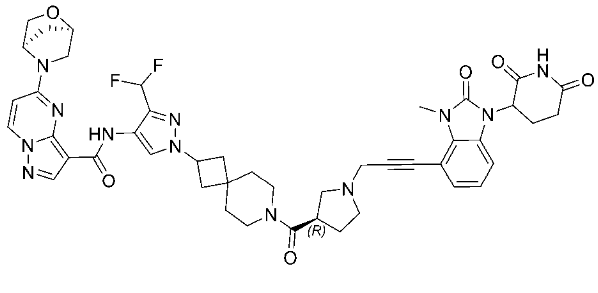

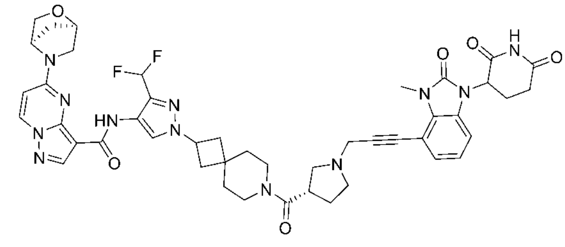

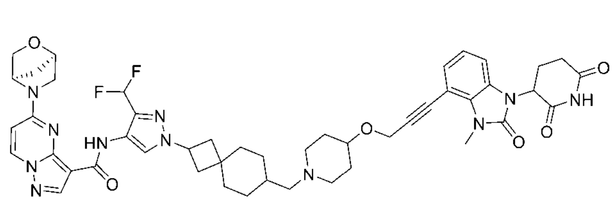

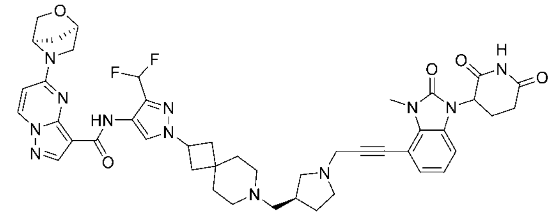

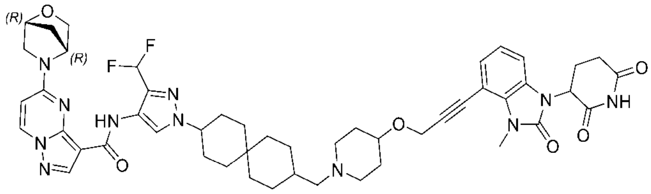

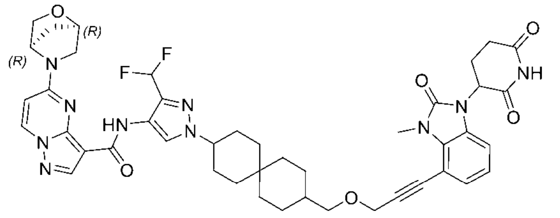

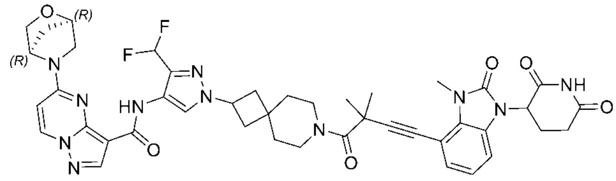

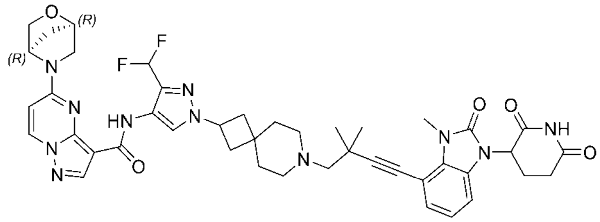

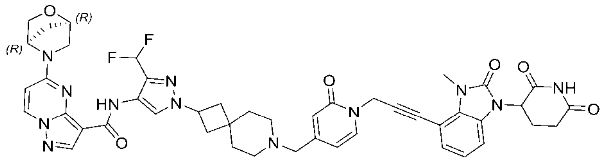

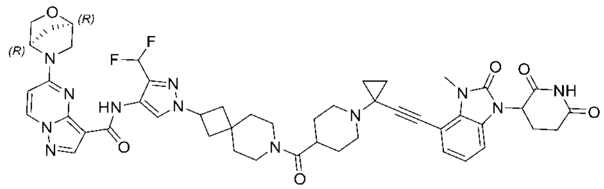

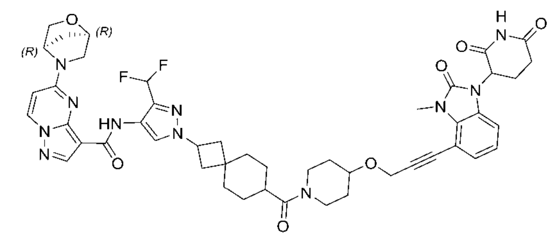

or

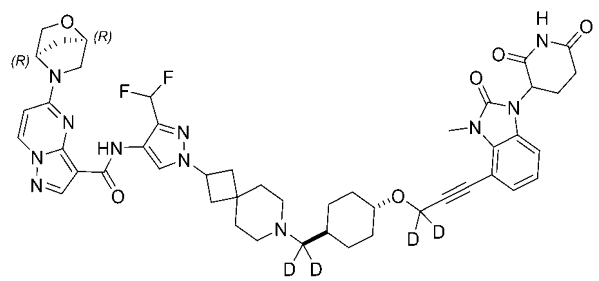

.

In another preferred embodiment, the bifunctional compound, the isomer thereof, the deuterated compound thereof, or the pharmaceutically acceptable salt thereof is selected from the group consisting of the following compounds:

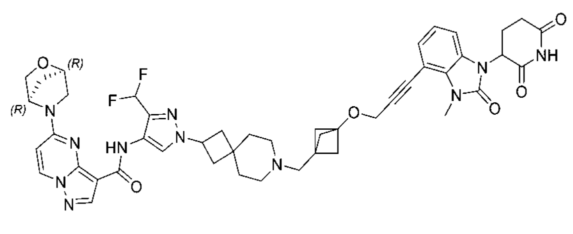

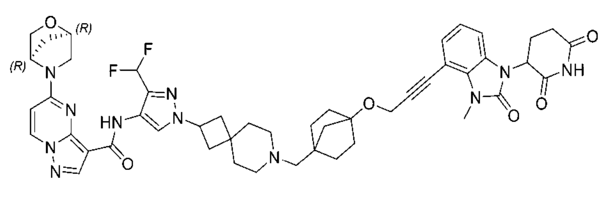

and

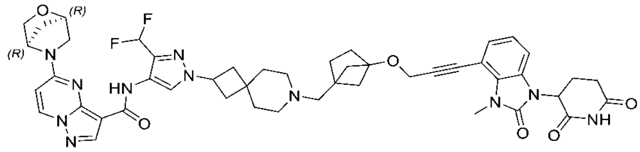 .

A second aspect of the present disclosure further provides a compound represented by the following formula (IB), an isomer thereof, a deuterated compound thereof, or a pharmaceutically acceptable salt thereof:

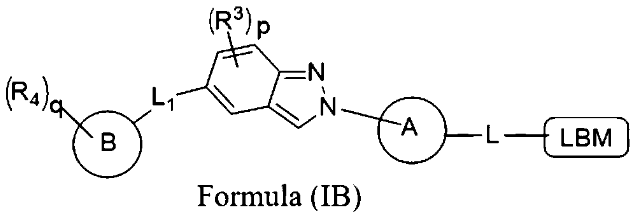

Formula (IB)

wherein ring B is 8- to 10-membered fused heteroaryl;
$L_1$ is selected from the group consisting of -N($R^d$)-C(O)- and -C(O)-N($R^d$)-;
$R^d$ is selected from the group consisting of H and $C_{1-4}$ alkyl;
$R^3$ is selected from the group consisting of $C_{1-4}$ alkyl, $C_{1-4}$ alkoxy, and halo-$C_{1-4}$ alkyl;
p is selected from the group consisting of 0, 1, 2, and 3;
$R^4$ is selected from the group consisting of $C_{1-4}$ alkyl, halo-$C_{1-4}$ alkyl, and 7- to 9-membered bridged heterocyclyl;
q is selected from the group consisting of 0, 1, 2, 3, and 4;
LBM is selected from the group consisting of

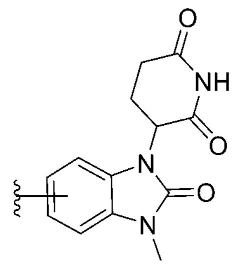

and

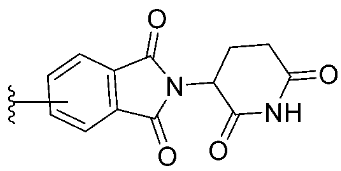 ;

ring A and L are as defined in any one of the embodiments of the first aspect of the present disclosure.

In another embodiment of the present disclosure, ring B is selected from the group consisting of

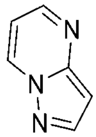 and 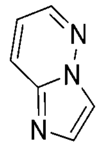 .

In another embodiment of the present disclosure, $R^3$ is selected from the group consisting of $-CH_3$, $-CH_2CH_3$, $-OCH_3$, and $-OCH_2CH_3$; p is selected from the group consisting of 0, 1, and 2.

In another embodiment of the present disclosure, the bifunctional compound, the isomer thereof, the deuterated compound thereof, or the pharmaceutically acceptable salt thereof has a structure as shown below:

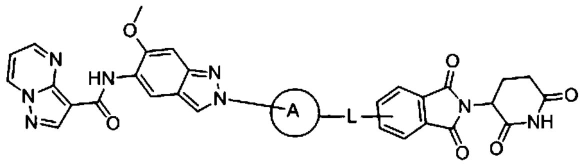

Formula (IB-1)

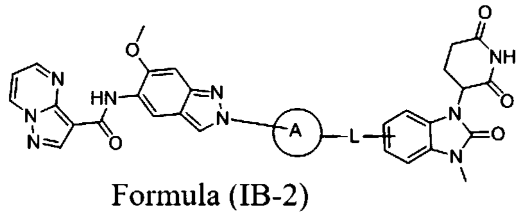

Formula (IB-2)

wherein ring A and L are as defined in any one of the preceding embodiments.

In another embodiment of the present disclosure, the compound represented by formula (IB), the isomer thereof, the deuterated compound thereof, or the pharmaceutically acceptable salt thereof is selected from the group consisting of:

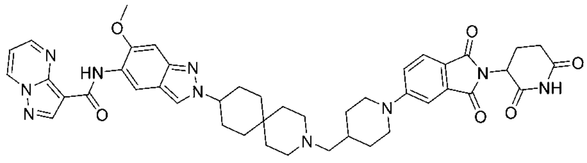

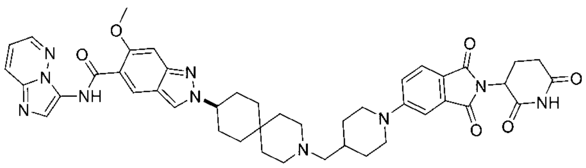

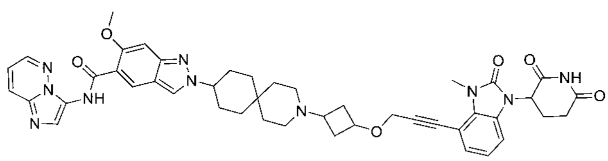

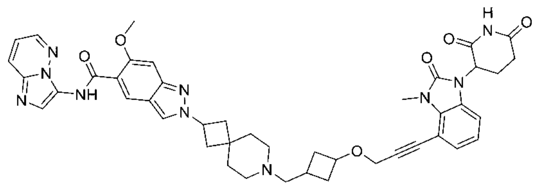

and

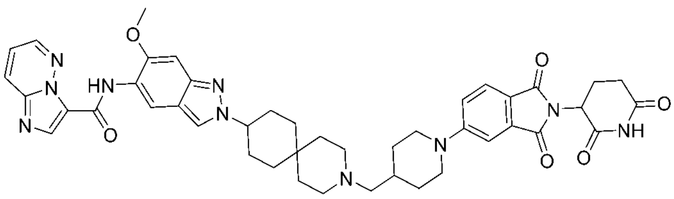

A third aspect of the present disclosure further provides a pharmaceutical composition comprising the compound, the isomer thereof, the deuterated compound thereof, or the pharmaceutically acceptable salt thereof according to any one of the embodiments in the first and second aspects of the present disclosure, and one or more pharmaceutically acceptable excipients, wherein the pharmaceutical composition may be in any one pharmaceutically acceptable dosage form. According to the present disclosure, the pharmaceutically acceptable excipient is a non-toxic substance that is compatible with the active ingredient and otherwise biologically suitable for use in an organism. The selection of a particular excipient will depend on the mode of administration for treating a particular patient or the type and status of the disease. Examples of pharmaceutically acceptable excipients include, but are not limited to: conventional solvents, diluents, dispersants, suspending agents, surfactants, isotonic agents, thickeners, emulsifiers, binders, lubricants, stabilizers, hydrating agents, emulsification accelerators, buffers, absorbents, colorants, ion exchangers, release agents, coating agents, flavoring agents, antioxidants, and the like in the pharmaceutical field. If necessary, fragrance agents, preservatives, sweeteners, and the like may also be added to the pharmaceutical composition.

In another preferred embodiment, in the pharmaceutical composition, the content of the compound, the isomer thereof, the deuterated compound thereof, or the pharmaceutically acceptable salt thereof is from 1% to 95%.

In certain embodiments of the present disclosure, in the pharmaceutical composition, the pharmaceutically acceptable excipient comprises one or more of a filler, a disintegrant, a binder, a glidant, or a lubricant.

A fourth aspect of the present disclosure further provides a use of the compound, the isomer thereof, the deuterated compound thereof, or the pharmaceutically acceptable salt thereof according to any one of the embodiments in the first and second aspects of the present disclosure in the manufacture of a medicament for preventing and/or treating an

IRAK4-mediated related disease.

In another preferred embodiment, the IRAK4-mediated related disease is selected from the group consisting of immune inflammatory diseases.

In another preferred embodiment, the IRAK4-mediated related disease is selected from the group consisting of hidradenitis suppurativa, rheumatoid arthritis, atopic dermatitis, lupus erythematosus, gouty arthritis, psoriasis, asthma, chronic obstructive pulmonary disease, polypoid sinusitis, and inflammatory bowel disease.

A fifth aspect of the present disclosure further provides a method for treating an IRAK4-mediated related disease, comprising administering a therapeutically effective amount of the compound, the isomer thereof, the deuterated compound thereof, or the pharmaceutically acceptable salt thereof according to any one of the embodiments in the first aspect of the present disclosure to a subject. The subject primarily refers to a human.

In another preferred embodiment, the IRAK4-mediated related disease is selected from the group consisting of immune inflammatory diseases.

In another preferred embodiment, the IRAK4-mediated related disease is selected from the group consisting of hidradenitis suppurativa, rheumatoid arthritis, atopic dermatitis, lupus erythematosus, gouty arthritis, psoriasis, asthma, chronic obstructive pulmonary disease, polypoid sinusitis, and inflammatory bowel disease.

A six aspect of the present disclosure provides a compound as shown below, an isomer thereof, a deuterated compound thereof, or a pharmaceutically acceptable salt thereof:

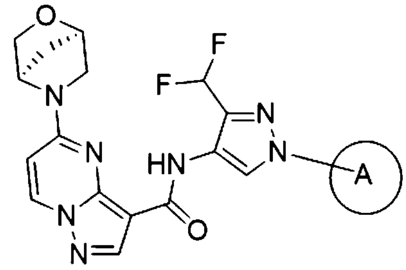

Formula Z

wherein ring A is as defined in any one of the preceding embodiments.

In another preferred embodiment, a compound represented by formula (Z), an isomer thereof, a deuterated compound thereof, or a pharmaceutically acceptable salt thereof is selected from the group consisting of the following structures:

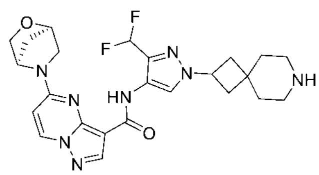
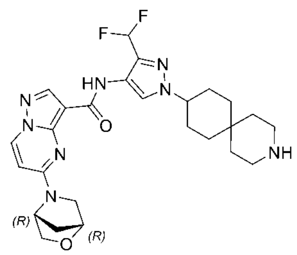
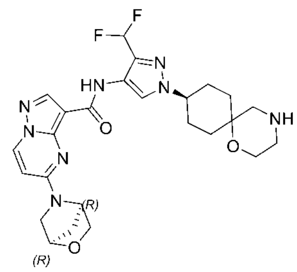
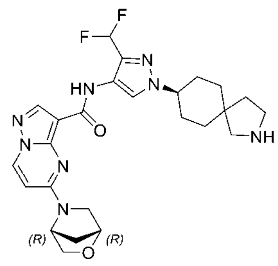

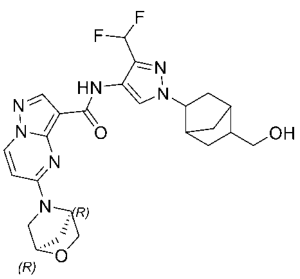
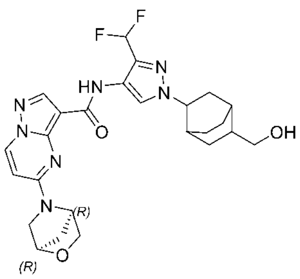
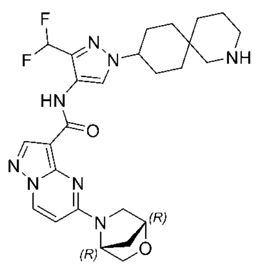

and

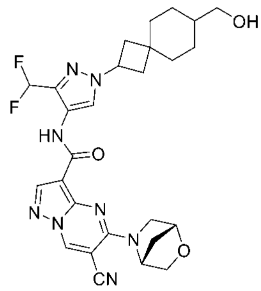

.

In another preferred embodiment, a compound represented by formula (Z), an isomer thereof, a deuterated compound thereof, or a pharmaceutically acceptable salt thereof has the following structure:

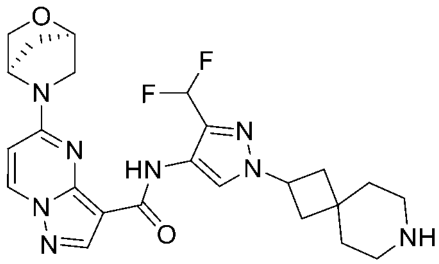

.

A seventh aspect of the present disclosure provides a compound represented by formula (X), an isomer thereof, a deuterated compound thereof, or a pharmaceutically acceptable salt thereof:

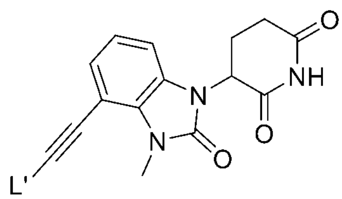

(X)

wherein L is $B^{1-1}$-$B^2$-$B^{3-1}$-;

$B^{1-1}$ is hydroxyl, amino, carboxyl, or hydroxy-substituted $C_{1-3}$ alkyl;

$B^{3-1}$ is a $C_{1-3}$ alkylene chain, any one methylene unit of which is optionally replaced by -O-, -C(O)-, -C($R^a$)($R^b$)-, -N($R^c$)-, or -S-; $R^a$ and $R^b$ are each independently selected from the group consisting of H, deuterium, halogen, $C_{1-6}$ alkyl, and halo-$C_{1-6}$ alkyl, and $R^a$ and $R^b$ are not simultaneously H;

$B^2$ is as defined in the first aspect of the present disclosure.

In another preferred embodiment, a compound represented by formula (X), an isomer thereof, a deuterated compound thereof, or a pharmaceutically acceptable salt thereof is selected from the group consisting of the following structures:

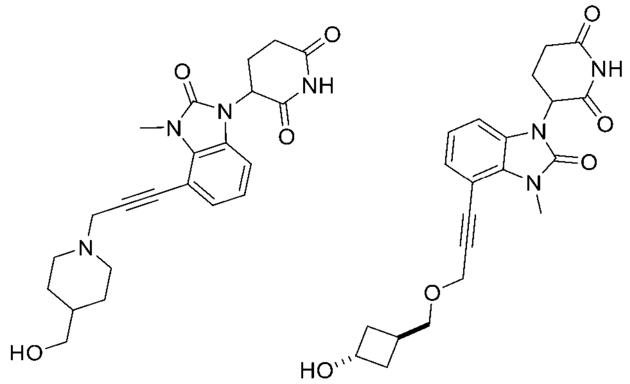

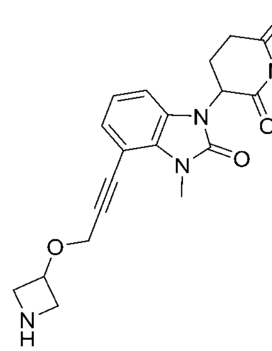

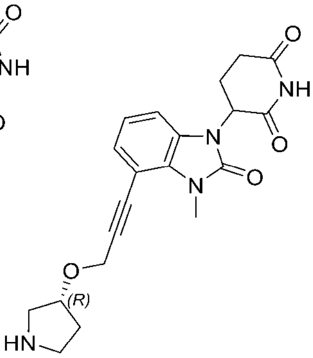

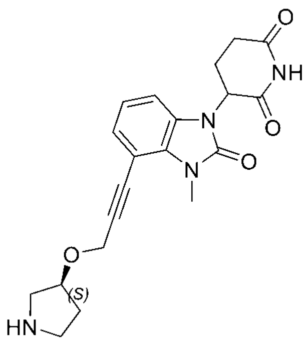

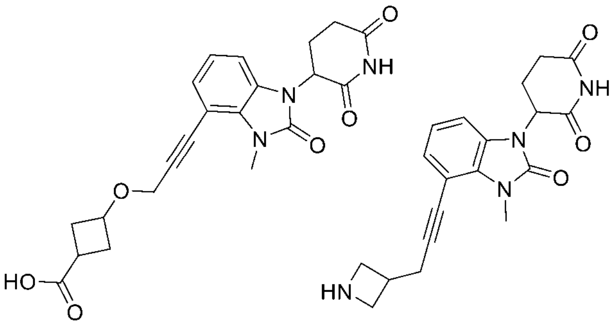

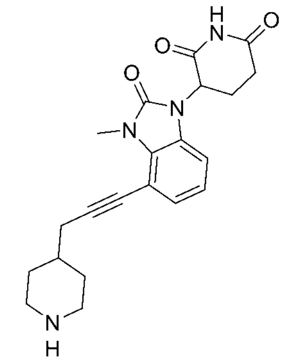

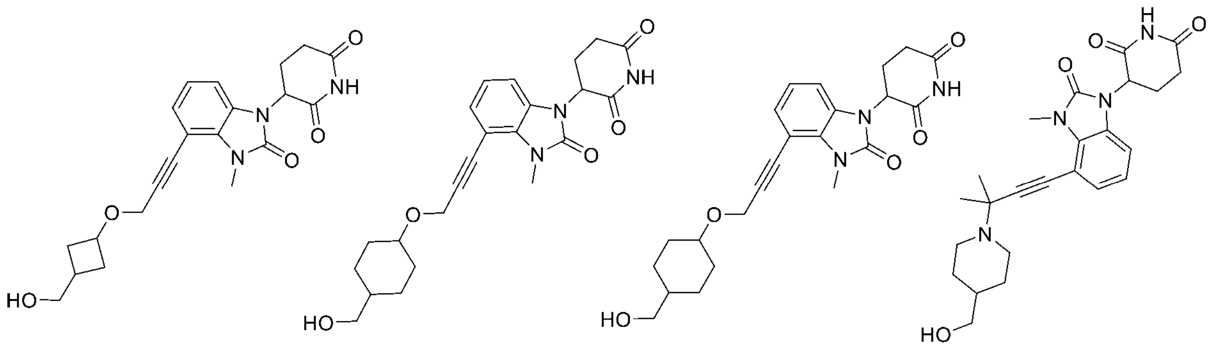

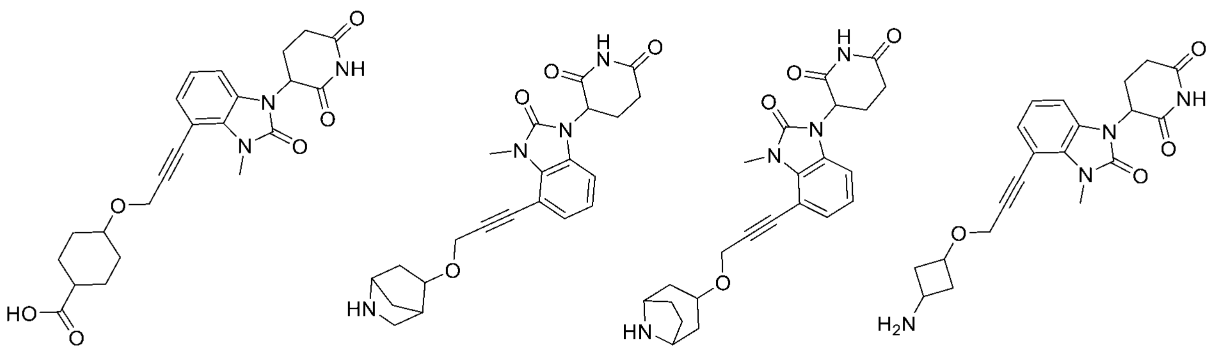

and

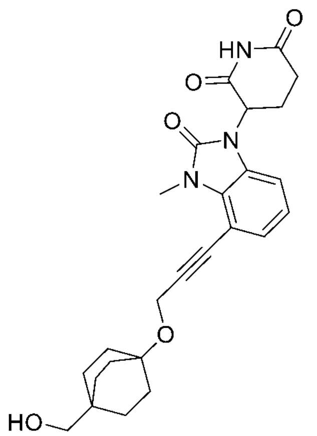

.

An eighth aspect of the present disclosure provides a compound as shown below, an isomer thereof, a deuterated compound thereof, or a pharmaceutically acceptable salt thereof:

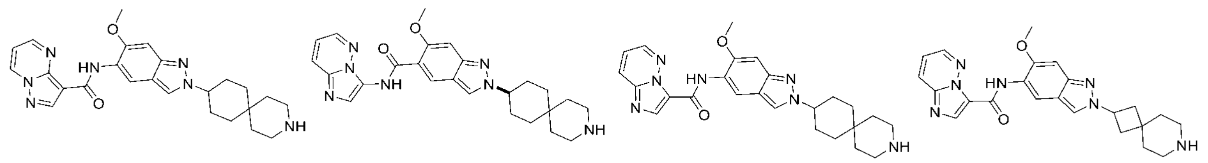

.

A ninth aspect of the present disclosure further comprises a use of the compound, the isomer thereof, the deuterated compound thereof, or the pharmaceutically acceptable salt thereof involved in the sixth aspect or the seventh aspect in the manufacture of an IRAK4 degrader; preferably, the IRAK4 degrader is as described in the first aspect of the present disclosure.

A tenth aspect of the present disclosure further comprises a use of the compound, the isomer thereof, the deuterated compound thereof, or the pharmaceutically acceptable salt thereof involved in the eighth aspect in the manufacture of an IRAK4 degrader.

Description and definition

In the present disclosure, unless otherwise specified, the scientific and technical terms used herein have the meanings commonly understood by those skilled in the art. However, for a better understanding of the present disclosure, definitions of some terms are provided below. When the definitions of terms provided in the present disclosure are inconsistent with the meanings commonly understood by those skilled in the art, the definitions and explanations of the terms provided in the present disclosure shall prevail.

In the present disclosure, the "E3 enzyme" includes enzyme species belonging to the E3 family that have been disclosed in the prior art, such as CRBN, VHL, MDM2, IAPs, TRIM24, DCAF15, DCAF16, and RNF114. Ligands for specific E3 enzyme species are as shown in the structure documented in the literature: "Jaeseok Lee, et al., Discovery of E3 Ligase Ligands for Target Protein Degradation, Molecules, 2022, 27(19):6515."

The term "pharmaceutically acceptable" refers to those compounds, materials, compositions and/or dosage forms, which are suitable for use in contact with human and animal tissues within the scope of reasonable medical judgment, without excessive toxicity, irritation, allergic reaction or other problems or complications, and is commensurate with a reasonable benefit/risk ratio.

The term "pharmaceutically acceptable salt" refers to a derivative prepared by the compound of the present disclosure with a relatively nontoxic acid or base. These salts may be prepared during the synthesis, separation and purification of the compound, or the free form of the purified compound may be used alone to react with a suitable acid or base. When the compound contains a relatively acidic functional group, the compound reacts with alkali metal, alkaline earth metal hydroxide or organic amine to obtain an alkali addition salt, including cations based on alkali metals and alkaline earth metals and non-toxic ammonium, quaternary ammonium and amine cations. Salts of amino acids are also covered. When the compound contains a relatively basic functional group, the compound reacts with an organic acid or an inorganic acid to obtain an acid addition salt.

The term "prophylactically or therapeutically effective amount" refers to a sufficient amount of the compound or the pharmaceutically acceptable salt thereof of the present disclosure, which is sufficient to treat a disorder at a reasonable

effect/risk ratio suitable for any medical treatment and/or prophylaxis. It should be recognized that the total daily dose of the compound, or the pharmaceutically acceptable salt thereof and the composition thereof in the present disclosure, is required to be determined by the attending physician within the scope of reliable medical judgment. For any specific patient, the specific therapeutically effective dose level depends on a variety of factors, including a disorder being treated and the severity of the disorder; activity of a specific compound used; a specific composition used; age, weight, general health status, sex and diet of the patient; administration time, administration route and excretion rate of the specific compound used; duration of treatment; drugs used in combination or simultaneously with the specific compounds used; and similar factors known in the medical field.

The "isomers" mentioned in the present disclosure include geometric isomers and stereoisomers, such as atropisomers, *cis-trans* isomers, enantiomers, diastereomers, tautomers, and racemic mixtures thereof, and other mixtures, all of which fall within the scope of the present disclosure. The term "enantiomer" refers to stereoisomers that are mirror images of each other. The term "tautomer" refers to a type of functional group isomer that has different hydrogen bonding sites through the displacement of one or more double bonds. For example, a ketone and its enol form constitute keto-enol tautomers. The term "diastereomer" refers to stereoisomers in which the molecules have two or more chiral centers and the relationship between the molecules is not mirror images. The term "cis-trans isomer" refers to different spatial configurations in which a double bond or a single bond of a ring-forming carbon atom in a molecule cannot rotate freely. The term "atropisomer" refers to a stereoisomer that can be isolated due to hindered or extremely slow rotation around a single bond.

Stereoisomers of the compounds of the present disclosure may be prepared by chiral synthesis or chiral reagents or other conventional techniques. For example, an enantiomer of a certain compound of the present disclosure may be prepared by an asymmetric catalysis technique or a chiral auxiliary derivatization technique. Alternatively, compounds with a single stereo configuration may be obtained from a mixture by a chiral resolution technique. Alternatively, it can be prepared directly from chiral starting materials. Separation of optically pure compounds in the present disclosure is usually accomplished by preparative chromatography, and a chiral chromatographic column is used to achieve the purpose of separating chiral compounds.

The "deuterated compound" mentioned in the present disclosure refers to a compound derived from the compound of the present disclosure in which one or more hydrogen atoms are substituted by deuterium.

The term "optionally substituted" means an atom can be substituted by a substituent or not, unless otherwise specified, the type and number of the substituent may be arbitrary as long as being chemically achievable. For example, the term "optionally substituted by one or more R" means that an atom may or may not be substituted by one or more R. When any variable occurs in the constitution or structure of the compound more than once, the definition of the variable at each occurrence is independent. For example, if a group is substituted by 0 to 2 $R_a$, the group can be optionally substituted by up to two $R_a$, wherein the definition of $R^a$ at each occurrence is independent.

When the substituent structure contains , it indicates that the atom is a bonding atom. For example,

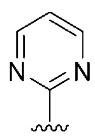

signifies that the C atom on the pyrimidine ring is the bonding atom. The presence of a dash "-" in a substituent structure indicates a point of attachment for the substituent, for example, $-CH_3$ is attached through a C atom. "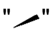" and "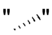" denote the absolute configuration of a stereocenter, namely the R or S configuration. "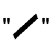" or "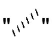" denotes the cis or trans configuration, where double solid bonds or double dashed bonds represent the cis configuration, and one solid bond and one dashed bond represent the trans configuration.

In the present disclosure, L is $-(B^1)n_1-(B^2)n_2-(B^3)n_3-$; when $n_1$, $n_2$, or $n_3$ is 0, it indicates that the corresponding group is absent, and the adjacent groups on both sides are directly connected by a chemical bond. Specifically, for example: when only $n_1$ is 0, L is $-(B^2)n_2-(B^3)n_3-$; when only $n_2$ is 0, L is $-(B^1)n_1-(B^3)n_3-$; when only $n_3$ is 0, L is $-(B^1)n_1-(B^2)n_2-$.

When the bond of a substituent may be cross-connected to two atoms on a ring, the substituent may be bonded to any atom on the ring. For example, the structure moiety

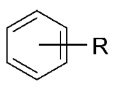

indicates that the substituent R may be substituted at any position on a benzene ring.

When a listed substituent does not specify the atom through which the substituent is attached to a given group or

to a given structural formula, the substituent may be attached through any bondable atom thereof.

The term "alkyl" mentioned in the present disclosure refers to a group derived from a branched or linear saturated aliphatic alkane with a specified number of carbon atoms by removing one hydrogen atom. For example, "$C_{1-10}$ alkyl" refers to including $C_1$, $C_2$, $C_3$, $C_4$, $C_5$, $C_6$, $C_7$, $C_8$, $C_9$, $C_{10}$ alkyl, including "$C_{1-6}$ alkyl", "$C_{1-4}$ alkyl", "$C_{1-3}$ alkyl"; specific examples include, but are not limited to, methyl, ethyl, *n*-propyl, isopropyl, *sec*-butyl, 2-methylbutyl, 1,1-dimethylbutyl, etc.

The term "haloalkyl" mentioned in the present disclosure refers to an alkyl group in which hydrogen is substituted by one or more halogens, such as "fluoromethyl" including monofluoromethyl, difluoromethyl, and trifluoromethyl; preferably, the "haloalkyl" mentioned in the present disclosure is "halo $C_{1-6}$ alkyl" or "halo $C_{1-4}$ alkyl". The alkyl is as defined above.

The "oxo" mentioned in the present disclosure refers to the substitution of the group with a "=O" structure, such as "$-CH_2-$" being oxo-substituted to form "-C(O)-", or "S" being oxo-substituted to form "S(O)" or "$S(O)_2$".

The term "hydrocarbylene" mentioned in the present disclosure refers to a group derived from a branched or linear alkane, alkene, or alkyne having a specified number of carbon atoms by removing two hydrogen atoms, including "alkylene", "alkenylene", and "alkynylene", wherein the hydrocarbylene may be further substituted by other groups; wherein "alkenylene" and "alkynylene" refer to unsaturated hydrocarbon chains containing at least one double bond or triple bond, respectively, in the hydrocarbon chain. The "hydrocarbylene" mentioned in the present disclosure includes "$C_{1-15}$ hydrocarbylene", preferably "$C_{2-10}$ hydrocarbylene", "$C_{4-6}$ alkylene", "$C_{2-10}$ alkylene", "$C_{5-7}$ alkynylene", "$C_{7-10}$ alkylene", "$C_{5-7}$ alkenylene", "$C_{1-6}$ hydrocarbylene", "$C_{1-3}$ alkylene", "$C_{2-6}$ alkynylene", etc. Preferably, the "alkylene" mentioned in the present disclosure is preferably "linear alkylene"; specific examples of the alkylene chain include, but are not limited to: $-CH_2-$, $-CH_2CH_2-$, $-CH_2CH_2CH_2-$, $-CH(CH_2)CH_2-$, $-CH_2CH_2CH_2CH_2-$, $-CH(CH_2)CH_2CH_2-$, $-CH(CH_2CH_2)CH_2-$, $-C(CH_2)(CH_2)CH_2-$, $-CH_2CH_2CH_2CH_2CH_2-$, etc.; preferably, the "methylene unit" mentioned in the present disclosure refers to $-CH_2-$. Specific examples of "alkenylene" mentioned in the present disclosure include, but are not limited to: $-CH=CH-$, $-CH=CHCH_2-$, $-CH=C(CH_3)CH_2-$, $-CH=CHCH_2CH=CH-$, $-CH_2CH=CHCH_2-$, $-CH_2CH=CHCH_2CH_2-$; specific examples of "alkynylene" mentioned in the present disclosure include, but are not limited to $-C{\equiv}C-$, $-C{\equiv}C-CH_2-$, $-C{\equiv}C-CH_2CH_2-$, $-CH_2-C{\equiv}C-CH_2CH_2-$, $-C{\equiv}C-CH_2C{\equiv}C-$, $-C{\equiv}C-CH_2CH=CH-$, etc. Furthermore, the term "any one methylene unit is optionally replaced" mentioned in the present disclosure, such as any one methylene unit in "$-CH_2CH_2CH_2-$" being replaced by -C(O)-, may form, but is not limited to, the following structures: $-C(O)CH_2CH_2-$, $-CH_2C(O)CH_2-$, $-CH_2CH_2C(O)-$, $-C(O)CH_2C(O)-$, etc.

The "ring" mentioned in the present disclosure includes, but is not limited to, "cycloalkane", "spirocycloalkane", "bridged cycloalkane", "heterocycle", "heterocycloalkene", "spiroheterocycle", "bridged heterocycle", "fused heterocycle", "heteroaromatic ring", "fused heteroaromatic ring", etc.; the monovalent rings derived therefrom may be "cycloalkyl", "spirocycloalkyl", "bridged cycloalkyl", "heterocyclyl", "heterocycloalkenyl", "spiroheterocyclyl", "bridged heterocyclyl", "heteroaryl", or "fused heteroaryl", respectively; and the "divalent ring" derived therefrom refers to a group derived from a ring structure by removing two hydrogen atoms, where the bonding sites may connect to different groups or different positions of the same group. The derived divalent rings may be "cycloalkylene", "bridged cycloalkylene", "spirocycloalkylene", "heterocyclylene", "heterocycloalkenylene", "spiroheterocyclylene", "bridged heterocyclylene", "fused heterocyclylene", "heteroarylene", etc., respectively.

The "cycloalkane" mentioned in the present disclosure refers to a monocyclic, saturated cyclic alkane structure, wherein the carbon atoms in the cycloalkane may be oxo-substituted, i.e., the carbon atoms are replaced by -C(O)-. It includes "$C_{3-8}$ cycloalkane", "$C_{3-6}$ cycloalkane", "$C_{3-5}$ cycloalkane", etc., with specific examples including, but not limited to: cyclopropane, cyclobutane, cyclopentane, cyclohexane, cycloheptane, cyclooctane.

The "spirocycloalkane" mentioned in the present disclosure refers to a cyclic structure formed by two or more adjacent cycloalkanes sharing one ring atom, wherein the carbon atoms in the cycloalkane may be oxo-substituted, i.e., the carbon atoms are replaced by -C(O)-. It includes "$C_{7-11}$ spirocycloalkane", "$C_{7-9}$ spirocycloalkane", etc., with specific examples including, but not limited to:

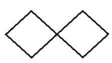, 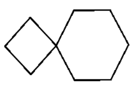, 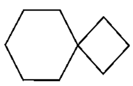, 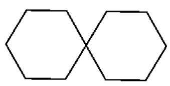, 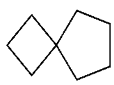,

and

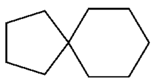.

The "bridged cycloalkane" mentioned in the present disclosure refers to a cyclic structure formed by two or more adjacent cycloalkanes sharing two non-adjacent ring atoms, wherein the carbon atoms in the cycloalkane may be oxo-substituted, i.e., the carbon atoms are replaced by -C(O)-. It includes "$C_{6-10}$ bridged cycloalkane", "$C_{6-8}$ bridged cycloalkane", etc., with specific examples including, but not limited to:

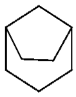, 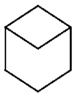, 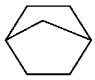, 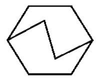,

etc.

The "heterocycle" mentioned in the present disclosure refers to a cyclic structure derived from a "cycloalkane" in which at least one carbon atom is replaced by a heteroatom/heteroatom group, wherein the heteroatom/heteroatom group is selected from the group consisting of O, NR, N(O), S, S(O), and $S(O)_2$, wherein R is H or any one substituent group that may exist, and the "cycloalkane" is as defined above. The heterocycle preferably contains 1 to 2 heteroatoms selected from the group consisting of NR and/or O, more preferably containing 1 NR and 0 to 1 NR or O heteroatom. The heterocycle is preferably a "nitrogen-containing heterocycle", which refers to a heterocycle in which at least one ring atom is NR. The heterocycle includes a 3- to 8-membered heterocycle, a 3- to 6-membered heterocycle, a 4- to 6-membered heterocycle, a 5- to 6-membered heterocycle, a 4- to 6-membered nitrogen-containing heterocycle, and a 5- to 6-membered nitrogen-containing heterocycle. Specific examples include, but are not limited to: tetrahydrofuran, pyrrolidine, piperidine, morpholine, piperazine,

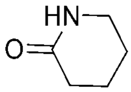,

etc.

The "heterocycloalkene" mentioned in the present disclosure refers to a heterocyclic group containing at least one ring-forming double bond in the "heterocycle" and lacking aromaticity. The heterocycloalkene preferably contains 1 to 2 heteroatoms selected from the group consisting of NR and/or O, more preferably containing 1 NR and 0 to 1 NR or O heteroatom. The "heterocycloalkene" includes "5- to 8-membered heterocycloalkene" and "6- to 8-membered heterocycloalkene". Specific examples include, but are not limited to:

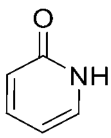, 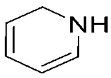, 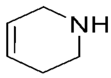, 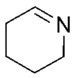, 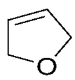, 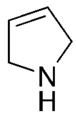,

etc.

The "spiroheterocycle" mentioned in the present disclosure refers to a cyclic structure derived from a "spirocycloalkane" in which at least one carbon atom is replaced by a heteroatom/heteroatom group, wherein the heteroatom/heteroatom group is selected from the group consisting of O, NR, N(O), S, S(O), and $S(O)_2$, wherein R is H or any one substituent group that may exist, and the "spirocycloalkane" is as defined above. It includes, but is not limited to, heterocyclic spiroheterocycles and cyclic structures formed by and heterocyclic spirocycloalkanes. The spiroheterocycle preferably contains 1 to 2 heteroatoms selected from the group consisting of NR and/or O, more preferably containing 1 NR and 0 to 1 NR or O heteroatom. The spiroheterocycle is preferably a "nitrogen-containing spiroheterocycle", which refers to a spiroheterocycle in which at least one ring atom is NR. The spiroheterocycle includes a 7- to 11-membered spiroheterocycle, a 7- to 9-membered spiroheterocycle, a 7- to 11-membered nitrogen-containing spiroheterocycle, and a 7- to 9-membered nitrogen-containing spiroheterocycle. Specific examples include, but are not limited to:

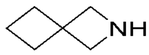,

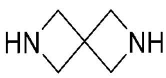, 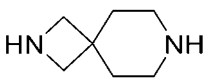, 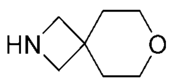, 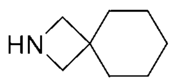, 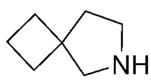,

etc.

The "bridged heterocycle" mentioned in the present disclosure refers to a cyclic structure derived from a "bridged

cycloalkane" in which at least one carbon atom is replaced by a heteroatom/heteroatom group, wherein the heteroatom/heteroatom group is selected from the group consisting of O, NR, N(O), S, S(O), and $S(O)_2$, wherein R is H or any one substituent group that may exist, and the "bridged cycloalkane" is as defined above. The bridged heterocycle is preferably a "nitrogen-containing bridged heterocycle", which refers to a bridged heterocycle in which at least one ring atom is NR. The bridged heterocycle includes "6- to 10-membered bridged heterocycle", "6- to 8-membered bridged heterocycle", etc., with specific examples including, but not limited to:

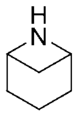 , 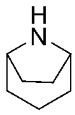 , 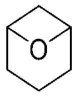 , 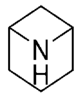 ,

etc.

The "fused heterocycle" mentioned in the present disclosure refers to a saturated cyclic structure formed by two or more rings sharing two adjacent ring atoms, wherein at least one ring is a heterocycle, and the "heterocycle" is as defined above. It includes, but is not limited to, heterocycle-fused heterocycles and structures formed by heterocycle-fused cycloalkanes. The fused heterocycle preferably contains 1 to 2 heteroatoms selected from the group consisting of NR and/or O, more preferably containing 1 NR and 0 to 1 NR or O heteroatom. The fused heterocycle is preferably a "nitrogen-containing fused heterocycle", which refers to a fused heterocycle in which at least one ring atom is NR. The fused heterocycle includes a 6- to 10-membered fused heterocycle and an 8- to 10-membered fused heterocycle. Specific examples include, but are not limited to,

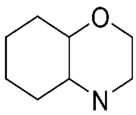 , 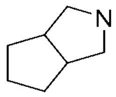 ,

and

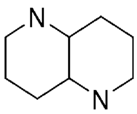 .

The "heteroaromatic ring" mentioned in the present disclosure refers to a monocyclic or polycyclic aromatic hydrocarbon in which at least one ring atom is a heteroatom, and the heteroatom is selected from the group consisting of N, O, and S. When the heteroaryl contains an N atom, a nitrogen oxide thereof is also included. The polycyclic heteroaromatic ring, i.e., a fused heteroaromatic ring, includes but is not limited to a benzo 5- to 6-membered heteroaromatic ring and a 5- to 6-membered heteroaromatic ring-fused 5- to 6-membered heteroaromatic ring. The heteroaromatic ring includes a 5- to 6-membered heteroaromatic ring, a 7- to 10-membered bicyclic fused heteroaromatic ring, and an 8- to 14-membered fused heteroaromatic ring. The heteroaromatic ring is preferably a "nitrogen-containing heteroaromatic ring", which refers to a heteroaromatic ring in which at least one ring atom is N. Examples of heteroaromatic rings include, but are not limited to, pyrrole, furan, thiophene, pyrazole, imidazole, pyrazine, pyridazine, triazine, oxazole, isoxazole, thiazole, isothiazole, pyridine, pyrimidine, indazole, indole, isoquinoline, quinoxaline, benzoxazole, benzofuran, benzothiophene, benzothiazole, benzimidazole, quinoline, quinazoline,

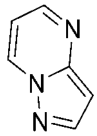 , 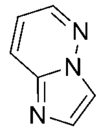 ,

etc.

Combinations of substituents and/or variables described in the present disclosure are permissible only when such combinations yield stable compounds or usable synthetic intermediates. A stable compound or stable structure refers to a compound that is sufficiently stable to undergo chemical reactions, can be isolated in a useful purity, and can be formulated into an effective therapeutic agent.

As used herein, the term "degrader" refers to a bifunctional compound capable of binding between the IRAK4

kinase and an E3 ligase, leading to ubiquitination and subsequent degradation of the IRAK4 kinase. In certain examples, the DC50 of the degrader is less than about 50 μM, less than about 1 μM, less than about 500 nM, less than about 100 nM, less than about 10 nM, or less than about 1 nM.

Beneficial effects of the compounds of the present disclosure:

(1) The compounds of the present disclosure exhibit good degradation activity against the IRAK4 target and can effectively inhibit the secretion of inflammatory factors.
(2) The compounds of the present disclosure exhibit favorable pharmacokinetic properties, such as high exposure, appropriate clearance rate, and other superior properties.
(3) The compounds of the present disclosure exhibit good safety, such as lower cardiotoxicity.

BRIEF DESCRIPTION OF THE DRAWINGS

FIG. 1 shows the concentration levels of inflammatory factors in mouse plasma and peritoneal lavage fluid after oral administration of KT-474 and the compound of Example 22 of the present disclosure to mice at three different dose levels (15 mg/kg, 50 mg/kg, and 150 mg/kg) in Test Example 6.
FIG. 2 shows the *in vivo* degradation levels of IRAK4 in mouse spleens after oral administration of KT-474 and the compound of Example 22 of the present disclosure to mice at three different dose levels (15 mg/kg, 50 mg/kg, and 150 mg/kg) in Test Example 6.

DETAILED DESCRIPTION OF THE PREFERRED EMBODIMENT

The preparation methods of some compounds in the present disclosure refer to the preparation methods of the aforementioned similar compounds. Those skilled in the art should know that when using or referring to the preparation methods cited therein, the feed ratio of reactants, reaction solvent, reaction temperature, etc. may be appropriately adjusted according to the different reactants.

The compounds of the present disclosure can be prepared by a variety of synthetic methods known to those skilled in the art, including the specific embodiments listed below, the embodiments formed by their combination with other chemical synthesis methods, and equivalent alternatives known to those skilled in the art, and preferred embodiments include, but are not limited to, the examples of the present disclosure.

Unless otherwise specified, all reactions in the present disclosure are conducted under continuous magnetic stirring in a dry nitrogen or argon atmosphere with the solvent being a dry solvent, wherein: (i) temperatures are given in degrees Celsius (°C), and operations are performed at room temperature, which generally refers to 15-35°C, preferably 20-30°C, more preferably 20-25°C; (ii) the solvent is removed by evaporation under reduced pressure using a rotary evaporator; (iii) reaction progress is monitored by LC-MS or thin-layer chromatography (TLC); (iv) final products exhibit satisfactory hydrogen nuclear magnetic resonance ($^1$H-NMR) and/or mass spectrometry (MS) data. The methods used for purifying the products of the present disclosure include, but are not limited to: preparative high-performance liquid chromatography, thin-layer chromatography, column chromatography, etc. The purification reagents used are conventional solvents in the art, such as dichloromethane, methanol, ethyl acetate, petroleum ether, acetonitrile, and water. The specific types and ratios used can be determined by conventional methods in the art.

Test instruments:

The structures of the compounds of the present disclosure are determined by nuclear magnetic resonance (NMR) or/and liquid chromatography-mass spectrometry (LC-MS). NMR chemical shifts (δ) are given in units of parts per million (ppm). NMR is determined using a Bruker Neo 400M or Bruker Ascend 400 NMR instrument, with deuterated dimethyl sulfoxide (DMSO-$d_6$), deuterated methanol ($CD_3OD$), and/or deuterated chloroform ($CDCl_3$) as solvents, and tetramethylsilane (TMS) as an internal standard.

Liquid chromatography-mass spectrometry (LC-MS) is determined using a Shimadzu 2030Plus-LCMS2020 mass spectrometer, an Agilent 1260-6125B single quadrupole mass spectrometer, or a Shimadzu LCMS-2020 mass spectrometer. HPLC is determined using a Shimadzu LCMS-2020 or Agilent 1260 high-performance liquid chromatography.

The preparative high-performance liquid chromatography is performed using a Shimadzu FRC-40 equipped with LC-20AP and PDA-20A (chromatographic column: Synergi Max-RP, 150 × 30 mm, 4 μm) or a GILSON Trilution LC (chromatographic columns: SunFire Prep C18, 10 μm, 19 × 250 mm; XBridge Prep C18, 10 μm, 19 × 250 mm).

The starting materials or intermediate compounds used in the present disclosure are commercially available or

self-synthesized, and the structures and preparations of the self-synthesized intermediates have been recorded in the present disclosure. The chirality of a chiral intermediate or chiral product in the present disclosure can be determined based on the chiral starting material used. When the reaction site is a non-chiral site, the chirality of the starting material is typically identical to that of the product.

The chemical abbreviations used in the present disclosure and their corresponding chemical names are as follows:

| Chemical Abbreviations | Chemical name |
|---|---|
| DHP | Dihexyl phthalate |
| TEA | Triethanolamine |
| DCM | Dichloromethane |
| DAST | Diethylaminosulfur trifluoride( |
| Dess-Martin | Dess-Martin reagent, 1,1,1-triacetoxy-1,1-dihydro-1,2-benziodoxol-3(1H)-one |
| PE | Petroleum ether |
| EtOAc | Ethyl acetate |
| DEAD | Diethyl azodicarboxylate |
| PMB | *p*-Methoxybenzyl |

Synthesis of IRAK4 Warhead A:

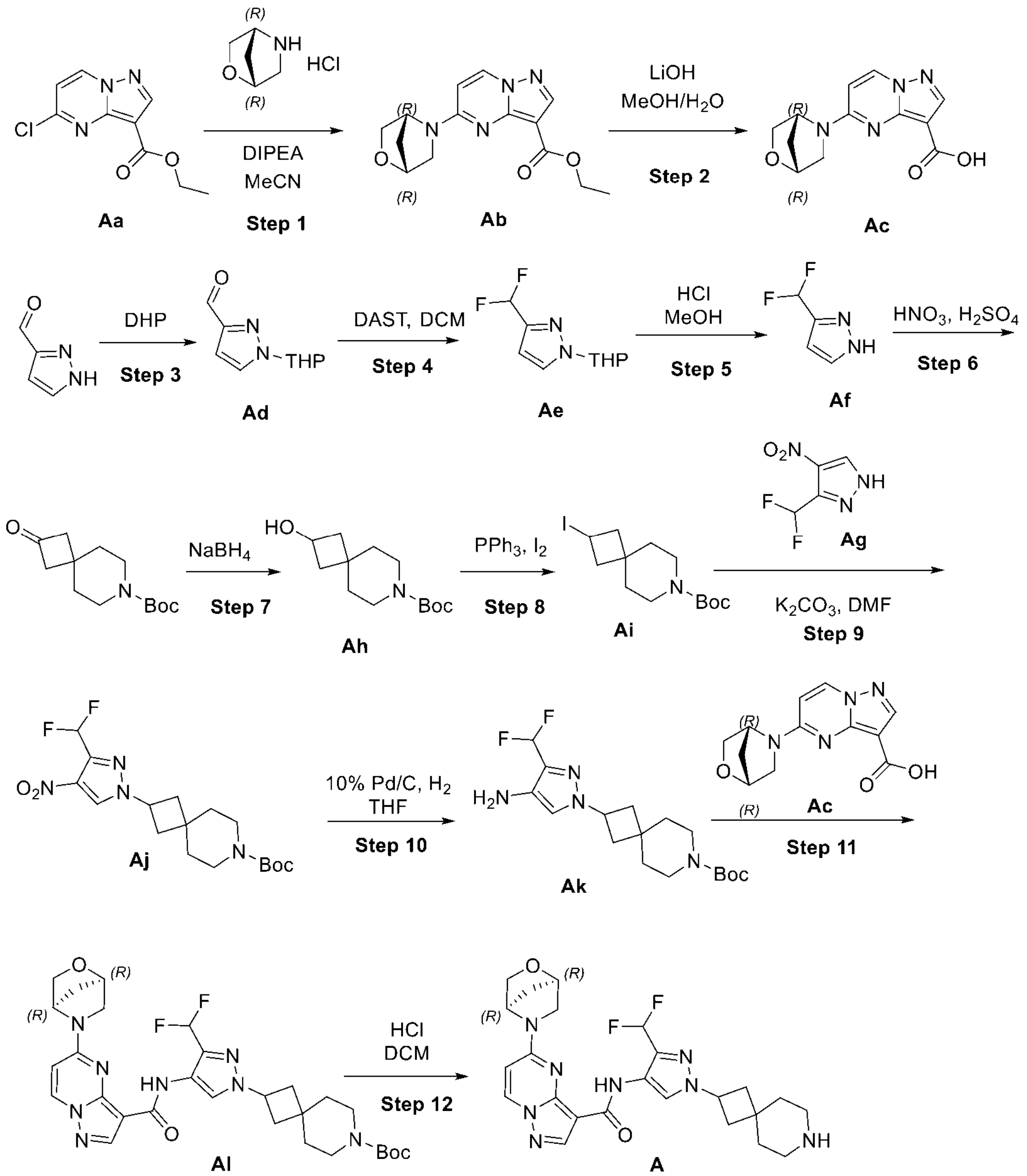


Step 1: Aa (8.32 g, 36.88 mmol) and (1*R*,4*R*)-2-oxa-5-azabicyclo[2.2.1]heptane hydrochloride (5 g, 36.88 mmol) were dissolved in acetonitrile (60 mL) at room temperature under an argon atmosphere, and *N*,*N*-diisopropylethylamine (14.3 g, 110.63 mmol) was slowly added dropwise with stirring. The reaction mixture was then stirred overnight at 60°C. LC-MS showed complete consumption of the starting material, with the formation of a single product Ab. Water (100 mL) was added to the cooled reaction mixture, and then the mixture was extracted with ethyl acetate (3 × 50 mL). The combined organic phases were washed with saturated brine (3 × 50 mL), dried over anhydrous sodium sulfate, and filtered. The filtrate was concentrated under reduced pressure, and the resulting residue was separated by silica gel column chromatography to obtain Ab (10.1 g). $^1$H NMR (400 MHz, $CDCl_3$) δ 8.32 - 8.20 (m, 2H), 6.12 (brs, 1H), 5.45 (brs, 1H), 4.82 - 4.71 (m, 1H), 4.42 - 4.26 (m, 2H), 4.02 - 3.84 (m, 2H), 3.51 - 3.37 (m, 2H), 2.12 - 1.87 (m, 2H), 1.41 - 1.39 (m, 3H).

Step 2: Ab (10.1 g, 35.03 mmol) was dissolved in anhydrous methanol/water (100 mL/25 mL) at room temperature, followed by the addition of lithium hydroxide monohydrate (8.82 g, 210.19 mmol) with stirring. The reaction mixture was then stirred overnight at 60°C. LC-MS showed that trace amounts of the starting material remained, and the main product was Ac. The cooled reaction mixture was diluted with water (100 mL), and the pH was adjusted to 5 with dilute hydrochloric acid (1 M) in an ice-water bath. The resulting mixture was extracted with ethyl acetate (3 × 50 mL). The combined organic phases were washed with saturated brine, dried over anhydrous sodium sulfate, and filtered. The filtrate was concentrated under reduced pressure to obtain Ac (8.7 g). $^1$H NMR (400 MHz, $CDCl_3$) δ 8.36 - 8.28 (m, 2H), 6.40 - 6.17 (m, 1H), 5.22, 4.82 (s, 1H), 4.68 (s, 1H), 3.99 - 3.90 (m, 2H), 3.72 - 3.51 (m, 2H), 2.13 - 2.03 (m, 2H).

Step 3: 1H-Pyrazole-5-carbaldehyde (58.7 g, 610.89 mmol) and $TsOH \cdot H_2O$ were dissolved in DCM (580 mL), cooled to 0°C, and DHP (61.6 g, 733.07 mmol) was slowly added. The ice bath was removed, and the reaction mixture was

warmed to room temperature and stirred overnight. TEA (23.5 mL) was added to the reaction mixture, and the mixture was concentrated by rotary evaporation. The residue was purified by silica gel column chromatography to obtain Ad (88 g). $^1$H NMR (400 MHz, $CDCl_3$) δ 10.00 (s, 1H), 7.68 (dd, *J* = 2.6, 0.6 Hz, 1H), 6.83 (d, *J* = 2.8 Hz, 1H), 5.49 - 5.45 (m, 1H), 4.09 - 4.05 m, 1H), 3.77 - 3.70 (m, 1H), 2.16 - 2.03 (m, 3H), 1.77 - 1.63 (m, 3H).

Step 4: Ad (88 g, 488.33 mmol) was dissolved in DCM (500 mL), and DAST (157 g, 976.65 mmol) was added dropwise at 0°C. The ice bath was removed, and the reaction was carried out overnight at room temperature. TLC (PE/EtOAc = 10/1) monitored that the reaction was not complete. The reaction mixture was slowly quenched with $NaHCO_3$ (3 L). The organic phase was separated, and the aqueous phase was further extracted with DCM (3 × 1 L). The organic phases were combined, dried over $Na_2SO_4$, filtered, and concentrated by rotary evaporation. The residue was purified by a silica gel column to obtain Ae (69.65 g). $^1$H NMR (400 MHz, $CDCl_3$) δ 7.63 (d, *J* = 2.4 Hz, 1H), 6.70 (t, *J* = 55.0 Hz, 1H), 6.51 (d, *J* = 2.4 Hz, 1H), 5.39 - 5.35 (m, 1H), 4.07 - 4.03 (m, 1H), 3.75 - 3.65 (m, 1H), 2.15 - 1.97 (m, 3H), 1.74 - 1.57 (m, 3H).

Step 5: Ae (25.7 g, 127.10 mmol) was dissolved in MeOH (254 mL), and HCl (4 M in 1,4-dioxane) (254 mL, 1.016 mol) was added at 0°C. The ice bath was removed, and the reaction was carried out overnight at room temperature. The completion of the reaction was monitored by TLC. The reaction mixture was concentrated, followed by addition of saturated $NaHSO_3$ (420 mL) and EtOAc (420 mL). The mixture was stirred at room temperature for 1 h, then extracted with EtOAc (3 × 200 mL). The combined organic phases were dried over $Na_2SO_4$, concentrated by rotary evaporation to obtain Af (16.8 g). $^1$H NMR (400 MHz, $CDCl_3$) δ 7.65 - 7.63 (m, 1H), 6.80 (t, *J* = 55.6 Hz, 1H), 6.56 - 6.54 (m, 1H).

Step 6: Af (14.0 g, 118.56 mmol) was dissolved in concentrated $H_2SO_4$ (140 mL), cooled to 0°C in an ice bath, and $HNO_3$ (65%, 40.23 g, 414.95 mmol) was added dropwise. The temperature was slowly warmed to room temperature, then gradually increased to 60°C, and the reaction was carried out overnight at 115°C. The reaction mixture was cooled to room temperature, poured into ice water (1 L), and extracted with EtOAc (3 × 1 L). The combined organic phases were dried over $Na_2SO_4$, concentrated by rotary evaporation, and purified to obtain Ag (7.79 g). $^1$H NMR (400 MHz, $CDCl_3$) δ 8.46 (s, 1H),7.35 (t, *J*= 53.6 Hz, 1H).

Step 7: *tert*-Butyl 2-oxo-7-azaspiro[3.5]nonane-7-carboxylate (9.57 g, 39.99 mmol) was dissolved in anhydrous methanol (100 mL) at room temperature, and sodium borohydride (1.82 g, 47.99 mmol) was slowly added in an ice-water bath with stirring. The reaction mixture was slowly returned to room temperature and stirred for 1 hour. TLC showed that the starting material was completely reacted. Saturated aqueous ammonium chloride solution (100 mL) was added to the reaction mixture, and then the mixture was extracted with ethyl acetate (3 × 100 mL). The combined organic phases were washed with saturated brine (200 mL), dried over anhydrous sodium sulfate, and filtered. The filtrate was concentrated, and the resulting residue was separated by silica gel column chromatography to obtain Ah (9.14 g). $^1$H NMR (400 MHz, $CDCl_3$) δ 4.37 - 4.28 (m, 1H), 3.35 - 3.27 (m, 4H), 2.31 - 2.25 (m, 2H), 1.80 - 1.78 (m, 1H), 1.71 - 1.65 (m, 2H), 1.54 - 1.47 (m, 4H), 1.45 (s, 9H).

Step 8: Ah (9.14 g, 37.87 mmol) was dissolved in toluene (90 mL) at room temperature, followed by sequential addition of triphenylphosphine (14.90 g, 56.81 mmol), imidazole (5.16 g, 75.75 mmol), and iodine (14.42 g, 56.81 mmol) in an ice-water bath with stirring. The reaction mixture was slowly returned to room temperature and stirred for 1 hour, then heated to 60°C and stirred for another hour. LC-MS showed that the starting material was completely reacted. Water (200 mL) was added to the reaction mixture, and then the mixture was extracted with ethyl acetate (3 × 200 mL). The combined organic phases were washed with saturated brine (400 mL), dried over anhydrous sodium sulfate, and filtered. The filtrate was concentrated, and the resulting residue was separated by silica gel column chromatography to obtain Ai (7.98 g). $^1$H NMR (400 MHz, $CDCl_3$) δ 4.54 - 4.45 (m, 1H), 3.34 - 3.30 (m, 2H), 3.29 - 3.25 (m, 2H), 2.69 - 2.63 (m, 2H), 2.45 - 2.39 (m, 2H), 1.69 - 1.65 (m, 2H), 1.57 - 1.53 (m, 2H), 1.44 (s, 9H).

Step 9: Ai (5 g, 14.24 mmol) and Ag (2.11 g, 12.94 mmol) were dissolved in *N,N*-dimethylformamide (50 mL) at room temperature, followed by the addition of potassium carbonate (3.58 g, 25.88 mmol) with stirring. The reaction mixture was then stirred overnight at 100°C. LC-MS showed that the starting material was completely reacted. The reaction mixture was cooled to room temperature, then added with water (100 mL), and extracted with ethyl acetate (3 × 100 mL). The combined organic phases were washed with saturated brine (200 mL), dried over anhydrous sodium sulfate, and filtered. The filtrate was concentrated, and the resulting residue was separated by silica gel column chromatography to obtain Aj (3.8 g, yield: 76%). $^1$H NMR (400 MHz, DMSO-$d_6$) δ 9.13 (s, 1H), 7.32 (t, *J*= 53.0 Hz, 1H), 5.05 - 5.00 (m, 1H), 3.32 - 3.29 (m, 2H), 3.25 - 3.21 (m, 2H), 2.44 - 2.38 (m, 2H), 2.30 - 2.23 (m, 2H), 1.60 - 1.53 (m, 4H), 1.39 (s, 9H).

Step 10: Aj (3.8 g, 9.83 mmol) was dissolved in tetrahydrofuran (60 mL) at room temperature, followed by the addition of 10% wet palladium on carbon (1.9 g). The reaction mixture was then stirred at 30°C under the protection of a hydrogen balloon for 3 hours. LC-MS showed that the starting material was completely reacted. The reaction mixture was filtered, and the filter cake was washed with ethyl acetate (100 mL). The combined filtrates were concentrated to obtain the desired product Ak (3.4 g). MS (ESI) m/z: 357 [M+H$^+$].

Step 11: Ak (2.00 g, 5.61 mmol) and Ac (1.50 g, 5.76 mmol) were dissolved in acetonitrile (40 mL) at room temperature under an argon atmosphere. 1-Methylimidazole (1.60 mL, 20.07 mmol) and *N,N,N',N'*-tetramethylchloroformamidinium hexafluorophosphate (TCFH, 2.40 g, 8.55 mmol) were added in an ice-water bath with stirring. The system was purged with argon three times, and the reaction mixture was then stirred overnight at 50°C under an argon

atmosphere. LC-MS showed that the starting material was completely reacted. Saturated aqueous sodium bicarbonate solution (40 mL) was added to the reaction mixture, and then the mixture was extracted with ethyl acetate (3 × 40 mL). The combined organic phases were washed with saturated brine (2 × 40 mL), dried over anhydrous sodium sulfate, and filtered. The filtrate was concentrated, and the resulting crude residue was separated by silica gel column chromatography to obtain the desired product Al (3.40 g). MS (ESI) m/z: 599.1 [M+H$^+$].

Step 12: A1 (3.40 g, 5.68 mmol) was dissolved in dichloromethane (40 mL) at room temperature, and a hydrochloric acid/1,4-dioxane solution (4 M, 14 mL, 56 mmol) was added in an ice-water bath with stirring. The reaction mixture was then stirred at room temperature for 2 hours. LC-MS showed that the starting material was completely reacted. The reaction mixture was concentrated to obtain the desired crude product A (3.87 g, hydrochloride). MS (ESI) m/z: 499.6 [M+H$^+$]. $^1$H NMR (400 MHz, DMSO-*d$_6$*) δ 9.51 (d, *J* = 5.2 Hz, 1H), 8.89 (s, 2H), 8.79 (d, *J* = 8.0 Hz, 1H), 8.42 (d, *J = 4.0* Hz, 1H), 8.26 (d, *J* = 5.6 Hz, 1H), 7.14 (t, *J* = 53.6 Hz, 1H), 6.89 - 6.45 (m, 1H), 5.28 - 5.08 (m, 1H), 5.00 - 4.92 (m, 1H), 4.80 - 4.74 (m, 1H), 3.87 - 3.73 (m, 2H), 3.65 - 3.43 (m, 2H), 3.04 - 2.95 (m, 4H), 2.47 - 2.41 (m, 2H), 2.30 - 2.24 (m, 2H), 2.06 - 1.92 (m, 2H), 1.88 - 1.84 (m, 2H), 1.82 - 1.79 (m, 2H).

Synthesis of intermediate B:

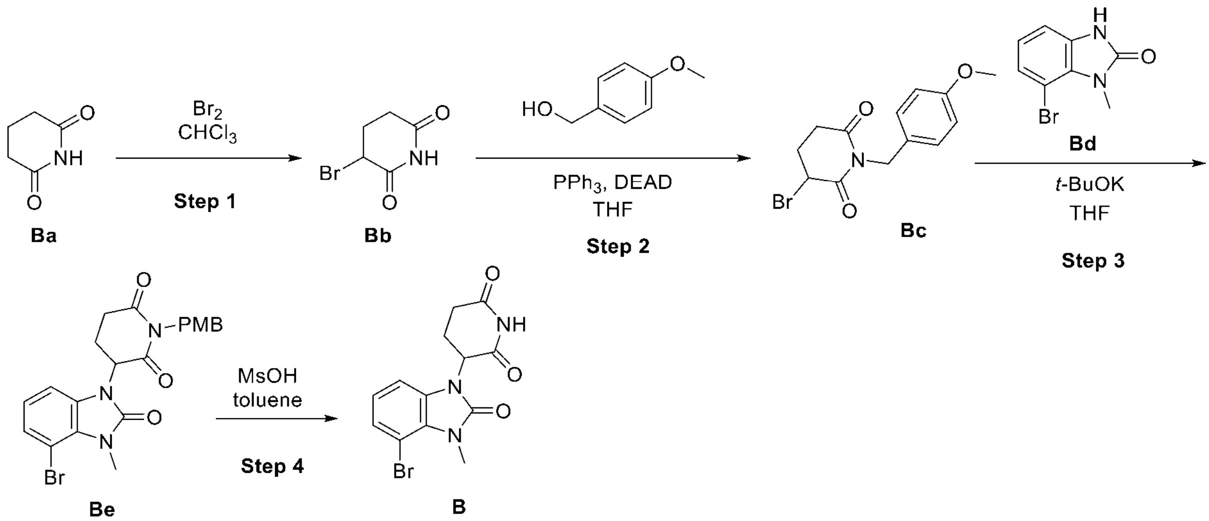


Step 1: Glutarimide (100 g, 884.0 mmol) was placed in a 500 mL sealed vessel at room temperature, then chloroform (200 mL) was added, and liquid bromine (141 g, 884.0 mmol) was slowly added with stirring. After sealing, the reaction mixture was stirred overnight at 105°C. LC-MS showed that the starting material was almost completely reacted, and the main product was Bb. The reaction mixture was cooled to room temperature and concentrated. The resulting residue was dissolved in ethyl acetate (1 L), washed with saturated aqueous sodium bicarbonate solution (500 mL) and saturated brine (500 mL), dried over anhydrous sodium sulfate, and filtered. The filtrate was concentrated, and the resulting residue was separated by silica gel column chromatography to obtain the desired product Bb (78 g). MS (ESI) m/z: 192.0 [M+H$^+$]. $^1$H NMR (400 MHz, DMSO-*d$_6$*) δ 11.05 (s, 1H), 4.88 (t, *J* = 4.8 Hz, 1H), 2.56 - 2.52 (m, 2H), 2.49 - 2.43 (m, 1H), 2.19 - 2.10 (m, 1H).

Step 2: *p*-Methoxybenzyl alcohol (20.6 g, 148.9 mmol) was dissolved in dry tetrahydrofuran (500 mL) at room temperature. Triphenylphosphine (39.1 g, 148.9 mmol) and Bb (26 g, 135.4 mmol) were added with stirring. The reaction mixture was then cooled to 0°C, and diethyl azodicarboxylate (25.9 g, 148.9 mmol) was slowly added dropwise under an argon atmosphere. After the dropwise addition was completed, the reaction mixture was naturally warmed to room temperature and stirred overnight. LC-MS showed that the starting material was completely reacted. The reaction mixture was concentrated, and the residue was purified by silica gel column chromatography to obtain the desired product Bc (29.7 g). $^1$H NMR (400 MHz, $CDCl_3$) δ 7.34 - 7.27 (m, 2H), 6.87 - 6.78 (m, 2H), 4.90 (dd, *J* = 37.9, 13.8 Hz, 2H), 4.74 - 4.68 (m, 1H), 3.78 (s, 3H), 3.10 - 2.96 (m, 1H), 2.80 - 2.69 (m, 1H), 2.42 - 2.30 (m, 1H), 2.27 - 2.17 (m, 1H).

Step 3: Bd (19.8 g, 87.4 mmol) was dispersed in dry tetrahydrofuran (400 mL) at room temperature, stirred and cooled to 0°C, and potassium *tert*-butoxide (16.7 g, 148.5 mmol) was added under an argon atmosphere. The reaction mixture was stirred at this temperature for another hour, then warmed to 45°C, and a solution of Bc (30.0 g, 96.1 mmol) in dry tetrahydrofuran (300 mL) was slowly added dropwise over 6 hours. After the dropwise addition was completed, the reaction mixture was stirred at 45°C for another 3 hours. LC-MS showed that the starting material was almost completely

reacted. The reaction mixture was cooled to 0°C, slowly quenched with saturated brine (300 mL), and the phases were separated. The organic phase was washed once with saturated brine (300 mL), dried over anhydrous sodium sulfate, and filtered. The filtrate was concentrated, and the resulting crude residue was separated by silica gel column chromatography to obtain the desired product Be (18.3 g). $^1$H NMR (400 MHz, DMSO-$d_6$) δ 7.29 - 7.15 (m, 3H), 7.13 - 7.04 (m, 1H), 6.94 (t, *J* = 8.0 Hz, 1H), 6.89 - 6.81 (m, 2H), 5.57 (dd, *J* = 13.0, 5.4 Hz, 1H), 4.79 (q, *J* = 14.3 Hz, 2H), 3.72 (s, 3H), 3.64 (s, 3H), 3.13 - 2.94 (m, 1H), 2.88 - 2.65 (m, 2H), 2.14 - 1.99 (m, 1H).

Step 4: Be (18.3 g, 19.9 mmol) was dissolved in toluene (140 mL) and methanesulfonic acid (70 mL) at room temperature, heated to 120°C, and stirred for 5 hours. LC-MS showed that the starting material was completely reacted. The reaction mixture was cooled to room temperature and concentrated. The resulting residue was slowly added dropwise to ice water (1 L), filtered, and dried to obtain the desired product B (10.5 g). $^1$H NMR (400 MHz, DMSO-$d_6$) δ 11.13 (s, 1H), 7.24 (d, *J* = 8.0 Hz, 1H), 7.17 (d, *J* = 8.0 Hz, 1H), 6.98 (t, *J* = 8.2 Hz, 1H), 5.41 (dd, *J* = 12.4, 5.2 Hz, 1H), 3.64 (s, 3H), 2.96 - 2.82 (m, 1H), 2.77 - 2.59 (m, 2H), 2.10 - 1.98 (m, 1H).

Example 1

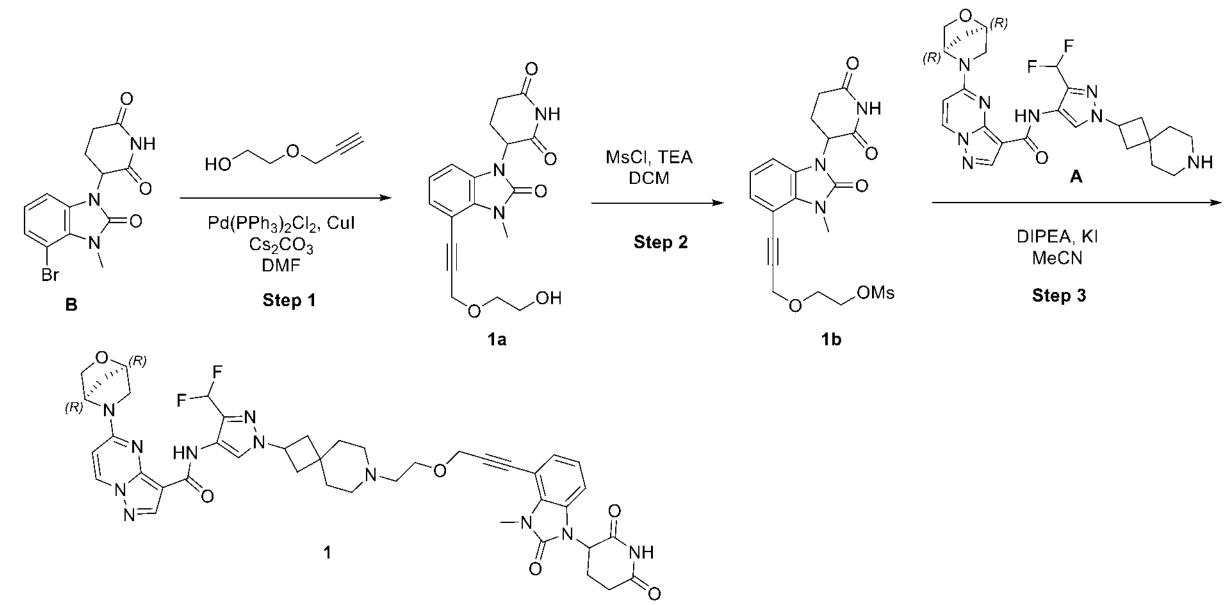


Step 1: B (300 mg, 0.89 mmol) and dry *N,N*-dimethylformamide (5 mL) were added to a dry 100 mL single-necked flask at room temperature, followed by the addition of hydroxyethyl propargyl ether (179 mg, 1.78 mmol), bis(triphenylphosphine)palladium(II) dichloride (62 mg, 0.089 mmol), copper(I) iodide (17 mg, 0.089 mmol), and cesium carbonate (870 mg, 2.67 mmol) under an argon atmosphere. The mixture was purged with argon three times, and heated to 80°C and stirred for 2 hours under an argon atmosphere. LC-MS showed that the reaction was complete. The reaction mixture was cooled to room temperature, and saturated aqueous ammonium chloride solution (20 mL) was added with stirring. The resulting mixture was extracted with dichloromethane (3 × 50 mL). The combined organic extracts were washed with saturated brine (100 mL), dried over anhydrous sodium sulfate, and filtered. The filtrate was concentrated, and the resulting residue was separated by silica gel column chromatography to obtain the desired 1a (103 mg).

Step 2: 1a (250 mg, 699.56 μmol) and triethylamine (155 mg, 1.54 mmol) were dissolved in dichloromethane (3 mL) at room temperature, and methanesulfonyl chloride (120 mg, 1.05 mmol) was added in an ice-water bath with stirring. The reaction mixture was then warmed to 30°C and stirred for 1 hour under an argon atmosphere. LC-MS showed the disappearance of the starting material. Water (30 mL) was added to the cooled reaction mixture, and then the mixture was extracted with ethyl acetate (3 × 20 mL). The combined organic phases were washed with saturated brine (3 × 0 mL), dried over anhydrous sodium sulfate, and filtered. The filtrate was concentrated, and the resulting residue was purified by preparative thin-layer chromatography to obtain 1b (80 mg). MS (ESI) m/z: 436.1 [M+H$^+$]; 434.1 [M-H$^-$]. $^1$H NMR (400 MHz, $CDCl_3$) δ 11.23 (s, 1H), 7.20 - 7.12 (m, 2H), 7.06 - 7.01 (m, 1H), 5.40 (dd, *J* = 12.2, 5.4 Hz, 1H), 4.52 (s, 2H), 4.39 - 4.36 (m, 2H), 3.83 - 3.80 (m, 1H), 3.71 - 3.63 (m, 1H), 3.61 (s, 3H), 3.20 (s, 3H), 2.95 - 2.83 (m, 1H), 2.77 - 2.60 (m, 2H), 2.07 - 1.99 (m, 1H).

Step 3: 1b (30 mg, 68.89 μmol) and A (crude hydrochloride, 51 mg, 103.34 μmol) were dissolved in acetonitrile (2 mL) at room temperature under an argon atmosphere, and potassium iodide (14 mg, 82.67 μmol) and *N,N*-diisopropy-

lethylamine (44 mg, 344.47 μmol) were added with stirring. The reaction mixture was then reacted overnight at 85°C under an argon atmosphere. LC-MS showed that a small amount of the starting material remained, and the main product was 1. The reaction mixture was cooled to room temperature, then saturated aqueous sodium bicarbonate solution (30 mL) was added thereto, and then the mixture was extracted with ethyl acetate (3 × 10 mL). The combined organic phases were washed with saturated brine (30 mL), dried over anhydrous sodium sulfate, and filtered. The filtrate was concentrated, and the resulting residue was purified by preparative thin-layer chromatography to obtain 1 (7.93 mg). MS (ESI) m/z: 838.5 [M+H$^+$]; $^1$H NMR (400 MHz, DMSO-$d_6$) δ 11.12 (s, 1H), 9.50 (d, *J* = 5.2 Hz, 1H), 8.78 (d, *J* = 7.6 Hz, 1H), 8.41 - 8.39 (m, 1H), 8.25 (d, *J* = 5.2 Hz, 1H), 7.27 - 6.96 (m, 4H), 6.88 - 6.44 (m, 1H), 5.43 - 5.36 (m, 1H), 5.28 - 5.07 (m, 1H), 5.00 - 4.90 (m, 1H), 4.79 - 4.74 (m, 1H), 4.40 (s, 2H), 3.84 - 3.57 (m, 8H), 3.47 - 3.43 (m, 1H), 2.93 - 2.84 (m, 1H), 2.76 - 2.60 (m, 2H), 2.50 - 2.10 (m, 9H), 2.06 - 1.90 (m, 4H), 1.89 - 1.55 (m, 4H).

Example 2

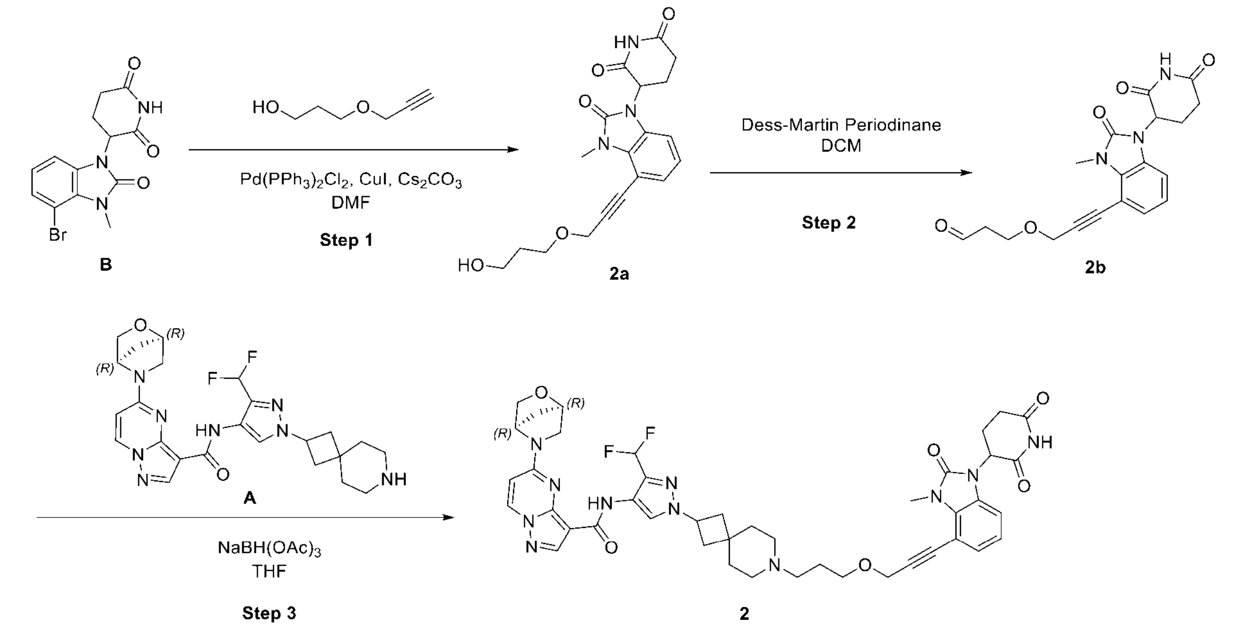


B, 2a, 2b, A, 2

Step 1: B (6.0 g, 17.7 mmol) and 3-(prop-2-yn-1-yloxy)propan-1-ol (4.0 g, 35.5 mmol) were dissolved in dry *N,N*-dimethylformamide (60 mL) at room temperature, and cesium carbonate (14.4 g, 44.4 mmol), bis(triphenylphosphine) palladium(II) chloride (1.25 g, 1.77 mmol), and copper(I) iodide (676 mg, 3.55 mmol) were added sequentially with stirring under an argon atmosphere. The reaction mixture was then stirred at 80°C under an argon atmosphere for 5 hours.

LC-MS showed that the starting material was completely reacted. The reaction mixture was cooled to room temperature and filtered. The filtrate was concentrated, and ethyl acetate (200 mL) and saturated brine (100 mL) were added to the resulting residue, followed by phase separation. The organic phase was then washed with saturated brine (100 mL), dried over anhydrous sodium sulfate, and filtered. The filtrate was concentrated, and the resulting crude residue was separated by silica gel column chromatography to obtain the desired product 2a (2.9 g). $^1$H NMR (400 MHz, DMSO-$d_6$) δ 11.12 (s, 1H), 7.17 (d, *J* = 7.3 Hz, 1H), 7.12 (d, *J* = 7.3 Hz, 1H), 7.03 (t, *J* = 7.8 Hz, 1H), 5.40 (dd, *J* = 12.6, 5.3 Hz, 1H), 4.54 - 4.31 (m, 3H), 3.64 (s, 3H), 3.60 (t, *J* = 6.5 Hz, 2H), 3.47 (t, *J* = 6.3 Hz, 2H), 2.97 - 2.82 (m, 1H), 2.78 - 2.57 (m, 2H), 2.10 - 1.98 (m, 1H), 1.77 - 1.61 (m, 2H).

Step 2: 2a (95.0 mg, 255.8 μmol) was dissolved in dichloromethane (4 mL) at room temperature, cooled in an ice-water bath, and Dess-Martin periodinane (162.7 mg, 383.7 μmol) was added with stirring. The reaction mixture was stirred at room temperature overnight. LC-MS showed the disappearance of the starting material. The reaction mixture was filtered, and water (20 mL) and dichloromethane (20 mL) were added to the filtrate for phase separation. The aqueous phase was further extracted with dichloromethane (10 mL × 2). The combined organic phases were washed with saturated aqueous sodium chloride solution, dried over anhydrous sodium sulfate, and filtered. The filtrate was concentrated, and the resulting crude product was purified by preparative thin-layer chromatography to obtain 2b (46 mg). MS (ESI) m/z: 370.1 [M+H$^+$]. $^1$H NMR (400 MHz, DMSO-$d_6$) δ 11.12 (s, 1H), 9.67 (t, *J* = 1.9 Hz, 1H), 7.18 (d, *J* = 7.6 Hz, 1H), 7.15 - 7.10 (m, 1H), 7.03 (t, *J* = 7.8 Hz, 1H), 5.40 (dd, *J* = 12.6, 5.4 Hz, 1H), 4.45 (s, 2H), 3.87 (t, *J* = 6.0 Hz, 2H), 3.64 (s, 3H), 2.96 - 2.82 (m, 1H), 2.81 - 2.56 (m, 4H), 2.10 - 1.95 (m, 1H).

Step 3: 2b (35 mg, 0.09 mmol) and A (crude hydrochloride, 47 mg, 0.09 mmol) were dissolved in tetrahydrofuran (2 mL) at room temperature, and sodium triacetoxyborohydride (100 mg, 0.47 mmol) was added in an ice-water bath with stirring. The reaction mixture was then stirred at room temperature for 2 hours. LC-MS showed that the starting material was completely reacted. Saturated aqueous sodium bicarbonate solution (20 mL) was added to the reaction mixture, and then the mixture was extracted with ethyl acetate (2 × 30 mL). The combined organic phases were washed with saturated brine (2 × 30 mL), dried over anhydrous sodium sulfate, and filtered. The filtrate was concentrated, and the resulting crude residue was purified by preparative thin-layer chromatography to obtain the desired product 2 (10.79 mg). MS (ESI) m/z: 852 [M+H$^+$]. $^1$H NMR (400 MHz, DMSO-$d_6$) δ 11.12 (s, 1H), 9.50 (d, $J$ = 5.6 Hz, 1H), 8.79 (d, $J$ = 7.6 Hz, 1H), 8.41 (d, $J$ = 4.0 Hz, 1H), 8.26 (d, $J$ = 5.6 Hz, 1H), 7.27 - 7.00 (m, 4H), 6.86 - 6.44 (m, 1H), 5.43 - 5.35 (m, 1H), 5.28 - 5.07 (m, 1H), 4.96 - 4.90 (m, 1H), 4.80 - 4.74 (m, 1H), 4.44 (s, 2H), 3.84 - 3.51 (m, 7H), 3.44 - 3.39 (m, 2H), 2.93 - 2.60 (m, 3H), 2.51 - 2.07 (m, 10H), 2.06 - 1.48 (m, 9H).

Example 3

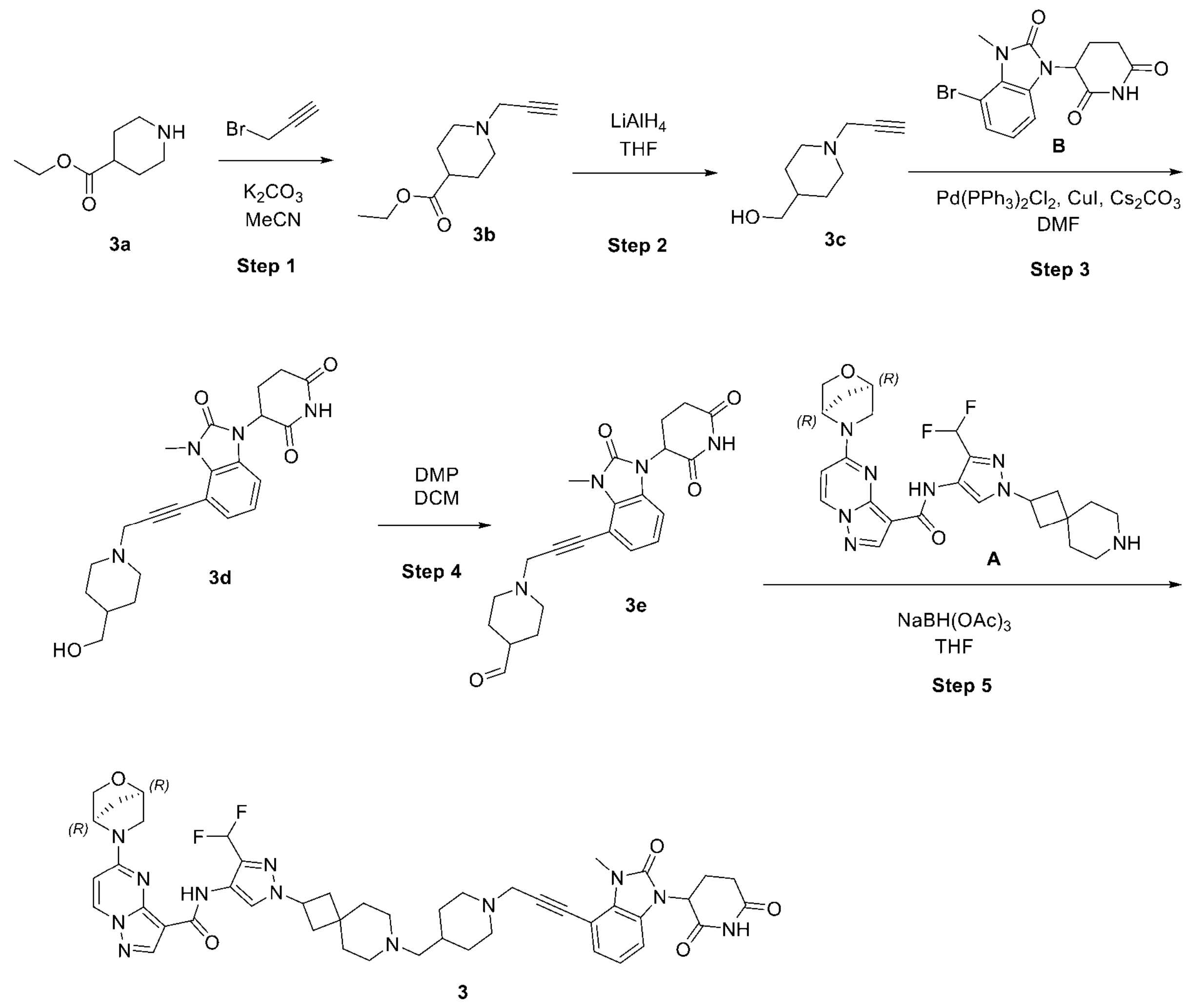


Step 1: 3a (2.0 g, 12.72 mmol) was dissolved in acetonitrile (63 mL) at room temperature, and potassium carbonate (4.4 g, 31.8 mmol) was added with stirring. The mixture was cooled to 0°C in an ice-water bath, and 3-bromopropyne (1.72 g, 13.99 mmol) was added with stirring. The reaction mixture was then stirred at room temperature for 1 hour. TLC showed complete consumption of the starting material and the formation of a new spot. The reaction mixture was slowly added dropwise to water, and then extracted with ethyl acetate (3 × 40 mL). The combined organic phases were washed with saturated brine (10 mL), dried over anhydrous sodium sulfate, and filtered. The filtrate was concentrated, and the resulting residue was separated by silica gel column chromatography to obtain 3b (2.75 g, yield: 81%) as a colorless liquid. $^1$H NMR (400 MHz, $CDCl_3$) δ 4.14 (q, $J$ = 7.1 Hz, 2H), 3.30 (d, $J$ = 2.4 Hz, 2H), 2.89 - 2.85 (m, 2H), 2.35 - 2.21

(m, 4H), 2.09 - 1.62 (m, 4H), 1.25 (t, *J* = 7.1 Hz, 3H).

Step 2: 3b (2.03 g, 10.40 mmol) was dissolved in tetrahydrofuran (15 mL) at room temperature. A tetrahydrofuran solution of lithium aluminum hydride (2.5 M, 12.48 mL, 31.2 mmol) was slowly added dropwise to the reaction mixture at 0°C with stirring, and the reaction mixture was then stirred at 0°C for 1 hour. TLC showed complete consumption of the starting material and formation of a product. The reaction mixture was quenched by slow dropwise addition into ice water, and the resulting mixture was extracted with ethyl acetate (3 × 50 mL). The combined organic phases were washed with saturated brine (10 mL), dried over anhydrous sodium sulfate, and filtered. The filtrate was concentrated, and the resulting residue was separated by silica gel column chromatography to obtain 3c (1.18 g). $^1$H NMR (400 MHz, $CDCl_3$) δ 3.50 (d, *J* = 6.5 Hz, 2H), 3.30 (d, *J* = 2.4 Hz, 2H), 2.92 (d, *J* = 11.5 Hz, 2H), 2.29 - 2.12 (m, 3H), 1.82 - 1.75 (m, 2H), 1.55 - 1.45 (m, 1H), 1.38 - 1.20 (m, 2H).

Step 3: 3c (159 mg, 1.04 mmol) was dissolved in *N*,*N*-dimethylformamide (10 mL) at room temperature, and B (422 mg, 1.25 mmol), copper(I) iodide (39.61 mg, 0.21 mmol), and cesium carbonate (1.36 g, 4.16 mmol) were added under an argon atmosphere. The reaction mixture was purged with argon three times, and then bis(triphenylphosphine) palladium(II) dichloride (146 mg, 0.21 mmol) was added. The reaction mixture was then stirred at 80°C for 2 hours under an argon atmosphere. LC-MS showed complete consumption of the starting material and formation of a product. The cooled reaction mixture was diluted with water, and the resulting mixture was extracted with ethyl acetate (3 × 50 mL). The combined organic phases were washed with saturated brine (10 mL), dried over anhydrous sodium sulfate, and filtered. The filtrate was concentrated, and the resulting residue was separated by silica gel column chromatography to obtain 3d (178 mg). MS (ESI) m/z: 411.2 [M+H$^+$].

Step 4: 3d (100 mg, 0.259 mmol) was dissolved in *N*,*N*-dimethylformamide (2 mL) at room temperature, cooled to 0°C in an ice-water bath, and Dess-Martin periodinane (165 mg, 0.389 mmol) was added with stirring. The reaction mixture was then stirred at room temperature for 4 hours. LC-MS showed complete consumption of the starting material and formation of a product. The reaction mixture was slowly added dropwise to saturated aqueous sodium bicarbonate solution, and then extracted with ethyl acetate (3 × 10 mL). The combined organic phases were washed with saturated brine (10 mL), dried over anhydrous sodium sulfate, and filtered. The filtrate was concentrated, and the resulting residue was separated by preparative thin-layer chromatography to obtain 3e (48 mg). MS (ESI) m/z: 409.2 [M+H$^+$]. $^1$H NMR (400 MHz, $CDCl_3$) δ 9.67 (s, 1H), 8.06 (s, 1H), 7.17 (d, *J*= 7.9 Hz, 1H), 6.98 (t, *J* = 7.9 Hz, 1H), 6.74 (d, *J* = 7.8 Hz, 1H), 5.19 (dd, *J* = 12.6, 5.3 Hz, 1H), 3.77 (s, 3H), 3.58 (s, 2H), 2.96 - 2.90 (m, 3H), 2.84 - 2.75 (m, 2H), 2.50 - 2.20 (m, 5H), 2.05 - 1.95 (m, 3H).

Step 5: 3e (24 mg, 0.058 mmol) and A (crude hydrochloride, 32 mg, 0.058 mmol) were dissolved in tetrahydrofuran (1 mL) at room temperature, stirred in an ice-water bath for 20 minutes, and sodium triacetoxyborohydride (50 mg, 0.235 mmol) was added. The reaction mixture was then stirred overnight at room temperature. LC-MS showed complete consumption of the starting material and formation of a product. The reaction mixture was quenched by slow addition into water, and the resulting mixture was extracted with ethyl acetate (3 × 5 mL). The combined organic phases were washed with saturated brine (5 mL), dried over anhydrous sodium sulfate, and filtered. The filtrate was concentrated, and the resulting residue was separated by preparative thin-layer chromatography to obtain 3 (13.8 mg, yield: 26%). MS (ESI) m/z: 891.4 [M+H$^+$]. $^1$H NMR (400 MHz, DMSO-$d_6$) δ 11.12 (s, 1H), 9.50 (d, *J* = 5.1 Hz, 1H), 8.78 (d, *J* = 7.7 Hz, 1H), 8.40 (s, 1H), 8.25 (d, *J* = *5.6* Hz, 1H), 7.28 - 7.00 (m, 4H), 6.89 - 6.45 (m, 1H), 5.43 - 5.37 (m, 1H), 5.28 - 5.08 (m, 1H), 4.94 (s, 1H), 4.80 - 4.75 (m, 1H), 3.84 - 3.71 (m, 2H), 3.65 (s, 3H), 3.58 - 3.43 (m, 4H), 2.98 - 2.65 (m, 7H), 2.33 - 2.20 (m, 7H), 2.20 - 1.98 (m, 5H), 1.71 - 1.64 (m, 7H), 1.24 - 1.15 (m, 3H).

Example 4

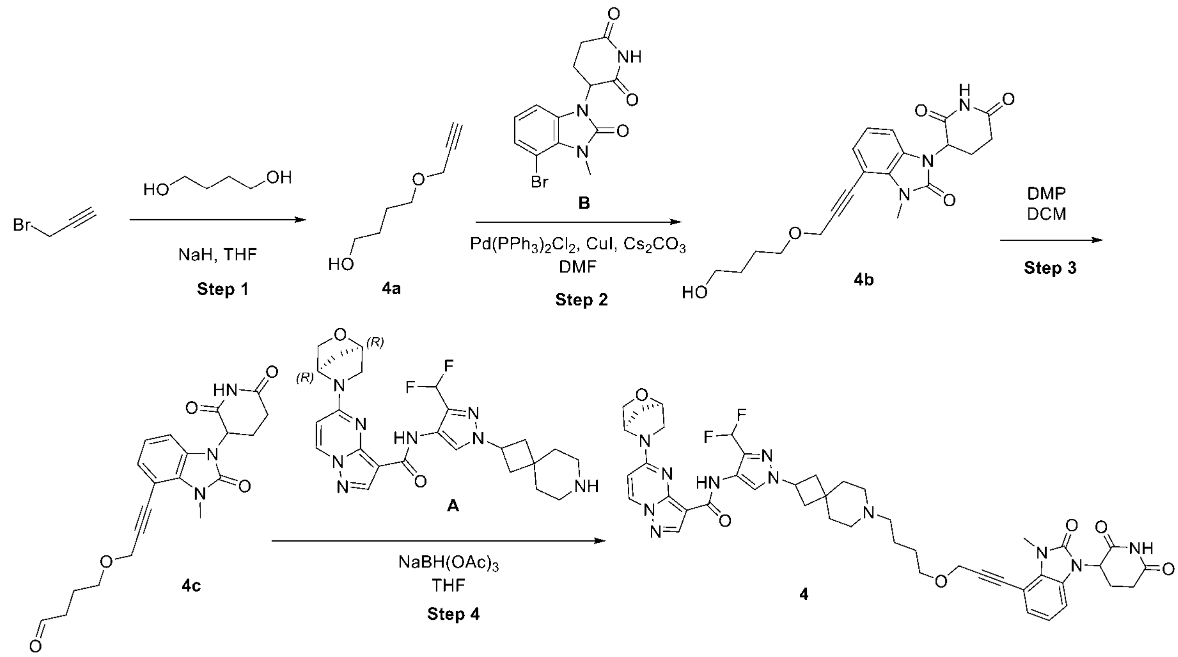


Step 1: 1,4-Butanediol (11.36 g, 126.09 mmol) was dissolved in tetrahydrofuran (30 mL) at room temperature, cooled to 0°C, and sodium hydride (60%, 2.02 g, 50.44 mmol) was added with stirring. The reaction mixture was then stirred at room temperature for 1 hour. The reaction mixture was cooled to 0°C, and a solution of 3-bromopropyne (3.0 g, 25.22 mmol) in tetrahydrofuran (10 mL) was slowly added dropwise with stirring. After the dropwise addition was completed, the reaction mixture was stirred overnight at room temperature. TLC showed complete consumption of the starting material and the formation of a new spot. The reaction mixture was diluted with water and then extracted with ethyl acetate (3 × 40 mL). The combined organic phases were washed with saturated brine (10 mL), dried over anhydrous sodium sulfate, and filtered. The filtrate was concentrated, and the resulting residue was separated by silica gel column chromatography to obtain 4a (2.75 g). $^1H$ NMR (400 MHz, $CDCl_3$) δ 4.15 (d, *J* = 2.4 Hz, 2H), 3.67 (t, *J* = 5.8 Hz, 2H), 3.57 (t, *J* = 5.9 Hz, 2H), 2.43 (t, *J* = 2.4 Hz, 1H), 1.78 - 1.65 (m, 5H).

The subsequent reaction procedures were performed with reference to Example 2. 4 (22.06 mg, yield: 42%) was obtained. MS (ESI) m/z: 866.5 $[M+H^+]$. $^1H$ NMR (400 MHz, DMSO-$d_6$) δ 11.12 (s, 1H), 9.50 (d, *J* = 4.8 Hz, 1H), 8.79 (d, *J* = 8.0 Hz, 1H), 8.40 (d, *J* = 3.6 Hz, 1H), 8.26 (d, *J* = 5.6 Hz, 1H), 7.28 - 7.00 (m, 4H), 6.89 - 6.44 (m, 1H), 5.44 - 5.39 (m, 1H), 5.28 - 5.08 (m, 1H), 4.95 - 4.90 (m, 1H), 4.80 - 4.74 (m, 1H), 4.43 (s, 2H), 3.84 - 3.72 (m, 2H), 3.64 - 3.62 (m, 4H), 3.59 - 3.55 (m, 3H), 3.47 - 3.43 (m, 1H), 2.91 - 2.85 (m, 1H), 2.75 - 2.57 (m, 3H), 2.38 - 2.28 (m, 4H), 2.20 - 2.11 (m, 3H), 2.06 - 1.92 (m, 4H), 1.75 - 1.50 (m, 8H).

Example 5

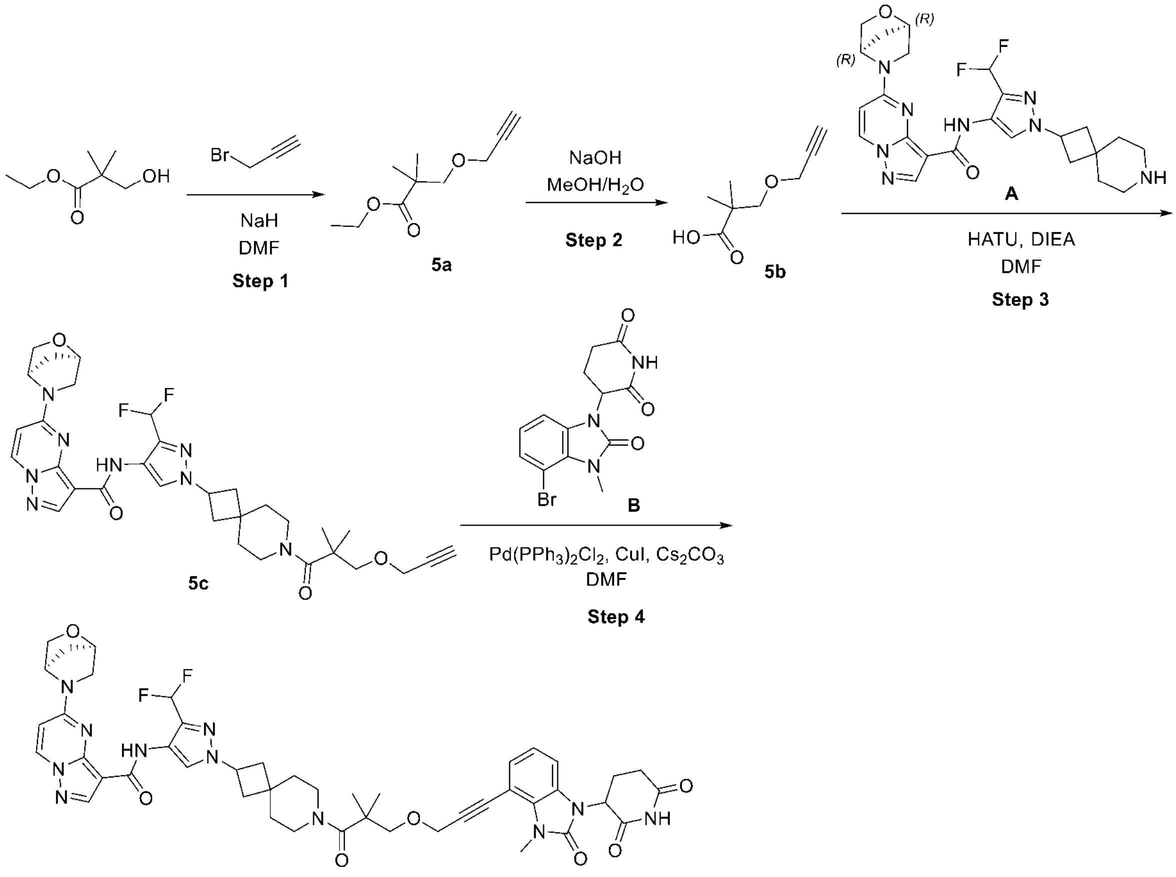


5

Step 1: Ethyl 2-(hydroxymethyl)-2-methylpropanoate (5.0 g, mmol) was dissolved in N,N-dimethylformamide (50 mL) at room temperature, and sodium hydride (60%, 2.05 g, 51.3 mmol) was added in an ice-water bath with stirring. After 20 minutes, 3-bromopropyne (3.25 mL, 4.48 g, 37.62 mmol) was added to the reaction mixture. After 20 minutes, the ice-water bath was removed, and the reaction mixture was stirred overnight at room temperature under an argon atmosphere. TLC showed the disappearance of the starting material and the formation of a new spot. Saturated aqueous ammonium chloride solution (about 50 mL) was added to the reaction mixture. The resulting mixture was extracted with ethyl acetate (3 × 00 mL). The combined organic phases were washed with saturated brine, dried over anhydrous sodium sulfate, and filtered. The filtrate was concentrated, and the resulting residue was separated by silica gel column chromatography to obtain 5a (2.12 g). $^1$H NMR (400 MHz, $CDCl_3$) δ 4.17 - 4.11 (m, 4H), 3.53 (s, 2H), 2.41 (t, *J* = 2.2 Hz, 1H), 1.25 (t, *J* = 7.0 Hz, 3H), 1.20 (s, 6H).

Step 2: 5a (1.0 g, 5.43 mmol) was dissolved in methanol/water (10 mL/5 mL) at room temperature, and sodium hydroxide (2.17 g, 54.3 mmol) was added. The reaction mixture was stirred at 100°C for 2 hours. TLC showed that the reaction was complete. The pH of the cooled reaction mixture was adjusted to 5 with concentrated hydrochloric acid, and the resulting mixture was extracted with ethyl acetate (3 × 50 mL). The combined organic phases were washed with saturated brine, dried over anhydrous sodium sulfate, and filtered. The filtrate was concentrated to obtain a crude product 5b (772 mg). $^1$H NMR (400 MHz, $CDCl_3$) δ 4.18 (d, *J* = 2.4 Hz, 2H), 3.55 (s, 2H), 2.43 (t, *J* = 2.2 Hz, 1H), 1.24 (s, 6H).

Step 3: 2-(7-Azabenzotriazol-1-yl)-*N,N,N'N'*-tetramethyluronium hexafluorophosphate (HATU, 72 mg, 0.56 mmol) was added to a solution of A (140 mg, 0.28 mmol) and 5b (50 mg, 0.32 mmol) in *N,N*-dimethylformamide (2 mL) in an ice-water bath. After 5 minutes, *N,N*-diisopropylethylamine (0.1 mL, 0.42 mmol) was added. The reaction mixture was then stirred at room temperature for 5 hours. LC-MS showed that the reaction was complete. Water (about 5 mL) was added to the reaction mixture, and then the mixture was extracted with ethyl acetate (3 × 10 mL). The combined organic phases were washed with saturated brine (2 × 10 mL), dried over anhydrous sodium sulfate, and filtered. The filtrate was concentrated, and the resulting crude residue was separated by silica gel column chromatography to obtain a crude product (106 mg). The crude product was further purified by preparative thin-layer chromatography to obtain 5c (58 mg). MS (ESI) m/z: 637.4 [M+H$^+$].

Step 4: B (15 mg, 0.044 mmol) and 5c (58 mg, 0.091 mmol) were dissolved in *N,N*-dimethylformamide (2 mL) under an argon atmosphere, followed by the sequential addition of cesium carbonate (29 mg, 0.090 mmol), copper(I) iodide (2 mg, 0.0089 mmol), and bis(triphenylphosphine)palladium(II) dichloride (3 mg, 0.0046 mmol). The reaction

mixture was then stirred at 80°C under an argon atmosphere for 6 hours. LC-MS showed completion of the reaction. The cooled reaction mixture was filtered through diatomite. The filter cake was washed with ethyl acetate (about 10 mL). The filtrate was added with water and then extracted with ethyl acetate (3 × 10 mL). The combined organic phases were washed with saturated brine, dried over anhydrous sodium sulfate, and filtered. The filtrate was concentrated, and the resulting residue was separated by preparative thin-layer chromatography to obtain 5 (8.81 mg). MS (ESI) m/z: 894.3 [M+H$^+$]. $^1$H NMR (400 MHz, DMSO-$d_6$) δ 11.11 (s, 1H), 9.51 (d, *J* = 5.6 Hz, 1H), 8.78 (d, *J* = 7.6 Hz, 1H), 8.39 (d, *J* = 4.0 Hz, 1H), 8.27 (d, *J* = 5.6 Hz, 1H), 7.28 - 7.01 (m, 5H), 6.88 - 6.44 (m, 1H), 5.42 - 5.37 (m, 1H), 5.28 - 5.07 (m, 1H), 4.94 - 4.85 (m, 2H), 4.77 (d, *J* = 16.0 Hz, 1H), 4.47 (s, 2H), 3.81 (s, 2H), 3.65 - 3.59 (m, 9H), 2.70 - 2.57 (m, 2H), 2.33 - 2.27 (m, 4H), 2.14 (t, *J* = 9.6 Hz, 3H), 2.06 - 1.90 (m, 5H), 1.60 - 1.44 (m, 6H).

Example 6

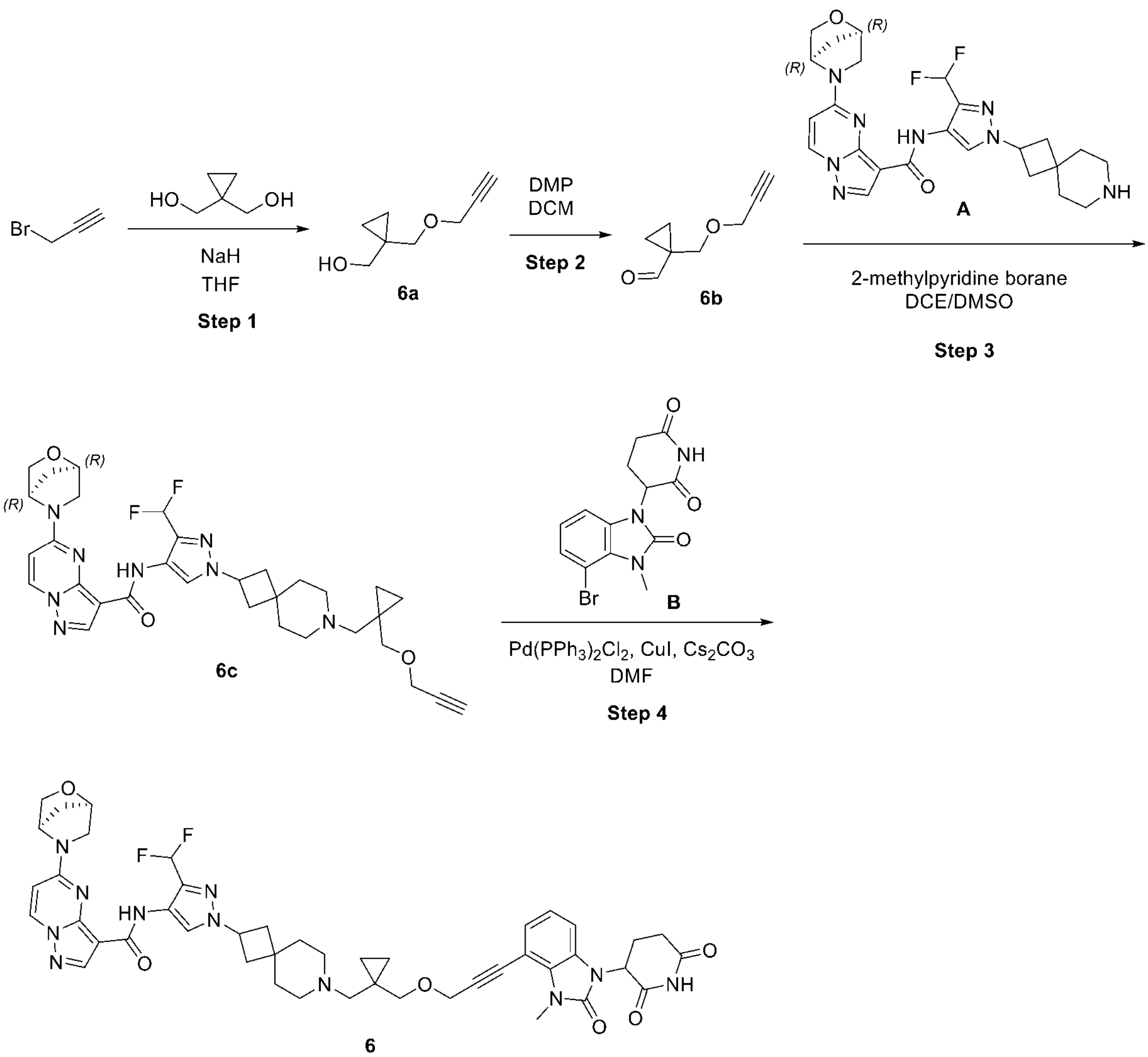


The desired product 6 (11.83 mg) was obtained with reference to Example 4. MS (ESI) m/z: 877.38 [M+H$^+$]. $^1$H NMR (400 MHz, DMSO-$d_6$) δ 11.11 (s, 1H), 9.50 (d, *J* = 5.2 Hz, 1H), 8.79 (d, *J* = 7.6 Hz, 1H), 8.40 (d, *J* = 2.8 Hz, 1H), 8.47 (d, *J* = 5.6 Hz, 1H), 7.28 - 7.00 (m, 4H), 6.87 - 6.44 (m, 1H), 5.44 - 5.07 (m, 2H), 4.98 - 4.74 (m, 2H), 4.45 (s, 2H), 3.84 - 3.72 (m, 2H), 3.66 - 3.59 ( m, 4H), 3.47 - 3.35 (m, 3H), 2.92 - 2.60 (m, 4H), 2.42 - 1.93 (m, 12H), 1.79 - 1.48 (m, 4H), 0.49 - 0.31 (m, 4H).

Example 7

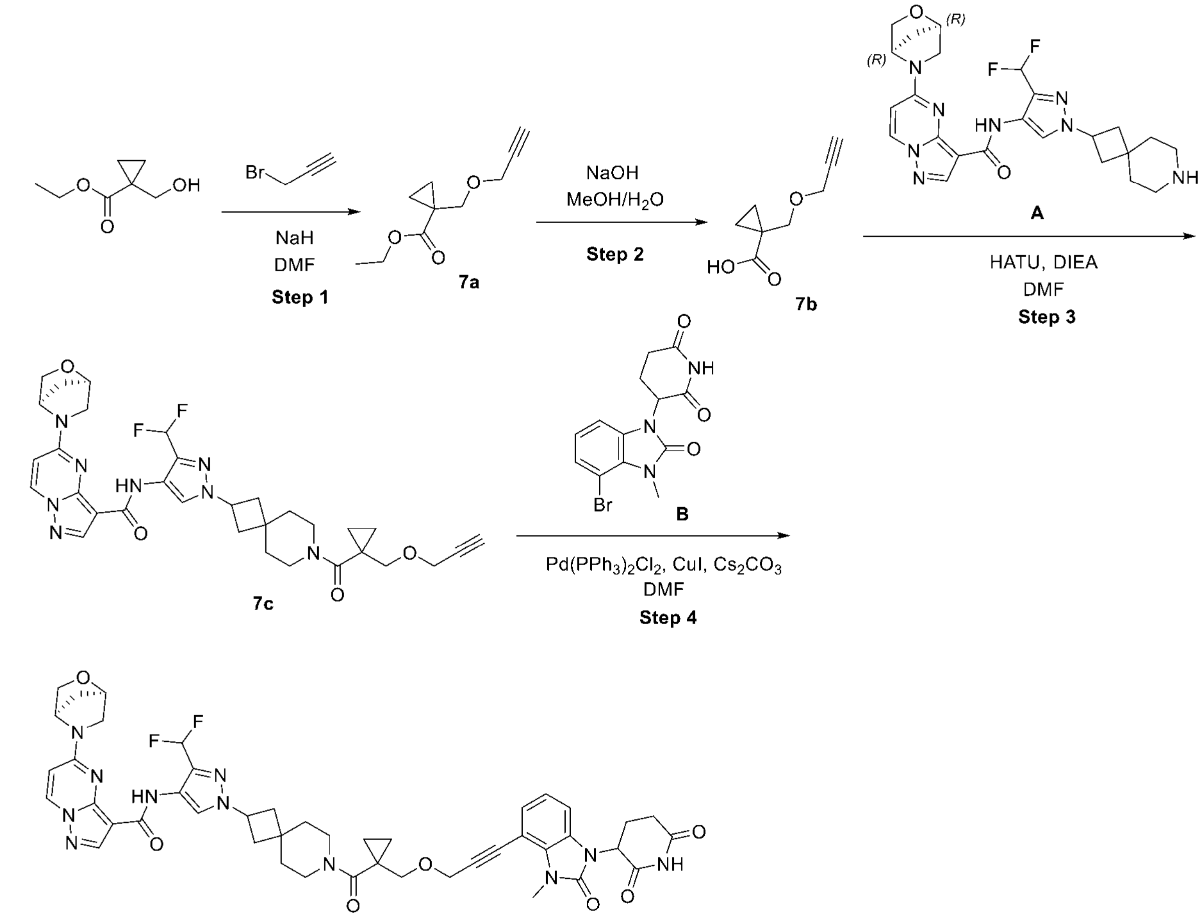


7

The desired product 7 (5.06 mg) was obtained with reference to Example 5. MS (ESI) m/z: 892.3 [M+H+]. $^1$H NMR (400 MHz, DMSO-$d_6$) δ 11.11 (s, 1H), 9.50 (d, $J$ = 5.6 Hz, 1H), 8.79 (d, $J$ = 8.0 Hz, 1H), 8.39 (d, d, $J$ = 3.6 Hz, 1H), 8.27 (d, $J$ = 5.6 Hz, 1H), 7.30 - 6.44 (m, 5H), 5.42 - 5.07 (m, 2H), 4.96 - 4.75 (m, 2H), 4.45 (s, 2H), 3.84 - 3.51 (m, 12H), 2.90 - 2.55 (m, 3H), 2.28 - 1.90 (m, 8H), 1.60 - 1.45 (m, 4H), 0.91 - 0.73 (m, 4H).

Example 8

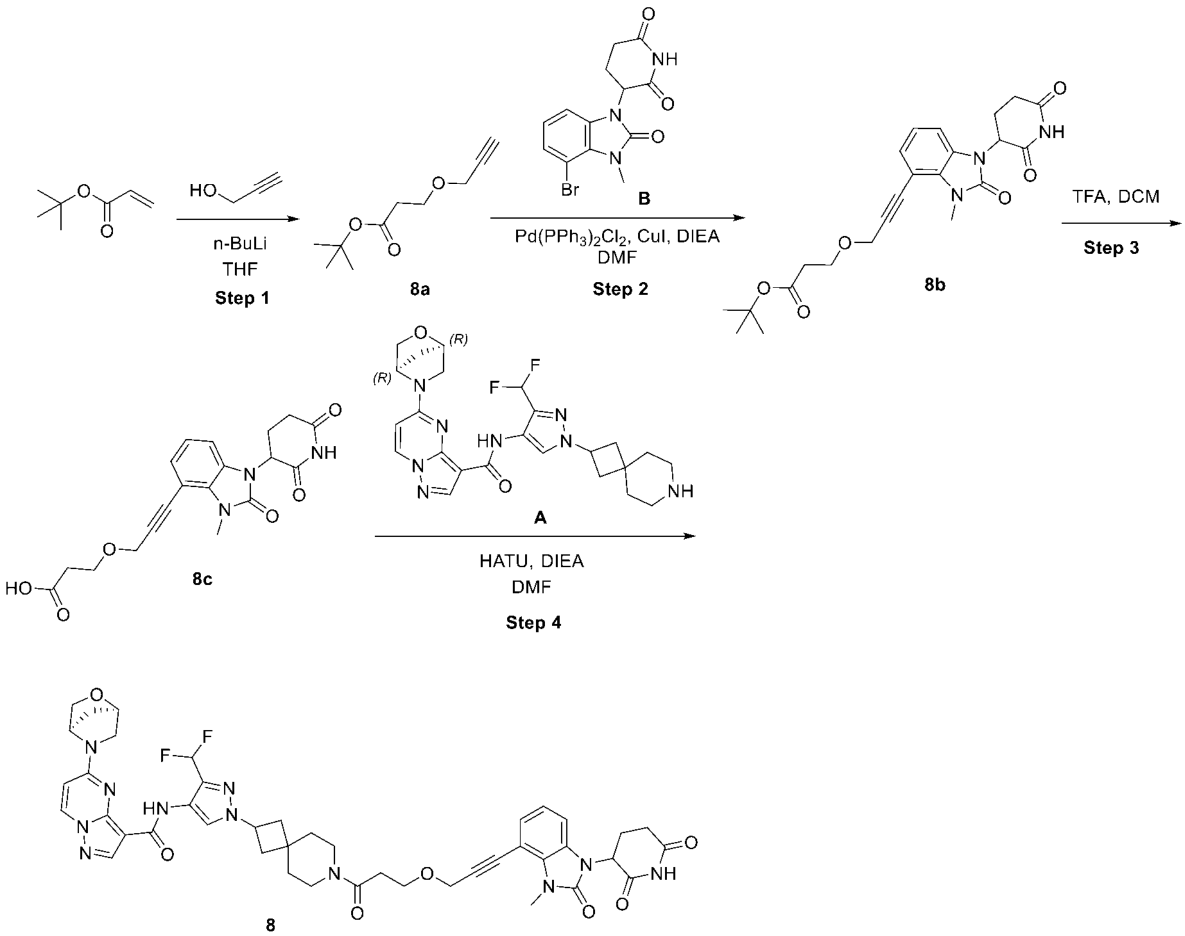


Step 1: n-Butyllithium (1.6 M in hexanes, 7 mL, 11.2 mmol) was added to a stirred solution of propargyl alcohol (6.4 mL, 110 mmol) in tetrahydrofuran (100 mL) via syringe at 0°C under an argon atmosphere. The reaction mixture was stirred at 0°C under an argon atmosphere for 10 minutes. *tert*-Butyl acrylate (14.6 mL, 110 mmol) was slowly added dropwise via syringe at 0°C. The reaction mixture was slowly returned to room temperature and stirred overnight under an argon atmosphere. TLC showed that the starting material was almost completely consumed. Acetic acid (1 mL) was added to the reaction mixture, and then the mixture was stirred for 10 minutes. The resulting mixture was added with water (100 mL) and extracted with ethyl acetate (3 × 50 mL). The combined organic phases were washed with saturated brine (200 mL), dried over anhydrous sodium sulfate, and filtered. The filtrate was concentrated, and the resulting residue was separated by silica gel column chromatography to obtain 8a (14 g). $^1H$ NMR (400 MHz, DMSO-$d_6$) δ 4.12 - 4.10 (m, 2H), 3.62 (t, *J* = 6.2 Hz, 2H), 3.42 - 3.40 (m, 1H), 2.44 (t, *J* = 6.2 Hz, 2H), 1.40 (s, 9H).

Step 2: N,N-Diisopropylethylamine (459 mg, 3.56 mmol) was added in one portion to a solution of dry B (200 mg, 0.59 mmol) and 8a (220 mg, 1.18 mmol) in dry *N,N*-dimethylformamide (8 mL) at room temperature, and then bis(triphenylphosphine)palladium(II) dichloride (28 mg, 0.04 mmol) and copper(I) iodide (24 mg, 0.12 mmol) were added under an argon atmosphere. The reaction mixture was heated to 80°C under an argon atmosphere and stirred for 5 hours. LC-MS showed that the starting material was completely reacted, and the main product was 8b (40%, 254 nm; $[M+H]^+$ = 442, R.T. = 1.57 min). The cooled reaction mixture was diluted with ethyl acetate (30 mL) and then filtered through diatomite. The filter cake was washed with ethyl acetate (30 mL). Water (50 mL) was added to the filtrate, and then the mixture was extracted with ethyl acetate (3 × 50 mL). The combined organic phases were washed with saturated brine (50 mL), dried over anhydrous sodium sulfate, and filtered. The filtrate was concentrated, and the resulting residue was separated by silica gel column chromatography to obtain pure 8b (146 mg). MS (ESI) m/z: 464.1 $[M+Na^+]$.

Step 3: Trifluoroacetic acid (2 mL) was added dropwise to a stirred solution of 8b (146 mg, 0.33 mmol) in dichloromethane (2 mL) in an ice-water bath. The reaction mixture was then slowly returned to room temperature and stirred for 2 hours. LC-MS showed that the main product was 8c, and the starting material was completely consumed. The reaction mixture was subjected to rotary evaporation to remove dichloromethane and trifluoroacetic acid. Purified water (30 mL) was added to the resulting crude residue, followed by lyophilization overnight to obtain the crude product 8c (58 mg). MS (ESI) m/z: 386.1 $[M+H^+]$.

Step 4: 8c (57.9 mg, 0.15 mmol), 2-(7-azabenzotriazol-1-yl)-*N,N,N',N'*-tetramethyluronium hexafluorophosphate (HATU, 76 mg, 0.2 mmol), and *N,N*-diisopropylethylamine (64.5 mg, 0.5 mmol) were added sequentially to a stirred solution of A (50 mg, 0.1 mmol) in *N,N*-dimethylformamide (3 mL) at room temperature under an argon atmosphere. The reaction mixture was slowly returned to room temperature and stirred for 1 hour under an argon atmosphere. LC-MS showed that the starting material was completely reacted, and the main product was 8 (34%). Saturated aqueous sodium bicarbonate solution (30 mL) was added to the reaction mixture, and then the mixture was extracted with ethyl acetate (3 × 30 mL). The combined organic phases were washed with saturated brine (50 mL), dried over anhydrous sodium sulfate, and filtered. The filtrate was concentrated, and the resulting residue was separated by preparative thin-layer chromatography to obtain pure 8 (2.07 mg + 16.31 mg, yield: 22%). MS (ESI) m/z: 866.55 [M+H+]; 864.30 [M-H-]. $^1H$ NMR (400 MHz, DMSO-$d_6$) δ 11.12 (s, 1H), 9.51 (d, *J* = 5.2 Hz, 1H), 8.79 (d, *J* = 7.6 Hz, 1H), 8.41 (d, *J* = 4.0 Hz, 1H), 8.26 (d, *J* = 5.6 Hz, 1H), 7.29 - 7.01 (m, 4H), 6.89 - 6.44 (m, 1H), 5.44 - 5.39 (m, 1H), 5.28 - 5.08 (m, 1H), 4.99 - 4.94 (m, 1H), 4.80 - 4.75 (m, 1H), 4.44 (s, 2H), 3.84 - 3.73 (m, 4H), 3.65 - 3.59 (m, 5H), 3.47 - 3.43 (m, 4H), 2.90 - 2.84 (m, 1H), 2.75 - 2.60 (m, 4H), 2.38 - 2.33 (m, 2H), 2.23 - 2.17 (m, 2H), 2.08 - 1.95 (m, 3H), 1.65-1.53 (m, 4H).

Example 9

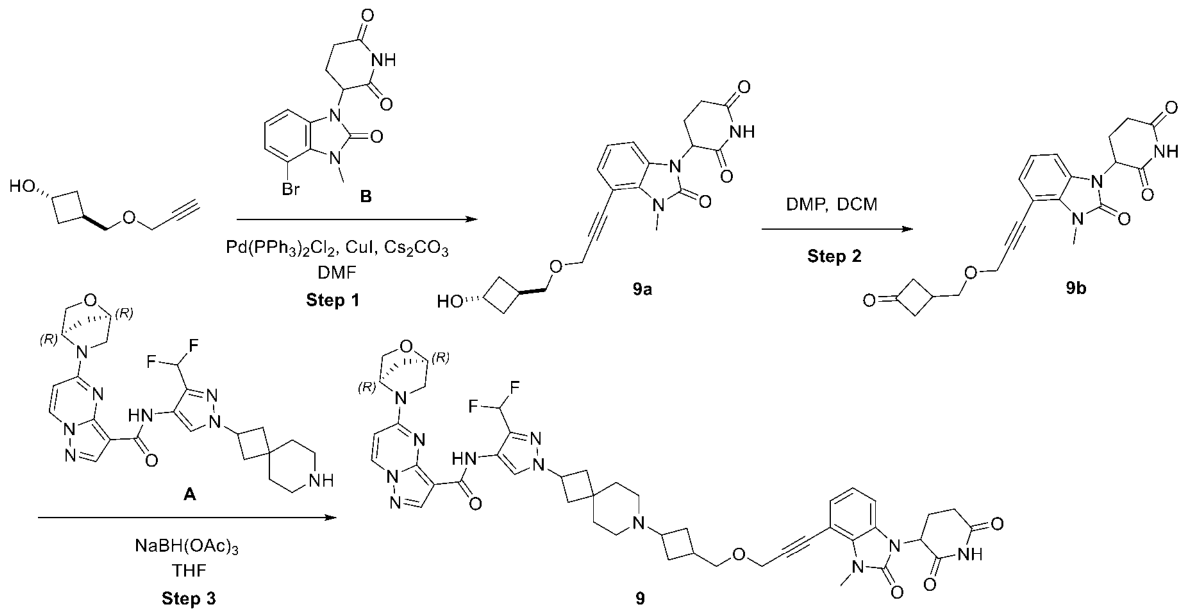


The desired product 9 (2.83 mg) was obtained with reference to Example 2. MS (ESI) m/z: 878.4 [M+H+]; $^1H$ NMR (400 MHz, $CDCl_3$) δ 9.61 (s, 1H), 8.47 - 8.41 (m, 2H), 8.32 (d, *J= 8.0* Hz, 2H), 7.22 - 7.17 (m, 1H), 7.04 - 6.97 (m, 2H), 6.80 - 6.73 (m, 2H), 6.12 (d, *J= 7.6* Hz, 1H), 5.45 (s, 1H), 5.35 - 5.17 (m, 2H), 4.84 - 4.60 (m, 3H), 4.40 (s, 2H), 4.10 - 4.07 (m, 1H), 4.00 - 3.95 (m, 2H), 3.81 (s, 3H), 3.68 - 3.64 (m, 2H), 3.61 - 3.56 (m, 2H), 3.51 - 3.48 (m, 2H), 2.98 - 2.92 (m, 2H), 2.88 - 2.67 (m, 4H), 2.48 - 2.36 (m, 8H), 2.12 - 2.09 (m, 2H), 2.03 - 1.97 (m, 2H).

Example 10

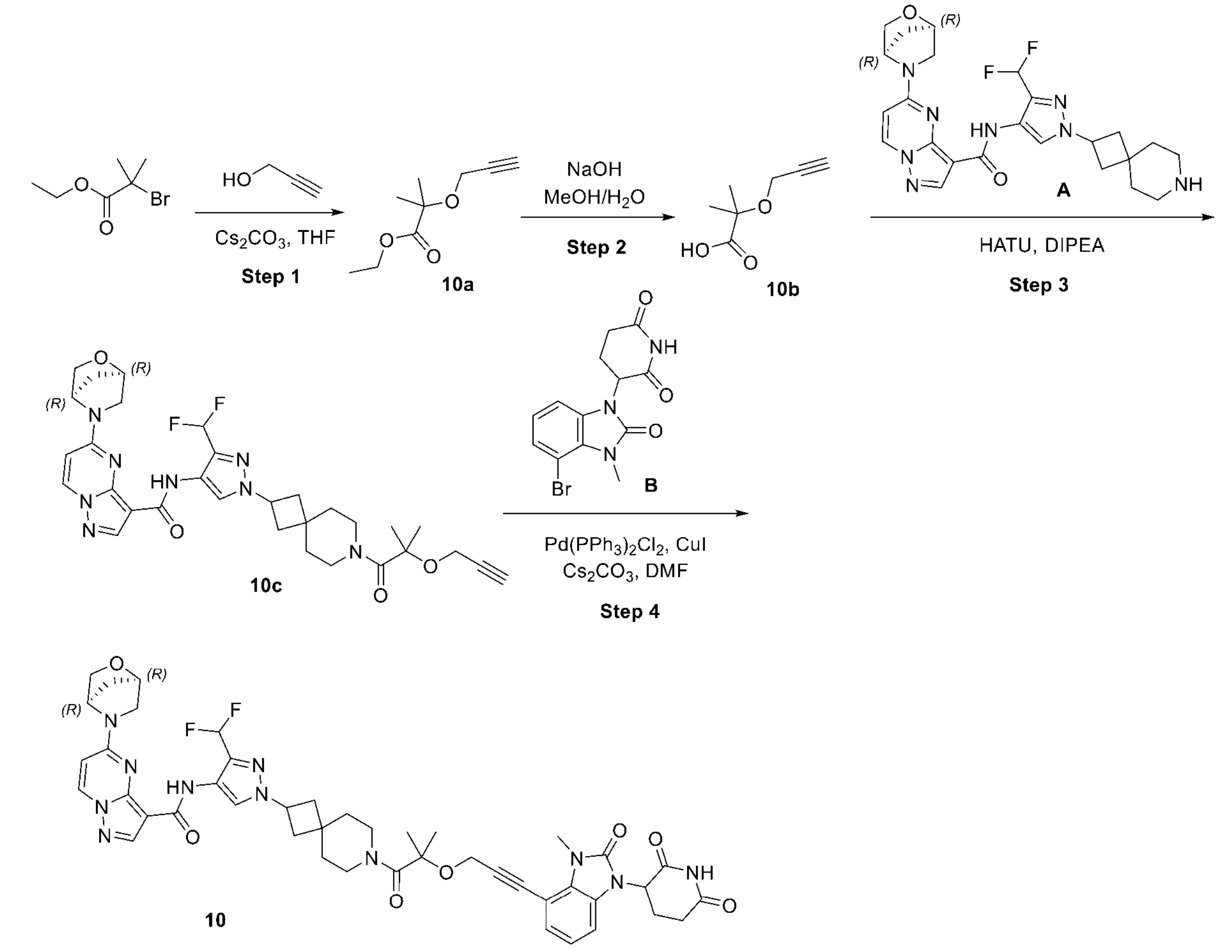


Step 1: Ethyl 2-bromo-2-methylpropanoate (1.0 g, 5.13 mmol) was dissolved in tetrahydrofuran (10 mL) at room temperature, and cesium carbonate (3.34 g, 10.26 mmol) was added in an ice-water bath with stirring. After 20 minutes, propargyl alcohol (0.30 mL, 5.13 mmol) was added slowly. After 20 minutes, the reaction mixture was returned to room temperature and stirred overnight under an argon atmosphere. TLC showed complete consumption of the brominated starting material and formation of a new spot. Saturated aqueous ammonium chloride solution (about 20 mL) was added to the reaction mixture in an ice-water bath with stirring. The resulting mixture was extracted with ethyl acetate (3 × 30 mL). The combined organic phases were washed with saturated brine, dried over anhydrous sodium sulfate, and filtered. The filtrate was concentrated, and the resulting residue was purified by preparative thin-layer chromatography to obtain 10a (200 mg). $^1$H NMR (400 MHz, $CDCl_3$) δ 4.27 - 4.15 (m, 4H), 2.42 (t, *J* = 2.4 Hz, 1H), 1.48 (s, 6H), 1.33 - 1.24 (m, 3H).

The subsequent procedures were performed with reference to Example 5 to obtain the desired product 10 (4.6 mg). MS (ESI) m/z: 880.5 [M+H$^+$]. $^1$H NMR (400 MHz, DMSO-$d_6$) δ 11.12 (s, 1H), 9.50 (d, *J* = 4.8 Hz, 1H), 8.79 (d, *J* = 8.0 Hz, 1H), 8.41 (d, *J* = 4.0 Hz, 1H), 8.26 (d, *J = 5.6* Hz, 1H), 7.28 - 7.00 (m, 4H), 6.88 - 6.44 (m, 1H), 5.43 - 5.08 (m, 2H), 5.01 - 4.97 (m, 1H), 4.77 (d, *J* = 17.2 Hz, 1H), 4.37 (s, 2H), 3.95 - 3.81 (m, 4H), 3.64 - 3.59 (m, 5H), 3.51 - 3.43 (m, 2H), 2.91 - 2.85 (m, 1H), 2.72 - 2.58 (m, 2H), 2.41 - 2.33 (m, 2H), 2.24 - 2.19 (m, 2H), 2.06 - 1.94 (m, 3H), 1.80 - 1.50 (m, 4H), 1.42 (s, 6H).

Example 11

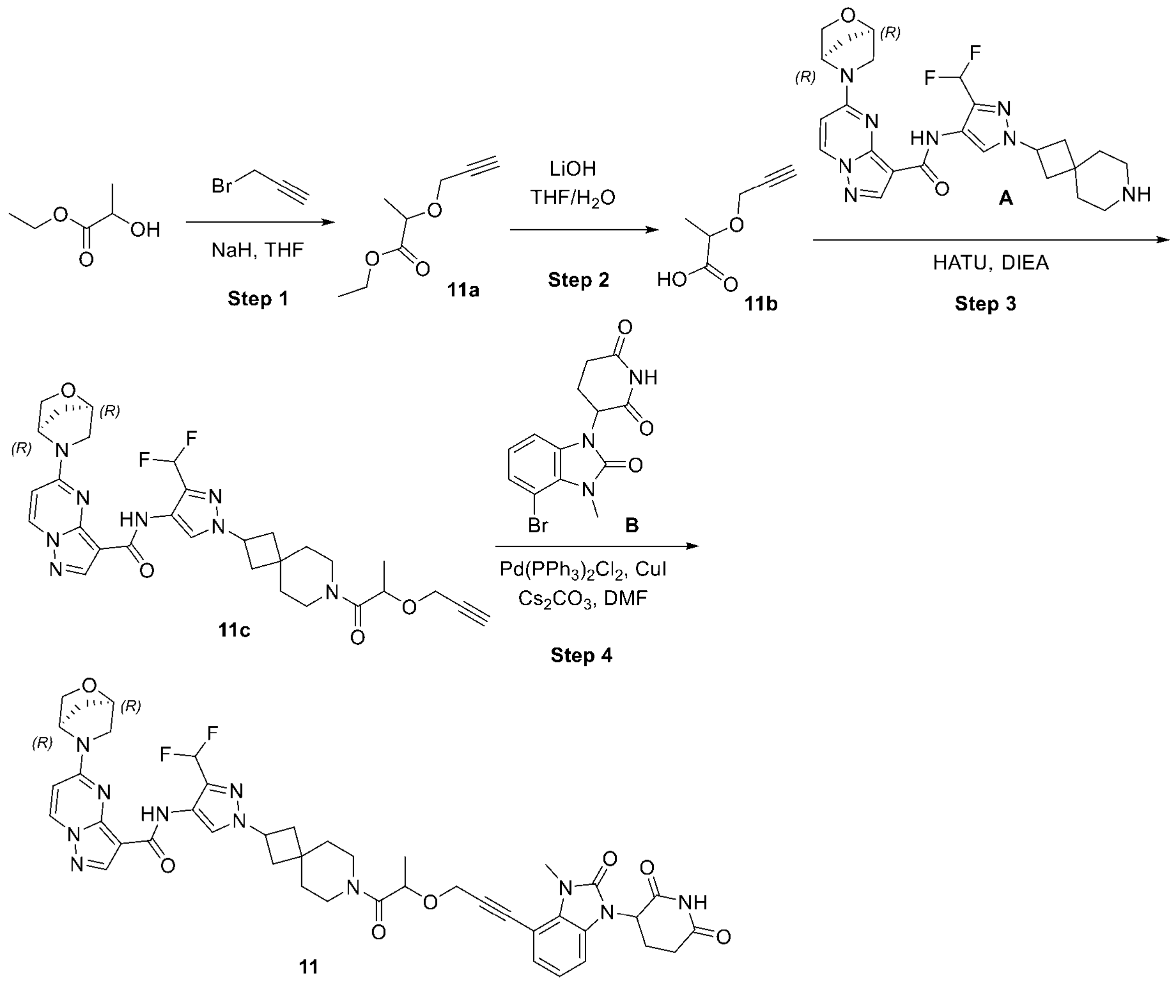


11

The desired product 11 (3.1 mg) was obtained with reference to Example 5. MS (ESI) m/z: 866.4 [M+H$^+$]. $^1$H NMR (400 MHz, DMSO-$d_6$) δ 11.12 (s, 1H), 9.50 (d, $J$ = 5.2 Hz, 1H), 8.79 (d, $J$ = 8.0 Hz, 1H), 8.41 (d, $J$ = 4.0 Hz, 1H), 8.26 (d, $J$ = 5.6 Hz, 1H), 7.29 - 7.00 (m, 4H), 6.88 - 6.44 (m, 1H), 5.42 - 5.38 (m, 1H), 5.28 - 5.08 (m, 1H), 4.98 - 4.93 (m, 1H), 4.80 - 4.75 (m, 1H), 4.62 - 4.56 (m, 1H), 4.52 - 4.37 (m, 2H), 3.84 - 3.80 (m, 2H), 3.75 - 3.72 (m, 1H), 3.64 - 3.63 (m, 3H), 3.59 (s, 1H), 3.54 - 3.43 (m, 4H), 2.92 - 2.84 (m, 1H), 2.76 - 2.59 (m, 3H), 2.49 - 2.15 (m, 4H), 2.07 - 1.93 (m, 3H), 1.66 - 1.49 (m, 4H), 1.29 - 1.27 (m, 2H).

Example 12

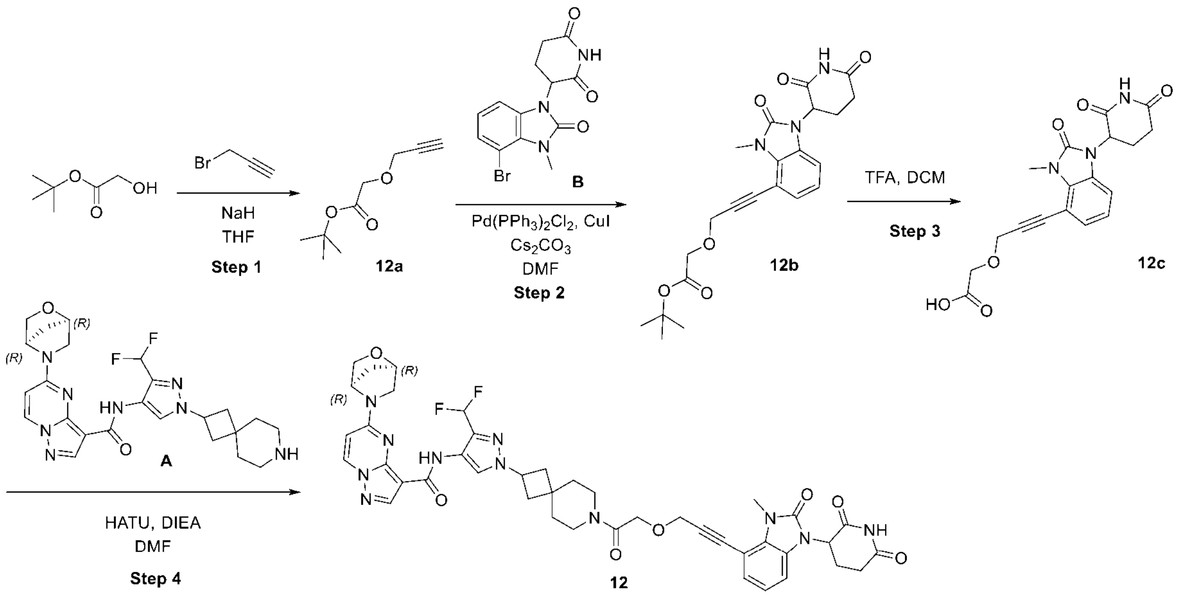


The desired product 12 (36.29 mg) was obtained with reference to Example 5. MS (ESI) m/z: 852.4 [M+H$^+$]; $^1$H NMR (400 MHz, DMSO-$d_6$) δ 11.12 (s, 1H), 9.50 (d, *J* = 5.2 Hz, 1H), 8.85 (s, 2H), 8.79 (d, *J* = 7.6 Hz, 1H), 8.69 (s, 1H), 8.41 (d, *J* = 3.6 Hz, 1H), 8.26 (d, *J* = 5.6 Hz, 1H), 7.55 - 7.46 (m, 1H), 7.20 - 7.01 (m, 4H), 6.88 - 6.45 (m, 1H), 5.41 (dd, *J* = 12.6, 5.4 Hz, 1H), 5.28 - 5.07 (m, 1H), 5.00 - 4.93 (m, 1H), 4.80 - 4.75 (m, 1H), 4.54 (s, 2H), 4.29 (d, *J* = 6.8 Hz, 2H), 3.81 (s, 3H), 3.63 - 3.57 (m, 4H), 3.55 - 3.30 (m, 1H), 2.95 - 2.55 (m, 3H), 2.40 - 2.35 (m, 2H), 2.24 - 2.19 (m, 2H), 2.06 - 1.93 (m, 2H), 1.69 - 1.50 (m, 4H).

Example 13

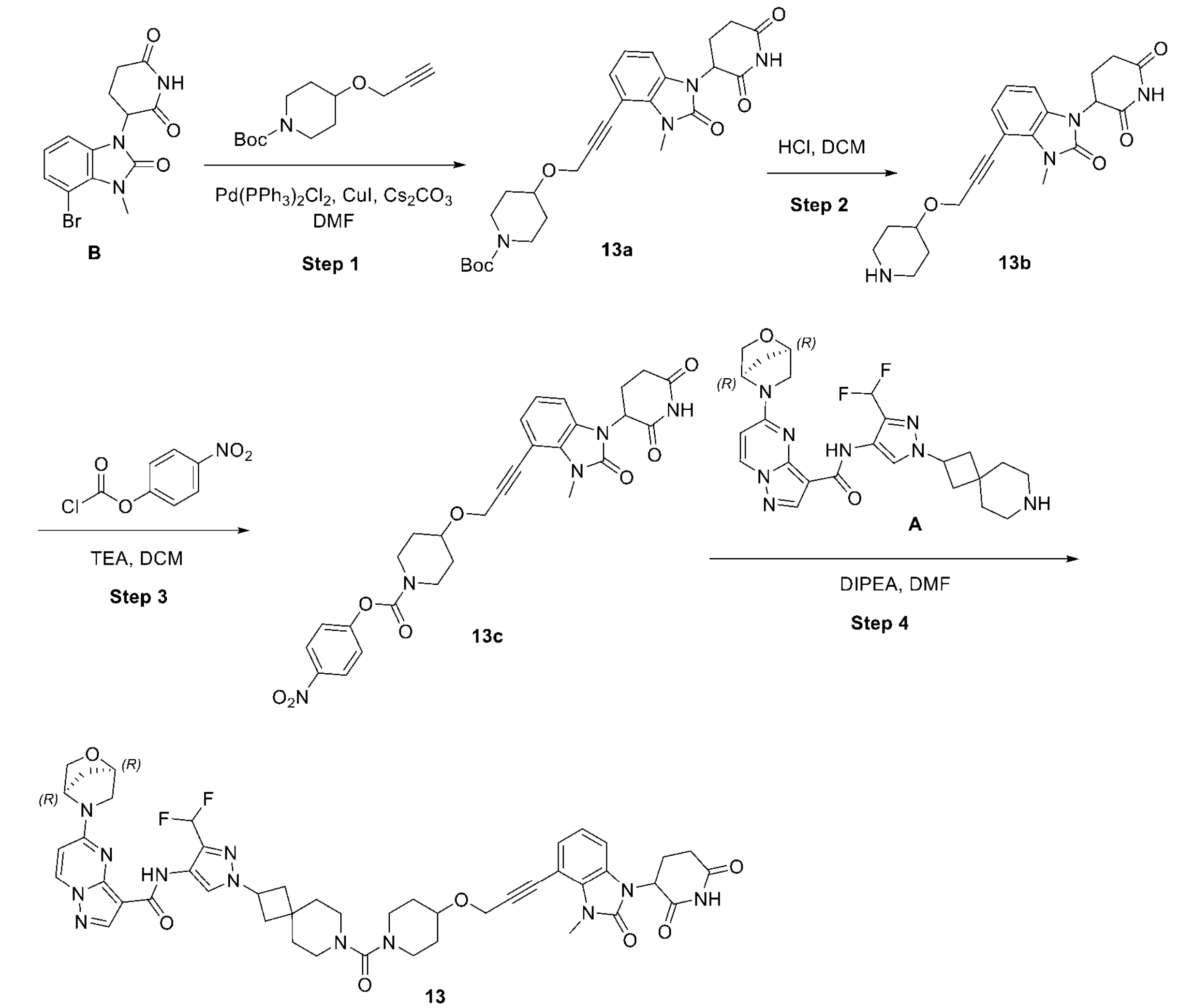


B, 13a, 13b, 13c, A, 13

Step 1: B (1.0 g, 2.96 mmol) was dissolved in N,N-dimethylformamide (10 mL) at room temperature under an argon atmosphere, and *tert*-butyl 4-(prop-2-yn-1-yloxy)piperidine-1-carboxylate (1.42 g, 5.91 mmol), cesium carbonate (1.93 g, 5.91 mmol), copper(I) iodide (112 mg, 591.43 μmol), and bis(triphenylphosphine)palladium(II) dichloride (207 mg, 295.72 μmol) were added sequentially. The reaction mixture was stirred at 80°C under an argon atmosphere for 2 hours. LC-MS showed that a small amount of the starting material B remained, and the main product was 13a. The reaction mixture was cooled to room temperature, then added with water (100 mL), and extracted with ethyl acetate (3 × 50 mL). The combined organic phases were washed with saturated brine (3 × 50 mL), dried over anhydrous sodium sulfate, and filtered. The filtrate was concentrated, and the resulting residue was separated by silica gel column chromatography to obtain 13a (1.03 g). $^1$H NMR (400 MHz, $CDCl_3$) δ 8.26 (s, 1H), 7.16 (d, *J* = 8.0 Hz, 1H), 6.99 (t, *J* = 7.8 Hz, 1H), 6.76 (d, *J* = 7.6 Hz, 1H), 5.20 (dd, *J* = 12.6, 5.4 Hz, 1H), 4.46 (s, 2H), 3.81 - 3.73 (m, 6H), 3.16 - 3.09 (m, 2H), 2.98 - 2.66 (m, 3H), 2.34 - 2.17 (m, 1H), 1.94 - 1.81 (m, 2H), 1.60 - 1.52 (m, 2H), 1.46 (s, 9H).

Step 2: 13a (300 mg, 604.15 μmol) was dissolved in dichloromethane (3 mL) in an ice-water bath, and trifluoroacetic acid (1.17 g, 12.08 mmol) was added with stirring. The reaction mixture was slowly returned to room temperature and stirred for 2 hours. LC-MS showed that the starting material was completely consumed and 13b was the main product. The reaction mixture was concentrated under reduced pressure to obtain 13b (236 mg, trifluoroacetate salt). $^1$H NMR (400 MHz, DMSO-$d_6$) δ 7.25 - 7.00 (m, 3H), 5.40 (dd, *J* = 12.6, 5.4 Hz, 1H), 4.50 (s, 2H), 3.72 - 3.67 (m, 1H), 3.64 (s, 3H), 3.08 - 3.02 (m, 2H), 2.93 - 2.83 (m, 1H), 2.76 - 2.60 (m, 3H), 2.04 - 2.00 (m, 1H), 1.94 - 1.91 (m, 2H), 1.53 - 1.45 (m, 2H).

Step 3: 13b (100 mg, 252.24 μmol) and *p*-nitrophenyl chloroformate (76 mg, 378.36 μmol) were dissolved in dichloromethane (3 mL), and triethylamine (76 mg, 756.72 μmol) was added at 0°C with stirring. The reaction mixture was stirred for 1 hour at room temperature under an argon atmosphere. LC-MS showed the disappearance of the starting material. Saturated aqueous sodium bicarbonate solution (30 mL) was added to the reaction mixture, and then the mixture was extracted with dichloromethane (3 × 10 mL). The combined organic phases were washed with saturated brine (30 mL), dried over anhydrous sodium sulfate, and filtered. The filtrate was concentrated, and the resulting residue was purified by preparative thin-layer chromatography to obtain 13c (77 mg). $^1$H NMR (400 MHz, $CDCl_3$) δ 8.28 - 8.23 (m, 2H),

8.20 - 8.13 (m, 1H), 7.32 - 7.28 (m, 2H), 7.18 (d, *J* = 8.8 Hz, 1H), 7.00 (t, *J* = 8.0 Hz, 1H), 6.78 (d, *J* = 7.2 Hz, 1H), 5.22 - 5.17 (m, 1H), 4.50 (s, 2H), 3.98 - 3.80 (m, 3H), 3.78 (s, 3H), 3.59 - 3.38 (m, 2H), 3.00 - 2.66 (m, 3H), 2.29 - 2.18 (m, 1H), 2.04 - 1.92 (m, 2H), 1.83 - 1.71 (m, 2H).

Step 4: 13c (57 mg, 101.50 μmol) and A (61 mg, 121.81 μmol) were dissolved in*N, N*-dimethylformamide (2 mL) at room temperature under an argon atmosphere, and potassium carbonate (42 mg, 304.51 μmol) was added with stirring. The reaction mixture was then reacted overnight at 80°C under an argon atmosphere. LC-MS showed that a small amount of the starting material remained, and the main product was 13. The reaction mixture was cooled to room temperature, then saturated aqueous sodium bicarbonate solution (30 mL) was added, and the resulting mixture was extracted with ethyl acetate (3 × 10 mL). The combined organic phases were washed with saturated brine (60 mL), dried over anhydrous sodium sulfate, and filtered. The filtrate was concentrated, and the resulting residue was separated by preparative thin-layer chromatography to obtain 13 (26.78 mg). MS (ESI) m/z: 921.4 [M+H$^+$]; $^1$H NMR (400 MHz, DMSO-$d_6$) δ 11.11 (s, 1H), 9.50 (d, *J* = 5.2 Hz, 1H), 8.78 (d, *J* = 7.6 Hz, 1H), 8.41 - 8.40 (m, 1H), 8.27 - 8.25 (m, 1H), 7.29 - 7.01 (m, 4H), 6.88 - 6.44 (m, 1H), 5.43 - 5.37 (m, 1H), 5.28 - 5.07 (m, 1H), 5.02 - 4.92 (m, 1H), 4.80 - 4.75 (m, 1H), 4.50 (s, 2H), 3.83 - 3.69 (m, 3H), 3.64 - 3.57 (m, 4H), 3.49 - 3.36 (m, 3H), 3.18 - 3.09 (m, 2H), 3.08 - 3.01 (m, 2H), 2.93 - 2.84 (m, 3H), 2.76 - 2.61 (m, 2H), 2.42 - 2.32 (m, 2H), 2.25 - 2.15 (m, 2H), 2.05 - 1.87 (m, 5H), 1.69 - 1.53 (m, 4H), 1.48 - 1.39 (m, 2H).

Example 14

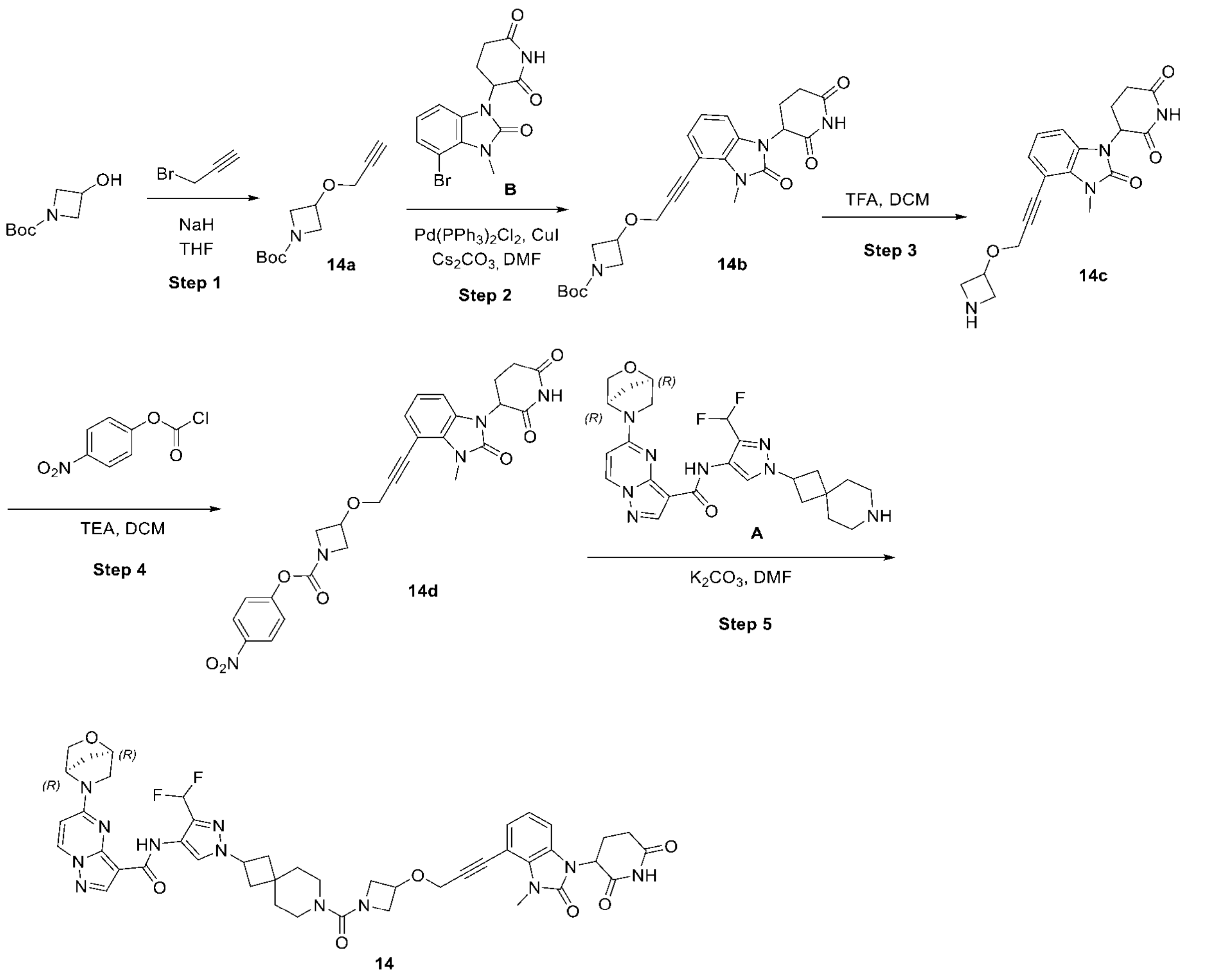


Step 1: Sodium hydride (60%, 2.80 g, 69.97 mmol) was added to a stirred solution of *N*-Boc-3-hydroxyazetidine (10.1 g, 58.31 mmol) in tetrahydrofuran (100 mL) in an ice-water bath. The reaction mixture was stirred for 0.5 hours in an ice-water bath, then returned to room temperature and stirred for another 0.5 hours. 3-Bromopropyne (8.32 g, 69.97 mmol, 6.03 mL) was added to the reaction mixture. The reaction mixture was then stirred overnight at 60°C under an argon atmosphere. LC-MS showed that the reaction was complete. The reaction mixture was cooled, and then quenched by the addition of saturated aqueous ammonium chloride solution (about 100 mL) in an ice-water bath with stirring. The resulting

mixture was diluted with water (about 100 mL) and then extracted with ethyl acetate (2 × 100 mL). The combined organic phases were washed with saturated brine, dried over anhydrous sodium sulfate, and filtered. The filtrate was concentrated under vacuum, and the resulting crude residue was separated by silica gel column chromatography to obtain the desired product 14a (11.60 g). $^1H$ NMR (400 MHz, DMSO-$d_6$) δ 4.39 - 4.32 (m, 1H), 4.14 (d, *J* = 2.4 Hz, 2H), 4.04 - 3.99 (m, 2H), 3.71 - 3.67 (m, 2H), 3.46 (t, *J* = 2.4 Hz, 1H), 1.37 (s, 9H).

The subsequent reactions were carried out with reference to Example 13 to obtain the desired product 14 (25.4 mg). MS (ESI) m/z: 893.2 [M+H$^+$]; $^1H$ NMR (400 MHz, $CDCl_3$) δ 9.63 (s, 1H), 8.83 (br, 1H), 8.49 (d, *J* = 10.0 Hz, 2H), 8.32 (d, *J* = 7.6 Hz, 1H), 7.17 (dd, *J* = 8.0, 0.8 Hz, 1H), 7.00 (t, *J* = 7.8 Hz, 1H), 6.92 - 6.64 (m, 2H), 6.12 (d, *J* = 7.6 Hz, 1H), 5.45 (s, 1H), 5.22 (dd, *J* = 12.8, 5.2 Hz, 1H), 4.83 - 4.71 (m, 2H), 4.48 - 4.39 (m, 3H), 4.20 - 4.16 (m, 2H), 4.05 - 3.95 (m, 4H), 3.78 (s, 3H), 3.59 (d, *J* = 9.2 Hz, 1H), 3.49 (d, *J* = 9.2 Hz, 1H), 3.33 - 3.24 (m, 2H), 3.22 - 3.12 (m, 2H), 2.99 - 2.92 (m, 1H), 2.89 - 2.83 (m, 1H), 2.81 - 2.67 (m, 1H), 2.42 - 2.36 (m, 2H), 2.31 - 2.20 (m, 3H), 2.12 - 2.09 (m, 1H), 2.04 - 1.97 (m, 1H), 1.57 - 1.51 (m, 4H).

Example 15

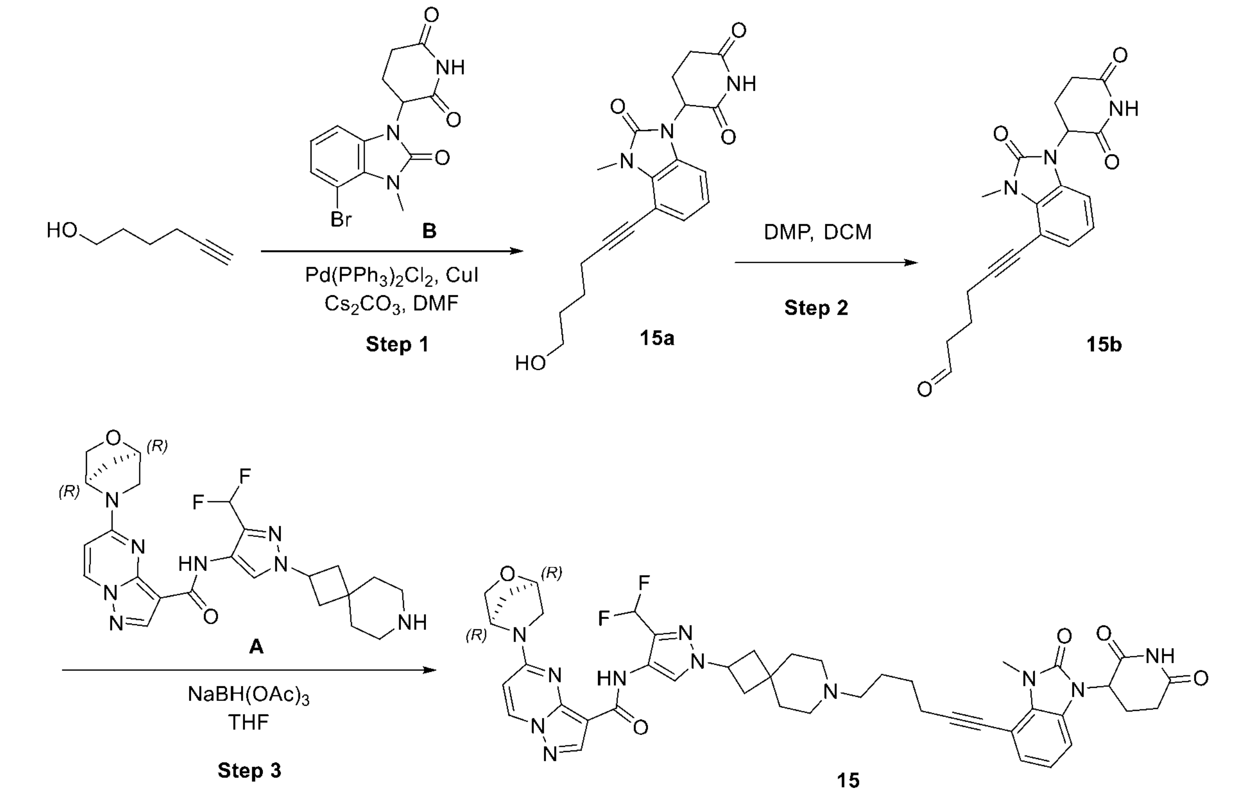


The desired product 15 (9.02 mg) was obtained with reference to Example 2. MS (ESI) m/z: 836.5 [M+H$^+$]; $^1H$ NMR (400 MHz, DMSO-$d_6$) δ 11.11 (s, 1H), 9.50 (d, *J* = 5.2 Hz, 1H), 8.79 (d, *J* = 7.6 Hz, 1H), 8.42 - 8.40 (m, 1H), 8.26 (d, *J* = 5.6 Hz, 1H), 7.28 - 6.97 (m, 4H), 6.88 - 6.44 (m, 1H), 5.42 - 5.36 (m, 1H), 5.28 - 5.07 (m, 1H), 5.01 - 4.90 (m, 1H), 4.80 - 4.74 (m, 1H), 3.83 - 3.72 (m, 2H), 3.65 (s, 3H), 3.62 - 3.59 (m, 1H), 3.45 - 3.39 (m, 2H), 2.94 - 2.85 (m, 1H), 2.75 - 2.60 (m, 3H), 2.39 - 2.13 (m, 9H), 2.05 - 1.90 (m, 4H), 1.84 - 1.44 (m, 8H).

Example 16

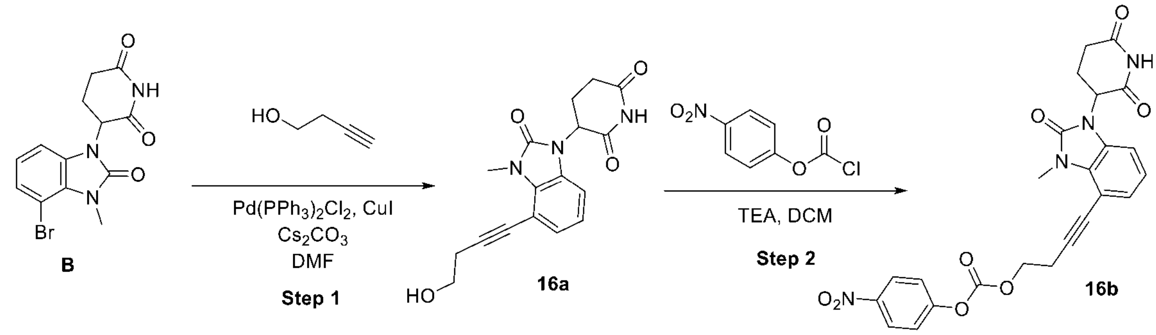


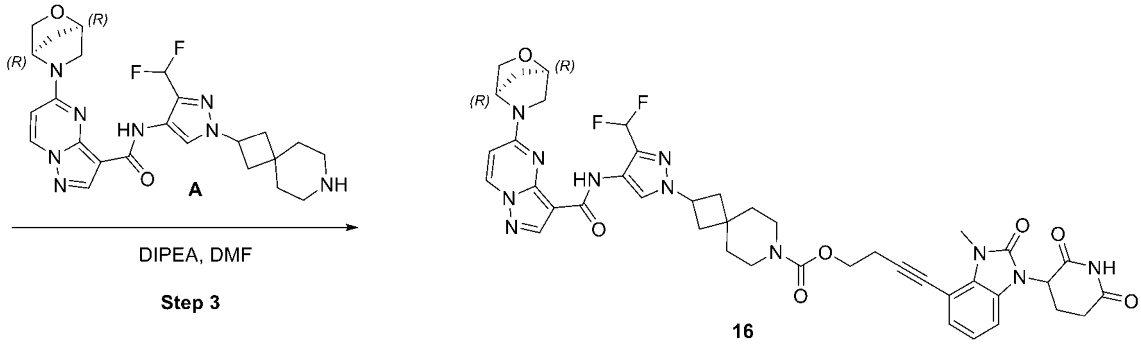


The desired product 16 (134.24 mg) was obtained with reference to Example 13. MS (ESI) m/z: 852.3 [M+H$^+$]; $^1$H NMR (400 MHz, DMSO-$d_6$) δ 11.11 (s, 1H), 9.49 (d, *J* = 4.8 Hz, 1H), 8.78 (d, *J* = 8.0 Hz, 1H), 8.40 (d, *J* = 4.4 Hz, 1H), 8.26 (d, *J* = 5.6 Hz, 1H), 7.28 - 6.98 (m, 4H), 6.88 - 6.44 (m, 1H), 5.42 - 5.37 (m, 1H), 5.28 - 5.07 (m, 1H), 4.99 - 4.90 (m, 1H), 4.80 - 4.75 (m, 1H), 4.23 - 4.19 (m, 2H), 3.83 - 3.73 (m, 2H), 3.64 (s, 3H), 3.62 - 3.57 (m, 1H), 3.46 - 3.30 (m, 5H), 2.92 - 2.82 (m, 3H), 2.75 - 2.59 (m, 2H), 2.35 - 2.30 (m, 2H), 2.20 - 2.15 (m, 2H), 2.07 - 1.92 (m, 3H), 1.60 - 1.57 (m, 2H), 1.53 - 1.51 (m, 2H).

Example 17

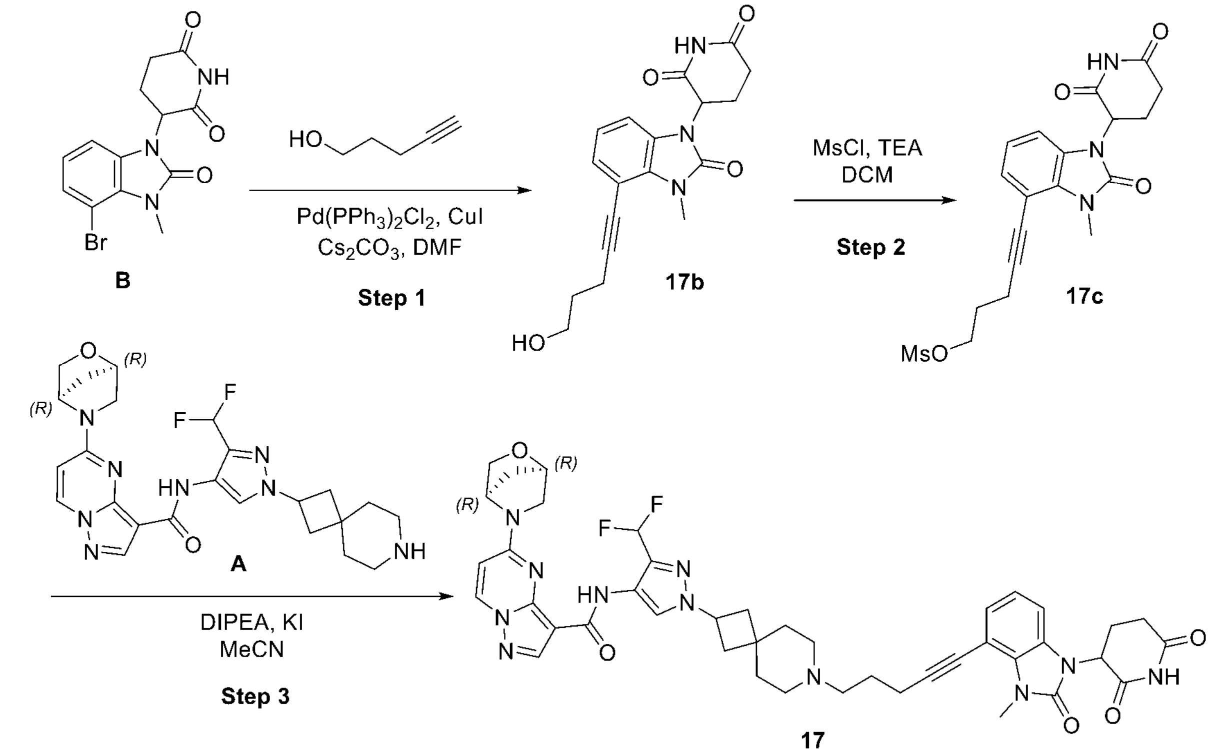


The desired product 17 (72.51 mg) was obtained with reference to Example 1. MS (ESI) m/z: 822.4 [M+H$^+$]; $^1$H NMR (400 MHz, DMSO-$d_6$) δ 11.11 (s, 1H), 9.50 (d, *J* = 4.8 Hz, 1H), 8.78 (d, *J* = 7.6 Hz, 1H), 8.42 (d, *J* = 3.6 Hz, 1H), 8.26 (d, *J* = 5.6 Hz, 1H), 7.28 - 6.98 (m, 4H), 6.88 - 6.44 (m, 1H), 5.42 - 5.37 (m, 1H), 5.28 - 5.07 (m, 1H), 5.01 - 4.90 (m, 1H), 4.80 - 4.75 (m, 1H), 3.84 - 3.72 (m, 2H), 3.65 - 3.59 (m, 5H), 3.50 - 3.40 (m, 1H), 2.95 - 2.85 (m, 1H), 2.76 - 2.54 (m, 7H), 2.44 - 2.13 (m, 6H), 2.07 - 1.52 (m, 9H).

Example 18

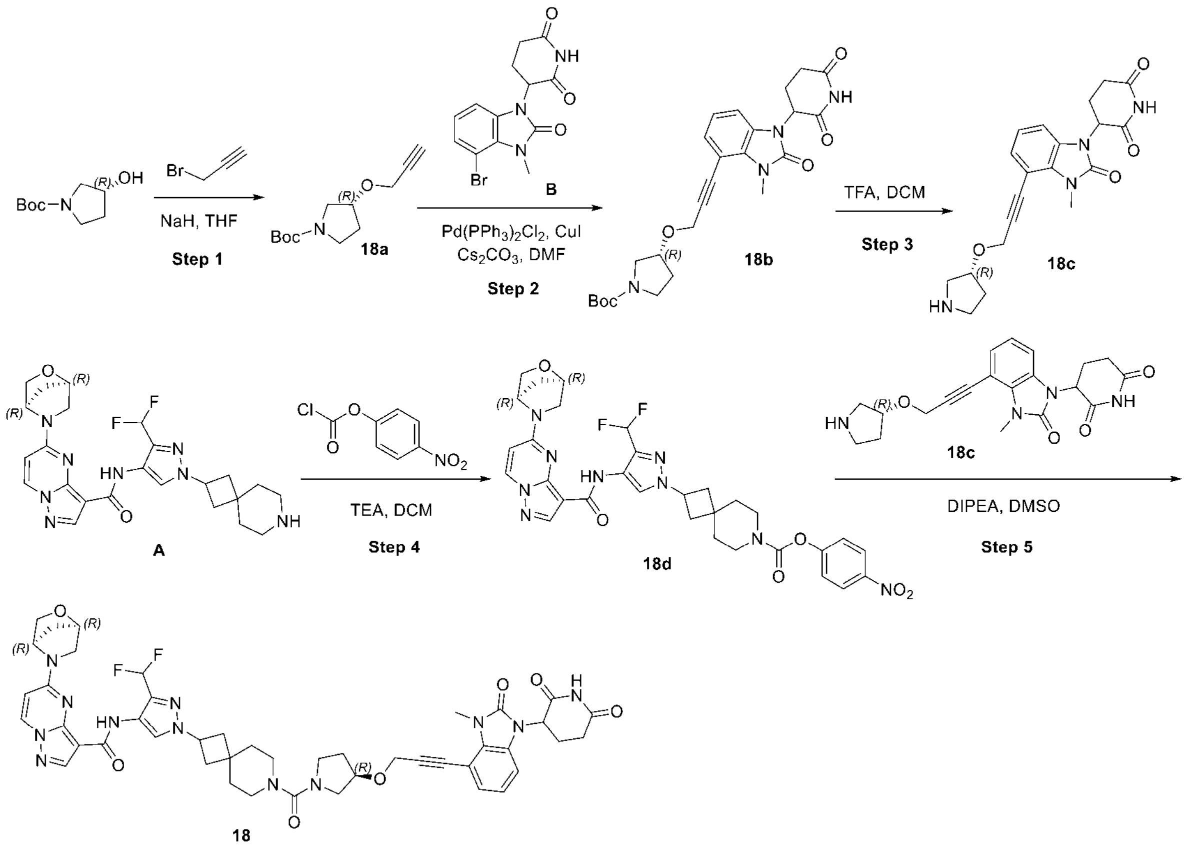

18

The desired intermediate 18c (5.8 g) was obtained with reference to Example 14. $^1$H NMR (400 MHz, $CDCl_3$) δ 4.28 - 4.20 (m, 1H), 4.18 - 4.11 (m, 2H), 3.51 - 3.35 (m, 4H), 2.44 (t, *J* = 2.4 Hz 1H), 2.10 - 1.89 (m, 2H), 1.46 (s, 9H).

Step 4: A (100 mg, 200.59 μmol) and *p*-nitrophenyl chloroformate (61 mg, 300.88 μmol) were dissolved in dichloromethane (3 mL) in an ice-water bath with stirring, and triethylamine (61 mg, 601.76 μmol) was added in an ice-water bath with stirring. The reaction mixture was then warmed to room temperature and stirred for 1 hour under an argon atmosphere. LC-MS showed the disappearance of the starting material. Saturated aqueous sodium bicarbonate solution (30 mL) was added to the reaction mixture, and then the mixture was extracted with dichloromethane (3 × 10 mL). The combined organic phases were washed with saturated brine (30 mL), dried over anhydrous sodium sulfate, and filtered. The filtrate was concentrated, and the resulting residue was separated by preparative thin-layer chromatography to obtain 18d (85 mg). $^1$H NMR (400 MHz, $CDCl_3$) δ 9.62 (s, 1H), 8.49 - 8.45 (m, 1H), 8.43 - 8.41 (m, 1H), 8.34 - 8.31 (m, 1H), 8.28 - 8.23 (m, 2H), 7.31 - 7.28 (m, 2H), 6.79 (t, *J* = 54.0 Hz, 1H), 6.13 (d, *J* = *8.0* Hz, 1H), 5.46 (s, 1H), 4.83 - 4.77 (m, 2H), 4.01 - 3.96 (m, 2H), 3.69 - 3.48 (m, 6H), 2.54 - 2.37 (m, 4H), 2.13 - 2.09 (m, 1H), 2.05 - 1.97 (m, 1H), 1.82 - 1.77 (m, 4H).

Step 5: 18d (70 mg, 105.48 μmol) and 18c (61 mg, 158.22 μmol) were dissolved in dimethyl sulfoxide (1 mL) at room temperature under an argon atmosphere, and *N,N*-diisopropylethylamine (136 mg, 1.05 mmol) was added with stirring. The reaction mixture was then stirred overnight at 100°C under an argon atmosphere. LC-MS showed that the main product was 18. The reaction mixture was cooled to room temperature, then saturated aqueous sodium bicarbonate solution (30 mL) was added to the reaction mixture, and then the resulting mixture was extracted with ethyl acetate (3 × 10 mL). The combined organic phases were washed with saturated brine (60 mL), dried over anhydrous sodium sulfate, and filtered. The filtrate was concentrated under reduced pressure, and the resulting residue was purified by preparative thin-layer chromatography to obtain 18 (5.11 mg). MS (ESI) m/z: 907.4 [M+H$^+$]; $^1$H NMR (400 MHz, DMSO-*$d_6$*) δ 11.12 (s, 1H), 9.50 (d, *J* = 4.8 Hz, 1H), 8.78 (d, *J* = 7.6 Hz, 1H), 8.41 - 8.40 (m, 1H), 8.26 (d, *J* = 5.6 Hz, 1H), 7.29 - 7.01 (m, 4H), 6.88 - 6.44 (m, 1H), 5.43 - 5.38 (m, 1H), 5.28 - 5.07 (m, 1H), 5.00 - 4.90 (m, 1H), 4.80 - 4.75 (m, 1H), 4.54 - 4.44 (m, 2H), 4.32 - 4.23 (m, 1H), 3.85 - 3.72 (m, 2H), 3.68 - 3.51 (m, 5H), 3.47 - 3.21 (m, 4H), 3.18 - 2.81 (m, 5H), 2.74 - 2.60 (m, 2H), 2.38 - 2.27 (m, 2H), 2.22 - 2.11 (m, 2H), 2.08 - 1.83 (m, 5H), 1.68 - 1.42 (m, 4H).

Example 19

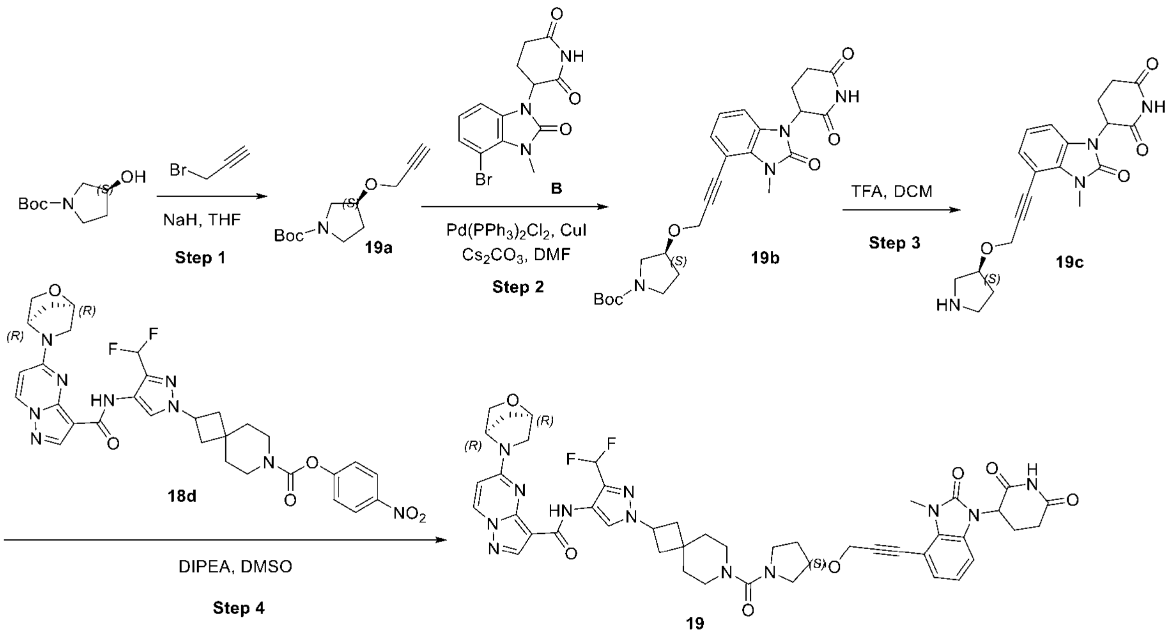


The desired product 19 (12.02 mg) was obtained with reference to Example 18. MS (ESI) m/z: 907.5 [M+H+]. $^1H$ NMR (400 MHz, $CDCl_3$) δ 9.73 - 9.20 (m, 2H), 8.64 - 8.52 (m, 2H), 8.32 (d, *J* = 6.8 Hz, 1H), 7.19 - 7.16 (m, 1H), 7.01 - 6.96 (m, 1H), 6.91 - 6.63 (m, 2H), 6.12 (d, *J* = 7.6 Hz, 1H), 5.45 (s, 1H), 5.32 - 5.22 (m, 1H), 4.79 (s, 1H), 4.69 (t, *J* = 8.0 Hz, 1H), 4.55 (t, *J* = 16.0 Hz, 1H), 4.40 - 4.33 (m, 1H), 4.26 - 4.21 (m, 1H), 3.97 (s, 2H), 3.78 (d, *J* = 1.2 Hz, 3H), 3.66 - 3.40 (m, 6H), 3.22 - 2.69 (m, 8H), 2.34 - 2.2 (m, 4H), 2.13 - 1.97 (m, 5H), 1.53 - 1.47 (m, 3H).

Example 20

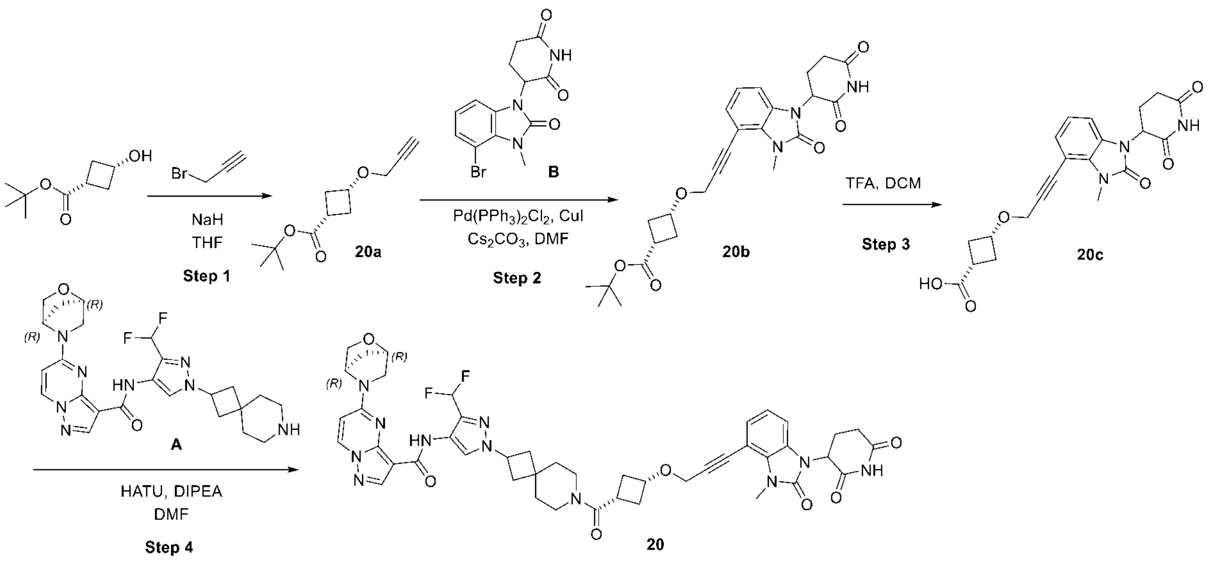


The desired product 20 (1.96 mg) was obtained with reference to Example 12. MS (ESI) m/z: 892.6 [M+H+]. $^1H$ NMR (400 MHz, DMSO-$d_6$) δ 11.12 (s, 1H), 9.51 (d, *J* = 8.8 Hz, 1H), 8.75 (d, *J* = 15.2 Hz, 1H), 8.41 (d, *J* = 4.4 Hz, 1H), 8.26 (d, *J* = 5.2 Hz, 1H), 7.00 - 7.29 (m, 4H), 6.40 - 6.90 (m, 1H), 5.35 - 5.45 (m, 1H), 5.28 (s, 1H), 5.05 (s, 1H), 4.90 - 5.00 (m,1H), 4.75 (d, *J*= 13.6 Hz, 2H), 4.38 (s, 2H), 4.10 (d, *J* = 8.4 Hz, 3H), 3.91 (s, 2H), 3.80 (s, 2H), 2.85 - 2.95 (m, 4H), 2.65 - 2.72 (m, 4H), 2.32 - 2.40 (m, 2H), 2.15 - 2.29 (m, 3H), 2.00 - 2.10 (m, 3H), 1.99 - 1.95 (m, 2H), 1.19 - 1.63 (m, 4H).

Example 21

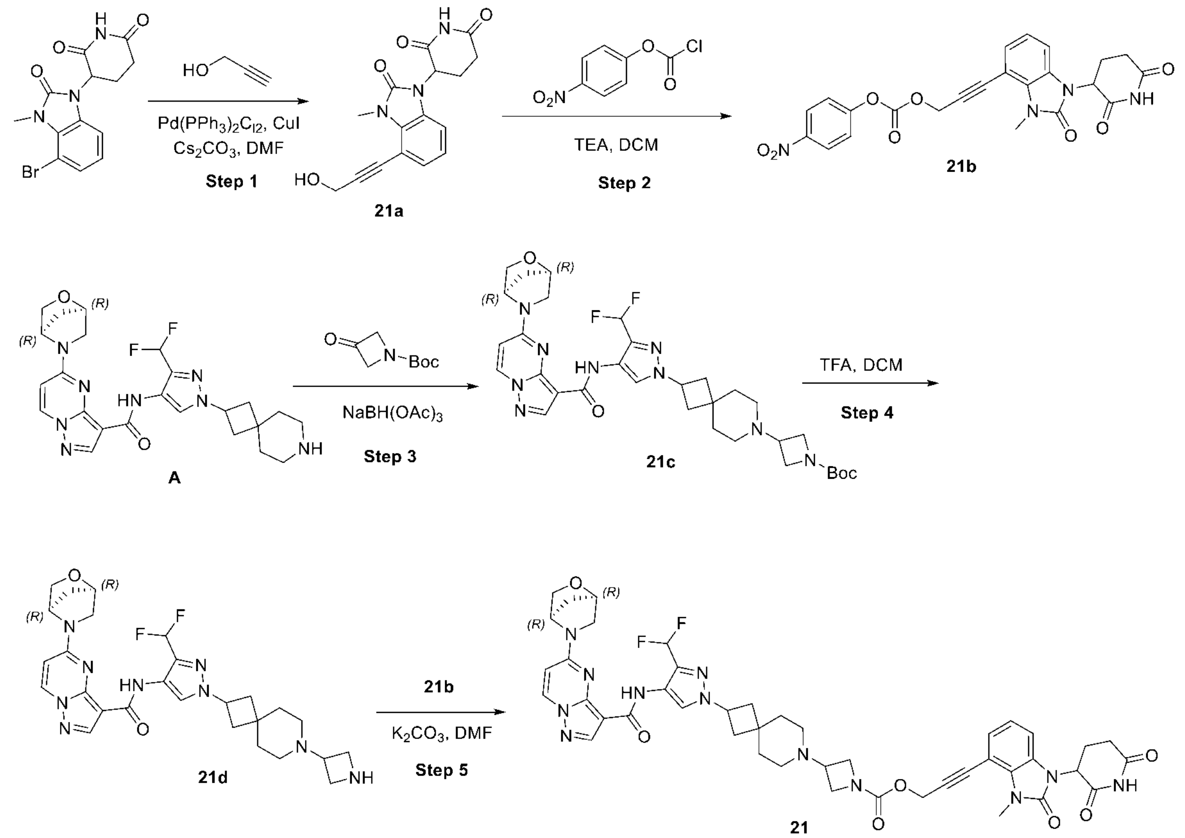


Step 1: B (300 mg, 891.84 μmol) was dissolved in *N,N*-dimethylformamide (3 mL) at room temperature under an argon atmosphere, and propargyl alcohol (100 mg, 1.78 mmol), cesium carbonate (581 mg, 1.78 mmol), copper(I) iodide (34 mg, 1.78 μmol), and bis(triphenylphosphine)palladium(II) dichloride (62 mg, 89.18 μmol) were added sequentially. The reaction mixture was stirred at 80°C under an argon atmosphere for 2 hours. LC-MS showed that a small amount of the starting material B remained, and the main product was 21a. The reaction mixture was cooled to room temperature, then added with water (50 mL), and extracted with ethyl acetate (30 mL × 3). The combined organic phases were washed with saturated brine (25 mL), dried over anhydrous sodium sulfate, and filtered. The filtrate was concentrated, and the resulting residue was separated by silica gel column chromatography to obtain 21a (81 mg, yield: 29%) as a yellow solid. $^1$H NMR (400 MHz, DMSO-$d_6$) δ 11.11 (s, 1H), 7.15 (d, *J* = 7.6 Hz, 1H), 7.08 (d, *J* = 8.0 Hz, 1H), 7.02 (t, *J* = 7.6 Hz, 1H), 5.42 - 5.36 (m, 2H), 4.36 (d, *J* = 6.0 Hz, 2H), 3.65 (s, 3H), 2.97 - 2.82 (m, 1H), 2.77 - 2.57 (m, 2H), 2.04 - 1.99 (m, 1H).

Step 2: Triethylamine (0.11 mL, 0.78 mmol) was added to a solution of 21a (80 mg, 0.26 mmol) and 4-nitrophenyl chloroformate (105 mg, 0.52 mmol) in dichloromethane (2.0 mL) in an ice-water bath with stirring. The reaction mixture was returned to room temperature after 10 minutes and stirred for 2 hours under an argon atmosphere. LC-MS showed that the reaction was complete. Saturated aqueous sodium bicarbonate solution (15 mL) was added to the reaction mixture, and then the mixture was extracted with ethyl acetate (3 × 10 mL). The combined organic phases were washed with saturated brine, dried over anhydrous sodium sulfate, and filtered. The filtrate was concentrated, and the resulting residue was separated by silica gel column chromatography and preparative thin-layer chromatography to obtain 21b (38 mg). MS (ESI) m/z: 479.1 [M+H$^+$]. $^1$H NMR (400 MHz, $CDCl_3$) δ 8.32 - 8.29 (m, 2H), 8.05 (s, 1H), 7.43 - 7.40 (m, 2H), 7.22 - 7.19 (m, 1H), 7.04 - 6.99 (m, 1H), 6.82 - 6.79 (m, 1H), 5.22 - 5.17 (m, 1H), 5.14 (s, 2H), 3.78 (s, 3H), 2.99 - 2.93 (m, 1H), 2.87 - 2.70 (m, 2H), 2.26 - 2.22 (m, 1H).

Step 3: A (300 mg, 601.76 μmol) and 1-Boc-3-azetidinone (515 mg, 3.01 mmol) were dissolved in tetrahydrofuran (5 mL) in an ice-water bath, and sodium triacetoxyborohydride (510 mg, 2.41 mmol) was added in an ice-water bath with stirring. The reaction mixture was then stirred at room temperature for 2 hours. LC-MS showed complete consumption of the starting material and formation of 21c. Saturated aqueous sodium bicarbonate solution (20 mL) was added to the reaction mixture, and then the mixture was extracted with ethyl acetate (3 × 20 mL). The combined organic phases were washed with saturated brine (3 × 30 mL), dried over anhydrous sodium sulfate, and filtered. The filtrate was

concentrated under reduced pressure, and the resulting residue was purified by preparative thin-layer chromatography to obtain 21c (240 mg). MS (ESI) m/z: 654.5 [M+H$^+$].

Step 4: 21c (240 mg, 367.12 μmol) was dissolved in dichloromethane (4 mL) in an ice-water bath, and trifluoroacetic acid (418 mg, 3.67 mmol) was added with stirring. The reaction mixture was slowly returned to room temperature and stirred for 1 hour. LC-MS showed that the starting material was completely consumed and 21d was the main product. The reaction mixture was concentrated under reduced pressure to obtain 21d (80 mg, crude trifluoroacetate salt). $^1$H NMR (400 MHz, DMSO-*d$_6$*) δ 9.51 (d, *J* = 5.2 Hz, 1H), 9.30 - 9.08 (m, 3H), 8.79 (d, *J* = 8.4 Hz, 1H), 8.44 (d, *J* = 4.4 Hz, 1H), 8.26 (d, *J* = 5.2 Hz, 1H), 7.27 - 7.00 (m, 1H), 6.88 - 6.45 (m, 1H), 5.27 - 5.08 (m, 1H), 5.03 - 4.95 (m, 1H), 4.79 - 4.74 (m, 1H), 4.40 - 4.26 (m, 3H), 4.20 - 4.08 (m, 2H), 3.87 - 3.44 (m, 5H), 2.49 - 2.44 (m, 1H), 2.36 - 2.21 (m, 3H), 2.08 - 1.64 (m, 7H).

Step 5: 21d (60 mg, 108.69 μmol), 21b (40 mg, 83.61 μmol), and potassium carbonate (35 mg, 250.83 μmol) were dispersed in *N*,*N*-dimethylformamide (2 mL) at room temperature. The reaction mixture was then heated to 60°C and stirred for 3 hours. LC-MS showed that a small amount of the starting material remained and a product was produced. The reaction mixture was cooled to room temperature, then saturated aqueous sodium bicarbonate solution (10 mL) was added, and then the resulting mixture was extracted with ethyl acetate (3 × 10 mL). The combined organic phases were washed with saturated brine (6 × 10 mL), dried over anhydrous sodium sulfate, and filtered. The filtrate was concentrated, and the resulting residue was separated by preparative thin-layer chromatography to obtain 21 (17.52 mg). MS (ESI) m/z: 893.4 [M+H$^+$]. $^1$H NMR (400 MHz, DMSO-*d$_6$*) δ 11.12 (s, 1H), 9.49 (d, *J* = 5.2 Hz, 1H), 8.78 (d, *J* = 7.6 Hz, 1H), 8.39 (d, *J* = 4.0 Hz, 1H), 8.25 (d, *J* = 5.6 Hz, 1H), 7.28 - 7.00 (m, 4H), 6.87 - 6.44 (m, 1H), 5.42 - 5.38 (m, 1H), 5.27 - 5.07 (m, 1H), 4.98 - 4.90 (m, 3H), 4.79 - 4.75 (m, 1H), 4.02 - 3.88 (m, 2H), 3.82 - 3.70 (m, 4H), 3.64 - 3.43 (m, 5H), 3.09 - 3.03 (m, 1H), 2.93 - 2.83 (m, 1H), 2.76 - 2.58 (m, 2H), 2.34 - 2.14 (m, 8H), 2.08 - 1.92 (m, 3H), 1.69 - 1.56 (m, 4H).

Example 22

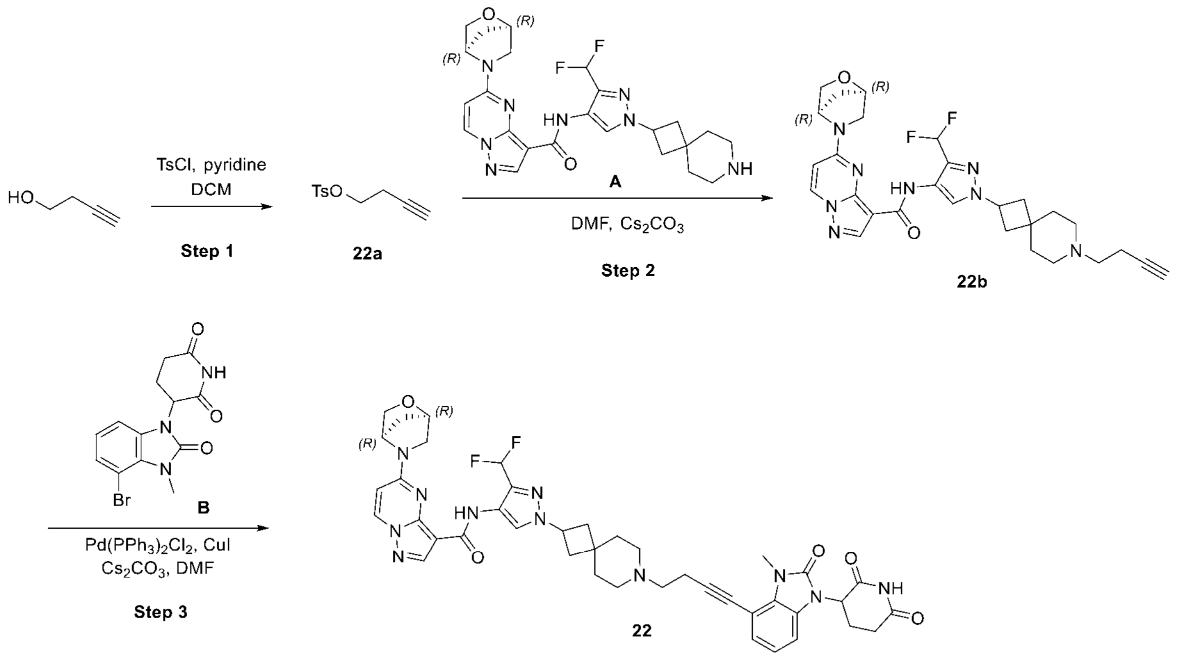


Step 1: 3-Butyn-1-ol (0.5 g, 7.13 mmol) was dissolved in dichloromethane (10 mL) at room temperature, and *p*-toluenesulfonyl chloride (1.63 g, 8.56 mmol) and pyridine (850 mg, 10.70 mmol) were added sequentially in an ice-water bath with stirring. The reaction mixture was then stirred at room temperature for 16 hours. TLC showed that the starting material was completely reacted. Saturated aqueous ammonium chloride solution (20 mL) was added to the reaction mixture, and then the mixture was extracted with ethyl acetate (2 × 50 mL). The combined organic phases were washed with saturated brine (3 × 50 mL), dried over anhydrous sodium sulfate, and filtered. The filtrate was concentrated, and the resulting crude residue was separated by silica gel column chromatography to obtain the desired product 22a (860 mg). $^1$H NMR (400 MHz, $CDCl_3$) δ 7.80 (d, *J* = 7.6 Hz, 2H), 7.36 (d, *J* = 8.0 Hz, 2H), 4.10 (t, *J* = 7.0 Hz, 2H), 2.57 - 2.52 (m, 2H), 2.45 (s, 3H), 1.99 (t, *J* = 2.8 Hz, 1H).

Step 2: 22a (201.8 mg, 0.90 mmL) was dissolved in N,N-dimethylformamide (4 mL) at room temperature, and A

(300 mg, 0.60 mmol) and cesium carbonate (586.5 mg, 1.80 mmol) were added with stirring. The reaction mixture was then stirred overnight at 60°C. LC-MS showed that the starting material was completely reacted. Water (30 mL) was added to the cooled reaction mixture, and then the mixture was extracted with ethyl acetate (3 × 30 mL). The combined organic phases were washed with saturated aqueous sodium bicarbonate solution (2 × 30 mL), washed with saturated brine (2 × 30 mL), dried over anhydrous sodium sulfate, and filtered. The filtrate was concentrated, and the resulting crude residue was separated by silica gel column chromatography to obtain the desired product 22b (123 mg). MS (ESI) m/z: 551.4 [M+H$^+$]; $^1$H NMR (400 MHz, $CDCl_3$) δ 9.61 (s, 1H), 8.43 (t, *J* = 8.4 Hz, 2H), 8.32 (d, *J* = 7.6 Hz, 1H), 6.78 (t, *J* = 54.0 Hz, 1H), 6.13 (d, *J* = 7.6 Hz, 1H), 5.46 (s, 1H), 4.81 (d, *J* = 10.4 Hz, 1H), 4.75 - 4.67 (m, 1H), 4.02 - 3.94 (m, 2H), 3.62 - 3.44 (m, 2H), 2.61 (t, *J* = 7.6 Hz, 2H), 2.55 - 2.35 (m, 8H), 2.33 - 2.24 (m, 2H), 2.14 - 2.09 (m, 1H), 1.99 (t, *J* = 2.6 Hz, 2H), 1.78 - 1.70 (m, 5H).

Step 3: 22b (123 mg, 0.22 mmol) and B (50 mg, 0.15 mmol) were dissolved in dry N,N-dimethylformamide (2 mL) at room temperature under an argon atmosphere, and then cesium carbonate (122.2 mg, 0.38 mmol), bis(triphenylphosphine)palladium(II) dichloride (16 mg, 0.022 mmol), and copper(I) iodide (9.0 mg, 0.045 mmol) were added sequentially under an argon atmosphere. The reaction was then stirred at 80°C under an argon atmosphere for 5 hours. LC-MS showed the formation of product 22. The cooled reaction mixture was filtered, and the filter cake was washed with ethyl acetate (20 mL). Saturated aqueous sodium bicarbonate solution (10 mL) was added to the filtrate, and then the mixture was extracted with ethyl acetate (2 × 20 mL). The combined organic phases were washed with saturated aqueous sodium chloride solution (5 × 20 mL), dried over anhydrous sodium sulfate, and filtered. The filtrate was concentrated, and the resulting crude residue was separated by preparative thin-layer chromatography to obtain the desired product 22 (6.9 mg). MS (ESI) m/z: 808.6 [M+H$^+$]; $^1$H NMR (400 MHz, $CDCl_3$) δ 9.61 (s, 1H), 8.43 (t, *J* = 10.8 Hz, 2H), 8.32 (d, *J* = 8.0 Hz, 1H), 8.10 (s, 1H), 7.12 (d, *J* = 8.0 Hz, 1H), 7.00 - 6.94 (m, 1H), 6.78 (t, *J* = 54.6 Hz, 1H), 6.71 (d, *J* = 8.0 Hz, 1H), 6.13 (t, *J* = 7.6 Hz. 1H), 5.46 (s, 1H), 5.25 - 5.16 (m, 1H), 4.83 - 4.68 (m, 2H), 4.01 - 3.95 (m, 2H), 3.80 (s, 3H), 3.62 - 3.48 (m, 2H), 2.99 - 2.96 (m, 1H), 2.88 - 2.70 (m, 2H), 2.68 (s, 4H), 2.50 - 2.35 (m, 11H), 1.75 (s, 4H).

Example 23

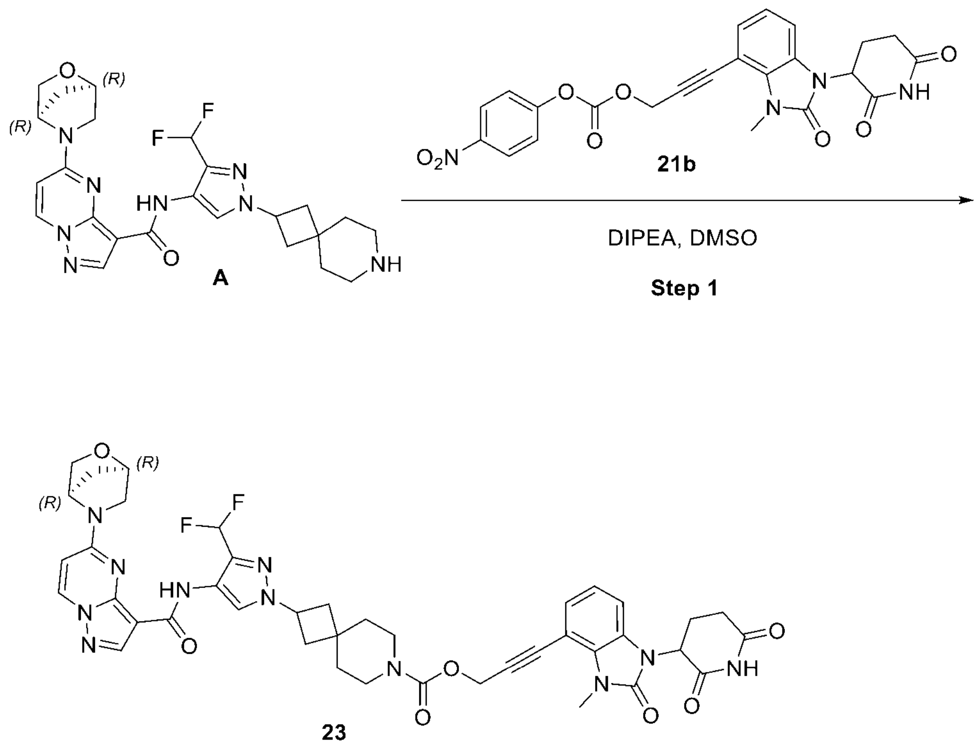


The desired product 23 (20.25 mg) was obtained with reference to Example 16. MS (ESI) m/z: 838.4 [M+H$^+$]; $^1$H NMR (400 MHz, DMSO-$d_6$) δ 11.12 (s, 1H), 9.50 (d, *J* = 5.2 Hz, 1H), 8.78 (d, *J* = 7.6 Hz, 1H), 8.41 (d, *J* = 4.0 Hz, 1H), 8.26 (d, *J* = 5.6 Hz, 1H), 7.29 - 7.01 (m, 4H), 6.88 - 6.44 (m, 1H), 5.43 - 5.38 (m, 1H), 5.28 - 5.07 (m, 1H), 5.01 - 4.94 (m, 3H), 4.77 (d, *J* = 15.2 Hz, 1H), 3.84 - 3.72 (m, 2H), 3.65 - 3.59 (m, 5H), 3.47 - 3.39 (m, 3H), 3.39 - 3.32 (m, 1H), 2.94 - 2.85 (m, 1H), 2.76 - 2.60 (m, 2H), 2.43 - 2.37 (m, 2H), 2.26 - 2.20 (m, 2H), 2.08 - 1.95 (m, 3H), 1.67 - 1.57 (m, 4H).

Example 24

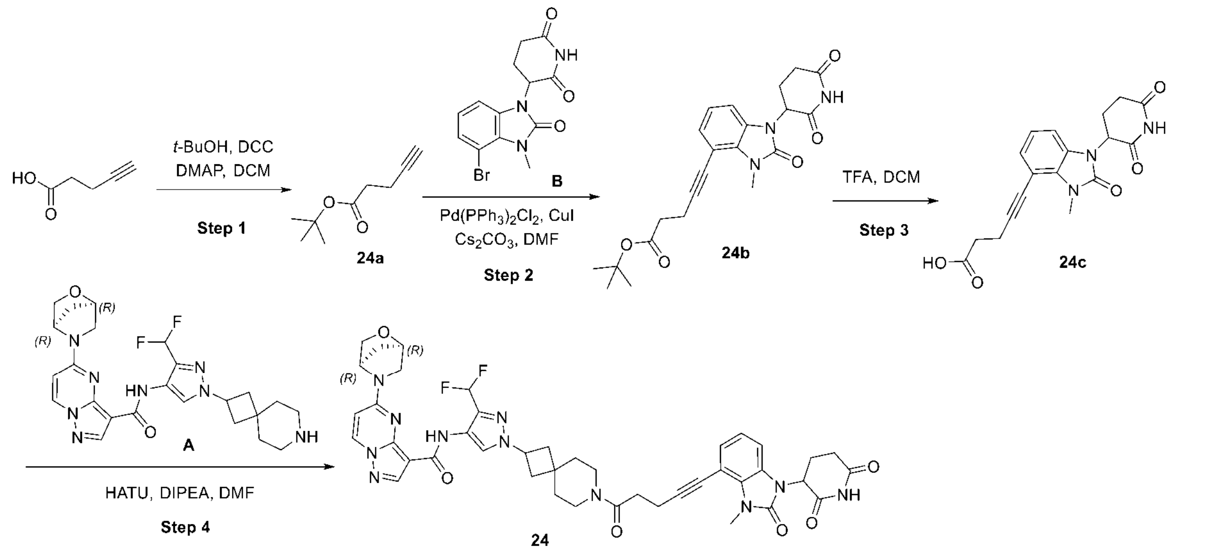


Step 1: 4-Pentynoic acid (2 g, 19.04 mmol) and tert-butanol (21.16 g, 285.46 mmol) were dissolved in dichloromethane (40 mL) at room temperature, and dicyclohexylcarbodiimide (DCC, 6.31 g, 30.59 mmol) and 4-dimethylaminopyridine (DMAP, 0.5 g, 4.08 mmol) were added in an ice-water bath with stirring. The reaction mixture was then stirred overnight at room temperature. TLC showed that the starting material was completely reacted. The reaction mixture was added with dilute hydrochloric acid (0.5 M, 50 mL), and then extracted with ethyl acetate (2 × 100 mL). The combined organic phases were washed with saturated brine (3 × 50 mL), dried over anhydrous sodium sulfate, and filtered. The filtrate was concentrated, and the resulting crude residue was separated by silica gel column chromatography to obtain the desired product 24a (1.27 g). $^1$H NMR (400 MHz, $CDCl_3$) δ 2.45 (s, 4H), 1.97 (s, 1H), 1.46 (s, 9H).

The subsequent procedures were performed with reference to Example 8 to obtain the desired product 24 (17.05 mg). MS (ESI) m/z: 836.6 [M+H$^+$]. $^1$H NMR (400 MHz, $CDCl_3$) δ 11.11 (s, 1H), 9.50 (d, *J* = 5.2 Hz, 1H), 8.79 (d, *J* = 7.6 Hz, 1H), 8.41 (d, *J* = 3.6 Hz, 1H), 8.26 (t, *J* = 5.6 Hz, 1H), 7.29 - 6.95 (m, 4H), 6.89 - 6.44 (m, 1H), 5.43 - 5.37 (m, 1H), 5.28 - 5.07 (m, 1H), 5.03 - 4.95 (m, 1H), 4.80 - 4.75 (m, 1H), 3.84 - 3.72 (m, 2H), 3.65 - 3.59 (m, 4H), 3.50 - 3.30 (m, 4H), 2.92 - 2.84 (m, 1H), 2.74 - 2.59 (m, 7H), 2.42 - 2.30 (m, 2H), 2.25 - 2.19 (m, 2H), 2.09 - 1.90 (m, 3H), 1.70 - 1.50 (m, 4H).

Example 25

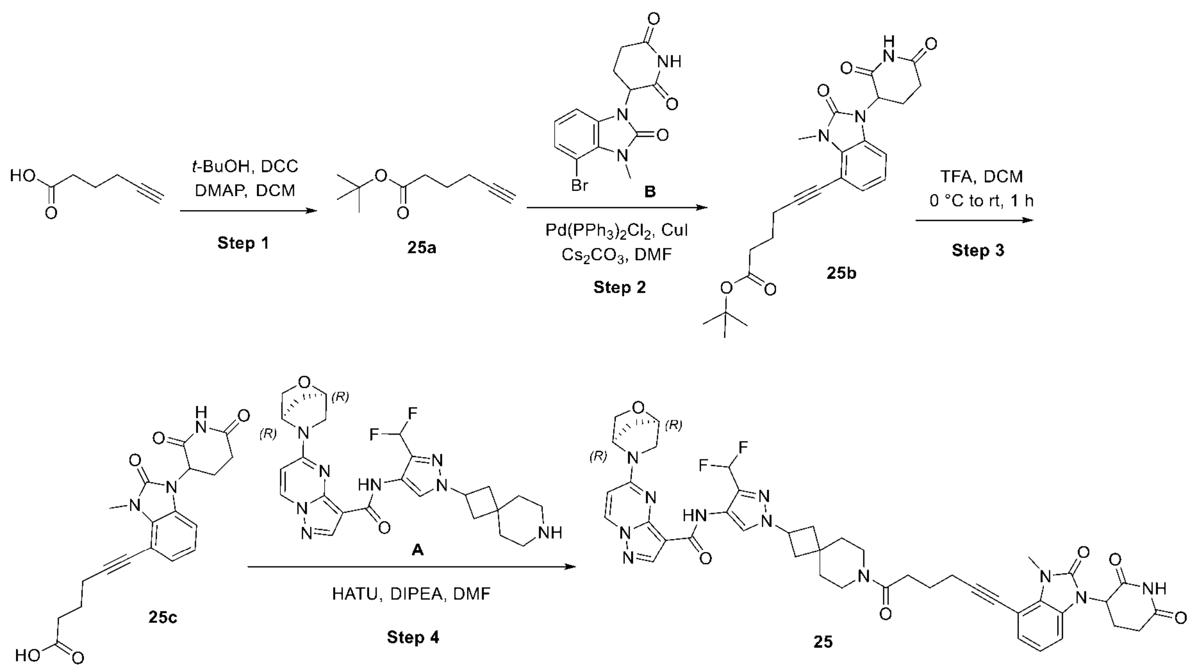


The desired product 25 (2.25 mg) was obtained with reference to Example 24. MS (ESI) m/z: 850.6 [M+H+]; $^1$H NMR (400 MHz, DMSO-$d_6$) δ 11.13 (s, 1H), 9.51 (d, $J$ = 5.2 Hz, 1H), 8.80 (d, $J$ = 7.2 Hz, 1H), 8.42 (d, $J$ = 2.4 Hz, 1H), 8.28 (d, $J$ = 4.8 Hz, 1H), 7.28 - 6.97 (m, 4H), 6.89 - 6.45 (m, 1H), 5.44 - 5.35 (m, 1H), 5.28 - 5.08 (m, 1H), 5.01 - 4.95 (m, 1H), 4.80 - 4.75 (m, 1H), 3.84 - 3.72 (m, 3H), 3.68 - 3.55 (m, 4H), 3.47 - 3.33 (m, 5H), 2.92 - 2.60 (m, 6H), 2.40 - 2.30 (m, 2H), 2.24 - 2.18 (m, 1H), 2.05 - 1.95 (m, 4H), 1.83 - 1.79 (m, 2H), 1.66 - 1.50 (s, 4H).

Example 26

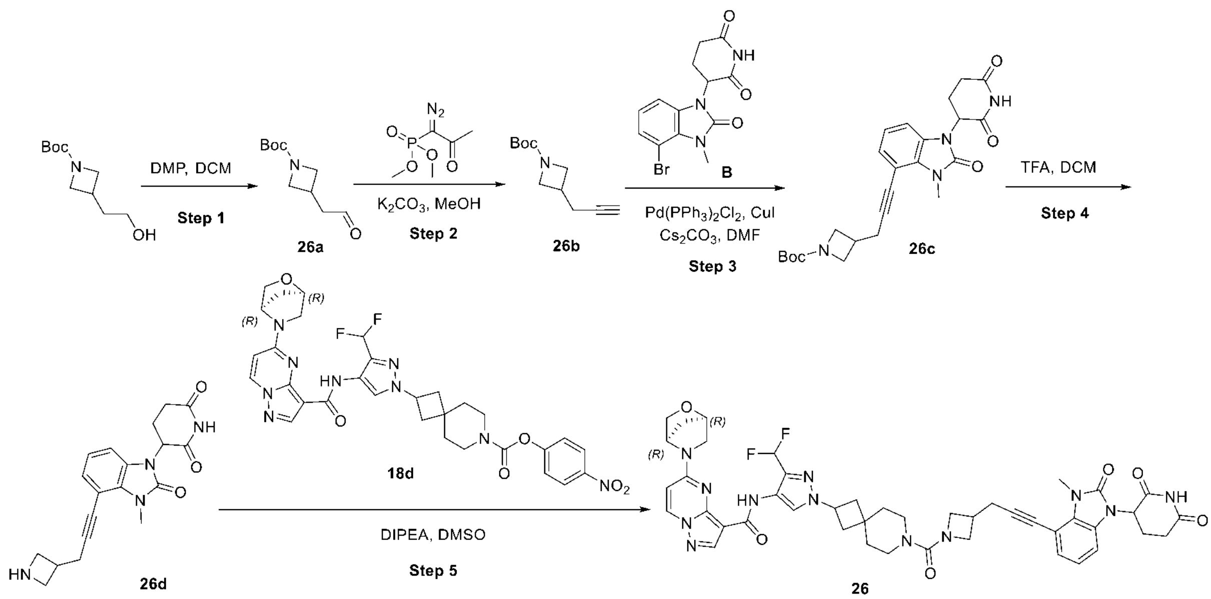


Step 1: Dess-Martin periodinane (4.22 g, 9.94 mmol) was added to a stirred solution of *tert*-butyl 3-(2-hydroxyethyl)azetidine-1-carboxylate (1.0 g, 4.97 mmol) in dichloromethane (20 mL) in an ice-water bath. The reaction mixture was returned to room temperature after 10 minutes and stirred for 1 hour under an argon atmosphere. TLC showed that the reaction was complete. The reaction mixture was filtered through diatomite. The filter cake was washed with ethyl acetate (30 mL), and the combined filtrates were added with water and extracted with ethyl acetate (3 × 30 mL). The combined organic phases were washed with saturated brine, dried over anhydrous sodium sulfate, and filtered. The filtrate was

concentrated, and the resulting residue was separated by silica gel column chromatography to obtain 26a (567 mg). $^1H$ NMR (400 MHz, $CDCl_3$) δ 9.77 (s, 1H), 4.13 (t, *J* = 8.6 Hz, 2H), 3.59 - 3.55 (m, 2H), 2.99 - 2.82 (m, 3H), 1.43 (s, 9H).

Step 2: Dimethyl (1-diazo-2-oxopropyl)phosphonate (682 mg, 3.55 mmol) and potassium carbonate (670 mg, 4.85 mmol) were added to a stirred solution of 26a (630 mg, 3.23 mmol) in methanol (10 mL) at room temperature. The reaction mixture was stirred overnight at room temperature under an argon atmosphere. TLC showed that the starting material was completely reacted. Water ( 30 mL) was added to the reaction mixture, and then the mixture was extracted with ethyl acetate (3 × 30 mL). The combined organic phases were washed with saturated brine, dried over anhydrous sodium sulfate, and filtered. The filtrate was concentrated to obtain 26b (600 mg). $^1H$ NMR (400 MHz, DMSO-$d_6$) δ 3.89 (brs, 2H), 3.55 (brs, 2H), 2.85 (t, *J* = 2.6 Hz, 1H), 2.70 - 2.62 (m, 1H), 2.43 - 2.40 (m, 2H), 1.37 (s, 9H).

The subsequent procedures were performed with reference to Example 18 to obtain the desired product 26 (14.70 mg). MS (ESI) M/Z:877.3 [M+H$^+$]; $^1H$ NMR (400 MHz, DMSO-$d_6$) δ 11.13 (s, 1H), 9.50 (d, *J* = 5.6 Hz, 1H), 8.79 (d, *J* = 8.0 Hz, 1H), 8.41 (d, *J* = 4.0 Hz, 1H), 8.26 (d, *J* = 5.6 Hz, 1H), 7.29 - 6.98 (m, 4H), 6.89 - 6.44 (m, 1H), 5.42 - 5.37 (m, 1H), 5.28 - 5.08 (m, 1H), 5.00 - 4.92 (m, 1H), 4.80 - 4.75 (m, 1H), 4.04 - 3.99 (m, 2H), 3.83 - 3.70 (m, 5H), 3.63 - 3.59 (m, 5H), 3.47 - 3.43 (m, 1H), 3.20 - 3.11 (m, 4H), 2.77 - 2.72 (m, 3H), 2.39 - 2.33 (m, 2H), 2.22 - 2.16 (m, 2H), 2.04 - 1.95 (m, 3H), 1.58 - 1.50 (m, 5H).

Example 27

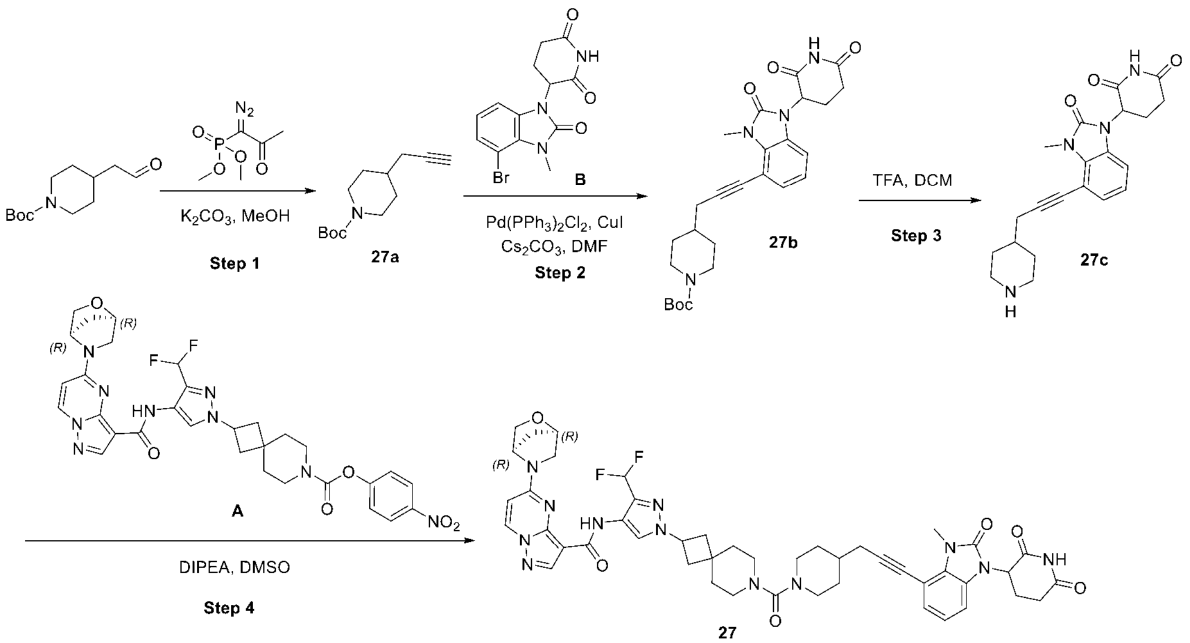


The desired product 27 (6.33 mg) was obtained with reference to Example 26. MS (ESI) m/z: 905.4 [M+H$^+$]. $^1H$ NMR (400 MHz, DMSO-$d_6$) δ 11.13 (s, 1H), 9.51 (d, *J* = 5.2 Hz, 1H), 8.79 (d, *J* = 7.6 Hz, 1H), 8.41 (d, *J* = 4.0 Hz, 1H), 8.26 (d, *J* = 5.6 Hz, 1H), 7.30 - 6.97 (m, 4H), 6.89 - 6.44 (m, 1H), 5.42 - 5.37 (m, 1H), 5.28 - 5.08 (m, 1H), 5.00 - 4.95 (m, 1H), 4.80 - 4.75 (m, 1H), 3.84 - 3.72 (m, 2H), 3.64 - 3.43 (m, 7H), 3.13 - 3.04 (m, 4H), 2.95 - 2.84 (m, 1H), 2.77 - 2.60 (m, 4H), 2.41 - 2.33 (m, 2H), 2.24 - 2.16 (m, 2H), 2.08 - 1.95 (m, 4H), 1.79 - 1.58 (m, 7H), 1.38 - 1.25 (m, 3H).

Example 28

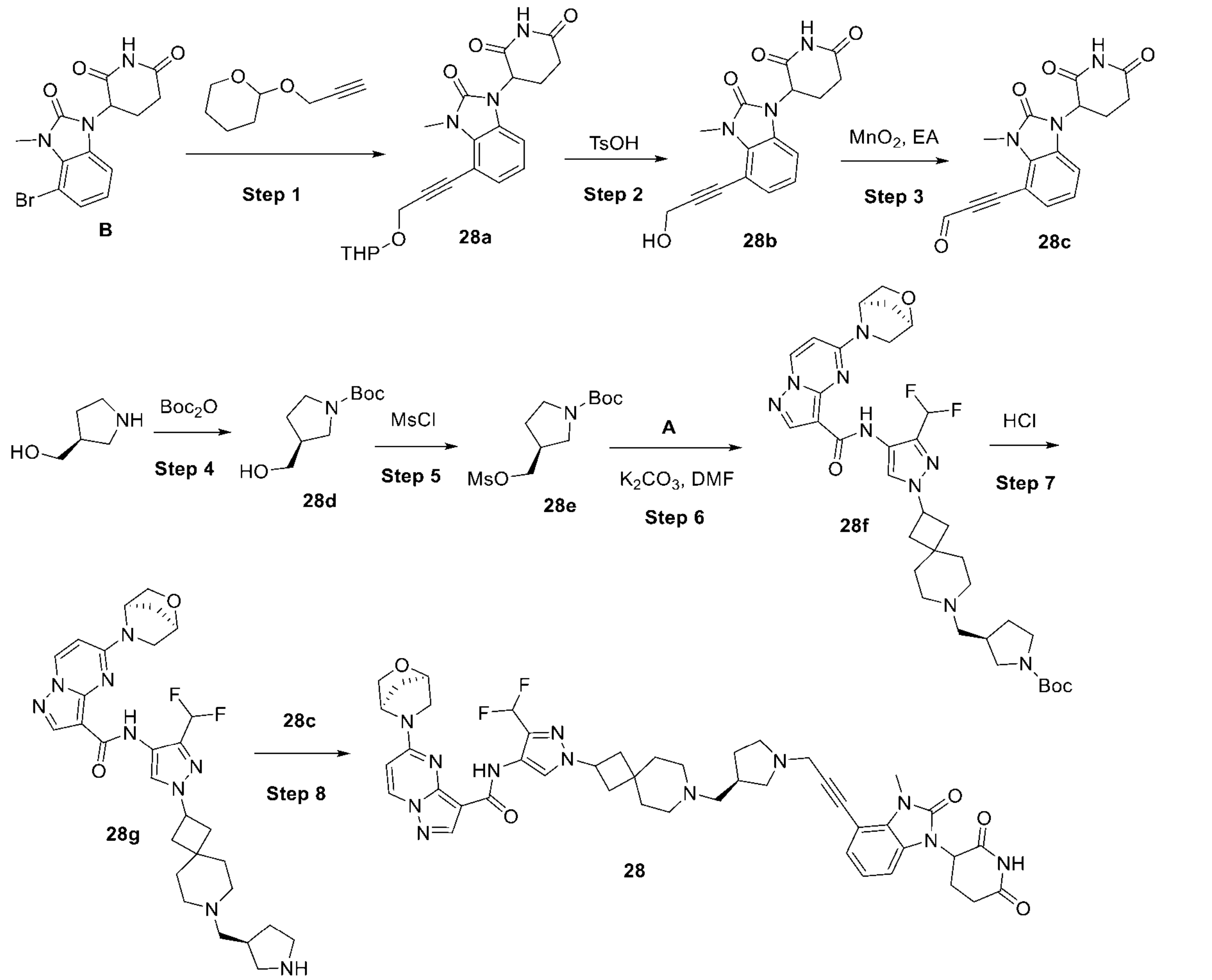


Step 1: 2-(Prop-2-en-1-yloxy)tetrahydro-2H-pyran (312 mg, 2.22 mmol), B (300 mg, 0.9 mmol), cesium carbonate (876.6 mg, 2.7 mmol), copper(I) iodide (618 mg, 0.09 mmol), and bis(triphenylphosphine)palladium(II) dichloride (60 mg, 0.09 mmol) were dissolved in *N,N*-dimethylformamide (6 mL) at room temperature. The reaction system was evacuated and purged with nitrogen several times. The reaction mixture was stirred in an oil bath at 85°C for 3 hours. After LCMS monitoring showed the disappearance of the starting material, the reaction mixture was quenched by adding water (30 mL). The mixture was extracted with ethyl acetate (30 mL × 3), and the organic phases were combined, washed with saturated brine (30 mL), then dried over anhydrous sodium sulfate, filtered, and finally concentrated under reduced pressure. The resulting residue was purified by silica gel column chromatography to obtain 28a (140 mg). MS (ESI) m/z: 398.1 [M+H$^+$]. $^1$H NMR (400 MHz, $CDCl_3$) δ 8.04 (s, 1H), 7.18 (d, J = 7.5 Hz, 1H), 6.98 (t, J = 7.9 Hz, 1H), 6.75 (d, J = 7.8 Hz, 1H), 5.19 (dd, J = 12.4, 5.2 Hz, 1H), 4.89 (t, J = 3.4 Hz, 1H), 4.53 (d, J = 3.6 Hz, 2H), 3.93 - 3.84 (m, 1H), 3.79 (d, J = 4.4 Hz, 3H), 3.60 - 3.53 (m, 1H), 1.85 - 1.73 (m, 2H), 1.67 - 1.50 (m, 9H).

Step 2: 28a (140 mg, 0.352 mmol) and p-toluenesulfonic acid (72 mg, 0.704 mmol) were dissolved in methanol (5 mL). The reaction system was purged with nitrogen and stirred at 25°C for 3 hours. After TLC monitoring showed the disappearance of the starting material, the reaction mixture was first diluted by adding dichloromethane (30 mL). The reaction mixture was washed with water (30 mL × 3) three times, and the organic phase was extracted. The organic phase was washed with saturated brine (300 mL × 3), dried over anhydrous sodium sulfate, filtered, and concentrated under reduced pressure. The resulting mixture was purified by silica gel column chromatography to obtain 28b (70 mg). MS (ESI) m/z: 314.1 [M+H$^+$]. $^1$H NMR (400 MHz, DMSO) δ 11.12 (s, 1H), 7.15 (d, J = 7.7 Hz, 1H), 7.10 - 7.06 (m, 1H), 7.02 (t, J = 7.8 Hz, 1H), 5.40 (dd, J = 12.7, 5.3 Hz, 1H), 4.36 (s, 2H), 2.96 - 2.83 (m, 1H), 2.65 (s, 2H), 1.99 (s, 1H), 1.17 (s, 1H).

Step 3: 28b (70 mg, 0.22 mmol) was dissolved in ethyl acetate (5 mL). The reaction system was purged with nitrogen, and manganese dioxide (350 mg, 2.2 mmol) was slowly added to the reaction mixture. The mixture was stirred overnight at room temperature. After TLC monitoring showed the disappearance of the starting material, the reaction mixture was first diluted by adding ethyl acetate (20 mL) and then filtered to obtain the filtrate. The organic phase was first washed with saturated brine (20 mL), then dried over anhydrous sodium sulfate, filtered, and finally concentrated under reduced pressure. The resulting residue was purified by silica gel column chromatography to obtain 28c (55 mg). MS (ESI) m/z: 312.1 [M+H$^+$].

Step 4: (S)-Pyrrolidin-3-ylmethanol (1.5 g, 14.5 mmol), di-tert-butyl dicarbonate (4.6 g, 22 mmol), 4-dimethylaminopyridine (180 mg, 1.45 mmol), and triethylamine (3.0 g, 29 mmol) were dissolved in dichloromethane (10 mL) at room temperature. The reaction system was evacuated and purged with nitrogen several times. The reaction mixture was stirred in an oil bath at 25°C for 12 hours. After LC-MS monitoring showed the disappearance of the starting material, the reaction mixture was quenched by adding water (30 mL). The mixture was extracted with ethyl acetate (30 mL × 3), and the organic phases were combined, washed with saturated brine (30 mL), then dried over anhydrous sodium sulfate, filtered, and finally concentrated under reduced pressure. The resulting residue was purified by silica gel column chromatography to obtain 28d (1.8 g).

Step 5: 28d (300 mg, 1.5 mmol) and triethylamine (450 mg, 4.5 mmol) were dissolved in dichloromethane (5 mL) at room temperature. The reaction system was cooled to 0°C, and methanesulfonyl chloride (258 mg, 2.25 mmol) was slowly added dropwise. Then, the system was evacuated and purged with nitrogen several times. The reaction mixture was stirred in an oil bath at 25°C for 12 hours. After LC-MS monitoring showed the disappearance of the starting material, the reaction mixture was quenched by adding water (30 mL). The mixture was extracted with ethyl acetate (15 mL × 3), and the organic phases were combined, washed with saturated brine (10 mL), then dried over anhydrous sodium sulfate, filtered, and finally concentrated under reduced pressure to obtain 28e (340 mg).

Step 6: 28e (309 mg, 0.99 mmol), A (450 mg, 0.9 mmol), potassium iodide (372 mg, 2.7 mmol), and potassium carbonate (150 mg, 0.9 mmol) were dissolved in *N,N*-dimethylformamide (10 mL) at room temperature. The reaction system was evacuated and purged with nitrogen several times. The reaction mixture was stirred in an oil bath at 90°C for 12 hours. After LC-MS monitoring showed the disappearance of the starting material, the reaction mixture was quenched by adding water (30 mL). The mixture was extracted with ethyl acetate (30 mL × 3), and the organic phases were combined, washed with saturated brine (30 mL), then dried over anhydrous sodium sulfate, filtered, and finally concentrated under reduced pressure. The resulting residue was purified by silica gel column chromatography to obtain 28f (270 mg). MS (ESI) m/z: 583.1 [M+H+].

Step 7: 28f (270 mg, 0.39 mmol) was dissolved in dioxane (3 mL) at room temperature, and then dioxane hydrochloride (3 mL) was added dropwise at 0°C. The reaction mixture was stirred in an oil bath at 25°C for 2 hours. After LCMS monitoring showed the disappearance of the starting material, the mixture was concentrated under reduced pressure, and then the pH was adjusted to 7-8. 28g (170 mg) was then obtained by reversed-phase preparative chromatography. MS (ESI) m/z: 583.1 [M+H+].

Step 8: 28g (300 mg, 0.9 mmol), cesium carbonate (876.6 mg, 2.7 mmol), copper(I) iodide (618 mg, 0.09 mmol), and bis(triphenylphosphine)palladium(II) dichloride (60 mg, 0.09 mmol) were dissolved in *N*,*N*-dimethylformamide (6 mL) at room temperature. The reaction system was evacuated and purged with nitrogen several times. The reaction mixture was stirred in an oil bath at 85°C for 3 hours. After LC-MS monitoring showed the disappearance of the starting material, the reaction mixture was quenched by adding water (30 mL). The mixture was extracted with ethyl acetate (30 mL × 3), and the organic phases were combined, washed with saturated brine (30 mL), then dried over anhydrous sodium sulfate, filtered, and finally concentrated under reduced pressure. The resulting residue was purified by silica gel column chromatography to obtain 28 (140 mg). MS (ESI) m/z: 398.1 [M+H+]. $^1$H NMR (400 MHz, $CDCl_3$) δ 8.04 (s, 1H), 7.18 (d, J = 7.5 Hz, 1H), 6.98 (t, J = 7.9 Hz, 1H), 6.75 (d, J = 7.8 Hz, 1H), 5.19 (dd, J = 12.4, 5.2 Hz, 1H), 4.89 (t, J = 3.4 Hz, 1H), 4.53 (d, J = 3.6 Hz, 2H), 3.93 - 3.84 (m, 1H), 3.79 (d, J = 4.4 Hz, 3H), 3.60 - 3.53 (m, 1H), 1.85 - 1.73 (m, 2H), 1.67 - 1.50 (m, 9H).

Example 29

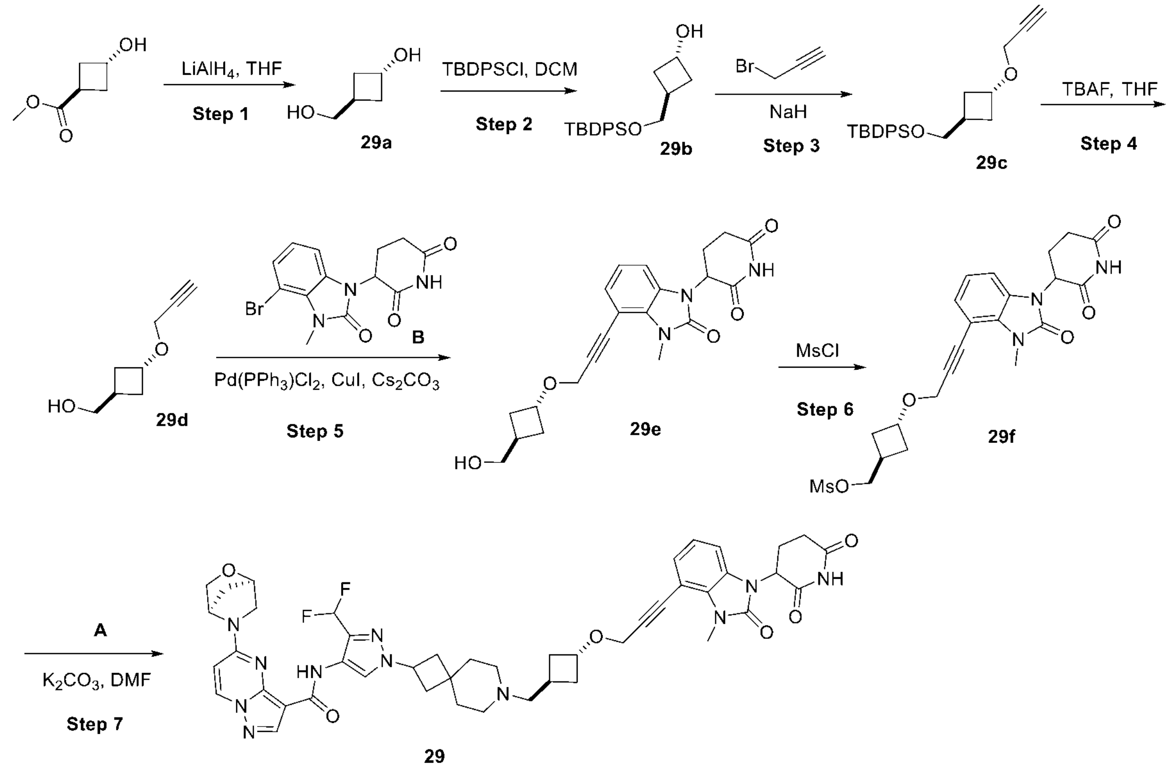


Step 1: Methyl (1R,3R)-3-hydroxycyclobutane-1-carboxylate (5.0 g, 38.4 mmol) was dissolved in dry tetrahydrofuran (32 mL) and placed in an ice-water bath. After the reaction system was cooled to 0°C, lithium aluminum hydride (2.5 M, 30.7 mL) was slowly added dropwise to the reaction mixture under a nitrogen atmosphere, and the reaction was stirred at 0°C for 3 hours. After TLC monitoring showed the disappearance of the starting material, the reaction mixture was first diluted by adding tetrahydrofuran (300 mL), and then $Na_2SO_4 \cdot 10H_2O$ was slowly added to quench the excess lithium aluminum hydride. A large amount of white solid was formed in the reaction mixture, which was filtered, and the filtrate was concentrated under reduced pressure. The resulting mixture was purified by silica gel column chromatography to obtain 29a (2.7 g). $^1$H NMR (400 MHz, DMSO) δ 4.86 (d, *J* = 6.3 Hz, 1H), 4.46 (t, *J* = 5.4 Hz, 1H), 4.16 - 4.09 (m, 1H), 3.35 (dd, *J* = 7.0, 5.5 Hz, 2H), 2.18 - 2.05 (m, 1H), 2.01 - 1.92 (m, 2H), 1.88 - 1.78 (m, 2H).

Step 2: 29a (2.5 g, 24.5 mmol) and imidazole (5.0 g, 73.5 mmol) were dissolved in dichloromethane (30 mL). The reaction system was purged with nitrogen and placed in an ice-water bath. After the reaction system was cooled to 0°C, *tert*-butyldiphenylsilyl chloride (8.1 g, 29.4 mmol) was slowly added dropwise to the reaction mixture under a nitrogen atmosphere, and the reaction was stirred at 25°C for 3 hours. After TLC monitoring showed the disappearance of the starting material, the reaction mixture was first diluted by adding dichloromethane (30 mL). The reaction mixture was washed with water (30 mL × 3) three times, and the organic phase was extracted. The organic phase was washed with saturated brine (300 mL × 3), dried over anhydrous sodium sulfate, filtered, and concentrated under reduced pressure. The resulting mixture was purified by silica gel column chromatography to obtain 29b (2.7 g). $^1$H NMR (400 MHz, DMSO) δ 7.65 - 7.54 (m, 5H), 7.54 - 7.36 (m, 5H), 4.92 (d, *J*= 6.3 Hz, 1H), 4.15 (dd, *J*= 13.7, 6.8 Hz, 1H), 3.61 (d, *J* = 6.5 Hz, 2H), 2.33 - 2.21 (m, 1H), 2.09 - 2.00 (m, 2H), 1.95 - 1.85 (m, 2H), 1.00 (s, 9H).

Step 3: 29b (220 mg, 0.65 mmol) was dissolved in dry tetrahydrofuran (3.5 mL), and the reaction system was purged with nitrogen and placed in an ice-water bath. After the reaction system was cooled to 0°C, sodium hydride (52 mg, 1.30 mmol) was slowly added to the reaction mixture under a nitrogen atmosphere. After the reaction was stirred at 0°C for 1 hour, propargyl bromide (153.4 mg, 1.29 mmol) was slowly added dropwise to the reaction system, and the reaction was slowly returned to room temperature and stirred overnight. After TLC monitoring showed the disappearance of the starting material, the reaction mixture was first quenched by adding saturated aqueous ammonium chloride solution (30 mL). The mixture was extracted with ethyl acetate (30 mL × 3). The organic phases were combined, first washed with saturated brine (50 mL), then dried over anhydrous sodium sulfate, filtered, and finally concentrated under reduced pressure. The resulting residue was purified by silica gel column chromatography to obtain 29c (190 mg). $^1$H NMR (400 MHz, DMSO) δ 7.74 - 7.52 (m, 4H), 7.52 - 7.37 (m, 6H), 4.27 - 4.13 (m, 1H), 4.00 (d, *J* = 2.4 Hz, 2H), 3.63 (d, *J* = 6.1 Hz,2H), 3.36 (t, *J* = 2.4 Hz, 1H), 2.41 - 2.29 (m, 1H), 2.12 - 2.04 (m, 2H), 2.04 - 1.93 (m, 2H), 1.01 (s, 9H).

Step 4: 29c (190 mg, 0.50 mmol) was dissolved in tetrahydrofuran (3 mL) at room temperature, and then tetrabutylammonium fluoride (1.0 M, 1.0 mL) was slowly added to the reaction mixture. The reaction mixture was stirred at

room temperature for another 2 hours. After LC-MS monitoring showed the disappearance of the starting material, the reaction mixture was quenched by adding saturated aqueous ammonium chloride solution (15 mL). The mixture was extracted with ethyl acetate (15 mL × 3). The organic phases were combined, first washed with saturated brine (50 mL), then dried over anhydrous sodium sulfate, filtered, and finally concentrated under reduced pressure. The resulting residue was purified by silica gel column chromatography to obtain 29d (70 mg). $^1$H NMR (400 MHz, DMSO) δ 4.56 (t, *J* = 5.3 Hz, 1H), 4.15 - 4.10 (m, 1H), 4.00 (d, *J* = 2.4 Hz, 2H), 3.42 - 3.34 (m, 3H), 2.28 - 2.14 (m, 1H), 2.04 - 1.88 (m, 4H).

Step 5: 29d (31.1 mg, 0.22 mmol), 3-(4-bromo-3-methyl-2-oxo-2,3-dihydro-1H-benzo[d]imidazol-1-yl)piperidine-2,6-dione (50.0 mg, 0.15 mmol), cesium carbonate (344.3 mg, 0.75 mmol), copper(I) iodide (2.8 mg, 0.015 mmol), and bis(triphenylphosphine)palladium(II) dichloride (10.5 mg, 0.015 mmol) were dissolved in *N,N*-dimethylformamide (1.5 mL) at room temperature. The reaction system was evacuated and purged with nitrogen several times. The reaction mixture was stirred in a sealed tube in an oil bath at 85°C for 3 hours. After LC-MS monitoring showed the disappearance of the starting material, the reaction mixture was quenched by adding water (30 mL). The mixture was extracted with ethyl acetate (30 mL × 3), and the organic phases were combined, washed with saturated brine (30 mL), then dried over anhydrous sodium sulfate, filtered, and finally concentrated under reduced pressure. The resulting residue was purified by silica gel column chromatography to obtain 29e (12 mg). $^1$H NMR (400 MHz, DMSO) δ 11.12 (s, 1H), 7.18 (d, *J* = 7.6 Hz, 1H), 7.14 - 7.09 (m, 1H), 7.03 (t, *J* = 7.9 Hz, 1H), 5.40 (dd, *J* = 12.7, 5.3 Hz, 1H), 4.58 (t, *J* = 5.3 Hz, 1H), 4.43 (s, 0.3H), 4.33 (s, 1.7H), 4.28 - 4.20 (m, 1H), 3.64 (s, 3H), 3.39 (dd, *J* = 6.5, 5.5 Hz, 2H), 2.96 - 2.82 (m, 1H), 2.77 - 2.59 (m, 2H), 2.28 - 2.18 (m, 1H), 2.08 - 1.97 (m, 5H).

Step 6: 29e (110 mg, 0.28 mmol) was dissolved in dichloromethane (3 mL) at room temperature. Triethylamine (85 mg, 0.84 mmol) and p-toluenesulfonyl chloride (47.8 mg, 0.42 mmol) were added at 0°C, and the reaction mixture was stirred at room temperature for 2 hours. After LC-MS monitoring showed the disappearance of the starting material, the reaction mixture was quenched by adding water (30 mL). The mixture was extracted with dichloromethane (30 mL × 3), and the organic phases were combined, washed with saturated brine (30 mL), then dried over anhydrous sodium sulfate, filtered, and finally concentrated under reduced pressure. The resulting residue was purified by silica gel column chromatography to obtain 29f (100 mg). MS (ESI) m/z: 476.2 [M+H$^+$].

Step 7: 29f (100 mg, 0.21 mmol) and A (115.0 mg, 0.23 mmol) were dissolved in *N,N*-dimethylformamide (1.5 mL) at room temperature. Potassium carbonate (86.9 mg, 0.63 mmol) was then added. The reaction system was evacuated and purged with nitrogen several times. The reaction mixture was stirred at 50°C for 18 hours. After LC-MS monitoring showed the disappearance of the starting material, the reaction mixture was quenched by adding water (30 mL). The mixture was extracted with ethyl acetate (30 mL × 3), and the organic phases were combined, washed with saturated brine (30 mL), then dried over anhydrous sodium sulfate, filtered, and finally concentrated under reduced pressure. The resulting residue was purified by preparative high-performance liquid chromatography to obtain the final product 29 (8.8 mg). MS (ESI) M/Z:878.3 [M+H$^+$]. $^1$H NMR (400 MHz, DMSO) δ 11.12 (s, 1H), 9.49 (d, *J* = 5.4 Hz, 1H), 8.78 (d, *J* = 7.7 Hz, 1H), 8.39 (d, *J* = 4.0 Hz, 1H), 8.25 (d, *J* = 5.6 Hz, 1H), 7.31 - 6.97 (m, 4H), 6.87 (d, *J* = 7.8 Hz, 0.5H), 6.45 (d, *J* = 7.8 Hz, 0.5H), 5.4 (dd, *J* = 12.7, 5.4 Hz, 1H), 5.27 (s, 0.5H), 5.07 (s, 0.5H), 4.98 - 4.87 (m, 1H), 4.77 (d, *J* = 16.2 Hz, 1H), 4.34 (s, 2H), 4.31 - 4.21 (m, 1H), 3.82 (d, *J* = 9.9 Hz, 2H), 3.74 (d, *J* = 7.5 Hz, 1H), 3.63 (s, 3H), 3.63 - 3.57 (m, 2H), 3.47 - 3.42 (m, 1H), 2.95 - 2.84 (m, 1H), 2.77 - 2.71 (m, 1H), 2.71 - 2.65 (m, 1H), 2.35 - 2.27 (m, 6H), 2.23 - 2.11 (m, 3H), 2.07 - 1.93 (m, 7H), 1.58 (dd, *J* = 16.5, 12.2 Hz, 4H).

Example 30

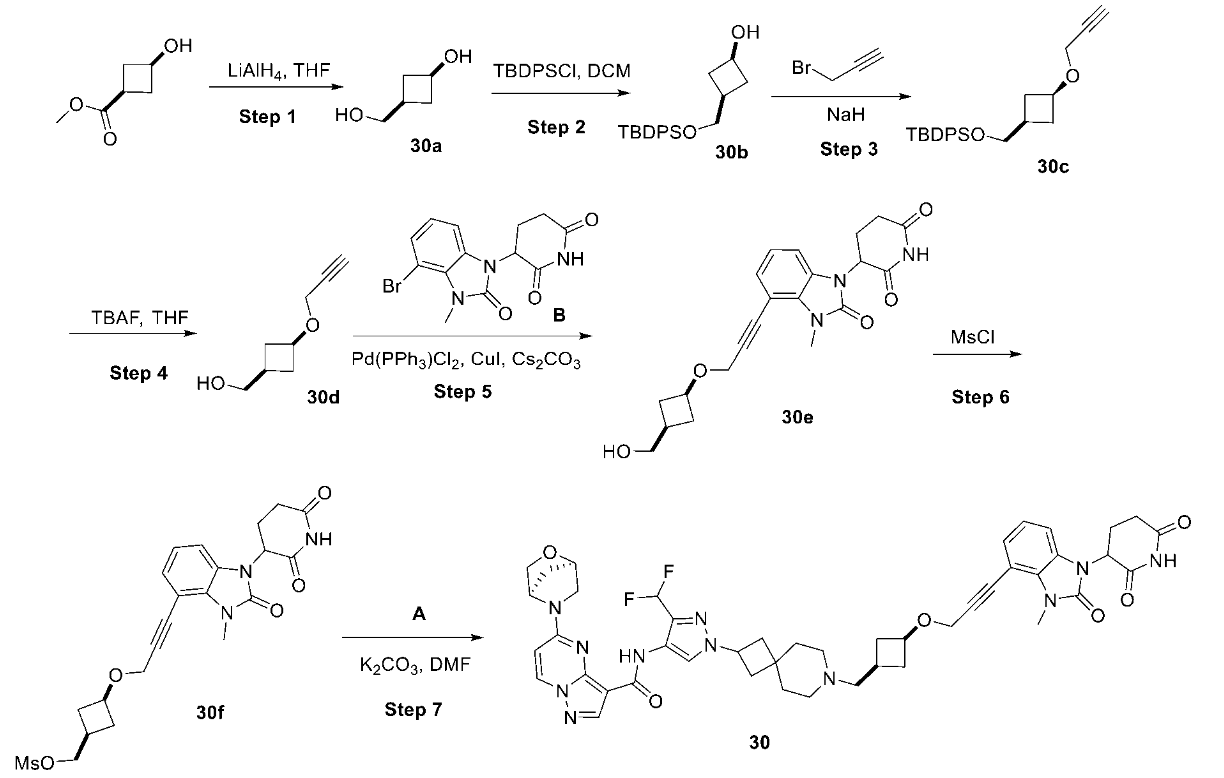


The desired product 30 (8.5 mg) was obtained with reference to Example 29. MS (ESI) M/Z:878.4 [M+H$^+$]. $^1$H NMR (400 MHz, DMSO) δ 11.12 (s, 1H), 9.50 (d, *J* = 5.3 Hz, 1H), 8.78 (d, *J* = 7.7 Hz, 1H), 8.39 (d, *J* = 4.0 Hz, 1H), 8.25 (d, *J* = 5.6 Hz, 1H), 7.29 - 6.97 (m, 4H), 6.87 (d, *J* = 7.9 Hz, 0.5H), 6.45 (d, *J* = 7.8 Hz, 0.5H), 5.40 (dd, *J* = 12.6, 5.3 Hz, 1H), 5.27 (s, 0.5H), 5.07 (s, 0.5H), 4.98 - 4.88 (m, 1H), 4.77 (d, *J* = 16.0 Hz, 1H), 4.43 (s, 2H), 3.85 - 3.72 (m, 3H), 3.63 (d, *J* = 3.8 Hz, 3H), 3.59 (s, 1H), 3.45 (d, *J* = 9.8 Hz, 0.5H), 3.33 (s, 0.5H), 2.88 (m, *J* = 20.3, 9.5 Hz, 1H), 2.78 - 2.57 (m, 2H), 2.31 (m, *J* = 12.5, 5.1 Hz, 4H), 2.22 (s, 1H), 2.19 - 2.12 (m, 2H), 2.10 - 1.90 (m, 6H), 1.79 (m, *J* = 10.7 Hz, 2H), 1.64 (m, 2H), 1.58 (m, 3H), 1.46 (m, *J* = 12.9 Hz, 4H), 1.25 - 1.18 (m, 2H).

Example 31

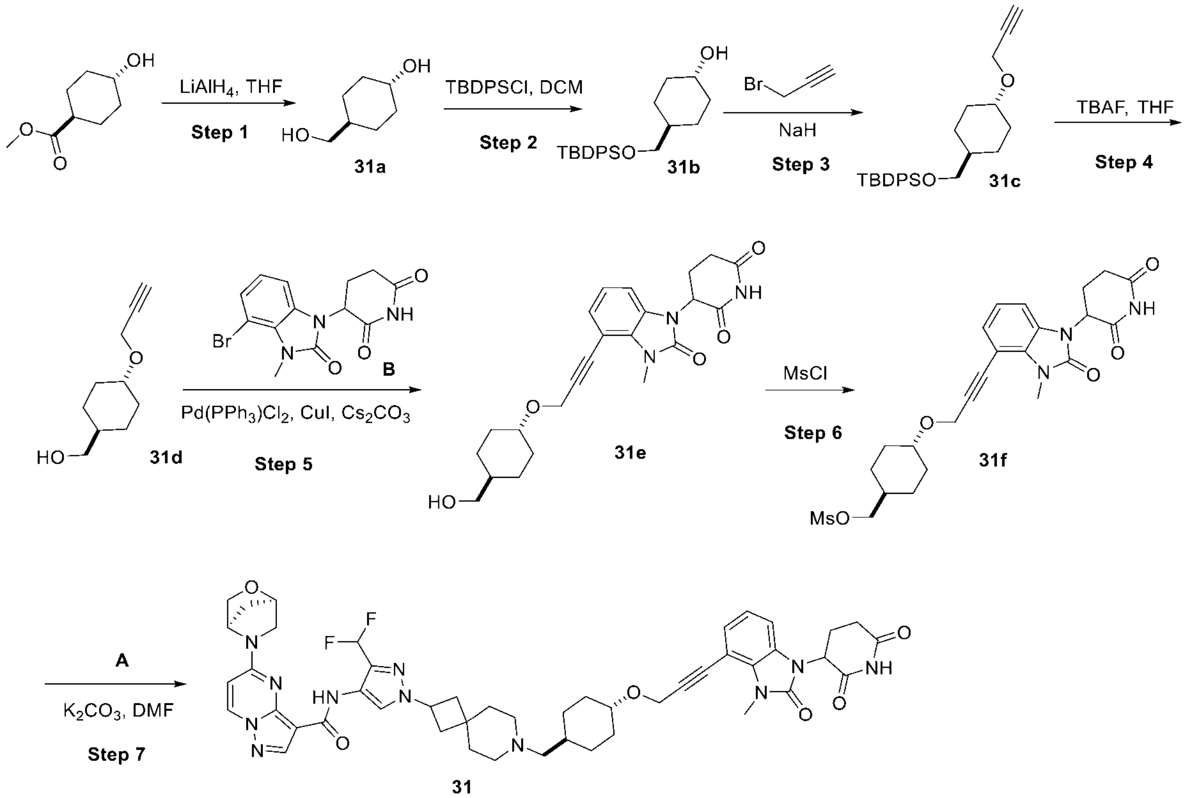


31a 31b 31c 31d 31e 31f 31

Step 1: Methyl (1r,4r)-4-hydroxycyclohexane-1-carboxylate (5.0 g, 31.6 mmol) was dissolved in dry tetrahydrofuran (50 mL) and placed in an ice-water bath. After the reaction system was cooled to 0°C, lithium aluminum hydride (2.5 M, 25.2 mL) was slowly added dropwise to the reaction mixture under a nitrogen atmosphere, and the reaction was stirred at 0°C for 3 hours. After TLC monitoring showed the disappearance of the starting material, the reaction mixture was first diluted by adding tetrahydrofuran (300 mL), and then $Na_2SO_4 \cdot 10H_2O$ was slowly added to quench the excess lithium aluminum hydride. A large amount of white solid was formed in the reaction mixture, which was filtered, and the filtrate was concentrated under reduced pressure. The resulting mixture was purified by silica gel column chromatography to obtain 31a (2.6 g). $^1H$ NMR (400 MHz, DMSO) δ 4.44 (d, *J*= 4.4 Hz, 1H), 4.34 (t, *J* = 5.3 Hz, 1H), 3.32 - 3.24 (m, 1H), 3.18 (t, *J* = 5.8 Hz, 2H), 1.88 - 1.75 (m, 2H), 1.74 - 1.61 (m, 2H), 1.31 - 1.16 (m, 1H), 1.15 - 0.98 (m, 2H), 0.94 - 0.76 (m, 2H).

Step 2: 31a (2.3 g, 17.7 mmol) and imidazole (3.6 g, 53.1 mmol) were dissolved in dichloromethane (40 mL). The reaction system was purged with nitrogen and placed in an ice-water bath. After the reaction system was cooled to 0°C, tert-butyldiphenylsilyl chloride (5.8 g, 21.2 mmol) was slowly added dropwise to the reaction mixture under a nitrogen atmosphere, and the reaction was stirred at 25°C for 3 hours. After TLC monitoring showed the disappearance of the starting material, the reaction mixture was first diluted by adding dichloromethane (50 mL). The reaction mixture was washed with water (50 mL × 3) three times, and the organic phase was extracted. The organic phase was washed with saturated brine (300 mL × 3), dried over anhydrous sodium sulfate, filtered, and concentrated under reduced pressure. The resulting mixture was purified by silica gel column chromatography to obtain 31b (3.8 g). $^1H$ NMR (400 MHz, MeOD) δ 7.72 - 7.58 (m, 4H), 7.47 - 7.32 (m, 6H), 3.52 - 3.38 (m, 3H), 1.98 - 1.91 (m, 2H), 1.90 - 1.74 (m, 2H), 1.53 - 1.40 (m, 1H), 1.32 - 1.25 (m, 2H), 1.11 - 0.94 (m, 11H).

Step 3: 31b (3.5 g, 9.5 mmol) was dissolved in dry tetrahydrofuran (3.5 mL), and the reaction system was purged with nitrogen and placed in an ice-water bath. After the reaction system was cooled to 0°C, sodium hydride (1.1 g, 28.4 mmol) was slowly added to the reaction mixture under a nitrogen atmosphere. After the reaction was stirred at 0°C for 1 hour, propargyl bromide (3.4 g, 28.4 mmol) was slowly added dropwise to the reaction system, and the reaction was slowly returned to room temperature and stirred overnight. After TLC monitoring showed the disappearance of the starting material, the reaction mixture was first quenched by adding saturated aqueous ammonium chloride solution (100 mL). The mixture was extracted with ethyl acetate (100 mL × 3). The organic phases were combined, first washed with saturated brine (100 mL), then dried over anhydrous sodium sulfate, filtered, and finally concentrated under reduced pressure. The resulting residue was purified by silica gel column chromatography to obtain 31c (2.0 g).

Step 4: 31c (1.8 g, 4.4 mmol) was dissolved in tetrahydrofuran (20 mL) at room temperature, and then tetrabutylammonium fluoride (1.0 M, 8.8 mL) was slowly added to the reaction mixture. The reaction mixture was stirred at room temperature for another 2 hours. After LC-MS monitoring showed the disappearance of the starting material, the

reaction mixture was quenched by adding saturated aqueous ammonium chloride solution (20 mL). The mixture was extracted with ethyl acetate (20 mL × 3). The organic phases were combined, first washed with saturated brine (50 mL), then dried over anhydrous sodium sulfate, filtered, and finally concentrated under reduced pressure. The resulting residue was purified by silica gel column chromatography to obtain 31d (670 mg). $^1$H NMR (400 MHz, DMSO) δ 4.38 (t, *J* = 5.3 Hz, 1H), 4.13 (d, *J* = 2.4 Hz, 2H), 3.37 - 3.33 (m, 1H), 3.33 - 3.25 (m, 1H), 3.24 - 3.14 (m, 2H), 2.03 - 1.93 (m, 2H), 1.78 - 1.65 (m, 2H), 1.35 - 1.22 (m, 1H), 1.15 - 1.02 (m, 2H), 0.96 - 0.80 (m, 2H).

Step 5: 31d (364.9 mg, 2.25 mmol), B (500.0 mg, 1.5 mmol), cesium carbonate (2.4 g, 7.5 mmol), copper(I) iodide (28.5 mg, 0.15 mmol), and bis(triphenylphosphine)palladium(II) dichloride (105.1 mg, 0.15 mmol) were dissolved in *N*,*N*-dimethylformamide (10 mL) at room temperature. The reaction system was evacuated and purged with nitrogen several times. The reaction mixture was stirred in a sealed tube in an oil bath at 85°C for 3 hours. After LC-MS monitoring showed the disappearance of the starting material, the reaction mixture was quenched by adding water (200 mL). The mixture was extracted with ethyl acetate (200 mL × 3), and the organic phases were combined, washed with saturated brine (30 mL), then dried over anhydrous sodium sulfate, filtered, and finally concentrated under reduced pressure. The resulting residue was purified by silica gel column chromatography to obtain 31e (300 mg). $^1$H NMR (400 MHz, DMSO) δ 11.12 (s, 1H), 7.17 (d, *J* = 7.7 Hz, 1H), 7.11 (d, *J* = 7.0 Hz, 1H), 7.03 (t, *J*= 7.9 Hz, 1H), 5.40 (dd, *J*= 12.7, 5.3 Hz, 1H), 4.46 (s, 2H), 3.64 (s,3H), 3.50 - 3.37 (m, 1H), 3.30 (s, 1H), 3.20 (m, 2H), 2.97 - 2.81 (m, 1H), 2.79 - 2.61 (m, 2H), 2.11 - 2.00 (m, 3H), 1.75 (d, *J*= 11.6 Hz, 2H), 1.37 - 1.25 (m, 1H), 1.16 - 1.06 (m, 1H), 0.99 - 0.77 (m, 2H).

Step 6: 31e (270 mg, 0.63 mmol) was dissolved in dichloromethane (8 mL) at room temperature. Triethylamine (318.8 mg, 3.1 mmol) and p-toluenesulfonyl chloride (291.9 mg, 2.5 mmol) were added at 0°C, and the reaction mixture was stirred at room temperature for 2 hours. After LC-MS monitoring showed the disappearance of the starting material, the reaction mixture was quenched by adding water (30 mL). The mixture was extracted with dichloromethane (30 mL × 3), and the organic phases were combined, washed with saturated brine (30 mL), then dried over anhydrous sodium sulfate, filtered, and finally concentrated under reduced pressure. The resulting residue was purified by silica gel column chromatography to obtain 31f (160 mg). MS (ESI) m/z: 504.0 [M+H$^+$].

Step 7: 31f (160 mg, 0.32 mmol) and A (207.2 mg, 0.42 mmol) were dissolved in *N,N*-dimethylformamide (4 mL) at room temperature. Potassium carbonate (176.6 mg, 1.28 mmol) was then added. The reaction system was evacuated and purged with nitrogen several times. The reaction mixture was stirred at 50°C for 18 hours. After LC-MS monitoring showed the disappearance of the starting material, the reaction mixture was quenched by adding water (30 mL). The mixture was extracted with ethyl acetate (30 mL × 3), and the organic phases were combined, washed with saturated brine (30 mL), then dried over anhydrous sodium sulfate, filtered, and finally concentrated under reduced pressure. The resulting residue was purified by preparative high-performance liquid chromatography to obtain the final product 31 (18.9 mg). MS (ESI) m/z: 906.8 [M+H$^+$]. $^1$H NMR (400 MHz, DMSO) δ 11.12 (s, 1H), 9.49 (d, *J* = 5.2 Hz, 1H), 8.78 (d, *J* = 7.7 Hz, 1H), 8.39 (d, *J* = 3.9 Hz, 1H), 8.25 (d, *J* = 5.7 Hz, 1H), 7.41 - 6.92 (m, 4H), 6.87 (d, *J* = 7.9 Hz, 0.5H), 6.45 (d, *J* = 7.7 Hz, 0.5H), 5.40 (dd, *J* = 12.7, 5.3 Hz, 1H), 5.28 (s, 0.5H), 5.08 (s, 0.5H), 5.00 - 4.86 (m, 1H), 4.77 (d, *J* = 16.4 Hz, 1H), 4.46 (s, 2H), 3.87 - 3.69 (m, 2H), 3.64 (s, 3H), 3.63 - 3.54 (m, 1H), 3.50 - 3.39 (m, 2H), 2.95 - 2.83 (m, 1H), 2.79 - 2.58 (m, 2H), 2.37 - 2.10 (m, 8H), 2.05 - 1.93 (m, 7H), 1.78 (d, *J*= 11.9 Hz, 2H), 1.61 (d, *J* = 22.8 Hz, 4H), 1.52 - 1.38 (m, 1H), 1.21 - 1.07 (m, 2H), 0.95 - 0.76 (m, 2H).

Example 32

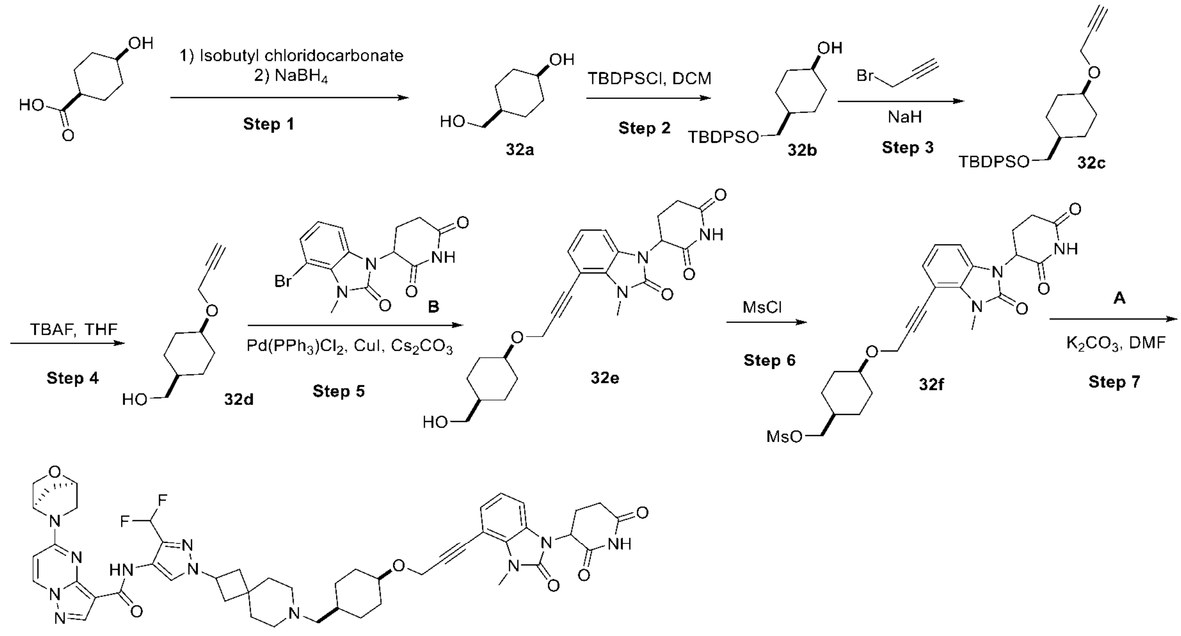


32

The desired product 32 (12.39 mg) was obtained with reference to Example 31. MS (ESI) M/Z:906.5 [M+H$^+$]. $^1$H NMR (400 MHz, DMSO) δ 11.12 (s, 1H), 9.50 (d, *J* = 5.3 Hz, 1H), 8.78 (d, *J* = 7.7 Hz, 1H), 8.39 (d, *J* = 4.0 Hz, 1H), 8.25 (d, *J* = 5.6 Hz, 1H), 7.29 - 6.97 (m, 4H), 6.87 (d, *J* = 7.9 Hz, 0.5H), 6.45 (d, *J* = 7.8 Hz, 0.5H), 5.40 (dd, *J* = 12.6, 5.3 Hz, 1H), 5.27 (s, 0.5H), 5.07 (s, 0.5H), 4.98 - 4.88 (m, 1H), 4.77 (d, *J* = 16.0 Hz, 1H), 4.43 (s, 2H), 3.85 - 3.72 (m, 3H), 3.63 (d, *J* = 3.8 Hz, 3H), 3.59 (s, 1H), 3.45 (d, *J* = 9.8 Hz, 0.5H), 3.33 (s, 0.5H), 2.88 (m, *J* = 20.3, 9.5 Hz, 1H), 2.78 - 2.57 (m, 2H), 2.31 (m, *J* = 12.5, 5.1 Hz, 4H), 2.22 (s, 1H), 2.19 - 2.12 (m, 2H), 2.10 - 1.90 (m, 6H), 1.79 (m, *J* = 10.7 Hz, 2H), 1.64 (m, 2H), 1.58 (m, 3H), 1.46 (m, *J* = 12.9 Hz, 4H), 1.25 - 1.18 (m, 2H).

Example 33

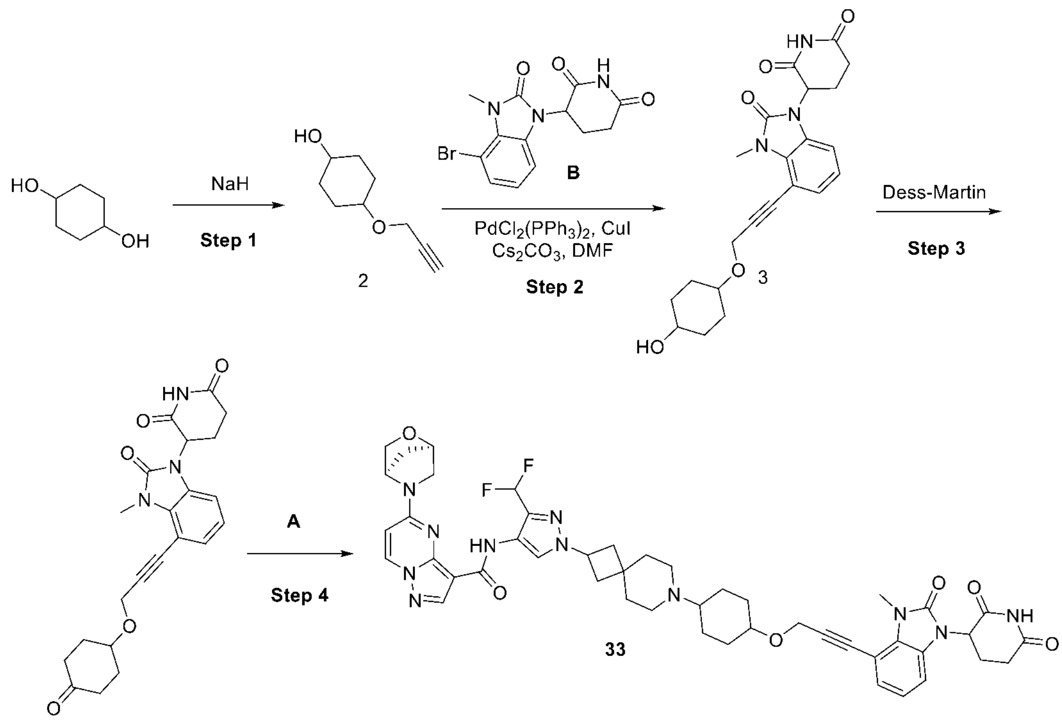

The desired product 33 (2.4 mg) was obtained with reference to Example 4. MS (ESI) m/z: 892.4 [M+H+]. $^{1}$H NMR (400 MHz, DMSO) δ 11.12 (s, 1H), 9.49 (d, J = 5.2 Hz, 1H), 8.79 (d, J = 7.7 Hz, 1H), 8.39 (d, J = 3.8 Hz, 1H), 8.26 (d, J = 5.6 Hz, 1H), 7.19 - 7.08 (m, 2.5H), 7.03 (t, J = 7.9 Hz, 1.4H), 6.87 (d, J = 7.8 Hz, 0.49H), 6.45 (d, J = 7.8 Hz, 0.53H), 5.40 (dd, J = 12.6, 5.4 Hz, 1H), 5.27 (s, 0.5H), 5.08 (s, 0.5H), 4.97 - 4.87 (m, 1H), 4.77 (d, J = 15.5 Hz, 1H), 4.47 - 4.41 (m, 2H), 3.82 (d, J = 9.9 Hz, 1.4H), 3.74 (d, J = 7.2 Hz, 1H), 3.65 - 3.63 (m, 3.64H), 3.59 (s, 1H), 3.48 - 3.39 (m, 1H), 3.29 (s, 1H), 2.93 - 2.84 (m, 1H), 2.75 - 2.60 (m, 2H), 2.46 - 2.42 (m, 1H), 2.38 - 2.23 (m, 5H), 2.07 - 1.98 (m, 3H), 1.94 - 1.86 (m, 2H), 1.75 (d, 1H), 1.65 - 1.56 (m, 3H), 1.49 - 1.42 (m, 2H), 1.27 - 1.21 (m, 1H).

Example 34

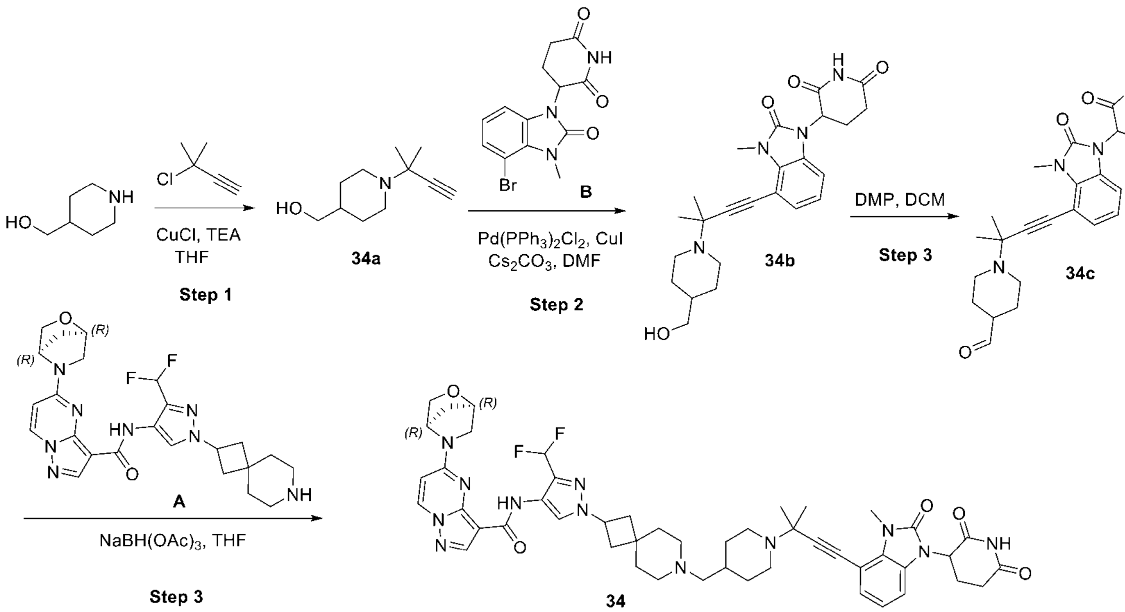


The desired product 34 (13.58 mg) was obtained with reference to Example 3. MS (ESI) M/Z:919.2 [M+H+]. $^{1}$H NMR (400 MHz, DMSO) δ 11.12 (s, 1H), 9.49 (d, *J* = 5.4 Hz, 1H), 8.78 (d, *J* = 7.7 Hz, 1H), 8.39 (d, *J* = 4.0 Hz, 1H), 8.25 (d, *J* = 5.6 Hz, 1H), 7.30 - 6.96 (m, 4H), 6.87 (d, *J* = 8.0 Hz, 0.5H), 6.45 (d, *J* = 7.8 Hz, 0.5H), 5.39 (dd, *J* = 12.8, 5.2 Hz, 1H), 5.27 (s, 0.5H), 5.07 (s, 0.5H), 4.99 - 4.88 (m, 1H), 4.77 (d, *J* = 17.4 Hz, 1H), 3.84 - 3.72 (m, 2H), 3.65 (d, *J* = 3.6 Hz, 3H), 3.60 (d, *J* = 11.7 Hz, 1H), 3.45 (d, *J* = 10.3 Hz, 1H), 3.11 (d, *J* = 10.0 Hz, 2H), 2.97 - 2.83 (m, 1H), 2.77 - 2.60 (m, 3H), 2.39 - 2.27 (m, 5H), 2.17 (t, *J*= 10.3 Hz, 6H), 2.09 - 1.86 (m, 4H), 1.79 - 1.58 (m, 6H), 1.43 (s, 6H), 1.18 - 1.03 (m, 2H).

Example 35

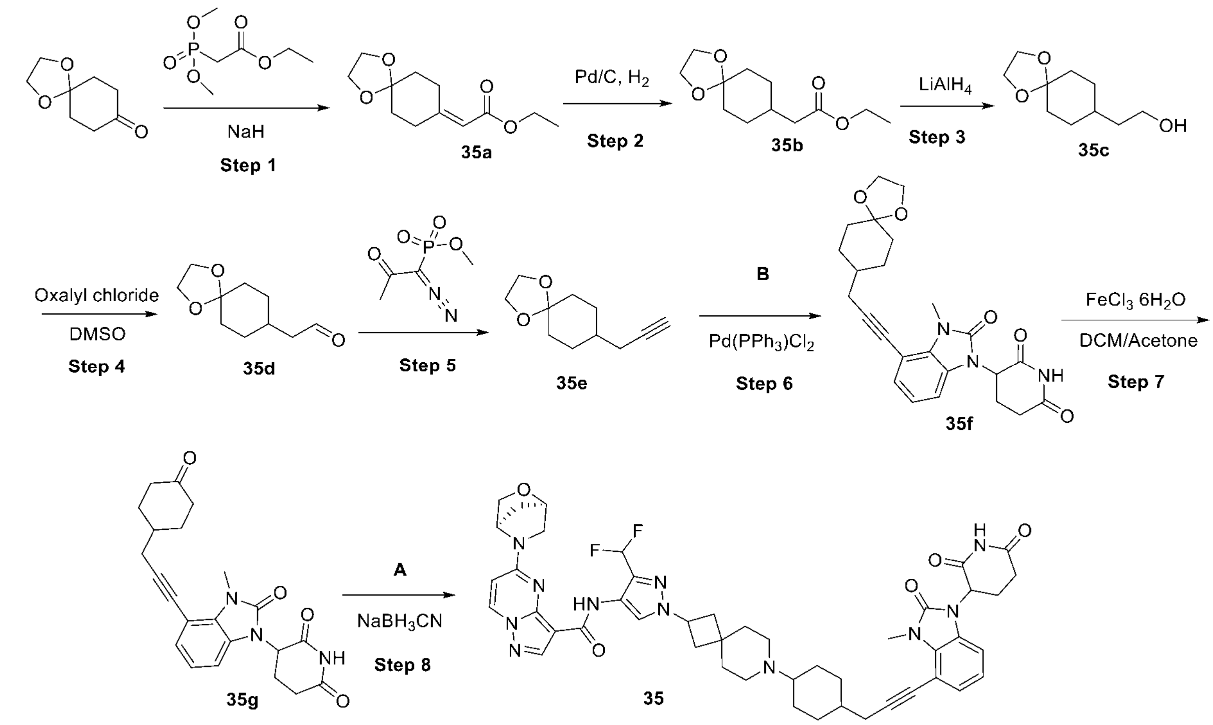


Step 1: Sodium hydride (8.6 g, 38.4 mmol) was dissolved in dry tetrahydrofuran (50 mL). The reaction system was purged with nitrogen and placed in an ice-water bath. After the reaction system was cooled to 0°C, 1,4-dioxaspiro[4.5] decan-8-one (5.0 g, 32.0 mmol dissolved in 10 mL of tetrahydrofuran) was slowly added to the reaction mixture under a nitrogen atmosphere. After the reaction was stirred at 0°C for 1 hour, the reaction mixture was cooled to -20°C, and ethyl 2-(dimethoxyphosphoryl)acetate (8.6 g, 38.4 mmol dissolved in 8 mL of tetrahydrofuran) was slowly added dropwise to the reaction system. The reaction was slowly returned to room temperature and stirred for 2.5 hours. After TLC monitoring showed the disappearance of the starting material, the reaction mixture was first quenched by adding water (500 mL). The mixture was extracted with ethyl acetate (500 mL × 3). The organic phases were combined, first washed with saturated brine (100 mL), then dried over anhydrous sodium sulfate, filtered, and finally concentrated under reduced pressure. The resulting residue was purified by silica gel column chromatography to obtain 35a (4.4 g). $^1$H NMR (400 MHz, DMSO) δ 5.45 - 5.32 (m, 1H), 4.05 (dt, *J* = 12.3, 4.2 Hz, 2H), 3.86 (s, 4H), 2.97 (s, 2H), 2.20 - 2.07 (m, 4H), 1.65 (t, *J* = 6.5 Hz, 2H), 1.17 (t, *J* = 7.1 Hz, 3H).

Step 2: 35a (4.0 g, 17.6 mmol) and palladium on carbon (4.0 g, 17.6 mmol) were dissolved in ethanol (25 mL), and the reaction system was stirred under a hydrogen atmosphere at room temperature for 3 hours. After TLC monitoring showed the disappearance of the starting material, the reaction mixture was first diluted by adding ethanol (100 mL), and the black solid was filtered through a Büchner funnel. The filter cake was washed twice with ethanol, and the filtrate was concentrated under reduced pressure. The resulting mixture was purified by silica gel column chromatography to obtain 35b (2.2 g). MS (ESI) m/z: 229.1 [M+H$^+$].

Step 3: 35b (2.2 g, 9.6 mmol) was dissolved in dry tetrahydrofuran (30 mL), and the reaction system was placed in an ice-water bath. After the reaction system was cooled to 0°C, lithium aluminum hydride (2.5 M, 7.7 mL) was slowly added dropwise to the reaction mixture under a nitrogen atmosphere, and the reaction was stirred at 0°C for 3 hours. After TLC monitoring showed the disappearance of the starting material, the reaction mixture was first diluted by adding tetrahydrofuran (300 mL), and then $Na_2SO_4 \cdot 10H_2O$ was slowly added to quench the excess lithium aluminum hydride. A large amount of white solid was formed in the reaction mixture, which was filtered, and the filtrate was concentrated under reduced pressure. The resulting mixture was purified by silica gel column chromatography, followed by distillation and suspension drying under reduced pressure to obtain 35c (1.45 g). $^1$H NMR (400 MHz, DMSO) δ 4.31 (t, *J* = 5.1 Hz, 1H), 3.83 (s, 4H), 3.47 - 3.37 (m, 2H), 1.63 (d, *J* = 10.1 Hz, 4H), 1.47 - 1.28 (m, 5H), 1.18 - 1.04 (m, 2H).

Step 4: Oxalyl chloride (0.2 mL, 2.42 mmol) was dissolved in dry dichloromethane (3 mL). The reaction system was purged with nitrogen and placed in a dry ice-acetone bath. After the reaction system was cooled to -78°C, dimethyl sulfoxide (0.35 mL, 4.84 mmol dissolved in 3 mL of dichloromethane) was slowly added to the reaction mixture under a nitrogen atmosphere. After the reaction was stirred at -78 for 0.5 hours, 35c (300 mg, 1.61 mmol dissolved in 3 mL of dichloromethane) was slowly added dropwise to the reaction system, and the reaction was stirred at -78 for another hour. After TLC monitoring showed the disappearance of the starting material, triethylamine (12 mL) was added to the reaction mixture, and the mixture was stirred at room temperature for 0.5 hours. Then, water (50 mL) was added to the reaction

mixture, and the mixture was extracted with dichloromethane (50 mL × 3). The organic phases were combined, washed with saturated brine (50 mL), then dried over anhydrous sodium sulfate, filtered, and finally concentrated under reduced pressure. The resulting residue was purified by silica gel column chromatography to obtain 35d (250 mg, yield: 84.4%). $^1$H NMR (400 MHz, DMSO) δ 9.65 (t, *J* = 2.0 Hz, 1H), 3.83 (s, 4H), 2.33 (dd, *J* = 6.8, 2.0 Hz, 2H), 1.92 - 1.78 (m, 1H), 1.69 - 1.57 (m, 4H), 1.53 - 1.39 (m, 2H), 1.27 - 1.12 (m, 2H).

Step 5: 35d (250 mg, 1.36 mmol) and potassium carbonate (563.1 mg, 4.08 mmol) were dissolved in methanol (5 mL) at room temperature. Then, dimethyl (1-diazo-2-oxopropyl)phosphonate (313.0 mg, 4.08 mmol) was added, and the reaction mixture was stirred at room temperature for 18 hours. After LC-MS monitoring showed the disappearance of the starting material, the reaction mixture was concentrated under reduced pressure, and water (30 mL) was added to the residue for quenching. The mixture was extracted with ethyl acetate (30 mL × 3), and the organic phases were combined, washed with saturated brine (30 mL), then dried over anhydrous sodium sulfate, filtered, and finally concentrated under reduced pressure. The resulting residue was purified by silica gel column chromatography to obtain 35e (220 mg, yield: 89.8%). $^1$H NMR (400 MHz, DMSO) δ 3.83 (s, 4H), 2.77 (t, *J* = 2.7 Hz, 1H), 2.09 (dd, *J* = 6.5, 2.7 Hz, 2H), 1.73 - 1.61 (m, 4H), 1.50 - 1.38 (m, 3H), 1.30 - 1.20 (m, 2H).

Step 6: 35e (167.1 mg, 0.93 mmol), B (200.0 mg, 0.62 mmol), cesium carbonate (1.0 g, 3.1 mmol), copper(I) iodide (11.8 mg, 0.062 mmol), and bis(triphenylphosphine)palladium(II) dichloride (43.5 mg, 0.062 mmol) were dissolved in *N,N*-dimethylformamide (16 mL) at room temperature. The reaction system was evacuated and purged with nitrogen several times. The reaction mixture was stirred in a sealed tube in an oil bath at 85°C for 3 hours. After LC-MS monitoring showed the disappearance of the starting material, the reaction mixture was quenched by adding water (200 mL). The mixture was extracted with ethyl acetate (200 mL × 3). The organic phases were combined, first washed with saturated brine (30 mL), then dried over anhydrous sodium sulfate, filtered, and finally concentrated under reduced pressure. The resulting residue was purified by silica gel column chromatography to obtain 35f (100 mg). MS (ESI) m/z: 438.3 [M+H$^+$].

Step 7: At room temperature, 35f (100 mg, 0.23 mmol) was dissolved in dichloromethane/acetone (4 mL/1 mL), and then iron(III) chloride hexahydrate (216.5 mg, 0.8 mmol) was slowly added to the reaction mixture. The reaction mixture was stirred overnight at room temperature. After LC-MS monitoring showed the disappearance of the starting material, the reaction mixture was quenched by adding aqueous sodium bicarbonate solution (20 mL). The mixture was extracted with dichloromethane (20 mL × 3). The organic phases were combined, first washed with saturated brine (50 mL), then dried over anhydrous sodium sulfate, filtered, and finally concentrated under reduced pressure. The resulting residue was purified by silica gel column chromatography to obtain 35g (62 mg). MS (ESI) m/z: 394.0 [M+H$^+$].

Step 8: At room temperature, 35g (60 mg, 0.15 mmol) was dissolved in a mixture of tetrahydrofuran/*N*,*N*-dimethylformamide (0.8 mL/0.4 mL), and then A (83.4 mg, 0.17 mmol) and potassium acetate (16.2 mg, 0.17 mmol) were added to the reaction mixture. The reaction system was evacuated and purged with nitrogen several times. The reaction mixture was stirred at 25°C for 2 hours. Then sodium cyanoborohydride (18.9 mg, 0.30 mmol) was slowly added to the reaction mixture. The reaction mixture was stirred at room temperature for another 2 hours. After LC-MS monitoring showed the disappearance of the starting material, the reaction mixture was quenched by adding saturated aqueous ammonium chloride solution (30 mL). The mixture was extracted with ethyl acetate (50 mL × 3). The organic phases were combined, first washed with saturated brine (50 mL), then dried over anhydrous sodium sulfate, filtered, and finally concentrated under reduced pressure. The resulting residue was purified by preparative high-performance liquid chromatography to obtain the final product 35 (12.0 mg). MS (ESI) m/z: 876.5 [M+H$^+$]. $^1$H NMR (400 MHz, DMSO) δ 11.11 (s, 1H), 9.50 (d, *J* = 5.1 Hz, 1H), 8.79 (d, *J* = 7.7 Hz, 1H), 8.39 (d, *J* = 4.1 Hz, 1H), 8.26 (d, *J* = 5.7 Hz, 1H), 7.20 - 6.92 (m, 4H), 6.87 (d, *J* = 7.9 Hz, 0.5H), 6.46 (d, *J* = 7.8 Hz, 0.5H), 5.38 (dd, *J* = 12.7, 5.4 Hz, 1H), 5.28 (s, 0.5H), 5.08 (s, 0.5H), 5.00 - 4.85 (m, 1H), 4.77 (d, *J* = 16.4 Hz, 1H), 3.85 - 3.71 (m, 2H), 3.66 - 3.57 (m, 4.5H), 3.48 - 3.41 (m, 0.5H), 3.29 (s, 2H), 2.95 - 2.82 (m, 1H), 2.78 - 2.57 (m, 2H), 2.40 - 2.26 (m, 6H), 2.21 - 2.10 (m, 3H), 2.08 - 1.74 (m, 6H), 1.74 - 1.41 (m, 10H).

Example 36

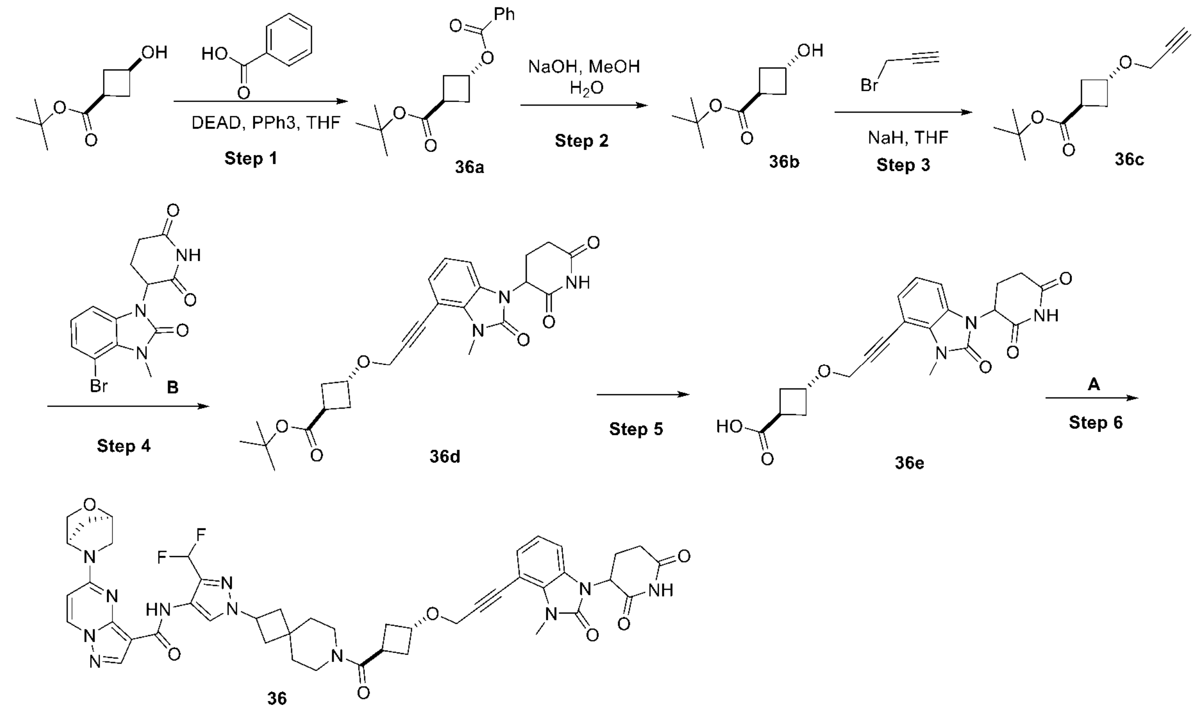


The desired product 36 (11.4 mg) was obtained with reference to Example 20. MS (ESI) m/z: 892.4 [M+H$^+$]. $^1$H NMR (400 MHz, DMSO) δ 11.12 (s, 1H), 9.50 (d, J = 5.1 Hz, 1H), 8.79 (d, J = 7.7 Hz, 1H), 8.41 (d, J = 4.1 Hz, 1H), 8.26 (d, J = 5.7 Hz, 1H), 7.35 - 6.94 (m, 4H), 6.87 (d, J = 7.9 Hz, 0.5H), 6.46 (d, J = 7.7 Hz, 0.5H), 5.40 (dd, J = 12.6, 5.3 Hz, 1H), 5.28 (s, 0.5H), 5.08 (s, 0.5H), 5.03 - 4.91 (m, 1H), 4.77 (d, J = 17.3 Hz, 1H), 4.37 (s, 2H), 4.23 - 4.13 (m, 1H), 3.87 - 3.71 (m, 2H), 3.64 (s, 3H), 3.63 - 3.57 (m, 1.5H), 3.52 - 3.37 (m, 2.5H), 3.26 - 3.17 (m, 1H), 2.96 - 2.81 (m, 1H), 2.77 - 2.60 (m, 2H), 2.43 - 2.34 (m, 4H), 2.26 - 2.18 (m, 5H), 2.06 - 1.91 (m, 4H), 1.65 - 1.50 (m, 4H)

Example 37

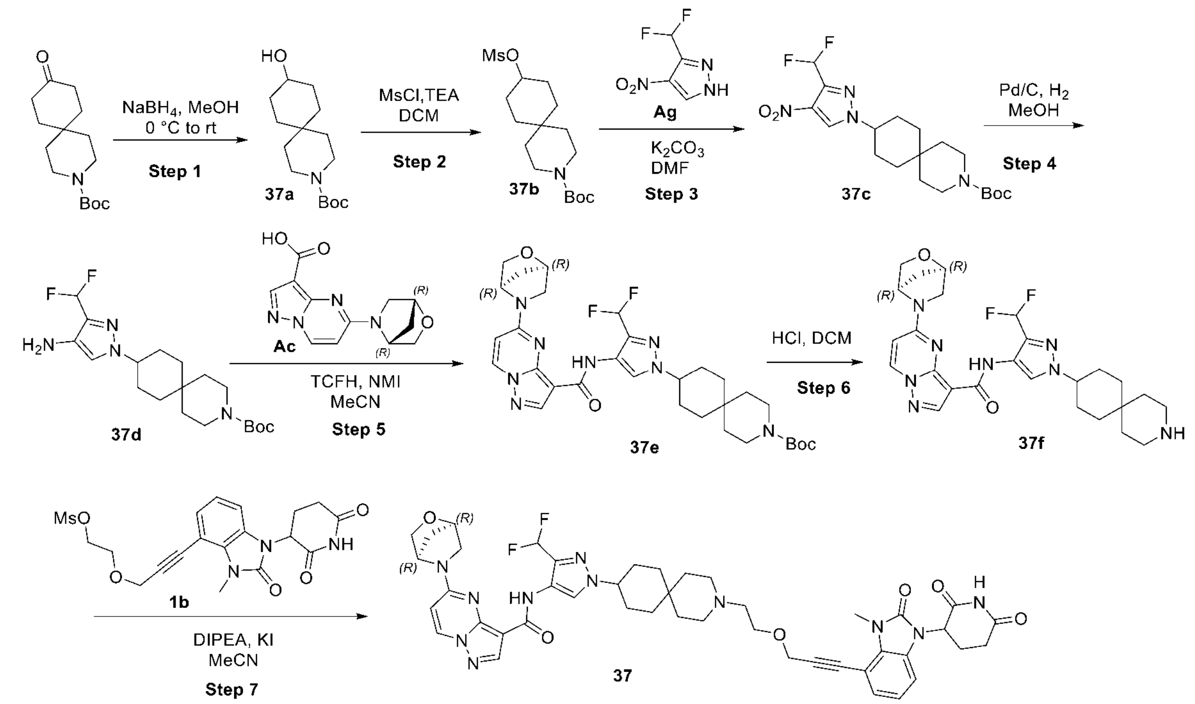

The desired product 37 (2.44 mg) was obtained with reference to Example 1. MS (ESI) m/z: 866.3 [M+H+]; $^1$H NMR (400 MHz, $CDCl_3$) δ 9.61 (s, 1H), 8.69 (s, 1H), 8.54 - 8.27 (m, 3H), 7.18 (d, *J*= 7.9 Hz, 1H), 6.99 (t, *J*= 7.9 Hz, 1H), 6.92 - 6.61 (m, 2H), 6.12 (d, *J = 7.5* Hz, 1H), 5.49 - 5.18 (m, 2H), 4.80 - 4.60 (m, 1H), 4.52 - 4.35 (m, 2H), 3.99 - 3.94 (m, 3H), 3.85 - 3.76 (m, 4H), 3.54 (dd, *J* = 36.4, 9.4 Hz, 2H), 3.02 - 2.57 (m, 7H), 2.25 (s, 1H), 2.09 (s, 1H), 2.01 - 1.59 (m, 12H), 1.30 - 1.20 (m, 4H).

Example 38

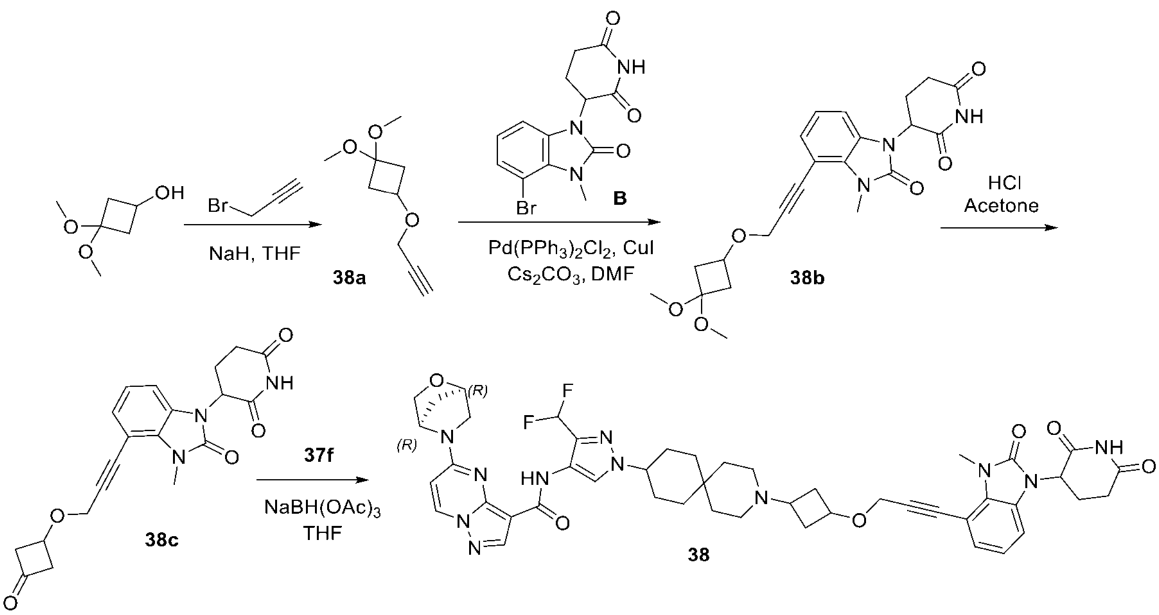


The desired product 38 (1.96 mg) was obtained with reference to the aforementioned examples. MS (ESI) m/z: 892.1 [M+H+]; $^1$H NMR (400 MHz, DMSO-*d$_6$*) δ 11.15 (s, 1H), 10.03 (s, 1H), 9.51 (s, 1H), 8.80 (s, 1H), 8.44 (s, 1H), 8.26 (s, 1H), 7.20 - 6.99 (m, 5H), 6.88 - 6.47 (m, 1H), 5.50 - 5.05 (m, 2H), 4.80 - 4.75 (m, 1H), 4.43 (s, 2H), 4.20 - 4.15 (m, 1H), 3.88 - 3.55 (m, 5H), 2.89 - 2.66 (m, 8H), 2.23 - 2.10 (m, 5H), 2.03 - 1.88 (m, 10H), 1.55 - 1.40 (m, 5H).

Example 39

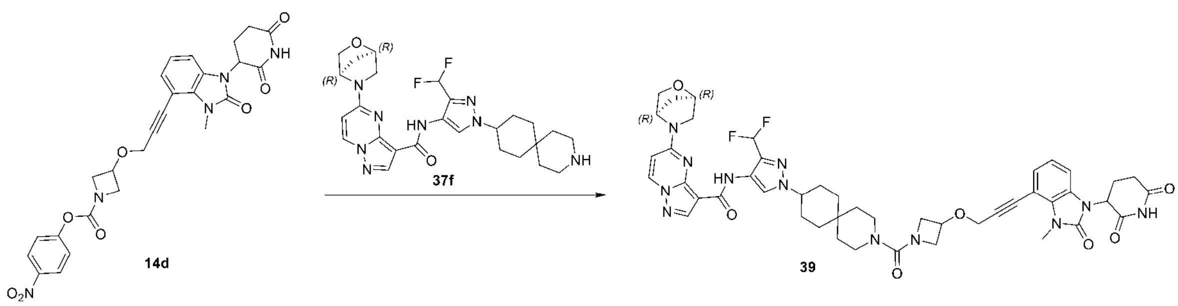


The desired product 39 (1.22 mg) was obtained with reference to the aforementioned examples. MS (ESI) m/z: 921.3 [M+H+]; $^1$H NMR (400 MHz, Methanol-*d$_4$*) δ 9.74 (s, 1H), 8.52 (d, *J* =7.9 Hz, 1H), 8.43 - 8.23 (m, 3H), 7.50 - 7.32 (m, 3H), 7.27 - 7.12 (m, 4H), 7.11 - 6.68 (m, 2H), 6.44 (d, *J*= 8.4 Hz, 1H), 5.46 - 5.29 (m, 2H), 4.73 - 4.45 (m, 3H), 4.27 - 4.17 (m, 2H), 4.00 - 3.94 (m, 3H), 3.83 - 3.63 (m, 6H), 2.95 - 2.75 (m, 6H), 2.23 - 1.85 (m, 11H).

Example 40

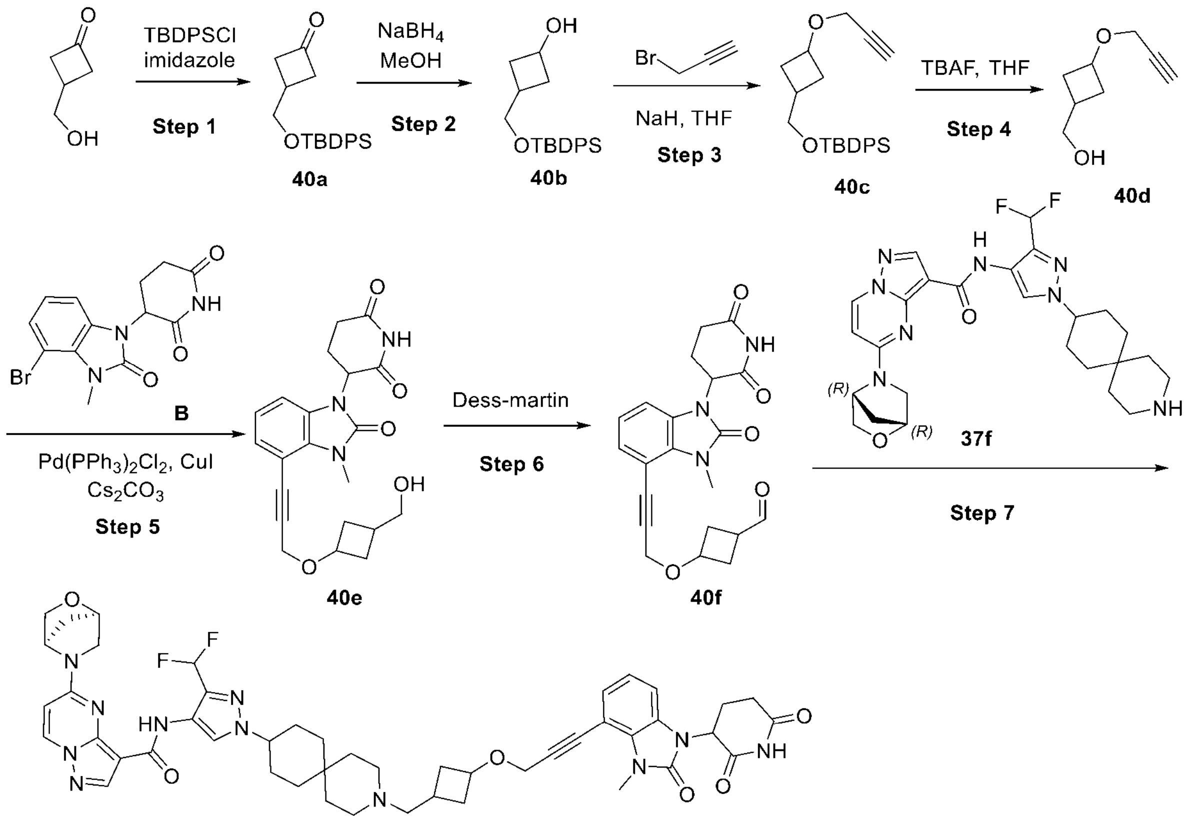


40a 40b 40c 40d 40e 40f

40

The desired product 40 (1.52 mg) was obtained with reference to the aforementioned examples. MS (ESI) M/Z: 906.2 $[M+H]^+$; $^1H$ NMR (400 MHz, Methanol-$d_4$) $\delta$ 8.51 *(d, J* = 7.8 Hz, 1H), 8.41 - 8.26 (m, 3H), 7.19 - 7.12 (m, 3H), 7.10 - 6.97 (m, 2H), 6.87 (d, *J*= 8.5 Hz, 1H), 6.73 (d, *J= 8.0* Hz, 1H), 6.43 (d, *J*= 7.8 Hz, 1H), 5.46 - 5.30 (m, 2H), 4.79 (d, *J*= 7.2 Hz, 1H), 4.38 (d, *J* = 3.9 Hz, 2H), 4.16 - 4.14 (m, 2H), 3.93 (d, *J* = 3.9 Hz, 2H), 3.74 (s, 4H), 3.73 - 3.62 (m, 2H), 3.50 (d, *J*= 9.8 Hz, 1H), 2.82 - 2.79 (m, 8H), 2.58 - 2.53 (m, 2H), 2.23 - 2.13 (m, 3H), 2.07 (d, *J*= 18.9 Hz, 2H), 2.01 - 1.95 (m, 5H), 1.56 (s, 3H).

Example 41

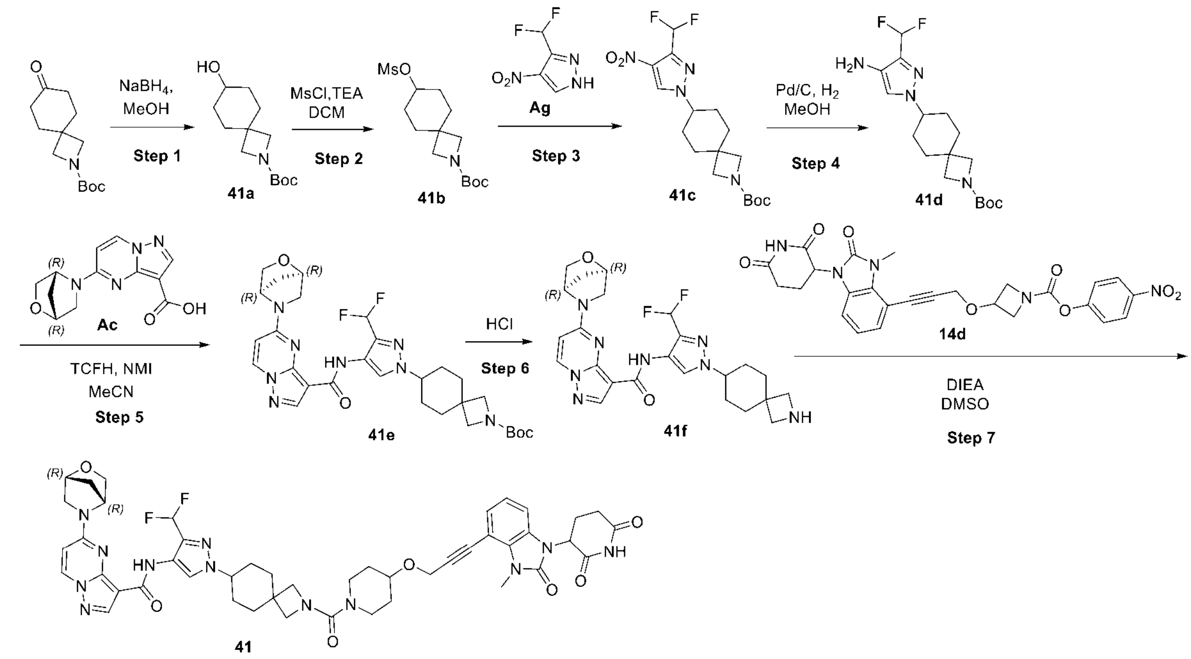


The desired product 41 (2.38 mg) was obtained with reference to the aforementioned examples. MS (ESI) m/z: 921.3 [M+H$^+$]; $^1$H NMR (400 MHz, DMSO-$d_6$) δ 11.13 (s, 1H), 9.50 (d, *J = 6.0* Hz, 1H), 8.78 (d, *J* = 7.7 Hz, 1H), 8.40 (d, *J*= 4.8 Hz, 1H), 8.26 (d, *J*= 5.4 Hz, 1H), 7.27 - 7.02 (m, 4H), 6.88 - 6.43 (m, 1H), 5.44 - 5.36 (m, 1H), 5.28 - 5.06 (m, 1H), 4.77 (d, J = 17.3 Hz, 1H), 4.51 (s, 2H), 4.26 - 4.15 (m, 1H), 3.82 - 3.51 (m, 14H), 3.03 - 2.83 (m, 3H), 2.78 - 2.60 (m, 3H), 2.08 - 1.89 (m, 8H), 1.81 - 1.71 (m, 2H), 1.65 - 1.55 (m, 2H), 1.45 - 1.36 (m, 2H).

Example 42

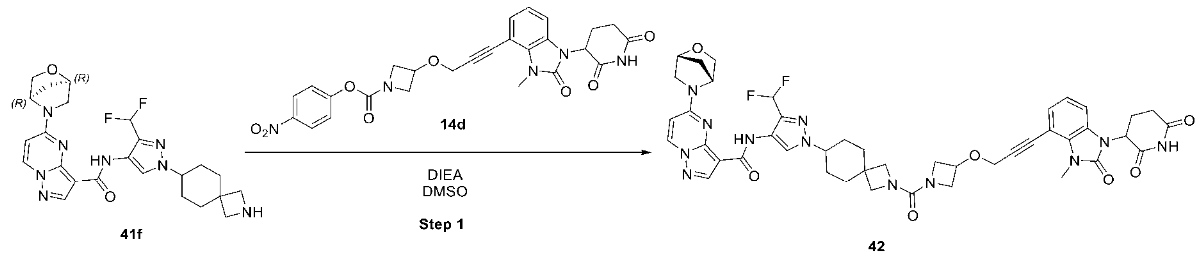


The desired product 42 (24.29 m, formate salt) was obtained with reference to the aforementioned examples. MS (ESI) m/z: 893.2 [M+H$^+$]; $^1$H NMR (400 MHz, DMSO-$d_6$) δ 11.13 (s, 1H), 9.56 - 9.44 (m, 1H), 8.78 (d, *J*= 7.8 Hz, 1H), 8.45 - 8.37 (m, 1H), 8.26 (d, *J*= 5.4 Hz, 1H), 7.27 - 7.02 (m, 4H), 6.89 - 6.43 (m, 1H), 5.45 - 5.36 (m, 1H), 5.28 - 5.07 (m, 1H), 4.77 (d, *J* = 17.4 Hz, 1H), 4.47 (s, 2H), 4.26 - 4.16 (m, 1H), 4.14 - 4.05 (m, 2H), 3.85 - 3.74 (m, 4H), 3.70 - 3.53 (m, 8H), 2.96 - 2.83 (m, 1H), 2.78 - 2.58 (m, 3H), 2.08 - 1.84 (m, 8H), 1.81 - 1.67 (m, 2H), 1.66 - 1.54 (m, 2H).

Example 43

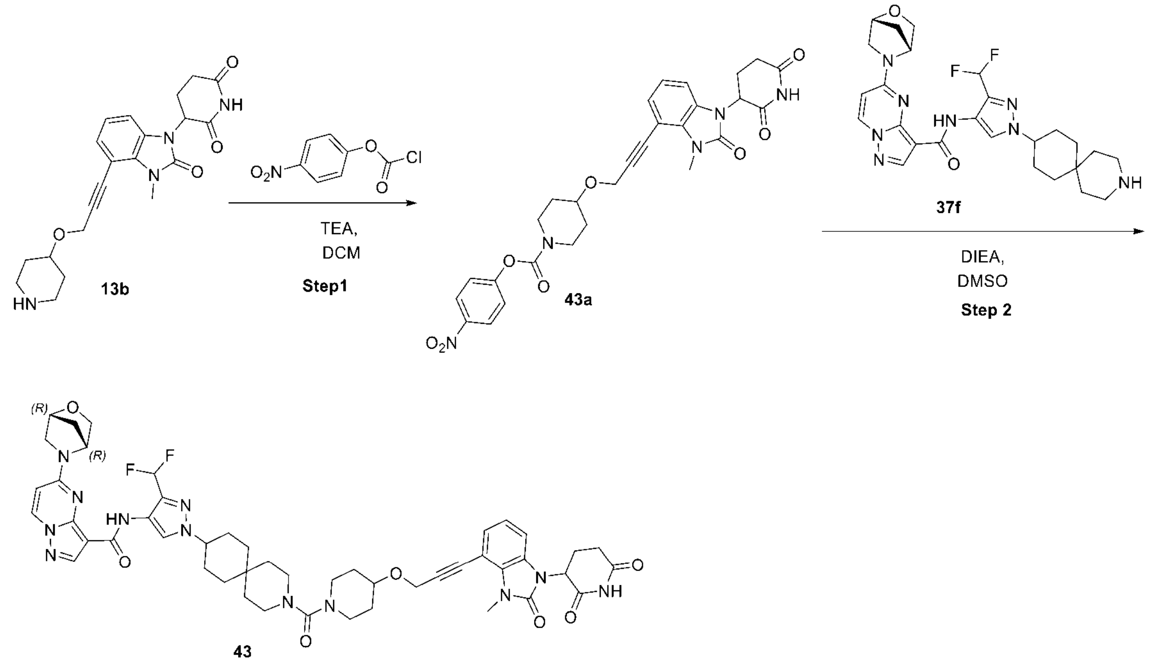


The desired product 43 (4.1 mg) was obtained with reference to the aforementioned examples. MS (ESI) m/z: 949.1 [M+H+]; $^1$H NMR (400 MHz, DMSO-$d_6$) δ 11.13 (s, 1H), 9.50 (d, *J* = 5.9 Hz, 1H), 8.79 (d, *J* = 7.7 Hz, 1H), 8.42 (d, *J* = 4.7 Hz, 1H), 8.26 (d, *J* = 5.7 Hz, 1H), 7.25 - 7.00 (m, 4H), 6.88 - 6.44 (m, 1H), 5.41 (dd, *J*= 12.7, 5.3 Hz, 1H), 5.28 - 5.07 (m, 1H), 4.77 (d, *J* = 16.4 Hz, 1H), 4.50 (s, 2H), 4.22 - 4.18 (m, 1H), 3.86 - 3.79 (m, 1H), 3.74 (d, *J= 7.9* Hz, 1H), 3.65 (s, 3H), 3.59 (s, 1H), 3.45 - 3.30 (m, 4H), 3.14 (s, 4H), 2.93 - 2.86 (m, 3H), 2.80 - 2.58 (m, 3H), 2.11 - 1.94 (m, 4H), 1.94 - 1.75 (m, 8H), 1.65 - 1.20 (m, 6H).

Example 44

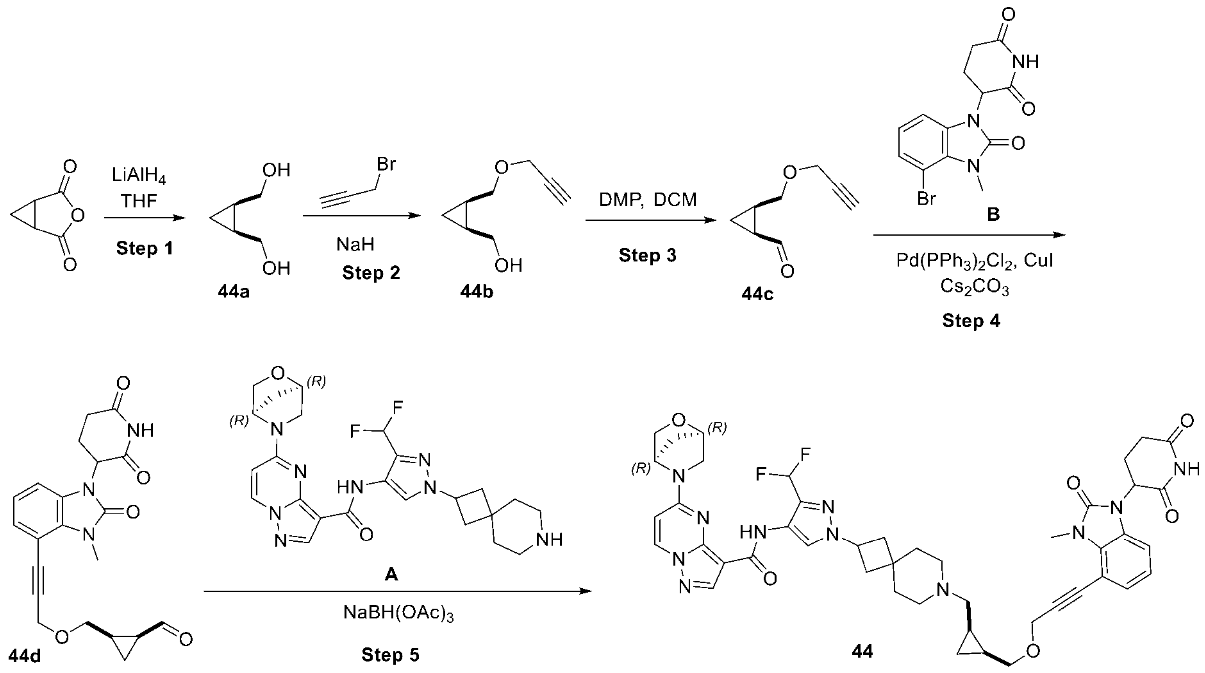


The desired product 44 (11.14 mg) was obtained with reference to the aforementioned examples. MS (ESI) m/z: 878.60 [M+H+]; $^1$H NMR (400 MHz, $CDCl_3$) δ 10.39 (s, 1H), 9.67 (d, *J = 12.4* Hz, 1H), 8.69 - 8.56 (m, 2H), 8.33 (dd, *J* = 7.6,

2.0 Hz, 1H), 7.20 (d, *J* = 8.0 Hz, 1H), 7.00 (s, 1H), 6.89 - 6.62 (m, 2H), 6.12 (d, *J*= 7.6 Hz, 1H), 5.44 (s, 1H), 5.37 - 5.17 (m, 1H), 4.80 (s, 1H), 4.64 - 4.61 (m, 1H), 4.52 - 4.46 (m, 1H), 4.32 - 4.22 (m, 1H), 3.97 (s, 3H), 3.80 (d, *J = 6.8* Hz, 4H), 3.58 (d, *J = 9.6* Hz, 1H), 3.49 (d, *J = 9.6* Hz, 1H), 3.39 - 3.20 (m, 2H), 2.95 - 2.80 (m, 4H), 2.40 - 2.16 (m, 8H), 2.11 - 2.08 (m, 2H), 2.00 - 1.97 (m, 1H), 1.46 - 1.43 (m, 2H), 1.29 - 1.24 (m, 3H), 1.07 - 0.84 (m, 2H).

Example 45

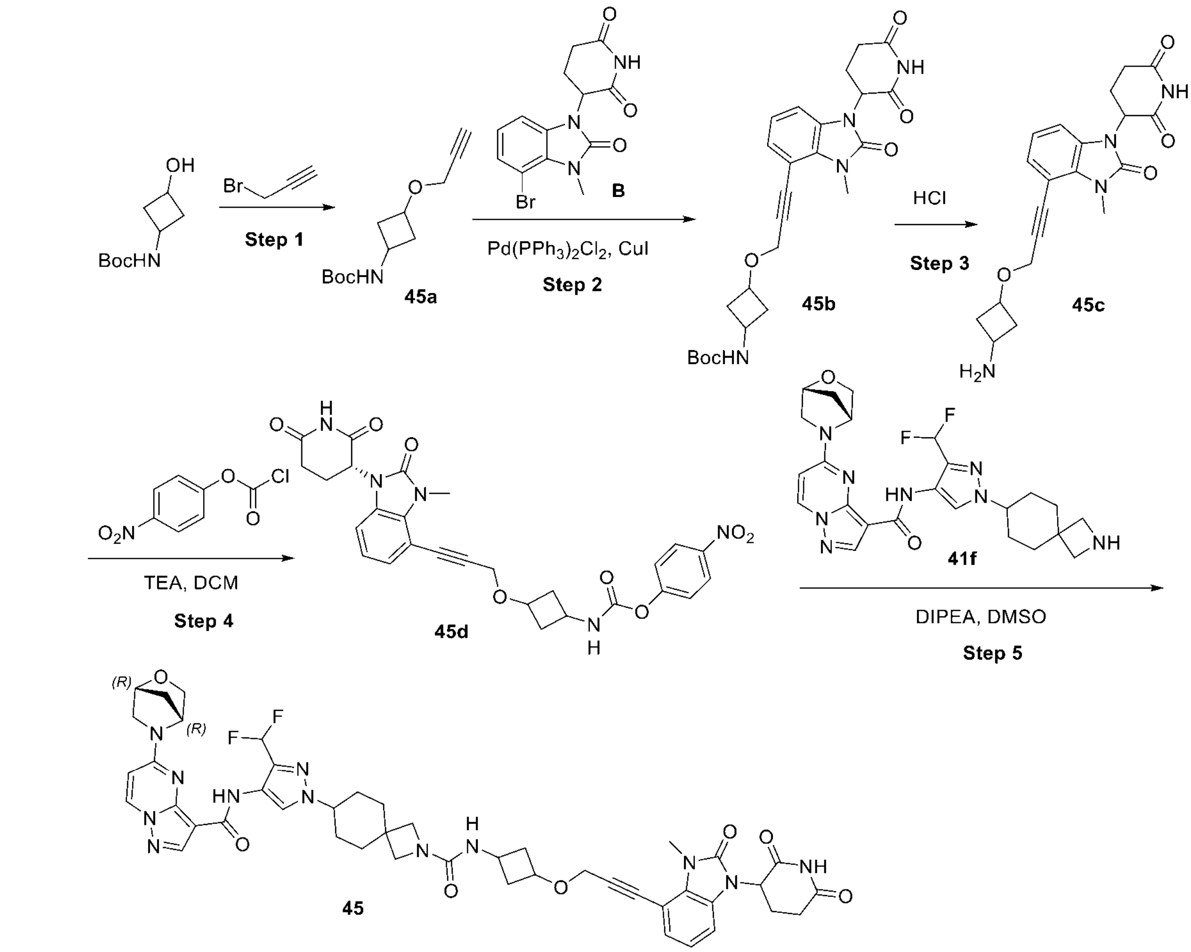


The desired product 45 (2.19 mg, formate salt) was obtained with reference to the aforementioned examples. MS (ESI) m/z: 907.2 [M+H$^+$]; $^1$H NMR (400 MHz, $CDCl_3$) δ 9.60 (s, 1H), 8.42 (s, 2H), 8.31 (s, 1H), 8.21 (s, 1H), 7.17 (d, *J*= 8.0 Hz, 1H), 6.98 (d, *J*= 7.6 Hz, 1H), 6.91 - 6.57 (m, 2H), 6.15 - 6.11 (m, 1H), 5.45 (s, 1H), 5.36 - 5.18 (m, 2H), 4.79 (s, 1H), 4.33 (s, 3H), 4.21 (d, *J* = 7.4 Hz, 1H), 4.05 - 3.95 (m, 3H), 3.77 (s, 3H), 3.69 (s, 2H), 3.65 - 3.45 (m, 4H), 2.95 - 2.72 (m, 4H), 2.50 (s, 1H), 2.25 - 2.20 (m, 2H), 2.15 - 1.95 (m, 7H), 1.85 - 1.15 (m, 6H).

Example 46

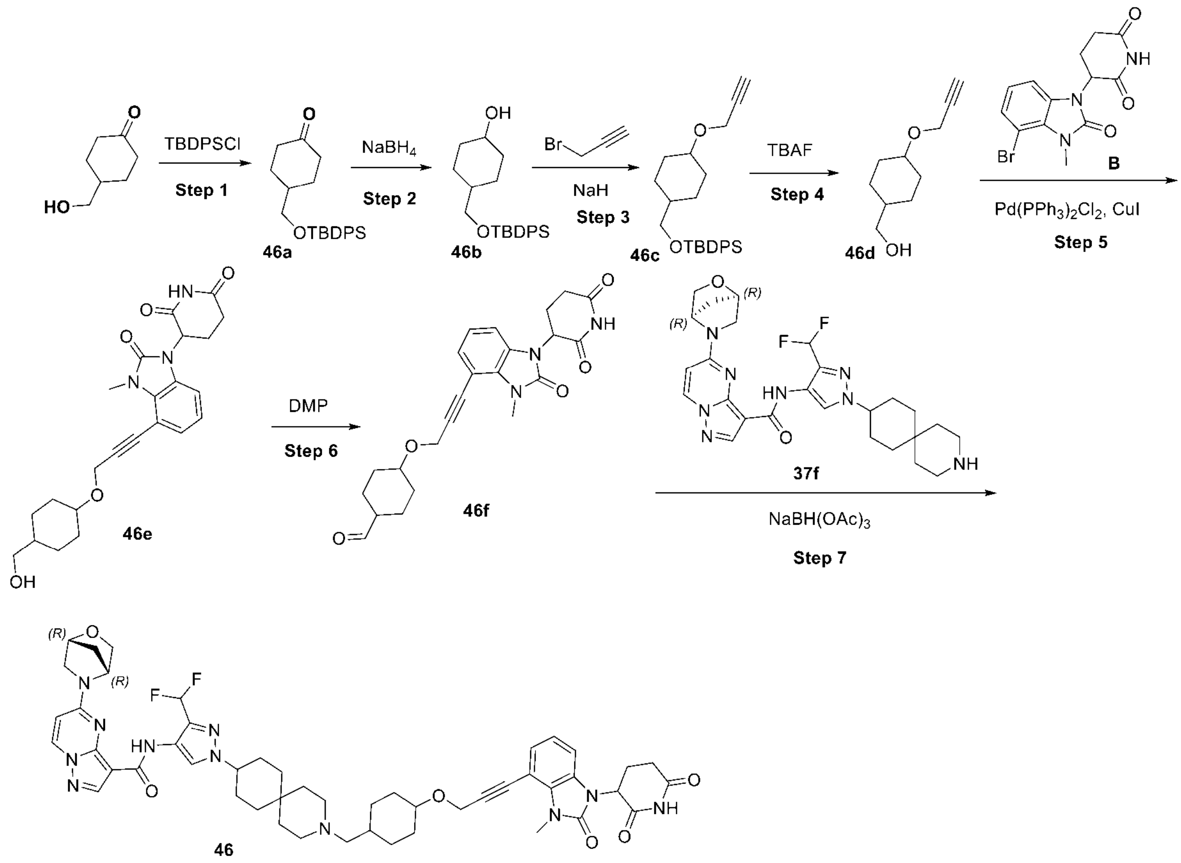


The desired product 46 (14.13 mg) was obtained with reference to the aforementioned examples. MS (ESI) m/z: 934.3 [M+H$^+$]; $^1$H NMR (400 MHz, DMSO-$d_6$) δ 11.12 (s, 1H), 9.49 (d, *J* = 5.4 Hz, 1H), 8.79 (d, *J* = 7.7 Hz, 1H), 8.41(d, *J* = 4.2 Hz, 1H), 8.25 (d, *J = 5.6* Hz, 1H), 7.22 (d, *J* = 26.4 Hz, 1H), 7.17 - 7.11 (m, 2H), 7.05 - 7.02 (m, 1H), 6.88 - 6.44 (m, 1H), 5.47 - 5.34 (m, 1H), 5.28 - 5.08 (m, 1H), 4.77 (d, *J* = 17.9 Hz, 1H), 4.47 (s, 2H), 4.19 (s, 1H), 3.86 - 3.70 (m, 2H), 3.64 (s, 3H), 3.62 - 3.57 (m, 1H), 3.50 - 3.40 (m, 1H), 3.30 - 3.27 (m, 1H), 2.95 - 2.80 (m, 1H), 2.78 - 2.56 (m, 3H), 2.36 - 2.25 (m, 3H), 2.05 - 1.80 (m, 16H), 1.57 - 1.10 (m, 9H).

Example 47

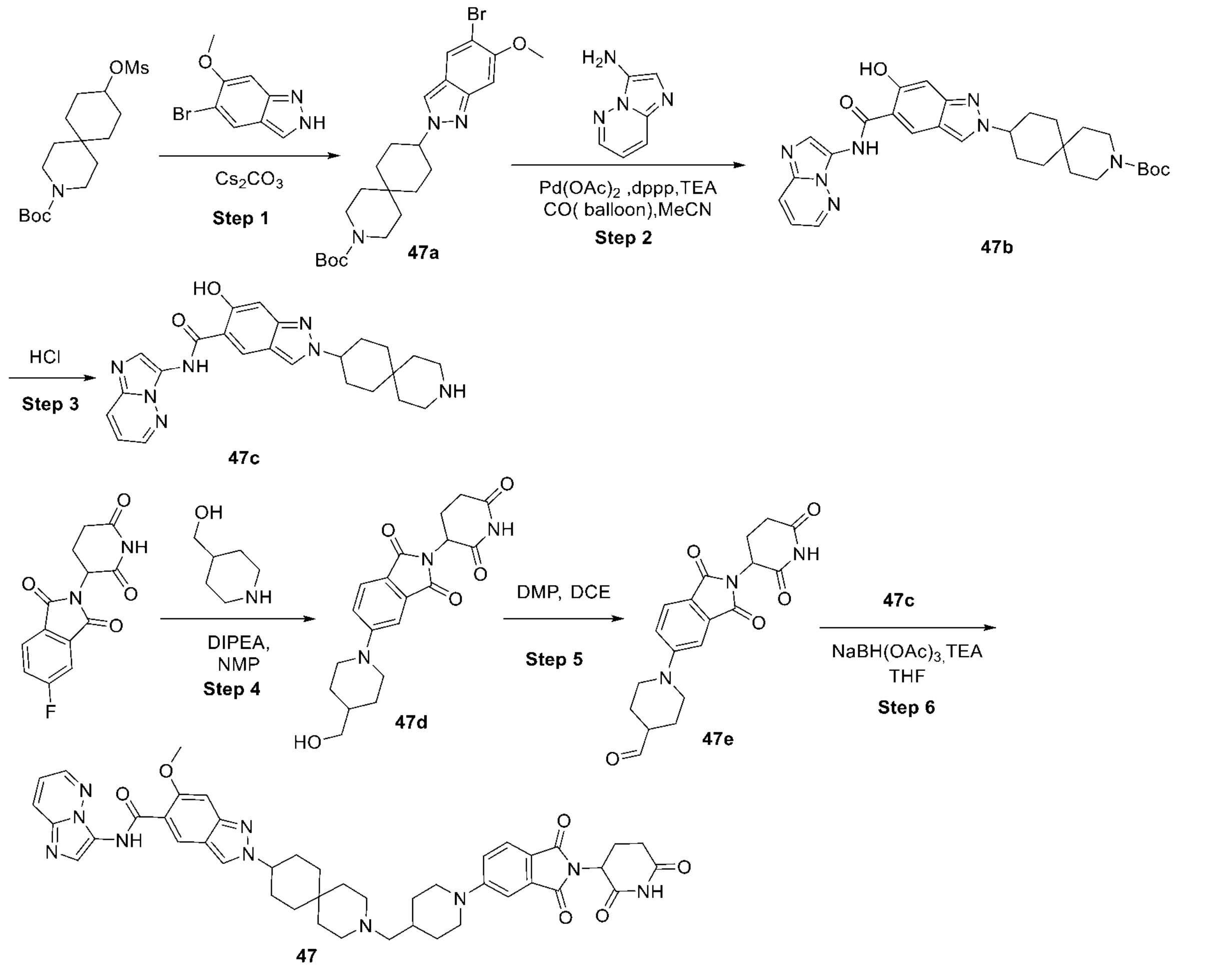


47a

47b

47c

47d

47e

47

The desired product 47 (2.06 mg) was obtained with reference to the aforementioned examples. MS (ESI) m/z: 813.3 [M+H+]; $^1$H NMR (400 MHz, DMSO-$d_6$) δ 11.10 (s, 1H), 11.08 (s, 1H), 9.24 (s, 1H), 8.71 (d, *J* = 3.4 Hz, 1H), 8.65 (s, 1H), 8.60 (s, 1H), 8.22 (d, *J*= *9.2* Hz, 1H), 8.12 (s, 1H), 7.68 (d, *J* = 8.6 Hz, 1H), 7.36 (s, 1H), 7.32 (dd, *J* = 9.3, 4.3 Hz, 1H), 7.26 (s, 2H), 5.07 (dd, *J*= 12.8, 5.2 Hz, 1H), 4.49 (s, 1H), 4.13 (s, 3H), 4.08 (s, 2H), 3.08 - 3.02 (m, 7H), 2.98 - 2.79 (m, 1H), 2.69 - 2.55 (m, 2H), 2.35 - 2.14 (m, 4H), 2.06 - 1.95 (m, 5H), 1.90 - 1.82 (m, 3H), 1.65 - 1.50 (m, 4H), 1.33 - 1.20 (m, 3H).

Example 48

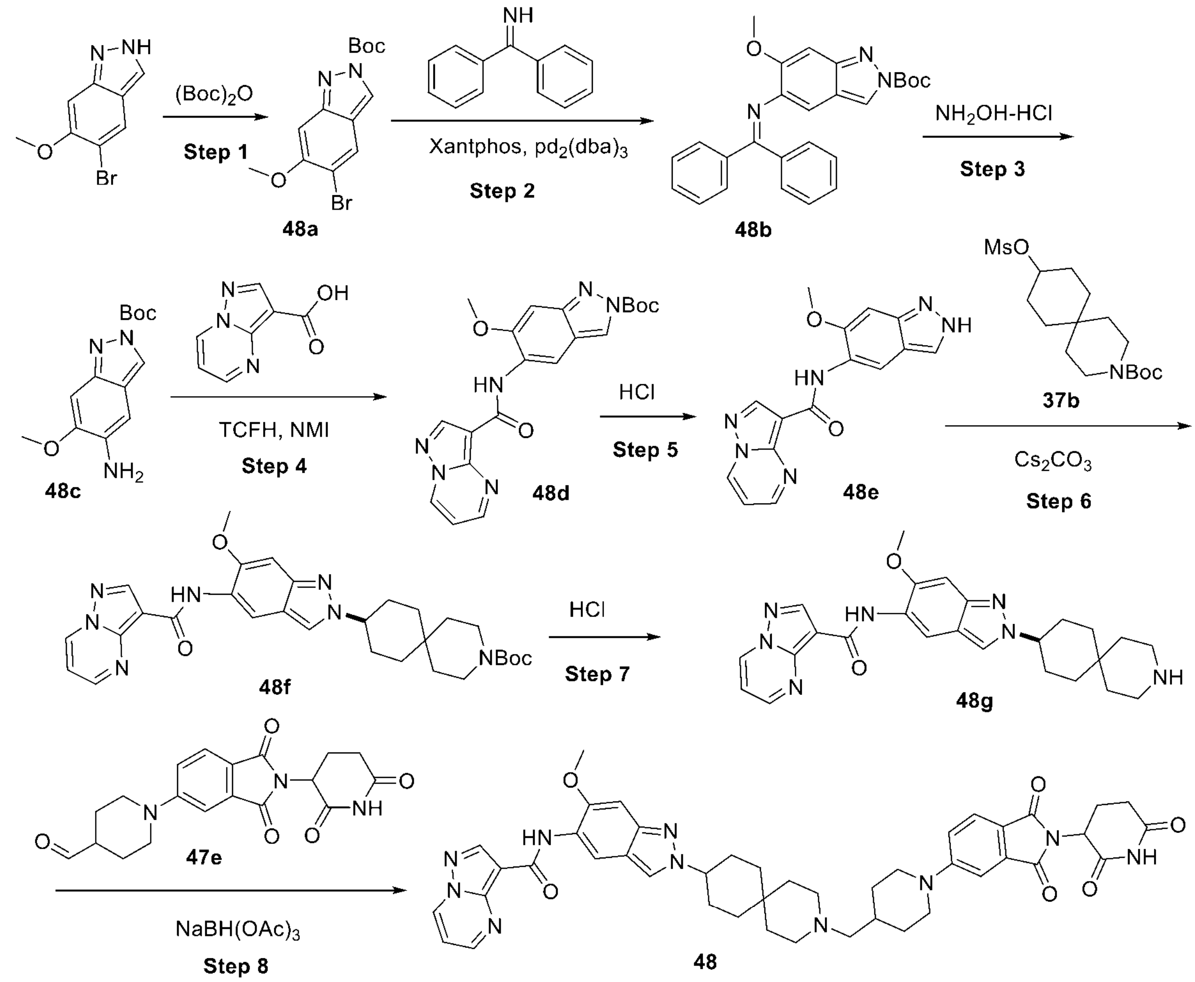


The desired product 48 (2.07 mg) was obtained with reference to the aforementioned examples. MS (ESI) m/z: 813.2 [M+H$^+$]; $^1$H NMR (400 MHz, DMSO-*d$_6$*) δ 11.08 (s, 1H), 10.53 (s, 1H), 9.38 (dd, *J* = 7.0, 1.5 Hz, 1H), 8.96 (dd, *J* = 4.2, 1.5 Hz, 1H), 8.70 (s, 1H), 8.68 (s, 1H), 8.31 (s, 1H), 7.65 (d, *J* = *8.5* Hz, 1H), 7.35 (dd, *J* = *7.0, 4.2* Hz, 1H), 7.31 (s, 1H), 7.23 (d, *J* = 8.6 Hz, 1H), 7.11 (s, 1H), 5.07 (dd, *J* = 12.9, 5.4 Hz, 1H), 4.37 - 4.33 (m, 1H), 4.07 - 4.02 (m, 1H), 4.02 (s, 3H), 3.00 - 2.82 (m, 3H), 2.65 - 2.33 (s, 5H), 2.28 - 2.10 (m, 1H), 2.05 - 1.80 (m, 11H), 1.66 - 1.30 (m, 6H), 1.25 - 1.16 (m, 4H).

Example 49

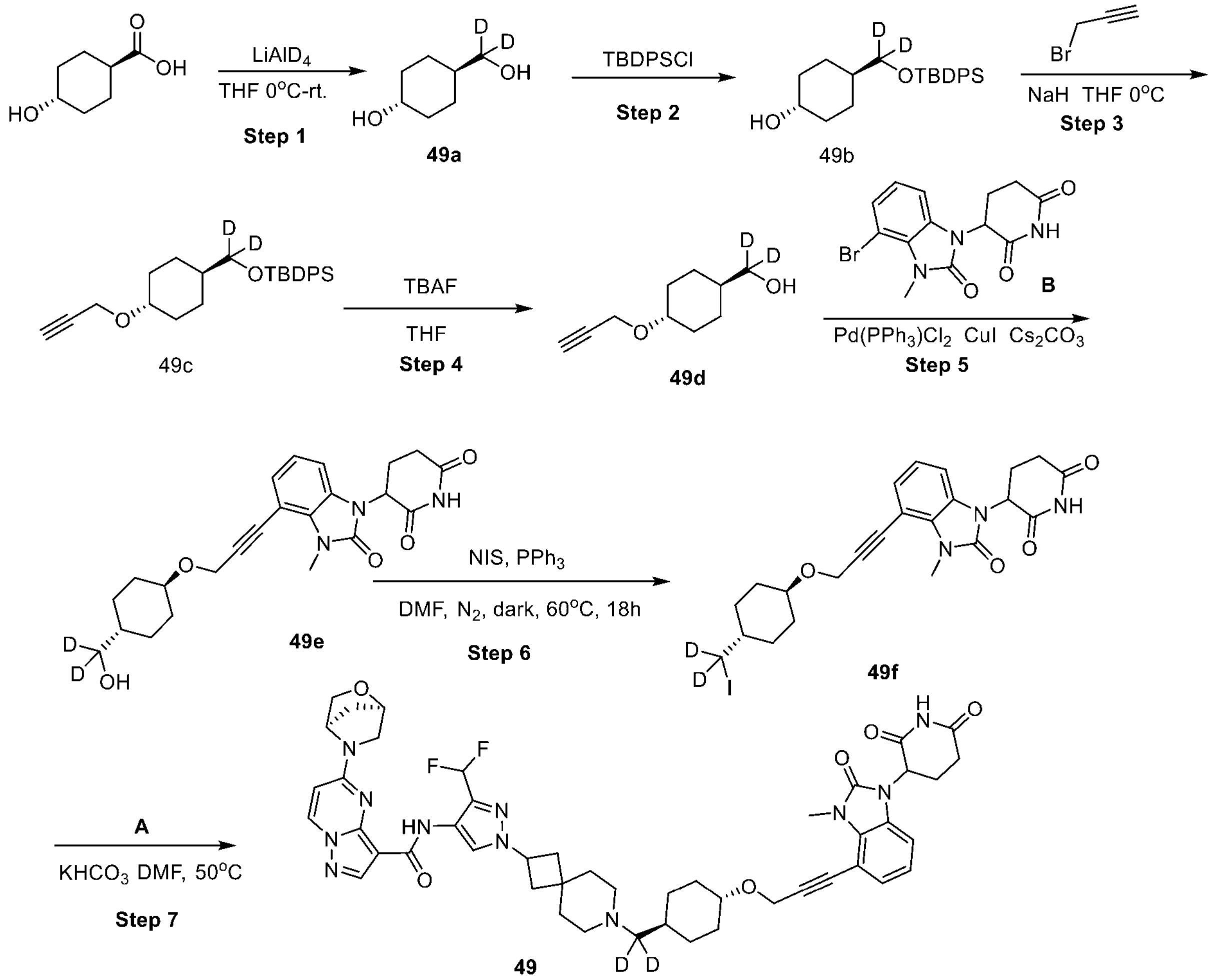


The desired product 49 (23 mg) was obtained with reference to Example 31. MS (ESI) m/z: 908.1 [M+H$^+$]; $^1$H NMR (400 MHz, DMSO) δ 11.13 (s, 1H), 9.50 (d, *J* = 5.2 Hz, 1H), 8.79 (d, *J* = 7.7 Hz, 1H), 8.42 (s, 1H), 8.26 (d, *J* = 5.6 Hz, 1H), 7.31 - 6.96 (m, 4H), 6.87 (d, *J* = *8.0* Hz, 0.5H), 6.46 (d, *J* = 7.7 Hz, 0.5H), 5.40 (dd, *J* = 12.6, 5.3 Hz, 1H), 5.27 (s, 0.5H), 5.08 (s, 0.5H), 4.96 (s, 1H), 4.77 (d, *J* = 18.3 Hz, 1H), 4.48 (s, 2H), 3.86 - 3.71 (m, 2H), 3.66 (s, 3H), 3.63 - 3.56 (m, 1.5H), 3.49 - 3.41 (m, 1.5H), 2.95 - 2.82 (m, 1H), 2.76 - 2.63 (m, 2H), 2.37 - 2.25 (m, 4H), 2.24 - 2.14 (m, 2H), 2.11 - 1.88 (m, 7H), 1.88 - 1.74 (m, 3H), 1.68 - 1.55 (m, 2H), 1.49 - 1.39 (m, 1H), 1.09 - 0.97 (m, 2H), 0.81 - 0.68 (m, 2H).

Example 50

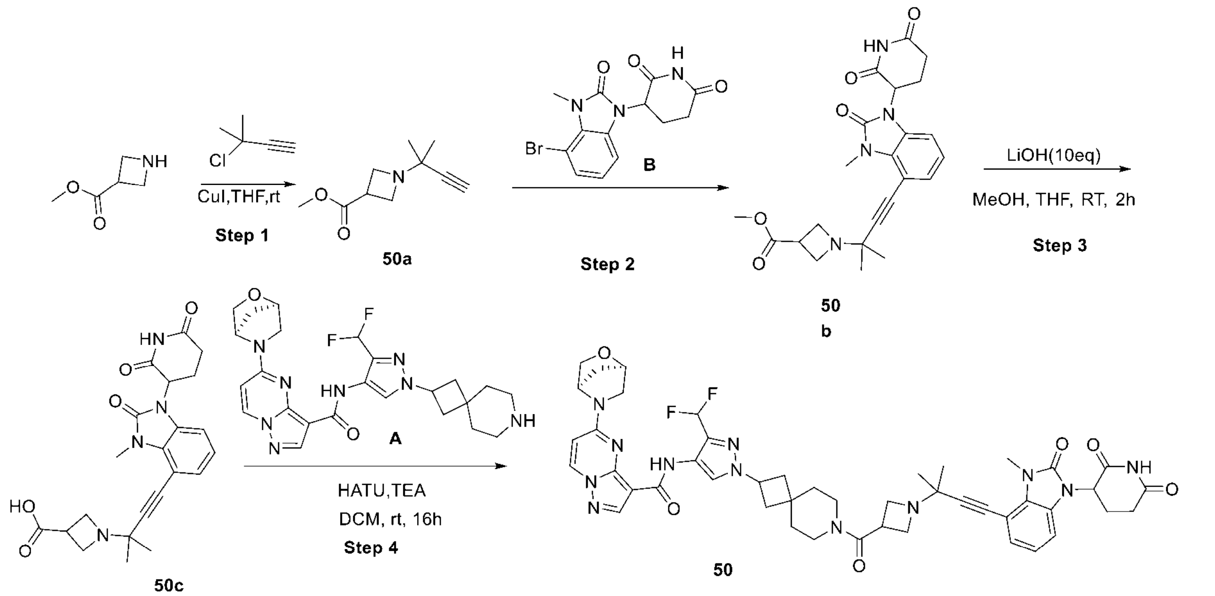


The desired product 50 (6 mg) was obtained with reference to the aforementioned examples. MS (ESI) m/z: 905.6 [M+H+]; $^1$H NMR (400 MHz, DMSO-$d_6$) $\delta$ 11.12 (s, 1H), 9.45 (d, *J=6,* 1H), 8.78 (d, J=7.6, 1H), 8.41 (d, J=2.8, 1H), 8.26 (d, J=5.2, 1H), 7.14 (t, J=6.6, 3H), 7.04-6.86 (m, 2H), 6.46 (d, J=8, 1H), 5.42-5.37 (m, 1H), 5.28 (s, 1H), 5.07(s, 1H), 4.97-4.95 (m, 1H), 4.79-4.75 (m, 1H), 3.81 (s,1H), 3.75-3.73 (m,1H), 3.67-3.59 (m, 5H), 2.89-2.85 (m, 1H), 2.73-2.61 (m, 3H), 2.39-2.33 (m, 3H), 2.23-2.18 (m,2H), 2.05-1.95 (m, 4H), 1.61-1.45 (m, 5H), 1.22 (s, 9H).

Example 51

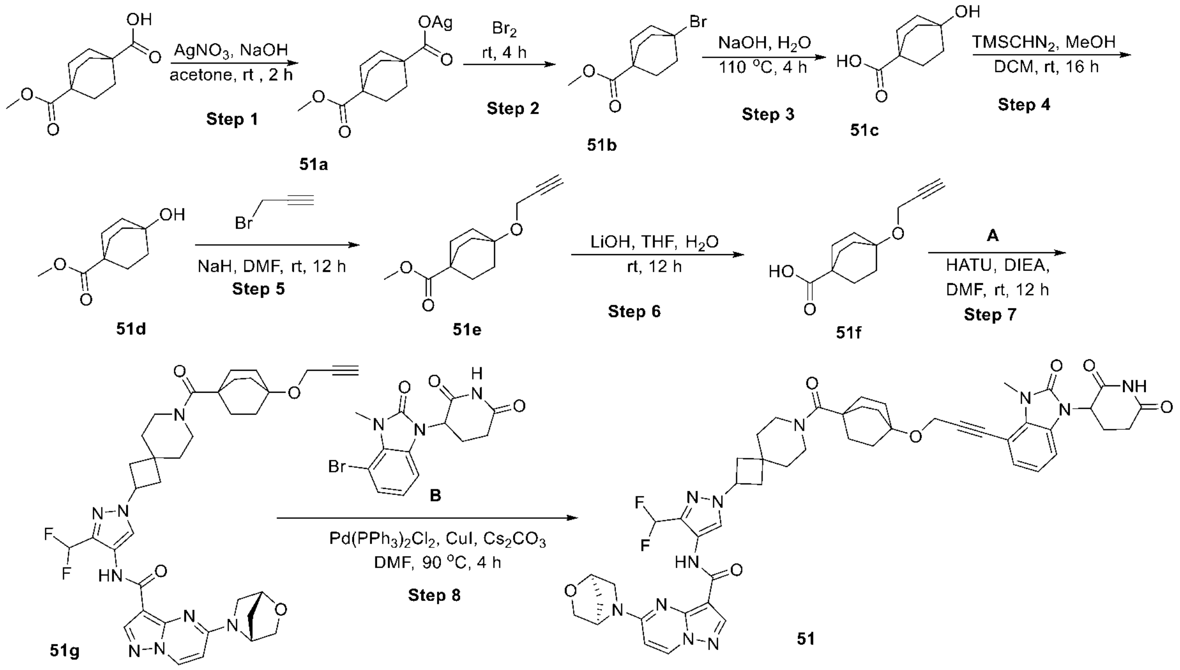


Step 1: 4-(Methoxycarbonyl)bicyclo[2.2.2]octane-1-carboxylic acid (1.22 g, 5.75 mmol) and acetone (10 mL) were added to a reaction flask and stirred, and then 1 M sodium hydroxide solution (5.8 mL, 5.8 mmol) was added dropwise at room temperature. After a clear solution was formed, 4 M aqueous silver nitrate solution (1.5 mL, 6.0 mmol) was added, and the mixture was stirred at room temperature for 2 hours. The mixture was filtered, and the filter cake was washed with water, methyl *tert*-butyl ether, and acetone. The solvent was removed under reduced pressure to obtain 51a (1.82 g).

Step 2: 51a (1.82 g, 5.75 mmol) was added to a reaction flask, which was then purged with $N_2$. n-Hexane (12 mL)

was added, followed by dropwise addition of liquid bromine (920 mg, 5.75 mmol), and the mixture was stirred for 4 hours. The reaction mixture was filtered, and the filtrate was collected. The filter cake was washed with methyl tert-butyl ether (15 mL × 3) and saturated sodium bicarbonate solution (20 mL × 3). The organic phase was washed with brine (20 mL), dried over anhydrous sodium sulfate, and subjected to rotary evaporation until dryness to obtain 51b (909 mg). $^1$H NMR (400 MHz, Chloroform-d) δ 3.63 (s, 3H), 2.29 - 2.19 (m, 6H), 2.00 - 1.93 (m, 6H).

Step 3: 51b (13.3 g, 53.8 mmol), sodium hydroxide (12.9 g, 323 mmol), and water (300 mL) were added to a reaction flask. The reaction was carried out at 110°C for 24 hours. After completion of the reaction, the pH was adjusted to 3-4 with 4 M hydrochloric acid solution, and the mixture was extracted with EA(50 mL × 3). The organic phase was washed with saturated sodium chloride (100 mL), dried over anhydrous sodium sulfate, and concentrated to obtain 51c (4.5 g). $^1$H NMR (400 MHz, DMSO-$d_6$) δ 11.96 (s, 1H), 4.21 (s, 1H), 1.79 - 1.72 (m, 6H), 1.55 - 1.44 (m, 6H).

Step 4: 51c (4.5 g, 26.5 mmol) was added to a reaction flask, which was then purged with nitrogen. Anhydrous dichloromethane (10 mL) and anhydrous methanol (10 mL) were added until the solution became clear. Trimethylsilyldiazomethane (2 M, 53 mmol, 26.5 mL) was added dropwise under a nitrogen atmosphere, and the reaction was carried out overnight at room temperature. After completion of the reaction, the reaction mixture was separated by column chromatography to obtain 51d (2.0 g). $^1$H NMR (400 MHz, Chloroform-d) δ 3.63 (s, 3H), 1.94 - 1.88 (m, 6H), 1.70 - 1.62 (m, 6H).

Step 5: 51d (2.0 g, 10.9 mmol) was added to a reaction flask, which was then purged with nitrogen. Anhydrous DMF (10 mL) and sodium hydride (60%wt, 872 mg, 21.8 mmol) were added, and the reaction was carried out at room temperature for 30 minutes. Subsequently, propargyl bromide (2.59 g, 21.8 mmol) was added, and the reaction was carried out overnight at room temperature. After completion of the reaction, the reaction mixture was quenched by adding water (30 mL) and extracted with EA (30 mL × 4). The organic phase was washed with saturated sodium chloride solution (100 mL), dried over anhydrous sodium sulfate, and separated by column chromatography to obtain 51e (720 mg). $^1$H NMR (400 MHz, Chloroform-d) δ 4.00 (d, *J* = 2.4 Hz, 2H), 3.57 (s, 3H), 2.31 (t, *J* = 2.4 Hz, 1H), 1.91 - 1.81 (m, 6H), 1.71 - 1.62 (m, 6H).

Step 6: 51e (100 mg, 0.45 mmol), LiOH (54 mg, 2.25 mmol), THF (2 mL), and water (0.4 mL) were added to a reaction flask, and the reaction was carried out overnight at room temperature. After completion of the reaction, the pH was adjusted to 3-4 by adding 1 M aqueous hydrochloric acid solution. The mixture was extracted with EA (10 mL × 3). The organic phase was washed with brine (20 mL), dried over anhydrous sodium sulfate, and subjected to rotary evaporation until dryness to obtain 51f (85 mg). $^1$H NMR (400 MHz, DMSO-$d_6$) δ 12.05 (s, 1H), 4.01 (d, *J* = 2.4 Hz, 2H), 3.27 (t, *J* = 2.4 Hz, 1H), 1.83 - 1.74 (m, 6H), 1.66 - 1.57 (m, 6H).

Step 7: 51f(20 mg, 0.096 mmol) was added to a reaction flask, which was then purged with nitrogen. Anhydrous DMF (2 mL) and HATU (40 mg, 0.106 mmol) were added, and the reaction was carried out at room temperature for 30 minutes. A (48 mg, 0.096 mmol) was added, and the reaction was stirred overnight at room temperature. After completion of the reaction, water (10 mL) was added, and the aqueous phase was extracted with EA (10 mL × 3). The organic phase was washed with saturated brine (10 mL × 5), dried over anhydrous sodium sulfate, and subjected to rotary evaporation until dryness to obtain compound 51g (38 mg). $[M+H]^+$= 689.5.

Step 8: B (110 mg, 0.32 mmol), 51g (450 mg, 0.65 mmol), cesium carbonate (212 mg, 0.65 mmol), CuI (12 mg, 0.06 mmol), and $Pd(PPh_3)_2Cl_2$ (46 mg, 0.06 mmol) were dissolved in ultra-dry DMF (3 mL) at room temperature, and the system was thoroughly purged with $N_2$. The mixture was stirred at 90°C for 12 hours. The reaction was monitored by LC-MS. The reaction was quenched with water (5 mL) and extracted with ethyl acetate (5 mL × 3). The organic phases were combined, dried over anhydrous sodium sulfate and purified by preparative TLC to obtain the desired product 51 (4.5 mg). MS (ESI) m/z: 946.7 $[M+H^+]$; $^1$H NMR (400 MHz, DMSO-$d_6$) δ 11.1 (s, 1H), 9.50 (d, *J* = 5.2 Hz, 1H), 8.78 (d, *J* = 8.0 Hz, 1H), 8.41 (d, *J* = 4.0 Hz, 1H), 8.25 (d, *J* = 5.2 Hz, 1H), 7.28 - 7.02 (m, 4H), 6.87 - 6.46 (m, 1H), 5.41 - 5.37 (m, 1H), 5.27 - 5.07 (m, 1H), 4.99 - 4.95 (m, 1H), 4.79 - 4.75 (m, 1H), 4.35 (s, 2H), 3.83 - 3.72 (m, 2H), 3.63 - 3.59 (m, 4H), 3.51 - 3.43 (m, 4H), 2.92 - 2.60 (m, 3H), 2.41 - 2.36 (m, 2H), 2.24 - 2.19 (m, 3H), 2.03 - 1.95 (m, 3H), 1.92 - 1.88 (m, 6H), 1.73 - 1.71 (m, 6H), 1.59 - 1.53 (m, 4H).

Example 52

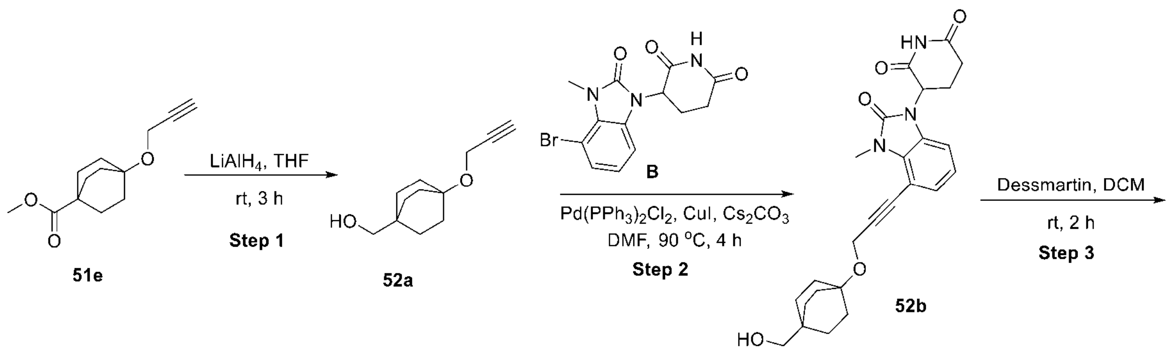


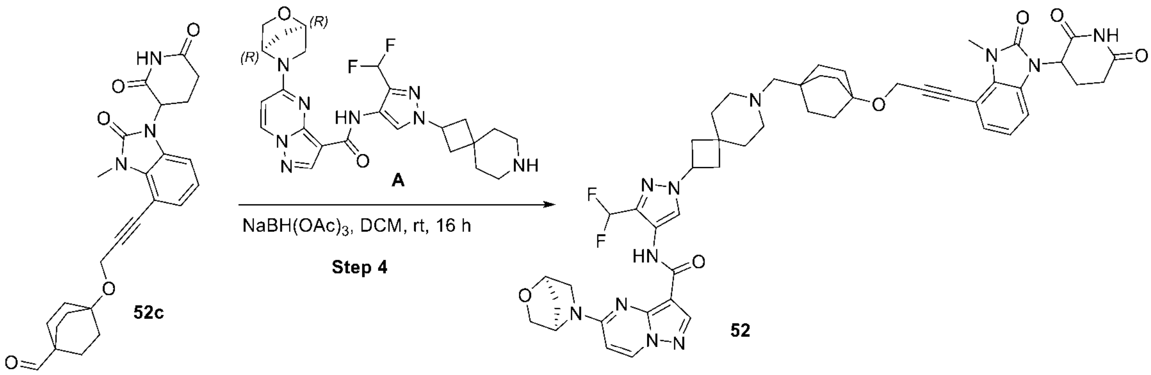


Step 1: 51e (260 mg, 1.17 mmol) was dissolved in ultra-dry THF (4 mL) at room temperature, and lithium aluminum hydride (1.75 mL, 1.75 mmol) was slowly added at 0°C. The reaction mixture was warmed to room temperature and stirred for 3 hours. After completion of the reaction, the reaction mixture was quenched with water and extracted with EA (5 mL × 3). The organic phases were combined, and dried over anhydrous sodium sulfate to obtain 52a (170 mg, 74.9%).

Step 2: 52a (170 mg, 0.87 mmol), B (300 mg, 0.62 mmol), cesium carbonate (395 mg, 1.24 mmol), CuI (24 mg, 0.12 mmol), and $Pd(PPh_3)_2Cl_2$ (89 mg, 0.12 mmol) were dissolved in ultra-dry DMF (5 mL) at room temperature, and the system was thoroughly purged with $N_2$. The mixture was stirred at 90°C for 4 hours. The reaction was monitored by LC-MS. The reaction was quenched with water (5 mL) and extracted with ethyl acetate (5 mL × 3). The organic phases were combined, dried over anhydrous sodium sulfate and purified by preparative TLC to obtain 52b (120 mg). MS (ESI) m/z: 452.3 [M+H$^+$].

Step 3: 52b (120 mg, 0.26 mmol) was dissolved in DCM (3 mL) at room temperature, and Dess-Martin periodinane (124 mg, 0.29 mmol) was added. The mixture was stirred at room temperature for 2 hours. The reaction was monitored by LCMS. After completion of the reaction, the reaction was quenched with water and extracted with DCM (5 mL × 3). The organic phases were combined, dried over anhydrous sodium sulfate, concentrated under reduced pressure, and then purified by preparative TLC to obtain 52c (90 mg, 75.6%). MS (ESI) m/z: 450.1 [M+H]$^+$.

Step 4: 52c (32 mg, 0.07 mmol) and A (32 mg, 0.07 mmol) were dissolved in DCM (3 mL) at room temperature, and $NaBH(OAc)_3$ (18 mg, 0.08 mmol) was added. The mixture was stirred at room temperature for 16 hours. The reaction was monitored by LC-MS. The reaction was quenched with water (5 mL) and extracted with DCM (5 mL × 3). The organic phases were combined, dried over anhydrous sodium sulfate, and purified by preparative TLC to obtain a crude product (30 mg). The crude product was further purified by high-pressure preparative liquid chromatography to obtain the desired product 52 (13.8 mg). MS (ESI) m/z: 932.8 [M+H$^+$]; $^1$H NMR (400 MHz, DMSO-*d*$_6$) δ 11.17 (s, 1H), 9.55 (d, *J* = 5.2 Hz, 1H), 8.82 (d, *J* = 7.6 Hz, 1H), 8.43 (d, *J* = 4.0 Hz, 1H), 8.31 (d, *J* = 5.6 Hz, 1H), 7.54 - 7.06 (m, 4H), 6.92 - 6.49 (m, 1H), 5.47 - 5.43 (m, 1H), 5.33 - 5.12 (m, 1H), 4.99 - 4.92 (m, 1H), 4.84 - 4.80 (m, 1H), 4.38 (s, 2H), 3.88 - 3.80 (m, 2H), 3.69 - 3.65 (m, 5H), 3.50 - 3.48 (m, 1H), 2.98 - 2.90 (m, 1H), 2.81 - 2.67 (m, 2H), 2.44 - 2.29 (m, 6H), 2.23 - 1.93 (m, 8H), 1.72 - 1.64 (m, 7H), 1.59 - 1.47 (m, 7H).

Example 53

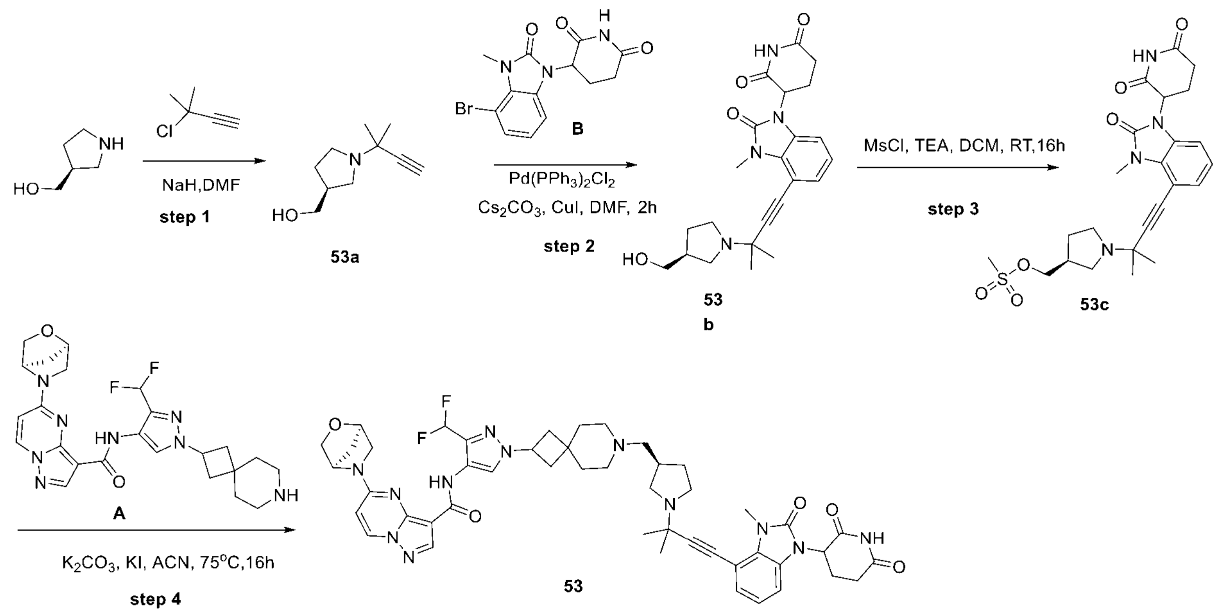


The desired product 53 (6.4 mg) was obtained with reference to the aforementioned examples. MS (ESI) m/z: 905.7 [M+H+]; $^1$H NMR (400 MHz, Chloroform-d) δ 9.63 (s, 1H), 8.54 (d, J = 9.2 Hz, 2H), 8.33 (s, 1H), 7.14 (d, J = 8.1 Hz, 1H), 7.02 - 6.94 (m, 1H), 6.76 (d, J = 7.6 Hz, 1H), 6.13 (s, 1H), 5.51 (s, 1H), 5.20 (d, J = 40.7 Hz, 1H), 4.80 (d, J = 10.5 Hz, 1H), 4.70 (t, J = 8.5 Hz, 1H), 3.96 (d, J = 7.4 Hz, 2H), 3.91 - 3.70 (m, 4H), 3.54 (dd, J = 33.2, 14.8 Hz, 2H), 3.18 - 2.57 (m, 8H), 2.53 - 1.89 (m, 17H), 1.64 (s, 6H).

Example 54

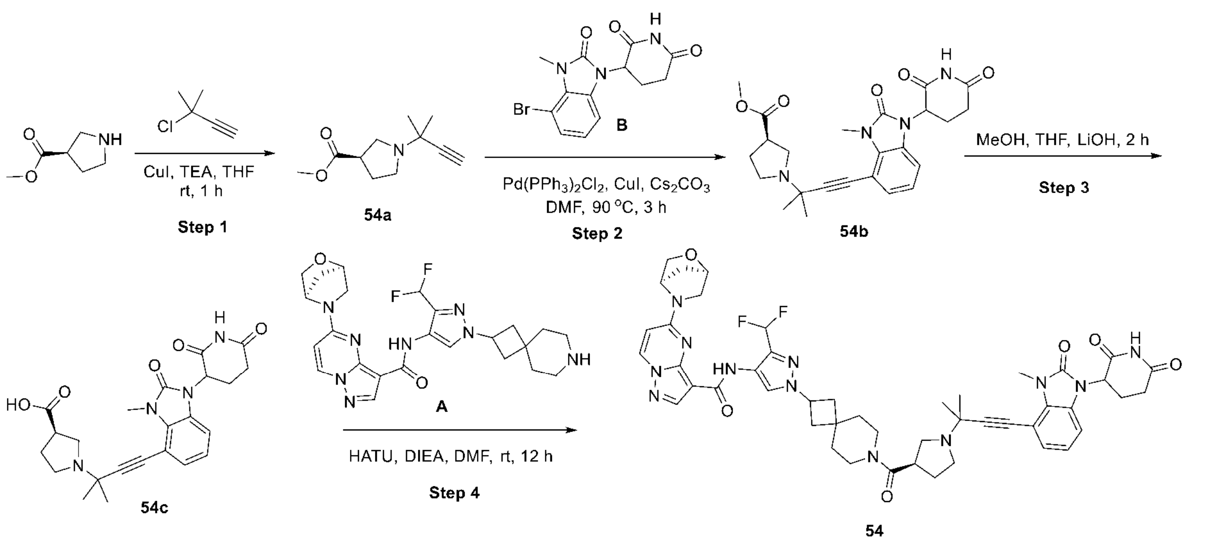


The desired product 54 (2.5 mg) was obtained with reference to the aforementioned examples. MS (ESI) m/z: 919.6 [M+H+]; $^1$H NMR (400 MHz, $CDCl_3$) δ: 9.46 (s, 1H), 8.53 - 8.41 (m, 2H), 8.35 - 8.24 (m, 1H), 7.11 - 7.08 (m, 1H), 6.93 - 6.87 (m, 1H), 6.71 - 6.65 (m, 1H), 6.05 (d, *J* = 2.8 Hz, 1H), 5.38 (s, 1H), 5.17 - 5.03 (m, 1H), 4.76 - 4,60 (m, 2H), 3.95 - 3.86 (m, 2H), 3.72 (s, 3H), 3.57 - 3.37 (m, 4H), 3.31 - 3.04 (m, 4H), 2.96 - 2.83 (m, 5H), 2.81 - 2.61 (m, 2H), 2.45 - 2.15 (m, 7H), 2.06 - 1.91 (m, 5H), 1.47 - 1.42 (m, 6H).

Example 55

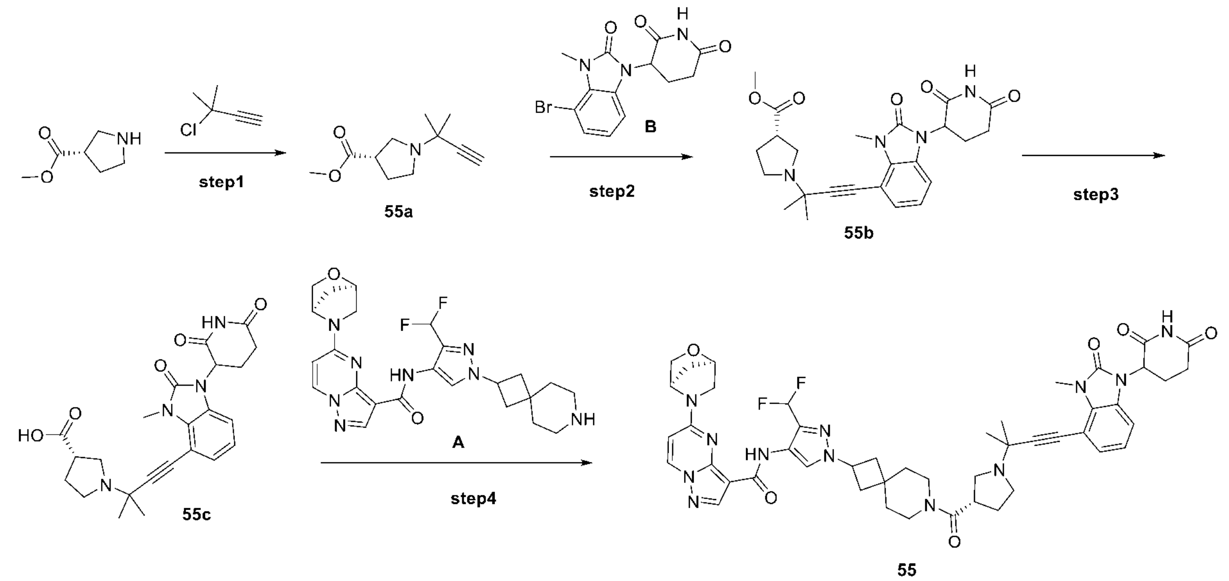


55a 55b 55c 55

The desired product 55 (16.1 mg) was obtained with reference to the aforementioned examples. MS (ESI) m/z: 919.7 [M+H+]; $^1$H NMR (400 MHz, Chloroform-d) δ 9.56 (s, 1H), 8.57 - 8.35 (m, 2H), 8.25 (d, *J* = 7.6 Hz, 1H), 7.10 (d, *J* = 7.5 Hz, 1H), 6.95 - 6.87 (m, 1H), 6.70 (d, *J = 7.8* Hz, 1H), 6.06 (d, *J = 7.7* Hz, 1H), 5.37 (s, 1H), 5.18 (d, *J* = 40.4 Hz, 1H), 4.76 - 4.52 (m, 2H), 3.90 (s, 2H), 3.72 (s, 3H), 3.54 - 3.40 (m, 3H), 3.29 (s, 1H), 3.16 - 3.07 (m, 6H), 3.00 - 2.72 (m, 6H), 2.44 - 1.88 (m, 11H), 1.45 (s, 6H).

Example 56

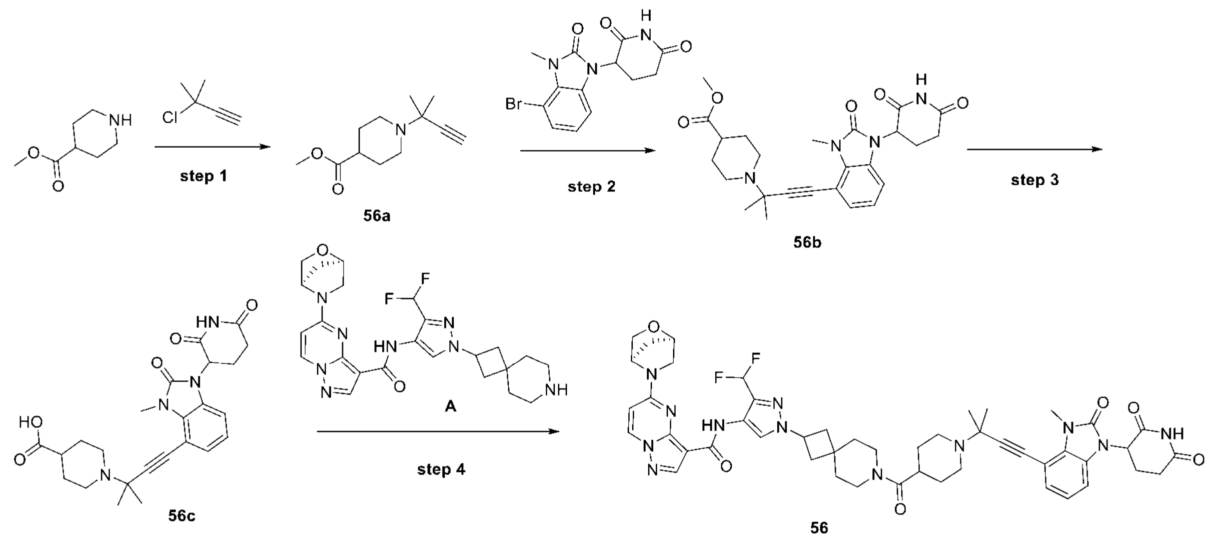


56a 56b 56c 56

Step 1: Methyl 4-piperidinecarboxylate (0.5 g, 3.49 mmol) was dissolved in tetrahydrofuran (5 mL) at room temperature, and 3-chloro-3-methyl-1-butyne (0.53 g, 5.2 mmol) and TEA (881 mg, 8.73 mmol) were added, and then the mixture was stirred for 10 minutes. CuI (66 mg, 0.349 mmol) was added to the system, and the mixture was stirred for 0.5 hours. Then, 40 mL of water and 80 mL of ethyl acetate were added, and the mixture was extracted three times. The organic phase was collected and dried over anhydrous sodium sulfate. After rotary evaporation until dryness, product 56a (470 mg) was obtained. $^1$H NMR (400 MHz, Chloroform-d) δ 3.61 (s, 3H), 2.99 (dt, *J*= 11.7, 3.6 Hz, 2H), 2.28 - 2.07 (m, 4H), 1.68 (qd, *J*= 11.6, 3.7 Hz, 2H), 1.33 (s, 6H).

Step 2: 56a (100 mg, 0.48 mmol), B (100 mg, 0.29 mmol), $Pd(PPh_3)_2Cl_2$ (37 mg, 0.05 mmol), CuI (10 mg, 0.05 mmol), and cesium carbonate (360 mg, 1.1 mmol) were dissolved in DMF (2 mL) at room temperature, and the reaction mixture was stirred at 90°C for 1 hour. After completion of the reaction, the reaction mixture was filtered through diatomite to

obtain a crude product, which was purified by preparative TLC to obtain the product 56b (90 mg). MS (ESI) m/z: 467.2 [M+H+].

Step 3: 56b (47 mg, 0.1 mmol) was dissolved in a mixed solvent of THF/MeOH = 1/1 (6 mL) at room temperature, and LiOH (36 mg, 1.5 mmol) was added. The reaction mixture was stirred at 40°C for 1 hour, then 40 mL of dichloromethane was added. After filtration to remove excess salts, the product was purified by preparative TLC to obtain 56c (25 mg). MS (ESI) m/z: 453.3 [M+H+].

Step 4: 56c (20 mg, 0.044 mmol) and HATU (18.5 mg, 0.048 mmol) were added to 0.5 mL of dichloromethane at room temperature, and the mixture was stirred at room temperature for 5 minutes. Then, A (22 mg, 0.044 mmol) and TEA (13 mg, 0.13 mmol) were dissolved in dichloromethane (0.5 mL) and added to the reaction system, which was stirred at 40°C for 2 hours. After completion of the reaction, the reaction mixture was purified by preparative TLC to obtain the desired product 56 (13.3 mg). MS (ESI) m/z: 933.6 [M+H+]; $^1$H NMR (400 MHz, Chloroform-d) δ 9.53 (s, 1H), 8.50 (s, 1H), 8.35 (d, *J*= 10.0 Hz, 1H), 8.24 (s, 1H), 7.11 (s, 1H), 6.92 (s, 1H), 6.69 (s, 1H), 6.06 (s, 1H), 5.37 (s, 1H), 5.13 (s, 1H), 4.69 (d, *J*= 21.9 Hz, 2H), 3.90 (s, 2H), 3.71 (s, 3H), 3.47 (d, *J*= 38.1 Hz, 4H), 3.27 (s, 1H), 3.19 (s, 2H), 2.76 (d, *J*= 69.2 Hz, 4H), 2.34 (d, *J*= 37.0 Hz, 8H), 1.83 (d, *J*= 80.8 Hz, 10H), 1.43 (s, 6H).

Example 57

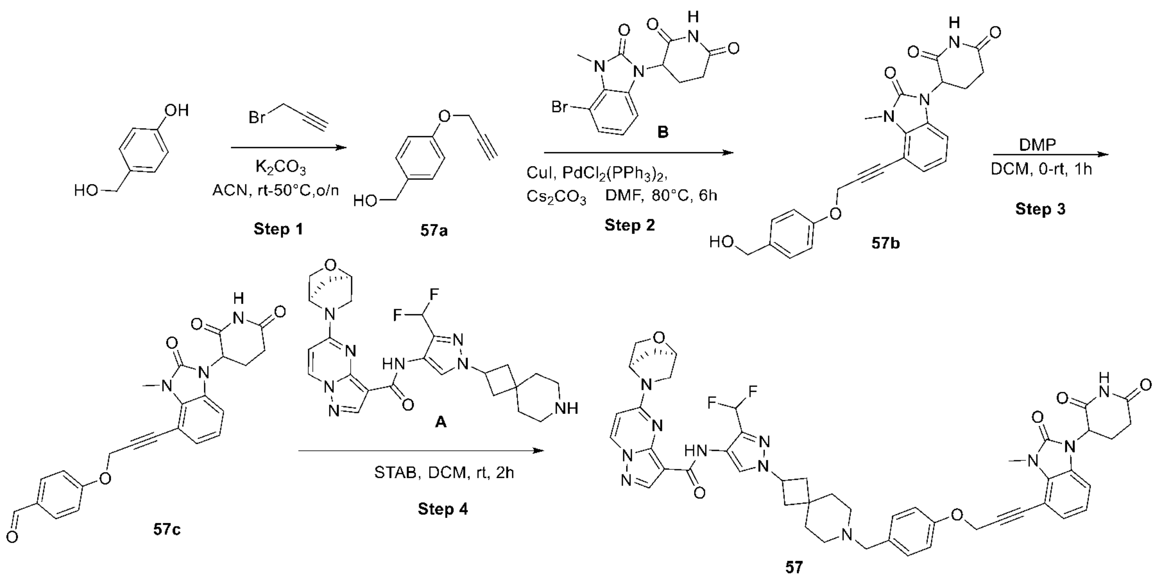


57

The desired product 57 (2.5 mg) was obtained with reference to the aforementioned examples. MS (ESI) m/z: 900.5[M+H+]; $^1$H NMR (400 MHz, DMSO-d6) δ 11.11 (s, 1H), 9.49 (d, *J*= 5.2 Hz, 1H), 8.78 (d, *J* = 7.6 Hz, 1H), 8.39 (d, *J*= *4.0* Hz, 1H), 8.25 (d, *J* = *5.2* Hz, 1H), 7.24 (d, *J* = 8.4 Hz, 2H), 7.14 (dd, *J* = 26.4, 7.8 Hz, 2H), 7.02 (dt, *J* = 7.6, 3.6 Hz, 3H), 6.86 (d, *J* = 7.8 Hz, 1H), 6.45 (d, *J* = 7.6 Hz, 1H), 5.38 (dd, *J* = 12.8, 5.4 Hz, 1H), 5.27 (s, 1H), 5.11 (d, *J* = 7.4 Hz, 2H), 5.07 (s, 1H), 4.98 - 4.88 (m, 1H), 4.77 (d, *J* = 17.2 Hz, 1H), 3.82 (d, *J*= *10.4* Hz, 2H), 3.74 (d, *J* = 7.6 Hz, 1H), 3.66 - 3.56 (m, 2H), 3.45 (s, 2H), 3.39 - 3.36 (m, 2H), 2.87 (t, *J* = 14.8 Hz, 1H), 2.68 - 2.59 (m, 2H), 2.33 (d, *J* = 8.8 Hz, 4H), 2.16 (d, *J* = 19.6 Hz, 3H), 1.99 (dd, *J* = 20.8, 10.3 Hz, 3H), 1.62 (d, *J* = *23.2* Hz, 4H), 1.23 (s, 2H).

Example 58

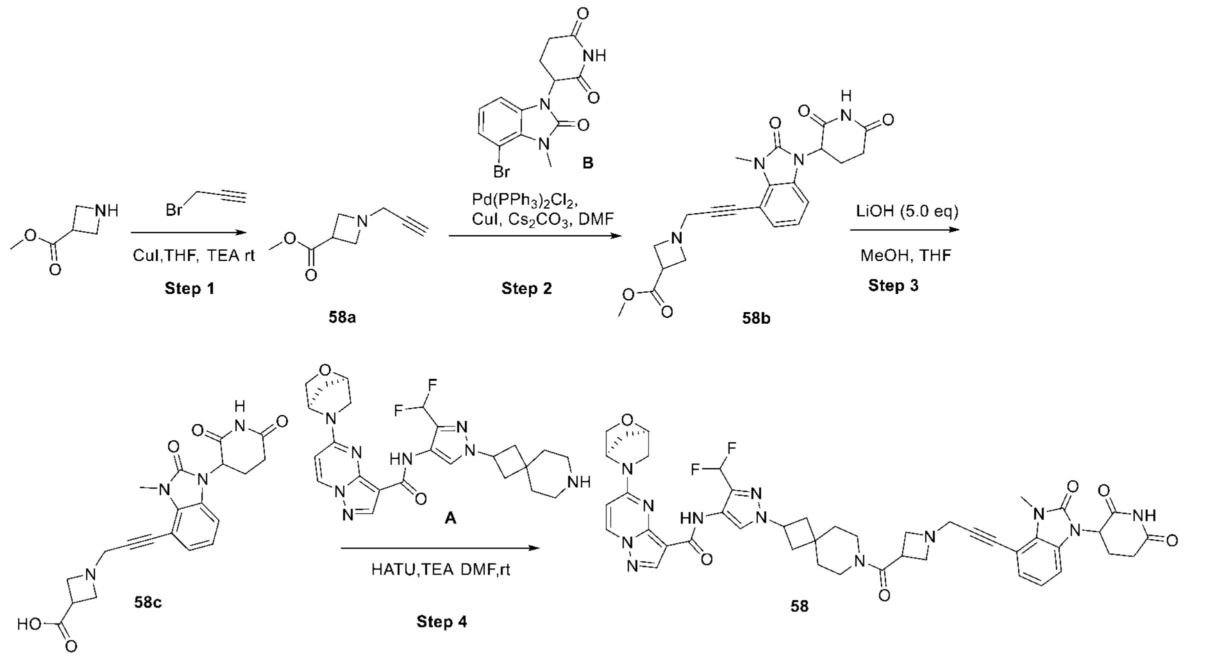


The desired product 58 (1.8 mg) was obtained with reference to the aforementioned examples. MS (ESI) m/z: 877.7 [M+H+]; $^1$H NMR (400 MHz, DMSO-*d$_6$*) δ 11.12 (s, 1H), 9.51 (d, *J*= 5.6 Hz, 1H), 8.79 (d, *J* = 7.8 Hz, 1H), 8.41 (d, *J* = 4.2 Hz, 1H), 8.26 (d, *J* = 5.6 Hz, 1H), 7.15 (dd, *J*= 13.2, 8.0 Hz, 2H), 7.03 (t, *J*= 7.8 Hz, 1H), 6.87 (d, *J*= 8.0 Hz, 1H), 6.46 (d, *J = 8.0* Hz, 1H), 5.40 (dd, *J*= 12.4, 5.6 Hz, 1H), 4.98 (t, *J*= 8.4 Hz, 1H), 4.78 (d, *J*= 17.2 Hz, 1H), 3.82 (d, *J = 9.6* Hz, 1H), 3.67 (s, 2H), 3.60 (s, 1H), 3.49 (d, *J*= 19.2 Hz, 4H), 3.17 (s, 2H), 2.88 (d, *J* = 17.2 Hz, 2H), 2.80 - 2.70 (m, 1H), 2.61 (s, 1H), 2.39 (t, *J* = 10.0 Hz, 2H), 2.33 (t, *J= 2.0* Hz, 1H), 2.22 (t, *J*= 10.0 Hz, 2H), 2.00 (dd, *J*= 19.6, 10.1 Hz, 3H), 1.59 (d, *J*= 18.0 Hz, 2H), 1.51 (s, 1H), 1.25 (d, *J*= 10.7 Hz, 6H), 0.86 (s, 1H).

Example 59

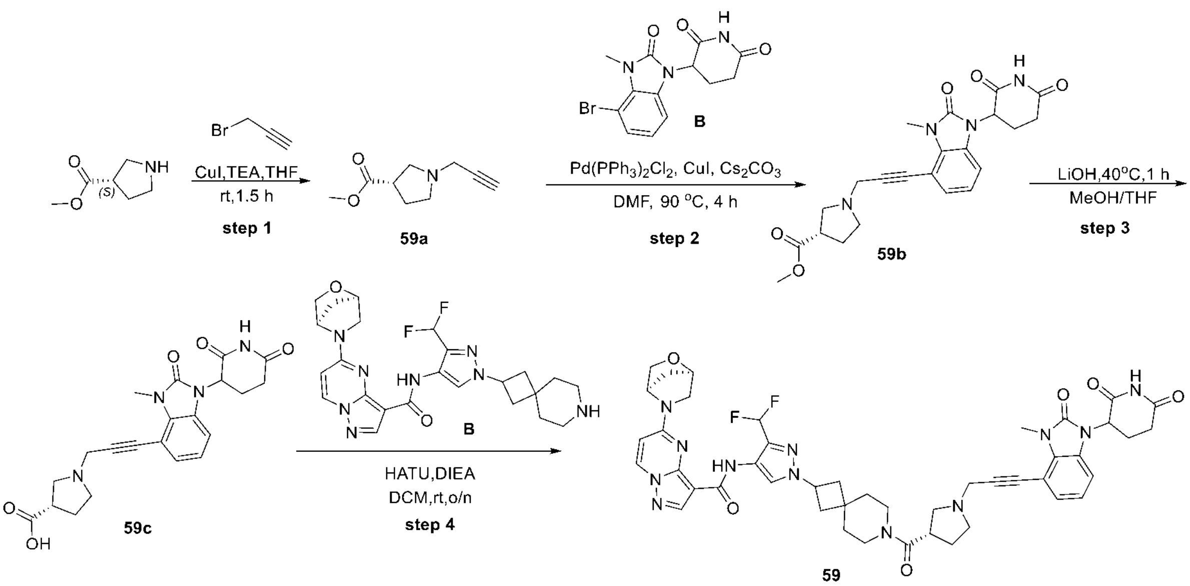


The desired product 59 (5.3 mg) was obtained with reference to the aforementioned examples. MS (ESI) m/z: 891.6 [M+H+]; $^1$H NMR (400 MHz, $CDCl_3$) δ 9.55 (s, 1H), 8.47 - 8.39 (m, 2H), 8.27 - 8.25 (m, 1H), 7.13 - 7.11 (m, 1H), 6.92 (t, *J*= 8 Hz, 1H), 6.84 - 6.57 (m, 2H), 6.05 (d, *J* = 7.6 Hz, 1H), 5.38 (s, 1H), 5.16 - 5.12 (m, 1H), 4.76 - 4.66 (m, 2H), 3.93-3.89 (m, 2H), 3.82 - 3.75 (m, 5H), 3.56 - 3.50 (m, 2H), 3.44 - 3.29 (m, 5H), 3.10 - 2.97 (m, 2H), 2.90 - 2.64 (m, 4H), 2.36 - 2.24 (m, 4H),

2.18 - 2.08 (m, 4H), 2.05 - 1.98 (m, 5H).

Example 60

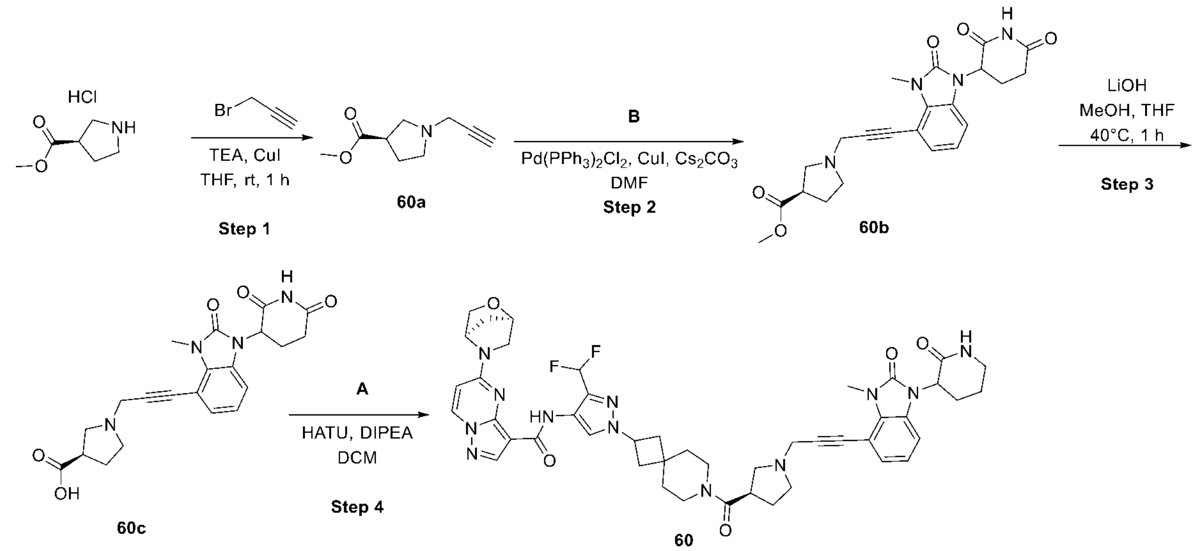


The desired product 60 (8.0 mg) was obtained with reference to the aforementioned examples. MS (ESI) m/z: 891.6 [M+H$^+$]; $^1$H NMR (400 MHz, DMSO-*d$_6$*) δ 11.12 (s, 1H), 9.50 (d, *J* = *5.3* Hz, 1H), 8.79 (d, *J*= 7.8 Hz, 1H), 8.42 (d, *J* = *4.1* Hz, 1H), 8.26 (d, *J* = *5.5* Hz, 1H), 8.13 (s, 0.6H), 7.17 (dd, *J* = 18.6, 7.3 Hz, 3H), 7.08 - 7.00 (m, 1H), 6.87 (d, *J* = 7.9 Hz, 0.5H), 6.46 (d, *J* = 7.8 Hz, 0.5H), 5.40 (dd, *J* = 12.7, 5.4 Hz, 1H), 5.18 (d, *J* = 79.8 Hz, 1H), 4.99 (q, *J*= 7.7 Hz, 1H), 4.77 (d, *J* = 17.9 Hz, 1H), 4.02 (s, 2H), 3.78 (d, *J* = 25.0 Hz, 2H), 3.63 (d, *J* = 23.6 Hz, 5H), 3.52 - 3.43 (m, 4H), 3.11 - 2.80 (m, 4H), 2.79 - 2.58 (m, 3H), 2.46 - 2.30 (m, 3H), 2.29 - 2.12 (m, 3H), 2.06 - 1.91 (m, 4H), 1.72 - 1.50 (m, 4H).

Example 61

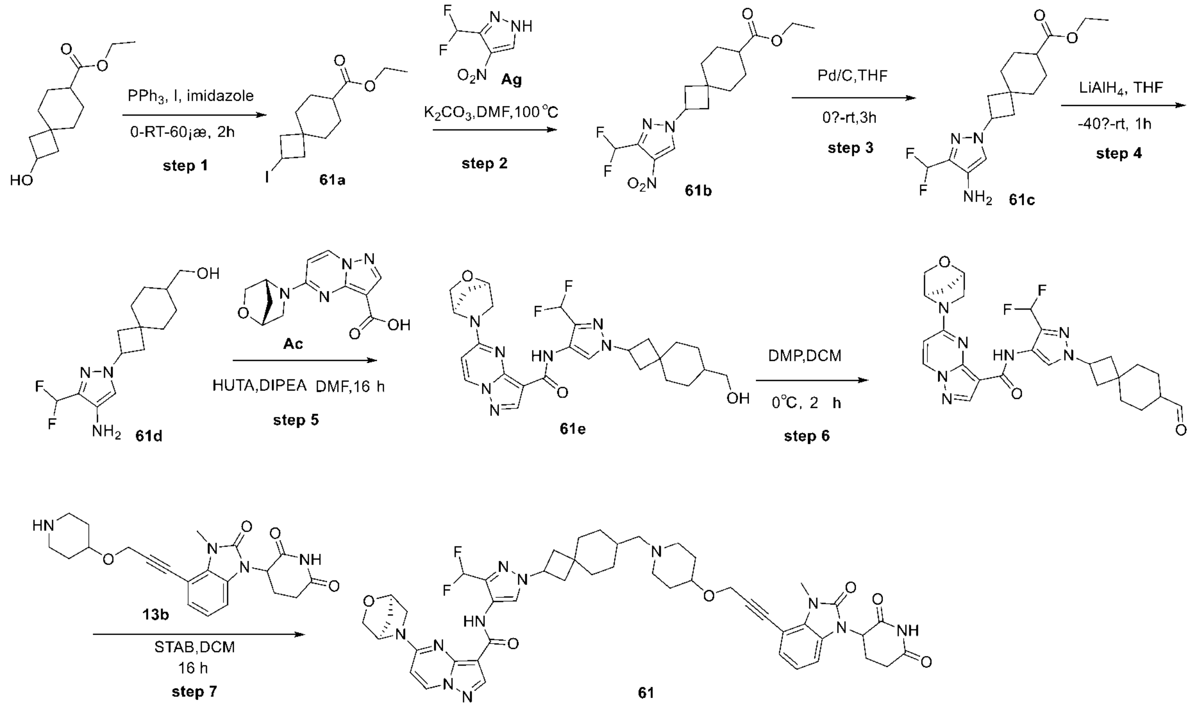


The desired product 61 (2.1 mg) was obtained with reference to the aforementioned examples. MS (ESI) m/z: 906.7 [M+H$^+$]; $^1$H NMR (400 MHz, Chloroform-d) δ 9.60 (s, 1H), 8.47 - 8.41 (m, 2H), 8.32 (d, *J = 7.6* Hz, 1H), 8.22 (s, 1H), 7.15 (d, *J*=7.6 Hz, 1H), 7.00 (t, *J* = 7.6 Hz, 1H), 6.79 (s, 1H), 6.77 (d, *J*= 2.8 Hz, 1H), 6.12 (d, *J*= 7.6 Hz, 1H), 5.46 (s, 1H), 5.20 (dd, *J*= 12.4, 5.2 Hz, 1H), 4.81 (d, *J*= 11.2 Hz, 1H), 4.68 (p, *J*= 8.4 Hz, 1H), 4.44 (s, 2H), 3.97 (d, *J= 9.2* Hz, 2H), 3.81 (s, 1H), 3.77 (s, 3H), 3.58 (d, *J= 9.2* Hz, 1H), 3.49 (d, *J* = 9.2 Hz, 1H), 2.89 - 3.01 (m, 3H), 2.89 - 2.68 (m, 4H), 2.51 (s, 2H), 2.36 - 2.45 (m, 1H), 2.32 - 2.21 (m, 5H), 1.78 - 1.86 (m, 4H), 1.58 - 1.73 (m, 4H), 1.36 - 1.47 (m, 2H), 1.21 - 1.30 (m, 1H), 0.95 - 1.15 (m, 2H).

Example 62

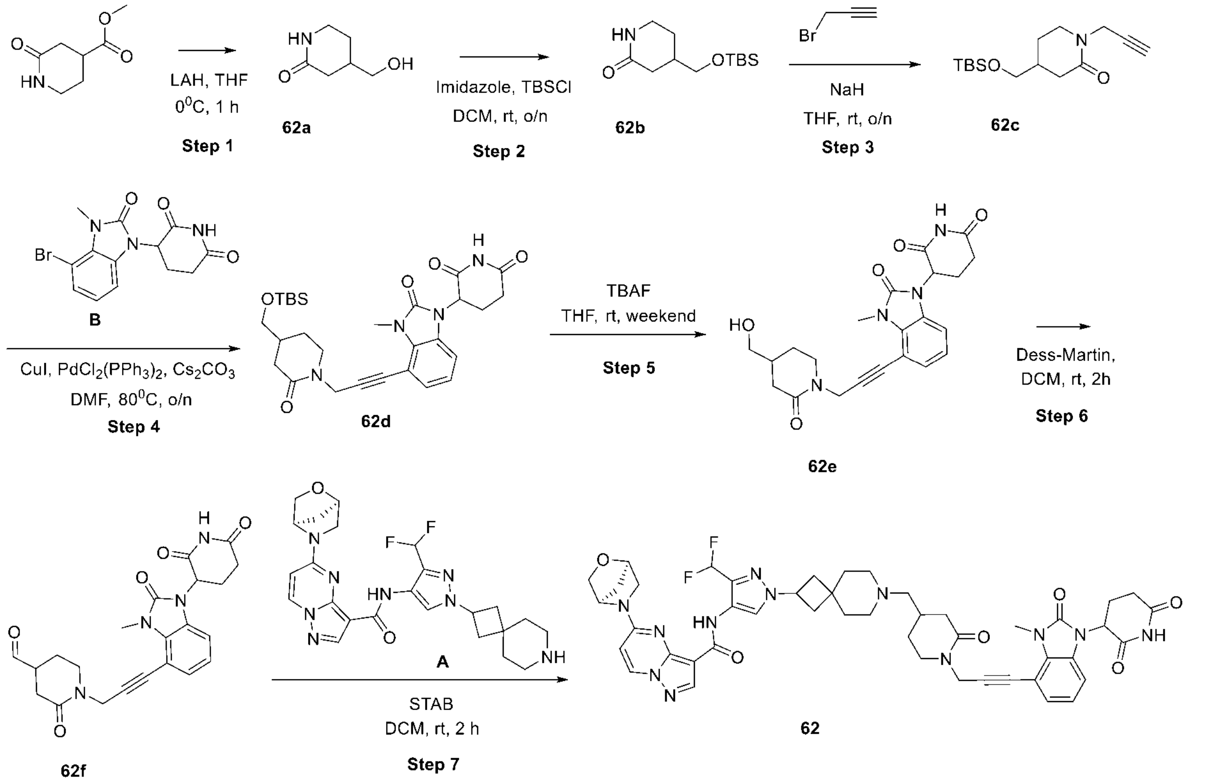


The desired product 62 (63.1 mg) was obtained with reference to the aforementioned examples. MS (ESI) m/z: 905.7 [M+H$^+$]; $^1$H NMR (400 MHz, DMSO-*d$_6$*) δ 11.12 (s, 1H), 9.50 (d, *J = 5.3* Hz, 1H), 8.78 (dd, *J*= 7.7, 1.0 Hz, 1H), 8.40 (d, *J = 4.0* Hz, 1H), 8.26 (d, *J*= 5.6 Hz, 1H), 7.27 (s, 0.3H), 7.19 - 7.06 (m, 2.6H), 7.05 - 6.99 (m, 1.3H), 6.87 (d, *J*= 7.9 Hz, 0.5H), 6.45 (d, *J = 7.8* Hz, 0.5H), 5.40 (dd, *J*= 12.7, 5.4 Hz, 1H), 5.28 (s, 0.5H), 5.07 (s, 0.5H), 4.99 - 4.87 (m, 1H), 4.77 (d, *J* = 16.5 Hz, 1H), 4.50 - 4.35 (m, 2H), 3.85 - 3.70 (m, 2H), 3.67 - 3.57 (m, 4H), 3.50 - 3.39 (m, 3H), 3.17 (d, *J* = 5.2 Hz, 1H), 2.96 - 2.84 (m, 1H), 2.78 - 2.66 (m, 1H), 2.68 - 2.59 (m, 1H), 2.43 - 2.20 (m, 7H), 2.20 - 2.10 (m, 4H), 2.07 - 2.00 (m, 2H), 1.99 - 1.89 (m, 3H), 1.71 - 1.54 (m, 4H), 1.52 - 1.38 (m, 1H).

Example 63

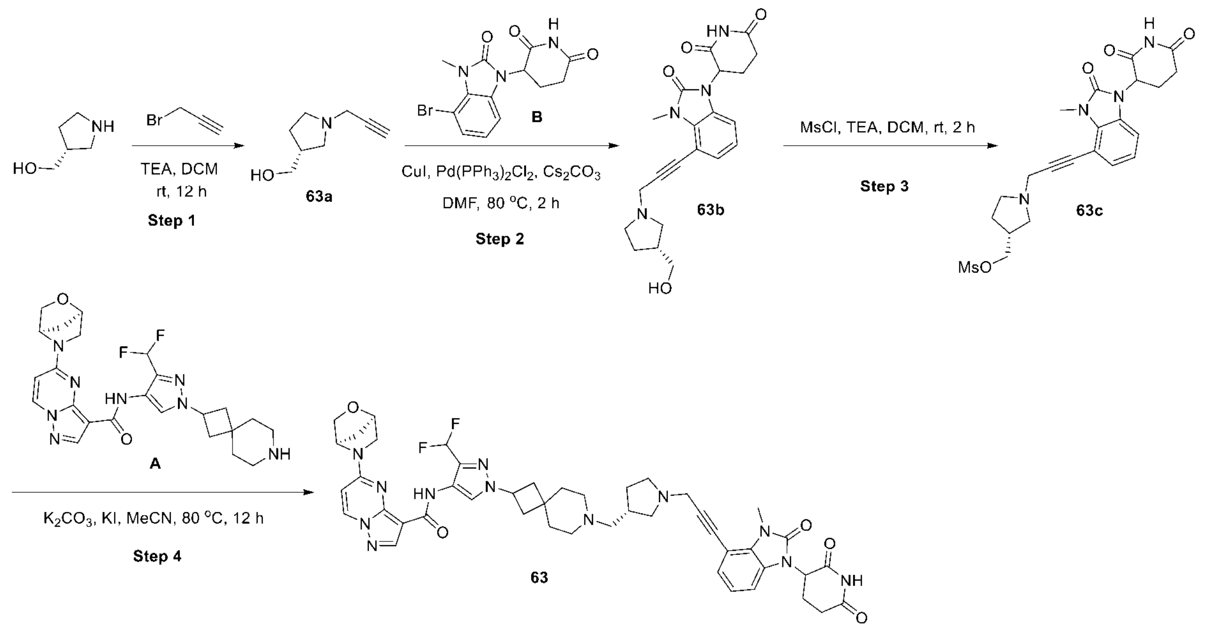

The desired product 63 (1.93 mg) was obtained with reference to the aforementioned examples. MS (ESI) m/z: 877.5 [M+H$^+$]; $^1$H NMR (400 MHz, $CD_3OD$) δ 8.52 - 8.48 (m, 1H), 8.36 - 8.30 (m, 2H), 7.19 - 7.17 (m, 1H), 7.13 - 7.10 (m, 1H), 7.07 - 7.03 (m, 1H), 6.90 - 6.72 (m, 1H), 6.43 (d, *J* = 2.8 Hz, 1H), 5.44 (s, 1H), 5.36 - 5.32 (m, 1H), 3.94 - 3.92 (m, 1H), 3.77 (s, 3H), 3.74 - 3.72 (m, 1H), 3.70 - 3.68 (m, 2H), 3.52 - 3.47 (m, 2H), 3.13 - 3.12 (m, 1H), 3.03 - 2.96 (m, 1H), 2.93 - 2.73 (m, 6H), 2.53 - 2.52 (m, 3H), 2.43 - 2.39 (m, 7H), 2.32 - 2.27 (m, 2H), 2.20 - 2.17 (m, 1H), 2.09 - 2.05 (m, 3H), 1.77 - 1.70 (m, 4H), 1.60 - 1.53 (m, 1H).

Example 64

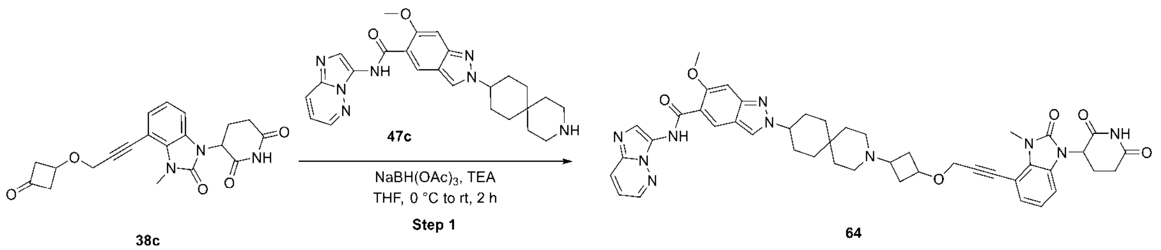


The desired product 64 (4.4 mg) was obtained with reference to the aforementioned examples. MS (ESI) m/z: 825.1 [M+H$^+$]; $^1$H NMR (400 MHz, $CDCl_3$) δ 11.22 (s, 1H), 8.82 (s, 1H), 8.37 (s, 2H), 8.25 (s, 1H), 8.08 (s, 1H), 7.96 (d, *J* = *9.0* Hz, 1H), 7.18 - 7.15 (m, 2H), 7.04 - 6.94 (m, 2H), 6.76 (d, *J* = *7.8* Hz, 1H), 5.20 (dd, *J*= 12.4, 5.2 Hz, 1H), 4.35 (s, 2H), 4.27 (s, 3H), 3.99 - 3.96 (m, 1H), 3.77 (s, 3H), 3.06 - 2.64 (m, 4H), 2.61 - 2.53 (m, 3H), 2.43 - 2.30 (m, 3H), 2.25 - 2.22 (m, 2H), 2.12 - 2.07 (m, 5H), 2.00 - 1.87 (m, 4H), 1.85 - 1.78 (m, 2H), 1.53 - 1.51 (m, 1H), 1.37 - 1.33 (m, 2H).

Example 65

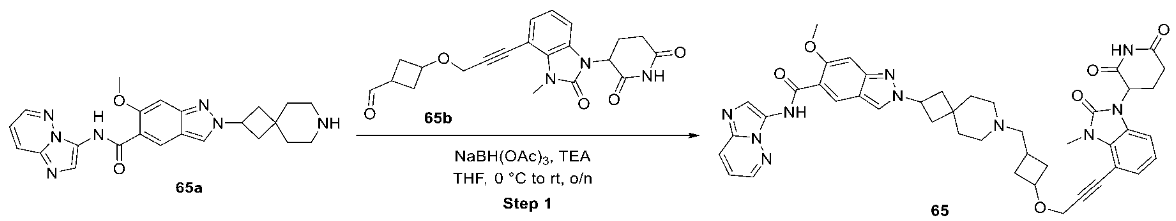


The desired product 65 (6.7 mg) was obtained with reference to the aforementioned examples. MS (ESI) m/z: 811.0 [M+H$^+$]; $^1$H NMR (400 MHz, DMSO-$d_6$) δ 11.26 (s, 1H), 11.13 (s, 1H), 10.71 (brs, 1H), 8.99 (d, *J* = 3.8 Hz, 1H), 8.66 (s, 1H), 8.59 (s, 1H), 8.45 (d, *J* = 9.2 Hz, 1H), 8.38 (s, 1H), 7.69 (dd, *J*= *9.2,* 4.5 Hz, 1H), 7.31 (s, 1H), 7.20 (d, *J*= 7.8 Hz, 1H), 7.13 (d, *J* = *7.7* Hz, 1H), 7.04 (t, *J*= 7.9 Hz, 1H), 5.43 (dd, *J* = 12.7, 5.4 Hz, 1H), 5.28 - 5.18 (m, 1H), 4.37 (s, 2H), 4.14 (s, 3H), 4.10 - 3.95 (m, 1H), 3.65 (s, 3H), 3.38 - 3.17 (m, 3H), 3.15 - 3.06 (m, 2H), 3.01 - 2.82 (m, 3H), 2.76 - 2.60 (m, 3H), 2.48 - 2.30 (m, 4H), 2.27 - 2.13 (m, 1H), 2.12 - 1.84 (m, 6H), 1.82 - 1.68 (m, 2H).

Example 66

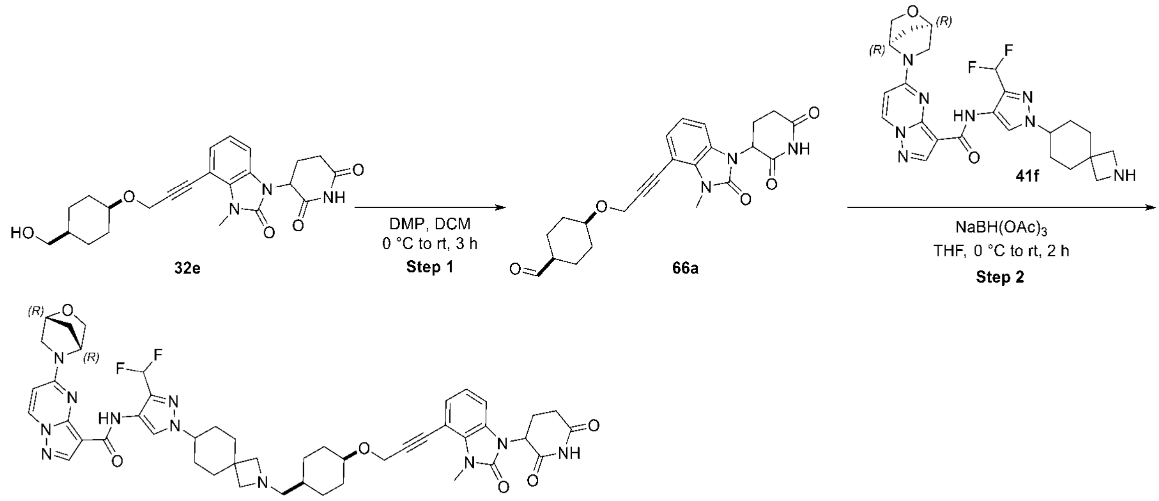

32e
66a
41f
66

The desired product 66 (19 mg) was obtained with reference to the aforementioned examples. MS (ESI) m/z: 904.4 [M+H$^+$]; $^1$H NMR (400 MHz, $CDCl_3$) δ 9.60 (s, 1H), 8.40 (d, *J = 6.8* Hz, 2H), 8.32 (d, *J*=7.6 Hz, 1H), 7.16 (d, *J = 7.2* Hz, 1H), 6.99 (t, *J* = 7.6 Hz, 1H), 6.90 - 6.58 (m, 2H), 6.12 (d, *J* = 7.6 Hz, 1H), 5.44 (s, 1H), 5.20 (dd, *J*= 12.4, 5.2 Hz, 1H), 4.79 (s, 1H), 4.39 (s, 2H), 4.06 - 3.92 (m, 3H), 3.90 - 3.72 (m, 5H), 3.68 - 3.45 (m, 5H), 2.96 - 2.94 (m, 1H), 2.87 - 2.71 (m, 4H), 2.35 - 2.24 (m, 3H), 2.17 - 2.09 (m, 4H), 2.00 - 1.92 (m, 4H), 1.78 - 1.69 (m, 4H), 1.68 - 1.66 (m, 2H), 1.56 - 1.37 (m, 4H).

Example 67

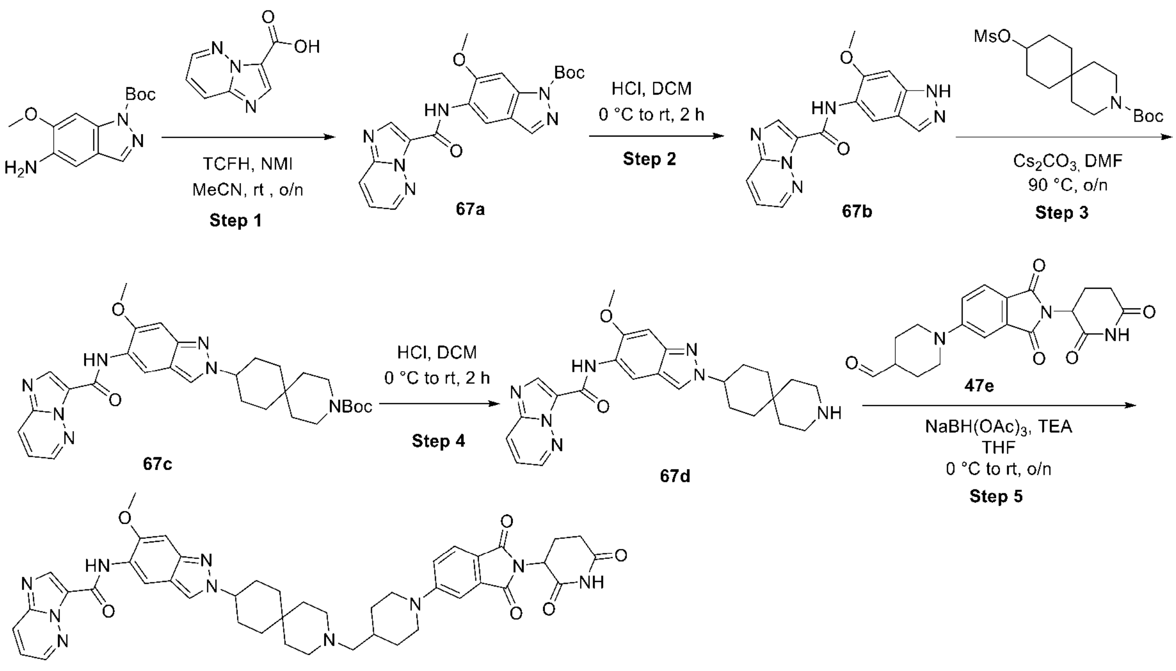

67a
67b
67c
67d
47e
67

The desired product 67 (5.3 mg) was obtained with reference to the aforementioned examples. MS (ESI) m/z: 813.4 [M+H$^+$]; $^1$H NMR (400 MHz, DMSO-*d$_6$*) δ 11.19 (s, 1H), 11.07 (s, 1H), 9.01 - 8.96 (m, 1H), 8.78 (s, 1H), 8.47 (s, 1H), 8.46 - 8.42 (m, 1H), 7.98 (s, 1H), 7.66 (d, *J* = 8.4 Hz, 1H), 7.56 (dd, *J*= 9.2, 4.5 Hz, 1H), 7.38 (s, 1H), 7.33 - 7.29 (m, 1H), 7.26 - 7.21 (m, 1H), 5.07 (dd, *J* = 12.8, 5.4 Hz, 1H), 4.65 - 4.54 (m, 1H), 4.13 (s, 3H), 4.09 - 3.99 (m, 2H), 3.29 (s, 1H), 3.08 - 2.76 (m, 4H), 2.41 - 2.30 (m, 4H), 2.21 - 1.93 (m, 8H), 1.85 - 1.72 (m, 6H), 1.67 - 1.61 (m, 2H), 1.40 - 1.33 (m, 4H).

Example 68

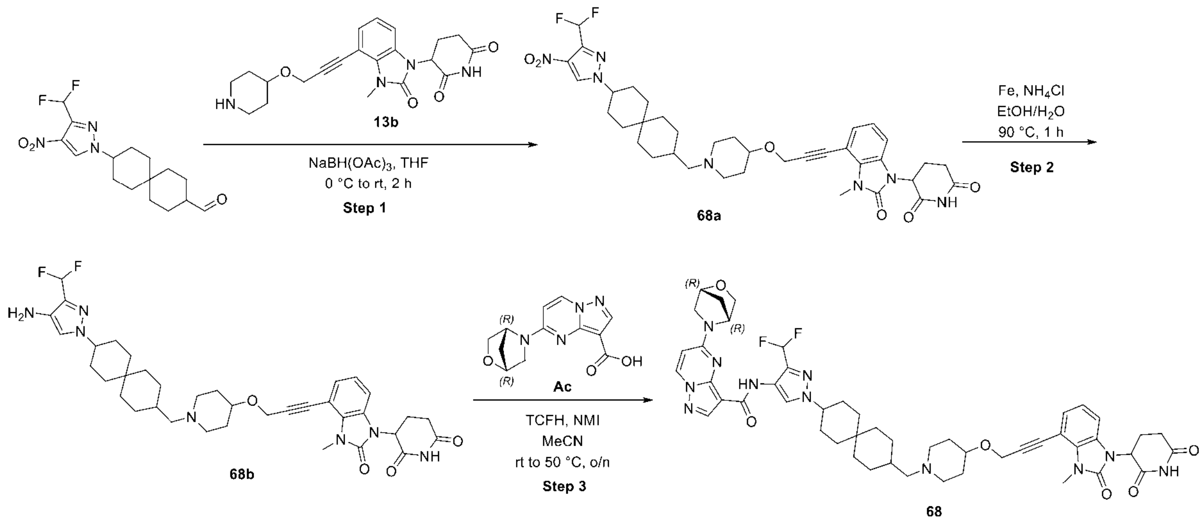


The desired product 68 (40 mg) was obtained with reference to the aforementioned examples. MS (ESI) m/z: 934.3 [M+H$^+$]; $^1$H NMR (400 MHz, DMSO-$d_6$) δ 11.13 (s, 1H), 9.49 (d, *J* = 5.2 Hz, 1H), 8.78 (d, *J* = 7.6 Hz, 1H), 8.41 (d, *J* = 4.4 Hz, 1H), 8.25 (d, *J* = 5.6 Hz, 1H), 7.26 - 6.98 (m, 4H), 6.88 - 6.44 (m, 1H), 5.41 (dd, *J* = 12.6, 5.4 Hz, 1H), 5.17 (d, *J* = 80.8 Hz, 1H), 4.77 (d, *J* = 17.6 Hz, 1H), 4.49 (s, 2H), 4.19 - 4.16 (m, 1H), 3.84 - 3.72 (m, 2H), 3.65 - 3.56 (m, 5H), 3.49 - 3.43 (m, 1H), 2.94 - 2.85 (m, 2H), 2.77 - 2.61 (m, 3H), 2.33 - 1.75 (m, 14H), 1.66 - 1.49 (m, 4H), 1.44 - 1.20 (m, 6H), 1.14 - 0.93 (m, 4H).

Example 69

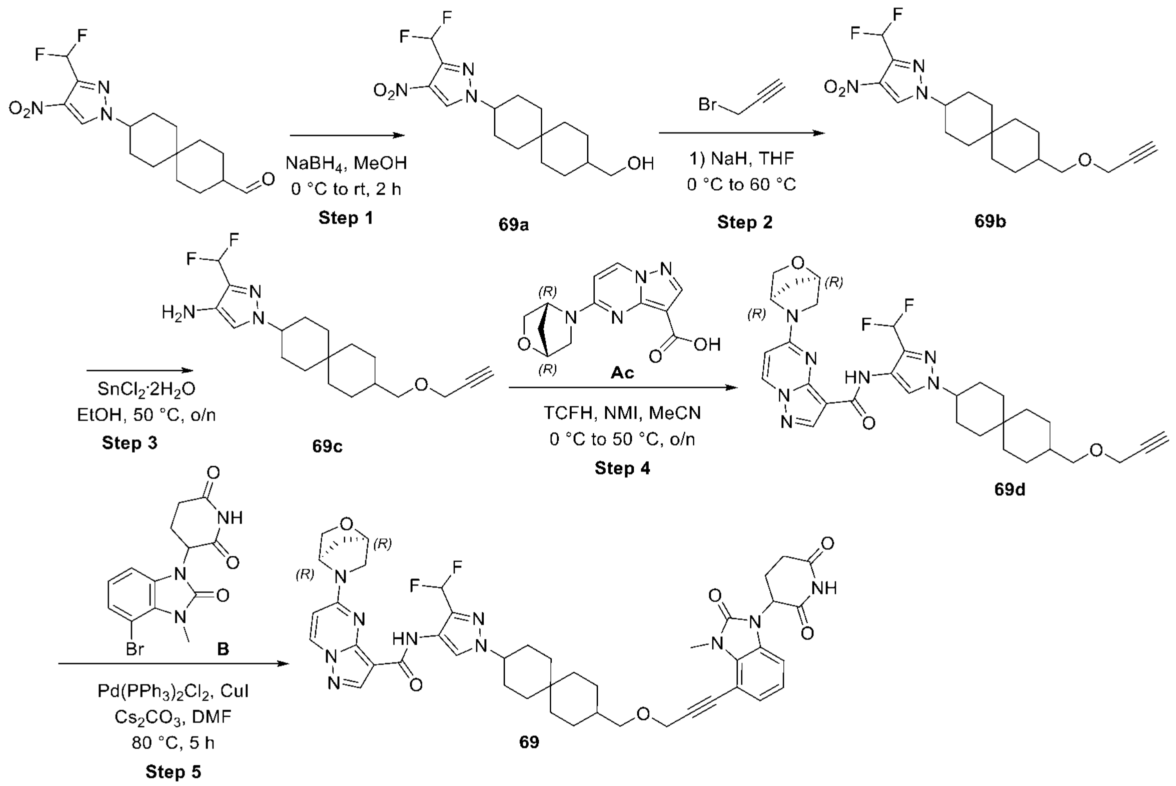

The desired product 69 (1 mg) was obtained with reference to the aforementioned examples. MS (ESI) m/z: 851.2 [M+H+]; $^1$H NMR (400 MHz, $CDCl_3$) δ 9.60 (s, 1H), 8.42 (d, *J* = 6.4 Hz, 2H), 8.31 (d, *J*= 7.6 Hz, 1H), 8.11 (d, *J* = 4.8 Hz, 1H), 7.18 (d, *J* = 7.6 Hz, 1H), 6.99 (t, *J = 8.0* Hz, 1H), 6.91 - 6.63 (m, 2H), 6.12 (d, *J* = 7.6 Hz, 1H), 5.46 (s, 1H), 5.36 - 5.33 (m, 1H), 5.22 - 5.17 (m, 1H), 4.82 - 4.79 (m, 1H), 4.40 (s, 2H), 4.07 - 3.95 (m, 3H), 3.79 (s, 3H), 3.57 (d, *J= 9.2* Hz, 1H), 3.49 (d, *J= 9.2* Hz, 1H), 3.43 (d, *J*= 6.0 Hz, 2H), 2.98 - 2.71 (m, 3H), 2.26 - 2.20 (m, 1H), 2.12 - 1.85 (m, 8H), 1.67 - 1.61 (m, 1H), 1.49 - 1.45 (m, 1H), 1.39 - 1.30 (m, 4H), 1.12 - 0.98 (m, 4H).

Example 70

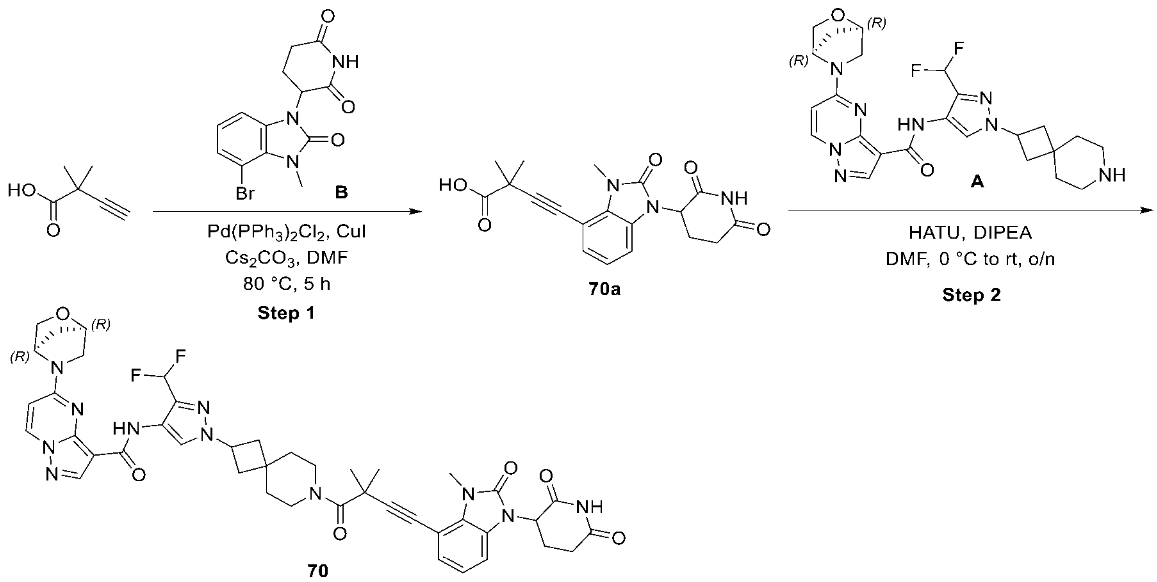


The desired product 70 (19 mg) was obtained with reference to the aforementioned examples. MS (ESI) m/z: 850.4 [M+H+]; $^1$H NMR (400 MHz, DMSO-$d_6$) δ 11.12 (s, 1H), 9.50 (d, *J = 5.2* Hz, 1H), 8.78 (d, *J* = 8.0 Hz, 1H), 8.40 (d, *J*= 3.6 Hz, 1H), 8.25 (d, *J* = 5.6 Hz, 1H), 7.28 - 7.01 (m, 4H), 6.88 - 6.44 (m, 1H), 5.45 - 5.36 (m, 1H), 5.28 - 5.07 (m, 1H), 5.02 - 4.94 (m, 1H), 4.80 - 4.74 (m, 1H), 3.85 - 3.72 (m, 4H), 3.65 - 3.59 (m, 5H), 3.51 - 3.43 (m, 2H), 2.93 - 2.85 (m, 1H), 2.72 - 2.60 (m, 2H), 2.44 - 2.38 (m, 2H), 2.27 - 2.21 (m, 2H), 2.05 - 1.95 (m, 3H), 1.76 - 1.64 (m, 4H), 1.51 (s, 6H).

Example 71

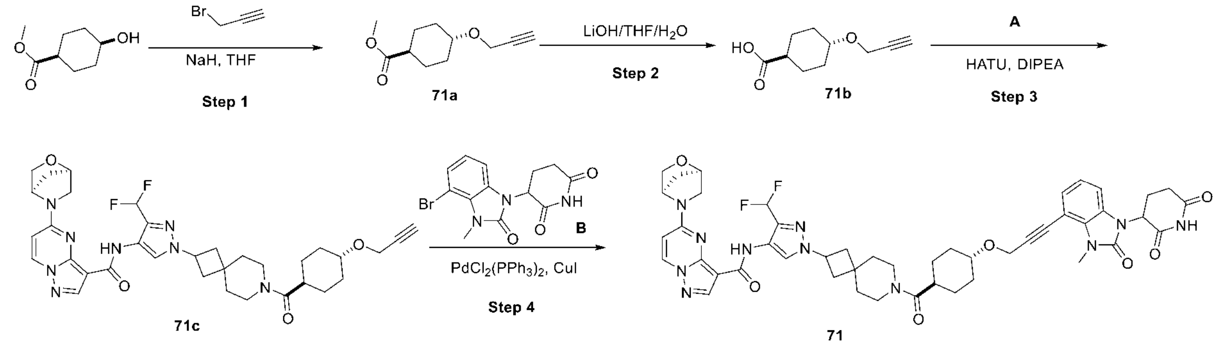


Step 1: Methyl (1s,4s)-4-hydroxycyclohexane-1-carboxylate (4.3 g, 27.2 mmol) was dissolved in dry tetrahydrofuran (40 mL), and the reaction system was purged with nitrogen and placed in an ice-water bath. After the reaction system was cooled to 0°C, sodium hydride (1.3 g, 32.6 mmol) was slowly added to the reaction mixture under a nitrogen atmosphere. After the reaction was stirred at 0°C for 1 hour, propargyl bromide (3.88 g, 32.6 mmol) was slowly added dropwise to the reaction system, and the reaction was slowly returned to room temperature and stirred for 3 hours. After

TLC monitoring showed the disappearance of the starting material, the reaction mixture was first quenched by adding saturated aqueous ammonium chloride solution (30 mL). The mixture was extracted with ethyl acetate (30 mL × 3). The organic phases were combined, first washed with saturated brine (50 mL), then dried over anhydrous sodium sulfate, filtered, and finally concentrated under reduced pressure. The resulting residue was purified by silica gel column chromatography to obtain $P_1$ (71a) (140 mg) and $P_2$ (72a) (200 mg).

$P_1$ $^1$H NMR (400 MHz, $CDCl_3$) δ 4.15 (t, *J*= 5.0 Hz, 2H), 3.75 - 3.69 (m, 1H), 3.67 (s, 3H), 2.44 - 2.32 (m, 2H), 1.94 - 1.79 (m, 4H), 1.70 - 1.64 (m, 2H), 1.60 - 1.51 (m, 2H).

$P_2$ $^1$H NMR (400 MHz, $CDCl_3$) δ 4.19 (d, *J* = 2.4 Hz, 2H), 3.67 (s, 3H), 3.47 (tt, *J* = 10.5, 4.1 Hz, 1H), 2.41 (t, *J* = 2.4 Hz, 1H), 2.28 (tt, *J* = 11.7, 3.7 Hz, 1H), 2.15 - 2.02 (m, 4H), 1.55 - 1.44 (m, 2H), 1.33 - 1.26 (m, 2H).

Step 2: 71a (200 mg, 1.1 mmol) was dissolved in tetrahydrofuran/water (4 mL/0.8 mL) at room temperature, and lithium hydroxide (230 mg, 5.49 mmol) was added. The reaction system was stirred overnight at 50°C. After LCMS monitoring showed the disappearance of the starting material, the reaction mixture was extracted with ethyl acetate (30 mL × 2). The aqueous phase was adjusted to pH 3-4 with 1 M hydrochloric acid and then extracted with ethyl acetate (30 mL × 2) to obtain intermediate 71b. (160 mg, pale yellow solid). $^1$H NMR (400 MHz, DMSO) δ 12.04 (s, 1H), 4.13 (t, *J* = 3.9 Hz, 2H), 3.35 (m, *J*= 2.4 Hz, 2H), 2.15 (tt, *J*= 11.5, 3.6 Hz, 1H), 2.00 - 1.94 (m, 2H), 1.91 - 1.83 (m, 2H), 1.38 - 1.28 (m, 2H), 1.21 - 1.12 (m, 2H).

Step 3: 71b (101 mg, 0.554 mmol), intermediate A (230 mg, 0.462 mmol), and 2-(7-azabenzotriazol-1-yl)-*N,N,N',N'*-tetramethyluronium hexafluorophosphate (263 mg, 0.693 mmol) were dissolved in N,N-dimethylformamide (4 mL), and N,N-diisopropylethylamine (150 mg, 1.155 mmol) was added. The reaction system was stirred at room temperature for 1 hour.

After TLC monitoring showed the disappearance of the starting material, ethyl acetate (30 mL) was added to the reaction mixture. The mixture was extracted with water (30 mL × 3). The organic phases were combined, washed with saturated brine, then dried over anhydrous sodium sulfate, filtered, and finally concentrated under reduced pressure. The resulting residue was purified by silica gel column chromatography to obtain 71c (150 mg). MS (ESI) m/z: 663.3 [M+H$^+$].

Step 4: 71c (150 mg, 0.226 mmol), intermediate B (92 mg, 0.271 mmol), cesium carbonate (220 mg, 0.679 mmol), copper(I) iodide (5 mg, 0.0226 mmol), and bis(triphenylphosphine)palladium(II) dichloride (16 mg, 0.0226 mmol) were dissolved in *N,N*-dimethylformamide (4 mL) at room temperature. The reaction system was evacuated and purged with nitrogen several times. The reaction mixture was stirred in an oil bath at 85°C for 3 hours. After LCMS monitoring showed the disappearance of the starting material, the reaction mixture was quenched by adding water (30 mL). The mixture was extracted with ethyl acetate (30 mL × 3), and the organic phases were combined, washed with saturated brine (30 mL), then dried over anhydrous sodium sulfate, filtered, and finally concentrated under reduced pressure. The resulting residue was purified by silica gel column chromatography, and the product was collected to obtain the final product 71 (5.6 mg). MS (ESI) m/z: 920.1 [M+H$^+$]; $^1$H NMR (400 MHz, DMSO) δ 11.10 (s, 1H), 9.50 (d, *J*= 5.2 Hz, 1H), 8.79 (d, *J*= 7.7 Hz, 1H), 8.42 (d, *J* = 4.1 Hz, 1H), 8.26 (d, *J* = 5.6 Hz, 1H), 7.31 - 7.09 (m, 3H), 7.03 (dd, *J*= 9.8, 5.9 Hz, 1H), 6.87 (d, *J= 7.9* Hz, 0.5H), 6.46 (d, *J*= 7.8 Hz, 0.5H), 5.40 (dd, *J* = 12.7, 5.4 Hz, 1H), 5.28 (s, 0.5H), 5.08 (s, 0.5H), 4.99 (d, *J* = 7.0 Hz, 1H), 4.77 (d, *J*= 16.0 Hz, 1H), 4.47 (s, 2H), 3.80 (t, *J* = 16.7 Hz, 2H), 3.65 (s, 3H), 3.61 (d, *J* = 10.7 Hz, 1.5H), 3.45 (d, *J* = 9.3 Hz, 3.5H), 3.37 (s, 2H), 2.93 - 2.84 (m, 1H), 2.77 - 2.68 (m, 1H), 2.65 (t, *J*= 5.4 Hz, 1H), 2.40 (s, 2H), 2.23 (t, *J* = 9.3 Hz, 2H), 2.12 - 2.02 (m, 4H), 2.01 - 1.91 (m, 2H), 1.67 (s, 3H), 1.59 (s, 2H), 1.51 (s, 1H), 1.40 (dd, *J*= 25.0, 11.6 Hz, 2H), 1.27 (d, *J*= 10.9 Hz, 2H).

## Example 72

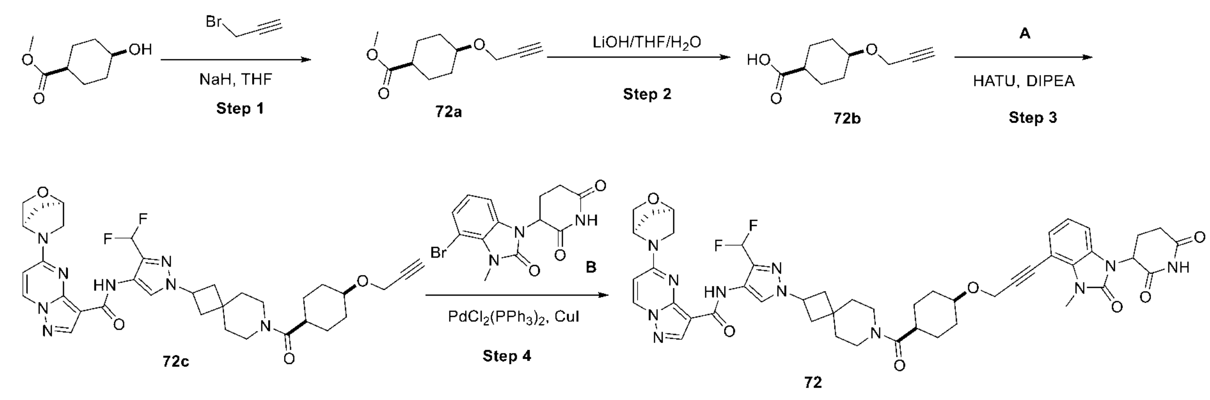


The desired product 72 (6.9 mg) was obtained with reference to Example 71. MS (ESI) m/z: 920.3 [M+H$^+$]; $^1$H NMR (400 MHz, DMSO) δ 11.10 (s, 1H), 9.50 (d, *J* = 5.2 Hz, 1H), 8.79 (d, *J* = 7.8 Hz, 1H), 8.41 (d, *J* = 4.1 Hz, 1H), 8.26 (d, *J* =

5.6 Hz, 1H), 7.19 - 7.00 (m, 4H), 6.87 (d, *J* = 7.9 Hz, 0.5H), 6.46 (d, *J* = 7.7 Hz, 0.5H), 5.40 (dd, *J* = 12.7, 5.4 Hz, 1H), 5.28 (s, 0.5H), 5.08 (s, 0.5H), 4.99 (s, 1H), 4.77 (d, *J* = 15.3 Hz, 1H), 4.45 (s, 2H), 3.82 (d, *J* = 8.9 Hz, 2H), 3.74 (d, *J* = 7.7 Hz, 0.5H), 3.64 (s, 3H), 3.58 (d, *J* = 11.3 Hz, 1.5H), 3.45 (d, *J* = 9.8 Hz, 3H), 3.37 (s, 2H), 2.88 (dd, *J* = 20.6, 9.4 Hz, 1H), 2.75 - 2.62 (m, 3H), 2.40 (t, *J*= 9.3 Hz, 2H), 2.23 (t, *J* = 10.0 Hz, 2H), 2.08 - 1.85 (m, 6H), 1.66 (s, 2H), 1.58 (d, *J*= 9.8 Hz, 3H), 1.52 (s, 2H), 1.41 (s, 2H).

Biological activity test examples

The control molecule used in this test example was KT-474, which was prepared with reference to the preparation method of compound I-417 in patent WO2020113233A1. Its structure is as follows:

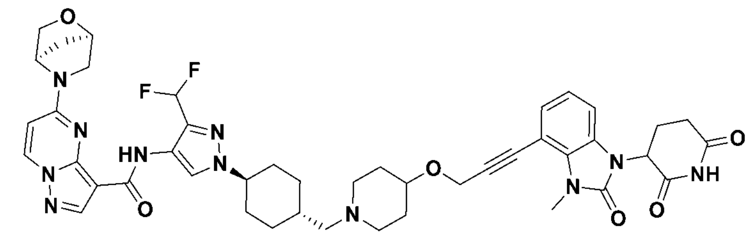

**KT-474**

Test Example 1: Degradation of IRAK4 by compounds in SU-DHL-2 and OCI-LY3 cells

(1) Experimental objective

The degradation level of IRAK4 protein in SU-DHL-2 cell line by the compounds was detected by flow cytometry.

(2) Experimental materials

Fix Buffer I, BD Phosflow™, 557870
Perm III buffer, BD Phosflow™, 558050
LIVE/DEAD™ Fixable Violet Dead Cell Stain Kit, Thermo Fisher, L34966
Alexa Fluor 647 Mouse anti-Human IRAK4 Clone L29-525 (RUO), BD Phosflow™, 560315

(3) Experimental instruments

Flow cytometer, BD, Model: BD LSRFortessa

(4) Experimental method

Step 1: The SU-DHL-2 cell line was cultured in RPMI 1640 medium supplemented with 10% fetal bovine serum and 1% double antibiotics.

Step 2: On day 1, the cells were seeded at a density of $2 \times 10^5$ cells per well in 100 μL in a 96-well plate. The compounds were dissolved in DMSO to a final concentration of 10 mM, and then diluted with culture medium at a 1:5 concentration gradient to prepare 10 concentrations. The diluted solutions were added to the cells to obtain a maximum final concentration of 2 μM. Wells containing cells treated with 0.1% DMSO served as the positive control The cells were incubated at 37°C in a 5% $CO_2$ incubator for 24 hours.

Step 3: After centrifugation, the cells were washed with FACS buffer. The cells were stained for viability discrimination using the LIVE/DEAD™ Fixable Blue Dead Cell Stain Kit, and surface staining of monocytes was performed with PE Mouse Anti-Human CD14, followed by incubation at 4°C for 30 minutes.

Step 4: After centrifugation, the cells were washed with FACS buffer. Fix buffer I, equal in volume to the cultured cells, was added, and the cells were fixed at 37°C for 10 minutes.

Step 5: After centrifugation, the cells were washed with FACS buffer. 150 μL of Perm III reagent was added, and the cells were incubated at 4°C for 30 minutes to permeabilize the membranes. After centrifugation, the cells were washed with FACS buffer.

Step 6: The cells were stained with Alexa Fluor 647 Mouse anti-Human IRAK4 Antibody and incubated at 4°C for 1 hour. Flow cytometry analysis was performed using a flow cytometer.

(5) Data processing

The flow cytometry data were analyzed using Flowjo software. Data presentation and $DC_{50}$ (half-maximal degradation concentration) calculation were performed using Prism 8.0.2 (GraphPad).

(6) Experimental results

The degradation activity results of the compounds of the present disclosure against IRAK4 are shown in Table 1.
The Dmax values of the compounds of the present disclosure for SU-DHL-2 and OCI-LY3 cells were generally 50% or more, with the highest reaching about 90%.
The results indicate that the compounds of the present disclosure have good degradation activity against the IRAK4 target in SU-DHL-2 and OCI-LY3 cells.

Table 1. Degradation results of IRAK4 in SU-DHL-2 and OCI-LY3 cells

| Compound No. | SU-DHL-2 $DC_{50}$ (nM) | OCI-LY3 $DC_{50}$ (nM) |
|---|---|---|
| Example 1 | 1.4 | 0.4 |
| Example 2 | 1.1 | 0.4 |
| Example 3 | 11 | 8 |
| Example 4 | 8 | 5 |
| Example 5 | 27 | 28 |
| Example 6 | 10 | 4 |
| Example 7 | 12 | 10 |
| Example 8 | 4 | 2 |
| Example 9 | 2.7 | 2.3 |
| Example 10 | 17 | 13 |
| Example 11 | 3 | 4 |
| Example 12 | 5 | 5 |
| Example 13 | 5.7 | 5 |
| Example 14 | 4.6 | 4.4 |
| Example 15 | 5 | 1.5 |
| Example 16 | 5 | 5 |
| Example 17 | 1 | 1 |
| Example 18 | 6 | 4 |
| Example 19 | 7 | 6 |
| Example 20 | 4 | 4 |
| Example 21 | 3 | 4 |
| Example 22 | 1.4 | 1.3 |
| Example 24 | 4 | 3 |
| Example 25 | 6 | 4 |
| Example 26 | 2.9 | 3.5 |
| Example 27 | 22 | 32 |
| Example 28 | 4 | 2 |
| Example 29 | 3 | 1 |

(continued)

| Compound No. | SU-DHL-2 $DC_{50}$ (nM) | OCI-LY3 $DC_{50}$ (nM) |
|---|---|---|
| Example 30 | 3 | 1 |
| Example 31 | 3.3 | 2.8 |
| Example 32 | 3.4 | 2.7 |
| Example 33 | 6.5 | |
| Example 34 | 21 | |
| Example 35 | 5 | |
| Example 36 | 5.6 | |
| Example 37 | 24 | |
| Example 38 | 68 | |
| Example 39 | 17 | |
| Example 40 | 33 | |
| Example 43 | 61 | |
| Example 44 | 5.4 | |
| Example 46 | 13 | |
| Example 47 | 0.5 | |
| Example 48 | 1 | |
| Example 49 | 4 | |
| Example 50 | 4 | |
| Example 51 | 7 | |
| Example 52 | 3 | |
| Example 53 | 6 | |
| Example 54 | 17 | |
| Example 55 | 7 | |
| Example 56 | 5 | |
| Example 57 | 22 | |
| Example 58 | 5 | |
| Example 59 | 3 | |
| Example 60 | 2.5 | |
| Example 61 | 4 | |
| Example 62 | 1.2 | |
| Example 63 | 6.5 | |
| Example 64 | 1 | |
| Example 65 | 2.8 | |
| Example 66 | 13 | |
| Example 67 | 45 | |
| Example 68 | 24 | |
| Example 70 | 16 | |
| Example 71 | 5 | |
| Example 72 | 4 | |

Test Example 2: Degradation of IRAK4 in human PBMCs by compounds

(1) Experimental objective

The degradation level of IRAK4 in monocytes of human peripheral blood mononuclear cells (hPBMCs) was detected by flow cytometry.

(2) Experimental materials

Fix Buffer I, BD Phosflow™, 557870
Perm III buffer, BD Phosflow™, 558050
LIVE/DEAD™ Fixable Blue Dead Cell Stain Kit, for UV excitation, Thermo Fisher, L34962
PE Mouse Anti-Human CD14, BD Pharmingen™, 555398
Alexa Fluor 647 Mouse anti-Human IRAK4 Clone L29-525 (RUO), BD Phosflow™, 560315

(3) Experimental instruments

Flow cytometer, BD, Model: BD LSRFortessa

(4) Experimental method

The cryopreserved hPBMCs were thawed and cultured in RPMI 1640 medium supplemented with 10% fetal bovine serum and 1% Pen + Strep, followed by recovery at 37°C for 1 hour.
On day 1, the cells were seeded at a density of $2 \times 10^5$ cells per well in 100 μL in a 96-well plate.
The compounds were dissolved in DMSO to a final concentration of 10 mM, and then diluted with culture medium at a 1:5 concentration gradient to prepare 10 concentrations. The diluted solutions were added to the cells to obtain a maximum final concentration of 2 μM. Wells containing cells treated with 0.1% DMSO served as the positive control
The cells were incubated at 37°C in a 5% $CO_2$ incubator for 24 hours.
After centrifugation, the cells were washed with FACS buffer.
The cells were stained for viability discrimination using the LIVE/DEAD™ Fixable Blue Dead Cell Stain Kit, and surface staining of monocytes was performed with PE Mouse Anti-Human CD14, followed by incubation at 4°C for 30 minutes.
After centrifugation, the cells were washed with FACS buffer.
Fix buffer I, equal in volume to the cultured cells, was added, and the cells were fixed at 37°C for 10 minutes.
After centrifugation, the cells were washed with FACS buffer.
150 μL of Perm III reagent was added, and the cells were incubated at 4°C for 30 minutes to permeabilize the membranes.
After centrifugation, the cells were washed with FACS buffer.
The cells were stained with Alexa Fluor 647 Mouse anti-Human IRAK4 Antibody and incubated at 4°C for 1 hour.
Flow cytometry analysis was performed using a flow cytometer.

(5) Data processing method

The flow cytometry data were analyzed using Flowjo software. Data presentation and $DC_{50}$ calculation were performed using Prism 8.0.2 (GraphPad).

(6) Experimental results

The degradation activity results of the compounds of the present disclosure against IRAK4 are shown in Table 2.
The results indicate that the compounds of the present disclosure have good degradation activity against the IRAK4 target in human PBMC cells.

Table 2. Degradation results of IRAK4 in human PBMC cells

| Compound No. | $DC_{50}$ in PBMC (nM) |
|---|---|
| Example 13 | 2.22 |

(continued)

| Compound No. | $DC_{50}$ in PBMC (nM) |
|---|---|
| Example 22 | 0.13 |
| Example 31 | 0.3 |
| Example 36 | 0.8 |
| Example 49 | 0.5 |
| Example 52 | 0.65 |
| Example 56 | 1.1 |
| KT-474 | 0.6 |

Test Example 3: Inhibition of inflammatory factor secretion induced in human PBMCs by compounds

(1) Experimental objective

The effect of PROTAC small molecules on inflammatory factors in PBMCs downstream of IRAK4 induced by TLR and IL-1 was detected based on the CBA (cytometric bead array) method.

(2) Experimental materials

| Name | Manufacturer | Cat NO. |
|---|---|---|
| 1640 | GIBCO | 11875093 |
| FBS | GIBCO | 10091-148 |
| human PBMC | Allcells | FPB004F-C |
| 3799 plate | Corsta | 3799 |
| IL-6 CBA kit | BD | 558276 |
| TNF$\alpha$ CBA kit | BD | 558273 |
| CBA buffer | BD | 558264 |
| IL-1$\beta$ | novoprotein | GMP-CG93 |
| R848 | Invivogen | tlrl-r848 |
| LPS | Invivogen | tlrl-b5lps |

(3) Experimental instruments

Flow cytometer, BD, Model: BD LSRFortessa

(4) Experimental method

Step 1: Thawing of Human PBMC

Step 2: The density of PBMCs was adjusted to 1 E6/mL, and 150 $\mu$L of PBMCs in 1640 complete medium were seeded into a 96-well plate, followed by incubation at 37°C for 1 hour.

Step 3: 50 $\mu$L of serially diluted small molecule (working concentration: 10000 nM, 1:3 dilution) drugs were inoculated into the plate in a 37°C incubator for 8 hours.

Step 4: 10 $\mu$L of stimulants were added respectively, with final concentrations of 100 ng/mL for LPS, 1 $\mu$g/mL for R848, and 100 ng/mL for IL-1$\beta$.

Step 5: CBA assay. The concentrations of IL-6 and TNF-$\alpha$ in the cell culture supernatant were determined using Hu IL-6 CBA Flex Set A7 and Hu TNF- CBA Flex Set D9.

Step 6: Standard samples were prepared: 2500 pg/mL, 1250 pg/mL,...0. A 1:2 serial dilution was performed to

obtain 10 concentrations.

Step 7: The mixed capture beads were resuspended in capture bead diluent (1:50), and cell culture supernatant (50 μL) was added, followed by incubation at room temperature for 10 minutes.

Step 8: The detection reagent prepared with detection diluent was added, and the mixture was incubated in the dark at room temperature for 3 hours. At the end of incubation, the samples were washed once with wash buffer and resuspended for detection.

(5) Data processing

The concentrations of IL-6 and TNFα were calculated using the standard curve method. Data presentation and $IC_{50}$ calculation were performed using Prism 8.0.2.

(6) Experimental results

The inhibitory effects of the compounds of the present disclosure on the secretion of inflammatory factors induced in human PBMCs are shown in Tables 3 and 4.

The results indicate that the compounds of the present disclosure can effectively inhibit the secretion of inflammatory factors, exhibiting potential therapeutic value for inflammatory diseases.

Table 3-1. TNFα secretion induced by LPS, R848, and IL-1β in human PBMCs

| Compound No. | $IC_{50}$ (nM) | | |
|---|---|---|---|
| | LPS | R848 | IL-1β |
| Example 13 | 22.3 | 20 | 13 |
| Example 22 | 1.2 | 1.4 | 0.16 |
| Example 31 | 7.7 | 8.2 | 2.1 |
| KT474 | 31 | 52 | 13 |

Table 3-2. TNFα secretion induced by LPS, R848, and IL-1β in human PBMCs

| Compound No. | $IC_{50}$ (nM) | | |
|---|---|---|---|
| | LPS | R848 | IL-1β |
| Example 52 | 8.5 | 21.5 | 13.9 |

Table 4-1. IL-6 secretion induced by LPS, R848, and IL-1β in human PBMCs

| Compound No. | $IC_{50}$ (nM) of compounds against induced secretion | | |
|---|---|---|---|
| | LPS | R848 | IL-1β |
| Example 13 | 19.7 | 10 | 13.2 |
| Example 22 | 0.5 | 0.16 | 1 |
| Example 31 | 4.6 | 0.9 | 9.3 |
| KT-474 | 41 | 14 | 18 |

Table 4-2. IL-6 secretion induced by LPS, R848, and IL-1β in human PBMCs

| Compound No. | $IC_{50}$ (nM) of compounds against induced secretion | | |
|---|---|---|---|
| | LPS | R848 | IL-1β |
| Example 52 | 8.4 | 20.3 | 8.9 |

Test Example 4: Inhibition of hERG potassium channel by compounds

(1) Experimental objective

The effects of compounds on hERG potassium channel (human *Ether-a-go-go* Related Gene potassium channel) currents were tested using the whole-cell manual voltage-clamp electrophysiological method.

(2) Experimental materials

Sodium chloride (NaCl) Sigma S7653, potassium chloride (KCl) Sigma P9333, magnesium chloride (MgCl2) Sigma M1028
Calcium chloride (CaCl2) Sigma 21115, glucose (Glucose) Sigma G7528, HEPES Sigma H3375, EGTA Sigma E3889, sodium hydroxide (NaOH) Sinopharm 10019718, potassium hydroxide (KOH) Sinopharm 10017018;
CHO-hERG cell line (Chinese Hamster Ovary): Chinese hamster ovary cells stably expressing the hERG channel
Extracellular solution formula (mM): 140 NaCl, 5 KCl, 1 $CaCl_2$, 1.25 $MgCl_2$, 10 HEPES, and 10 Glucose, adjusted to pH 7.4 with NaOH.
Intracellular solution formula (mM): 140 KCl, 1 $MgCl_2$, 1 $CaCl_2$, 10 EGTA, and 10 HEPES, adjusted to pH 7.2 with KOH.

(3) Experimental instruments

Patch-clamp amplifier (Multiclamp 700B, Axon, USA)
Digital-to-analog converter (DigiData 1440A, Axon, USA)
Inverted microscope (IX71, Olympus, Japan)
Fast perfusion system (RSC-200, Bio-Logic, France)
Micromanipulator (MX7600R, Syskiyou, USA)
Electrode puller (P-97, Sutter, USA)
Glass electrode (BF150-86-10, Sutter, USA)
Vibration isolation table and Faraday cage (63-534, TMC, USA)
Data acquisition and analysis software (pClamp, Axon, USA)
$CO_2$ incubator (HERAcell 150i, Thermo, USA)
Biosafety cabinet (MODEL 1384, Thermo, USA)
Water purifier (Milli Q, Millipore, USA)

(4) Experimental method

(4.1) Cell culture and treatment

CHO cells stably expressing hERG were cultured in cell culture dishes with a diameter of 35 mm and incubated at 37°C in a 5% $CO_2$ incubator. The cells were passaged at a ratio of 1:5 every 48 hours. The culture medium was formulated as follows: 90% F12 (Invitrogen), 10% fetal bovine serum (Gibco), 100 g/mL G418 (Invitrogen), and 100 g/mL Hygromycin B (Invitrogen). On the day of the experiment, the cell culture medium was aspirated, and the cells were rinsed once with extracellular solution. Then, 0.25% Trypsin-EDTA (Invitrogen) solution was added, and the cells were digested for 3-5 minutes at room temperature. The digestion solution was aspirated, and the cells were resuspended in extracellular solution and then transferred to experimental dishes for electrophysiological recording for later use.

(4.2) Preparation of compounds

On the day of testing, the compounds were prepared as 10 mM stock solutions in DMSO, then diluted to 1 mM with DMSO, and further diluted 1000-fold with extracellular solution to obtain the desired final test concentration. Preparation of the positive control compound cisapride: 10 μL of a 150 μM cisapride DMSO stock solution was added to 4990 μL of extracellular solution, and diluted 500-fold to obtain the desired final test concentration of 300 nM. The DMSO content in the final test concentration did not exceed 0.2%, and DMSO at this concentration had no effect on the hERG potassium channel.

(4.3) Electrophysiological recording process

hERG potassium channel currents were recorded at room temperature using the whole-cell patch-clamp technique in CHO cells stably expressing the hERG channel. The glass microelectrode was prepared by pulling the glass electrode blank (BF150-86-10, Sutter) with a puller, and the tip resistance after filling with the internal electrode solution was about 2-5 MΩ. The glass microelectrode was inserted into the amplifier probe and then connected to the patch-clamp amplifier. The clamping voltage and data recording were controlled and recorded by pClamp software via a computer, with a sampling frequency of 10 kHz and a filtering frequency of 2 kHz. After obtaining the whole-cell recording, cells were clamped at -100 mV. The hERG potassium current (*I*hERG) was evoked by applying a 2 s depolarizing voltage step from -100 mV to +20 mV, followed by repolarization to -50 mV for 1 s before returning to -100 mV. This voltage stimulus was applied every 5 s, and the drug administration process was initiated after the hERG potassium current was confirmed to be stable (1 minute). Each test concentration of the compounds was applied for at least 1 minute to reach steady-state effect or for a maximum of 3 minutes, and at least two cells ($n \geq 2$) were tested for each concentration.

(5) Data processing

Data were analyzed and processed using pClamp, GraphPad Prism 8 and Excel software. The degree of inhibition of hERG potassium current (peak hERG tail current elicited at -50 mV) by different compound concentrations was calculated using the following formula:

$$\text{Inhibition } \% = [1 - (I / I\text{o})] \times 100\%$$

where Inhibition % represents the percentage inhibition of hERG potassium current by the compound, and *I* and *I*o represent the amplitudes of hERG potassium current after and before drug administration, respectively.

(6) Experimental results

Table 5. Inhibition results of hERG potassium channel

| Compound | hERG (inhibition%) |
|---|---|
| Example 22 | 36% @ 1 μM |
| Example 52 | 16% @ 1 μM |
| KT-474 | 47% @ 1 μM |
| Note: @ 1 μM refers to the concentration of the test compound. | |

The experimental results show that the compounds of the present disclosure have no significant inhibitory activity on hERG, indicating that the compounds of the present disclosure have good safety.

Test Example 5: Determination of *in vivo* pharmacokinetics of compounds

Experiment 1:

C57BL/6 mice were used as test animals to study the pharmacokinetic behavior of the compounds of the present disclosure in mouse plasma after oral administration at a dose of 100 mg/kg.

1. Experimental scheme

1.1 Test drugs:

Examples 13, 22, and 31 of the present disclosure.

1.2 Test animals

C57BL/6, male, transferred from the animal repository of the laboratory institution (999M-018).

1.3 Administration:

Three male C57BL/6 mice were fasted overnight (10-14 hours) with free access to water one day before administration. The mice were fed 4 hours after administration. The dose for PO administration was 100 mg/kg, with an administration volume of 20 mL/kg.

1.4 Experimental equipment

The centrifuge (5810 R) was purchased from Eppendorf Company, the pipette was purchased from Eppendorf Company, and the vortex mixer was purchased from Scientific Industries Company.

1.5 Sample collection

After administration, blood samples (0.03 mL) were collected from the mice via venipuncture at 0.083, 0.25, 0.5, 1, 2, 4, 8, and 24 hours. The samples were placed in EDTA-K2 tubes and centrifuged at 6800 rpm for 5 minutes at 2-8°C. The plasma was separated and stored at -80°C.

1.6 Sample processing

1) 10 μL of plasma sample was added to 200 μL of methanol for precipitation. After mixing, the mixture was centrifuged at 18000 g for 10 minutes.
2) The processed supernatant solution was taken for LC/MS/MS analysis to determine the concentration of the test compound.

1.7 Liquid phase analysis

Liquid phase conditions: Shimadzu LC-30AD pump
Mass spectrometry conditions: AB Sciex API 5500 mass spectrometer
Chromatographic column: Phenomenex Kinetex 2.6 μm C18 50 3.0 mm
Mobile phase: Solution A was 0.1% formic acid in water, and solution B was 0.1% formic acid in acetonitrile
Flow rate: 0.6 mL/min
Elution time: Gradient elution for 0-1.6 minutes.

2. Experimental results and analysis

The main pharmacokinetic parameters were calculated using WinNonlin 8.0.
The experimental results show that the compounds of the examples of the present disclosure have good pharmacokinetic properties.

Table 6. Pharmacokinetic parameters of some compounds of the present disclosure administered orally to mice

| Example | Dose (mpk) | $T_{1/2}$ (hr) | Tmax (hr) | Cmax (ng/mL) | $MRT_{Inf}$ (hr) | $AUC_{last}$ (hr*ng/mL) | $AUC_{Inf}$ (hr*ng/mL) |
|---|---|---|---|---|---|---|---|
| Example 13 | 100 | 7.6 | 1 | 2158 | 10.9 | 16306 | 19243 |
| Example 22 | 100 | 5.4 | 1.7 | 1396 | 8 | 17628 | 18423 |
| Example 31 | 100 | 7.3 | 3.3 | 364 | 10.4 | 4892 | 5463 |

Experiment 2:

1.1 Test drug:

Example 52 of the present disclosure.

1.2 Test animals

C57BL/6J, male, purchased from JH Laboratory Animal Co. LTD. Qualification No.: SCXK (SH) 2022-0009 20220009007223

1.3 Administration:

Two male C57BL/6 mice were allowed free access to food and water. The dose for PO administration was 100 mg/kg, with an administration volume of 10 mL/kg.

1.4 Experimental equipment

The centrifuge (5810 R) was purchased from Eppendorf Company, the pipette was purchased from Eppendorf Company, and the vortex mixer was purchased from Scientific Industries Company.

1.5 Sample collection

After administration, blood samples (0.025 mL) were collected from the mice via venipuncture at 0.083, 0.25, 0.5, 1, 2, 4, 8, and 24 hours. The samples were placed in EDTA-K2 tubes and centrifuged at 2000 g for 5 minutes at 4°C. The plasma was separated and stored at -70°C.

1.6 Sample processing

1) 5 μL of plasma sample was added to 100 μL of acetonitrile for precipitation. After mixing, the mixture was centrifuged at 5800 rmp for 10 minutes.
2) The processed supernatant solution was taken for LCMS/MS analysis to determine the concentration of the test compound.

1.7 Liquid phase analysis

LC-MS/MS instrument: LCMS/MS-45 (Triple Quad 6500+)
Chromatographic column: Waters BEH C18 (2.1 × 50 mm, 1.7 μm)
Mobile phase: Solution A was $H_2O$-0.025% FA-1 mM $NH_4OAc$, and Solution B was MeOH-0.025% FA-1 mM $NH_4OAc$.
Flow rate: 0.6 mL/min
Elution time: Gradient elution for 0-2.5 minutes.

2. Experimental results and analysis

The main pharmacokinetic parameters were calculated using WinNonlin 8.2. The pharmacokinetic parameters of the drug administered orally to mice are shown in Table 7 below. The experimental results indicate that compound 52 of the present disclosure has good pharmacokinetic properties.

Table 7. Pharmacokinetic parameters of some compounds of the present disclosure administered orally to mice

| Example | Dose (mpk) | $T_{1/2}$ **(hr)** | Tmax (hr) | Cmax (ng/mL) | $MRT_{Inf}$ (hr) | $AUC_{last}$ (hr*ng/mL) |
|---|---|---|---|---|---|---|
| Example 52 | 100 | NA | 8 | 2205 | NA | 40075 |

Experiment 3:

1.1 Test drugs:

Examples 13, 22, 31, and 52 of the present disclosure.

1.2 Test animals

Healthy Beagle dogs, male, weighing about 8-10 kg, 3 animals per group.

1.3 Experimental scheme:

Beagle dogs (3 animals per group) were administered with the compound at 2.5 mg/kg by oral gavage, with a dosing volume of 5 mL/kg, which was prepared with 10% DMSO + 50% PEG400 + 40% Water (w/v). The animals were not fasted and had free access to water.

Venous blood (0.5-0.8 mL) was collected at 15 minutes, 30 minutes, 1 hour, 2 hours, 4 hours, 8 hours, and 24 hours after oral gavage administration, placed in K2EDTA tubes, and centrifuged at 4°C to separate plasma. The concentration of the compound in plasma was determined by liquid chromatography-tandem mass spectrometry.

1.4 Experimental results:

The main pharmacokinetic parameters were calculated using WinNonlin. The pharmacokinetic parameters of the drug administered orally to dogs are shown in Table 8 below. The experimental results indicate that the compounds of the examples of the present disclosure have good pharmacokinetic properties.

Table 8. Pharmacokinetic parameters of some compounds of the present disclosure administered orally to dogs

| Example | Dose (mpk) | $T_{1/2}$ (hr) | Cmax (ng/mL) | $MRT_{Inf}$ (hr) | $AUC_{last}$ (hr*ng/mL) |
|---|---|---|---|---|---|
| Example 13 | 2.5 | 2.1 | 102 | 4.5 | 521 |
| Example 22 | 2.5 | 7.6 | 100 | 11.2 | 1194 |
| Example 31 | 2.5 | 8 | 15 | 12 | 198 |
| Example 52 | 2.5 | 13.4 | 78 | 20.2 | 1156 |
| KT-474 | 2.5 | 6.7 | 31 | 10.3 | 380 |

Test Example 6: Evaluation of the efficacy of compounds in IL-33-induced acute inflammation in mice

(1) Experimental objective

IL-33, a member of the IL-1 cytokine family, is the ligand for IL-1 receptor-like 1 or ST2. IL-33 transgenic mice, which specifically express IL-33 in keratinocytes, can spontaneously develop AD (atopic dermatitis)-like eczema, indicating that the IL-33 pathway plays a key role in AD. The effect of the compounds on inflammation in the IL-1R signaling pathway was investigated using an acute inflammatory model in mice induced by intraperitoneal injection of IL-33.

(2) Experimental materials and methods

Experimental animals: SPF-grade male C57BL/6J mice aged 6-8 weeks.

Preparation of compound: 10% DMSO + 50% PEG400 + 40% sterile water for injection. The compound was dissolved in DMSO, followed by the addition of PEG400 and sterile water for injection, and the solution was adjusted to clarity with 6N hydrochloric acid.

Preparation of IL-33 inducer: The mouse recombinant IL-33 powder was prepared as a 1 μg/μL stock solution. When using, 40 μL of the 1 μg/μL IL-33 solution was added to 3960 μL of PBS, mixed well by inversion, to obtain 4 mL of IL-33 solution with a concentration of 1 μg/100 μL, which was prepared freshly before use.

Compound administration and IL-33 inflammatory induction: The mice were randomly grouped according to body weight and orally administered with the compound at final doses of 15 mg/kg, 50 mg/kg, and 150 mg/kg, twice daily. At 4 hours after the last administration of the compound, the mice were intraperitoneally injected with 1 μg/100 μL PBS of IL-33 for inflammatory induction.

Termination, sample collection, and data: Plasma, peritoneal lavage fluid, and spleen were collected for

subsequent sample index detection.

The experiment was terminated 8 hours after the last administration. The mice were anesthetized, and cardiac blood was collected. The blood samples were anticoagulated with EDTA-K2, centrifuged at 3500 rpm for 15 minutes, and the upper plasma was collected. The euthanized mice were disinfected by immersion in 75% ethanol for 3 minutes inside a sterile biosafety cabinet. 2.5 mL of 4°C pre-cooled PBS was intraperitoneally injected, and the abdomens of mice were massaged for 5 minutes. The abdominal skin was cut open with sterile scissors and forceps to expose the peritoneum. The sternum at the xiphoid process was lifted with sterile forceps, and a 1 $mm^2$ incision was made in the peritoneum at the xiphoid process. A pipette was inserted into the peritoneal cavity for gentle aspiration and blowing, and 2 mL of lavage fluid was collected and aliquoted into 1.5 mL centrifuge tubes for storage at -80°C. The expression levels of inflammatory factors in mouse plasma and peritoneal lavage fluid were detected by ELISA, and the expression level of IRAK4 protein in mouse spleen was detected by LC-MS.

(3) Experimental results and analysis

Table 9. Drug concentrations in terminal plasma and tissues

| $C_{8hr}$ | KT474 | | | Example 22 | | |
|---|---|---|---|---|---|---|
| Dose (mpk) | 15 | 50 | 150 | 15 | 50 | 150 |
| Plasma (ng/mL) | 100 | 262 | 578 | 54 | 517 | 1392 |
| Spleen (ng/mL) | 896 | 1740 | 7262 | 369 | 6135 | 16257 |

The results in Table 9 show that Example 22 at a dose of 50 mpk achieves the same concentration levels in plasma and spleen as KT-474 at 150 mpk, indicating that Example 22 requires a lower dosage.

FIG. 1 shows the concentration-dependent reduction of inflammatory factors in mouse plasma and peritoneal lavage fluid after three PO doses of Example 22. At comparable drug concentrations in plasma, the anti-inflammatory effect of Example 22 is superior to that of KT474.

FIG. 2 shows the *in vivo* degradation of IRAK4 in mouse spleen after three PO doses of Example 22. At comparable drug concentrations in the spleen, the degradation effect of Example 22 at 50 mpk is 2-3 times better than that of KT-474 at 150 mpk.

The above description merely represents preferred examples of the present application and is not intended to limit the present application. Any modifications, equivalent substitutions, improvements, etc. made within the spirit and principles of the present application shall fall within the scope of protection of the present application.

## Claims

**1.** A bifunctional compound represented by general formula (I), an isomer thereof, a deuterated compound thereof, or a pharmaceutically acceptable salt thereof,

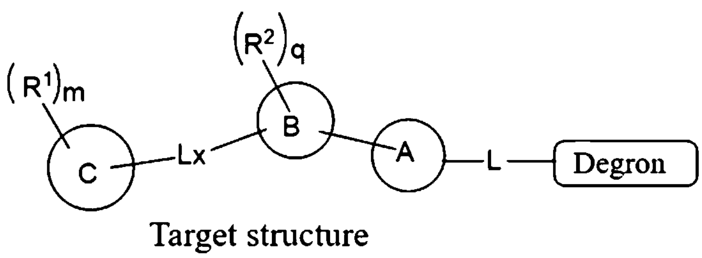

Formula (I)

wherein ring C is phenyl, 5- to 6-membered heteroaryl, or 9- to 10-membered fused heteroaryl;
$R^1$ is selected from the group consisting of -CN, $-NR^{1a}R^{1b}$, -OH, halogen, $C_{1-6}$ alkyl, halo-$C_{1-6}$ alkyl, hydroxy-$C_{1-6}$ alkyl, $C_{1-6}$ alkoxy, halo-$C_{1-6}$ alkoxy, 3- to 6-membered cycloalkyl, 3-to 6-membered heterocyclyl, 7- to 9-membered bridged heterocyclyl, 6- to 10-membered fused heterocyclyl, 3- to 6-membered cycloalkyl-NH-,

phenyl, 5- to 6-membered heteroaryl, and 9-to 10-membered fused heteroaryl, wherein the heterocyclyl, bridged heterocyclyl, fused heterocyclyl, cycloalkyl, heteroaryl, and fused heteroaryl are optionally substituted by one or more Rx, and the Rx is selected from the group consisting of $-NH_2$, $-NR^{1a}R^{1b}$, -CN, -OH, halogen, $C_{1-6}$ alkyl, and halo-$C_{1-6}$ alkyl;

$R^{1a}$ and $R^{1b}$ are each independently selected from the group consisting of H, $C_{1-6}$ alkyl, halo-$C_{1-6}$ alkyl, 3- to 6-membered cycloalkyl, 3- to 6-membered cycloalkyl-$C_{1-6}$ alkyl-, 3- to 6-membered heterocyclyl, and 3- to 6-membered heterocyclyl-$C_{1-6}$ alkyl-;

m is selected from the group consisting of 0, 1, 2, 3, and 4;

ring B is selected from the group consisting of 5- to 6-membered heteroaryl and 9- to 10-membered fused heteroaryl;

$R^2$ is selected from the group consisting of H, halogen, $C_{1-6}$ alkyl, halo-$C_{1-6}$ alkyl, hydroxy-$C_{1-6}$ alkyl, $C_{1-6}$ alkoxy, and halo-$C_{1-6}$ alkoxy;

q is selected from the group consisting of 0, 1, 2, 3, and 4;

Lx is $-C(O)-NR^{La}-$;

$R^{La}$ is selected from the group consisting of H, $C_{1-6}$ alkyl, halo-$C_{1-6}$ alkyl, and 3- to 6-membered cycloalkyl;

ring A is selected from the group consisting of divalent groups optionally substituted by one or more Rz: 7- to 11-membered spirocycloalkylene, 7- to 11-membered spiroheterocyclylene, 7- to 9-membered bridged cycloalkylene, and 7- to 9-membered bridged heterocyclylene, wherein the Rz is selected from the group consisting of halogen, -OH, $-NH_2$, $C_{1-6}$ alkyl, halo-$C_{1-6}$ alkyl, $C_{1-6}$ alkoxy, halo-$C_{1-6}$ alkoxy, 3- to 6-membered cycloalkyl, and 3- to 6-membered heterocyclyl;

L is $-(B^1)n_1-(B^2)n_2-(B^3)n_3-$;

$B^1$ is a $C_{1-6}$ alkylene chain, any one methylene unit of which is optionally replaced by -O-, -C(O)-, $-C(R^a)(R^b)-$, $-N(R^c)-$, or -S-; $n_1$ is selected from the group consisting of 0 and 1;

$B^2$ is selected from the group consisting of the following divalent rings optionally substituted by one or more Ry: $C_{3-6}$ cycloalkylene, $C_{5-8}$ bridged cycloalkylene, 4- to 6-membered heterocyclylene, 6- to 8-membered bridged heterocyclylene, phenylene, and 6- to 8-membered heterocycloalkenylene, wherein the Ry is selected from the group consisting of oxo, halogen, $C_{1-6}$ alkyl, and halo-$C_{1-6}$ alkyl; $n_2$ is selected from the group consisting of 0 and 1;

$B^3$ is a saturated or unsaturated $C_{1-6}$ hydrocarbylene chain, any one methylene unit of which is optionally replaced by -O-, -C(O)-, $-C(R^a)(R^b)-$, $-N(R^c)-$, or -S-; $n_3$ is selected from the group consisting of 0 and 1;

$R^a$ and $R^b$ are each independently selected from the group consisting of H, deuterium, halogen, $C_{1-6}$ alkyl, and halo-$C_{1-6}$ alkyl, and $R^a$ and $R^b$ are not simultaneously H;

alternatively, $R^a$ and $R^b$ on the same carbon atom together with the carbon atom to which they are attached form a 3- to 6-membered cycloalkyl ring or a 3- to 6-membered heterocyclyl ring;

$R^c$ is selected from the group consisting of H and $C_{1-6}$ alkyl;

the degron is a ligand that binds to an E3 enzyme.

**2.** The bifunctional compound, the isomer thereof, the deuterated compound thereof, or the pharmaceutically acceptable salt thereof as claimed in claim 1, wherein the bifunctional compound is represented by the following formula (IA),

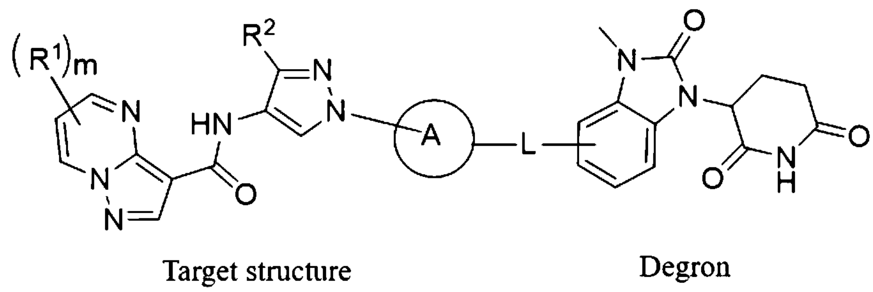

Formula (IA)

wherein $R^1$ is selected from the group consisting of -CN, halogen, $C_{1-4}$ alkyl, halo-$C_{1-4}$ alkyl, $C_{1-4}$ alkoxy, 5- to 6-membered heterocyclyl, 7- to 9-membered bridged heterocyclyl, 6-to 10-membered fused heterocyclyl, and 3- to 6-membered cycloalkyl-NH-, wherein the heterocyclyl, bridged heterocyclyl, fused heterocyclyl, and cycloalkyl are optionally substituted by one or more Rx, and the Rx is selected from the group consisting of $-NH_2$, -OH, halogen, $C_{1-4}$ alkyl, and halo-$C_{1-4}$ alkyl;

m is selected from the group consisting of 0, 1, 2, 3, and 4;
$R^2$ is selected from the group consisting of $C_{1-4}$ alkyl and halo-$C_{1-4}$ alkyl;
ring A is selected from the group consisting of the following divalent groups: 7- to 11-membered spirocycloalkylene, 7- to 11-membered spiroheterocyclylene, 7- to 9-membered bridged cycloalkylene, and 7- to 9-membered bridged heterocyclylene;
L is $-(B_1)n_1-(B^2)n_2-(B^3)n_3-$;
$B^1$ is a $C_{1-3}$ alkylene chain, any one methylene unit of which is optionally replaced by -O-, -C(O)-, -C($R^a$)($R^b$)-, -N($R^c$)-, or -S-; $n_1$ is selected from the group consisting of 0 and 1;
$B^2$ is selected from the group consisting of the following divalent rings optionally substituted by one or more Ry: $C_{3-6}$ cycloalkylene, $C_{5-8}$ bridged cycloalkylene, 4- to 6-membered heterocyclylene, 6- to 8-membered bridged heterocyclylene, phenylene, and 6- to 8-membered heterocycloalkenylene, wherein the Ry is selected from the group consisting of oxo, halogen, $C_{1-4}$ alkyl, and halo-$C_{1-4}$ alkyl; $n_2$ is selected from the group consisting of 0 and 1;
$B^3$ is a saturated or unsaturated $C_{1-6}$ hydrocarbylene chain, any one methylene unit of which is optionally replaced by -O-, -C(O)-, -C($R^a$)($R^b$)-, -N($R^c$)-, or -S-; $n_3$ is selected from the group consisting of 0 and 1;
$R^a$ and $R^b$ are each independently selected from the group consisting of H, deuterium, halogen, $C_{1-4}$ alkyl, and halo-$C_{1-4}$ alkyl, and $R^a$ and $R^b$ are not simultaneously H;
alternatively, $R^a$ and $R^b$ on the same carbon atom together with the carbon atom to which they are attached form a 3- to 6-membered cycloalkyl ring;
$R^c$ is selected from the group consisting of H and $C_{1-4}$ alkyl.

3. The bifunctional compound, the isomer thereof, the deuterated compound thereof, or the pharmaceutically acceptable salt thereof as claimed in claim 1 or 2, wherein the bifunctional compound, the isomer thereof, the deuterated compound thereof, or the pharmaceutically acceptable salt thereof satisfies one or more of the following conditions:

(1) the heteroatom in the heterocyclyl, bridged heterocyclyl, fused heterocyclyl, heteroaryl, fused heteroaryl, or spiroheterocyclyl is independently selected from the group consisting of one, two, or three types of N, O, and S, and the number of heteroatoms is independently 1, 2, 3, or 4;
(2) $R^1$ is selected from the group consisting of -CN, halogen, $C_{1-6}$ alkyl, halo-$C_{1-6}$ alkyl, $C_{1-6}$ alkoxy, 3- to 6-membered heterocyclyl, 7- to 9-membered bridged heterocyclyl, 6- to 10-membered fused heterocyclyl, and 3- to 6-membered cycloalkyl-NH-;
(3) Rx is selected from the group consisting of $-NH_2$ and $C_{1-6}$ alkyl;
(4) m is selected from the group consisting of 1 and 2, preferably 1;
(5) ring B is 5- to 6-membered heteroaryl;
(6) $R^2$ is $C_{1-6}$ alkyl or halo-$C_{1-6}$ alkyl, preferably halo-$C_{1-6}$ alkyl;
(7) q is 1;
(8) Lx is -C(O)-$NR^{La}$-*, wherein the * terminus is connected to ring B;
(9) in L, $-(B^3)n_3-$ terminus is connected to the degron;
(10) ring A is selected from the group consisting of the following unsubstituted divalent groups: 7- to 11-membered spirocycloalkylene, 7- to 11-membered spiroheterocyclylene, 7- to 9-membered bridged cycloalkylene, and 7- to 9-membered bridged heterocyclylene.

4. The bifunctional compound, the isomer thereof, the deuterated compound thereof, or the pharmaceutically acceptable salt thereof as claimed in any one of claims 1 to 3, wherein $R^1$ is 7- to 9-membered bridged heterocyclyl.

5. The bifunctional compound, the isomer thereof, the deuterated compound thereof, or the pharmaceutically acceptable salt thereof as claimed in any one of claims 1 to 3, wherein $R^1$ is selected from the group consisting of -F, $-CH_3$, -CN, $-CHF_2$, $-CF_3$, $-OCH_3$,

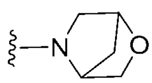
,

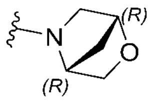, 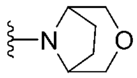, 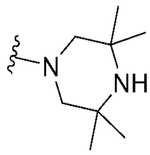, 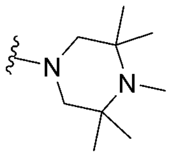, 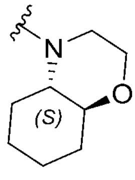, and 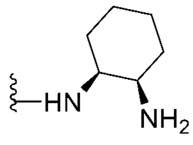;

preferably

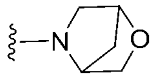 and 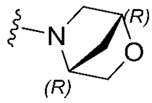;

m is selected from the group consisting of 1 and 2.

**6.** The bifunctional compound, the isomer thereof, the deuterated compound thereof, or the pharmaceutically acceptable salt thereof as claimed in any one of claims 1 to 5, wherein $R^2$ is selected from the group consisting of $-CH_3$, $-CH_2F$, $-CHF_2$, and $-CF_3$; preferably $-CHF_2$.

**7.** The bifunctional compound, the isomer thereof, the deuterated compound thereof, or the pharmaceutically acceptable salt thereof as claimed in any one of claims 1 to 6, wherein ring A is 7- to 9-membered nitrogen-containing spiroheterocyclylene.

**8.** The bifunctional compound, the isomer thereof, the deuterated compound thereof, or the pharmaceutically acceptable salt thereof as claimed in any one of claims 1 to 6, wherein ring A is selected from the group consisting of

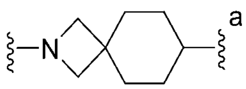, 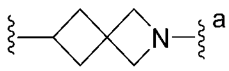,

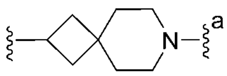, 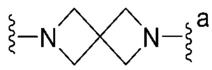, 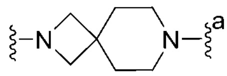, 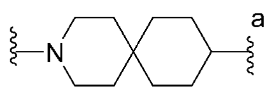,

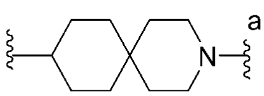, 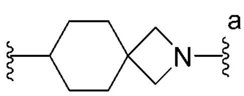, 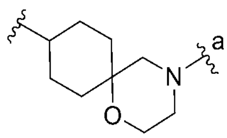, 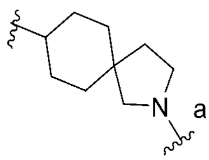,

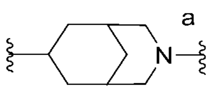, 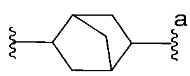, 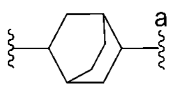, 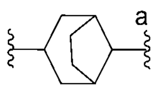, 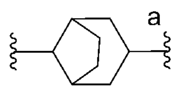,

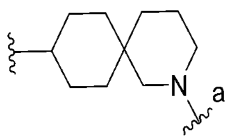, 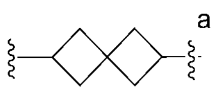, 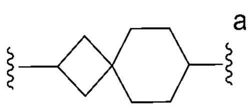, 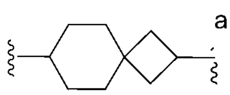,

and

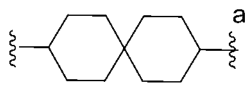,

wherein the "a" terminus denotes the end connected to L in general formula (IA); preferably, ring A is

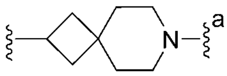.

**9.** The bifunctional compound, the isomer thereof, the deuterated compound thereof, or the pharmaceutically acceptable salt thereof as claimed in any one of claims 1 to 8, wherein L is selected from the group consisting of $-B^1-B^2-B^3-$, $-B^1-B^3-$, and $-B^2-B^3-$; preferably, L is $-B^1-B^2-B^3-$; more preferably, the $-B^3-$ terminus is connected to the degron; alternatively, L is $-B^3-$.

**10.** The bifunctional compound, the isomer thereof, the deuterated compound thereof, or the pharmaceutically acceptable salt thereof as claimed in any one of claims 1 to 9, wherein the bifunctional compound, the isomer thereof, the deuterated compound thereof, or the pharmaceutically acceptable salt thereof satisfies one or more of the following conditions:

(1) $B^1$ is a $C_{1-3}$ alkylene chain, wherein 1 to 2 methylene units are optionally replaced by -O-, -C(O)-, $-C(R^a)(R^b)-$, or $-N(R^c)-$; preferably, 1 methylene unit is optionally replaced by - C(O);
(2) $B^3$ is an unsaturated $C_{2-6}$ hydrocarbylene chain, wherein 1 to 2 methylene units are optionally replaced by -O-, -C(O)-, or $-C(R^a)(R^b)-$; the hydrocarbylene chain is connected to the degron via the alkyne terminus; preferably, 1 methylene unit is optionally replaced by -O;
(3) $R^a$ and $R^b$ are each independently selected from the group consisting of H, deuterium, and $-CH_3$; and $R^a$ and $R^b$ are not simultaneously H; alternatively, $R^a$ and $R^b$ on the same carbon atom together with the carbon atom to which they are attached form a cyclopropyl ring or a cyclobutyl ring;
(4) $R^c$ is selected from the group consisting of H and $-CH_3$; preferably H;
(5) $B^2$ is selected from the group consisting of the following groups:

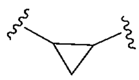,

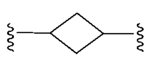, 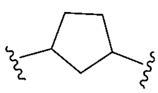, 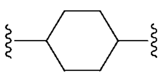, 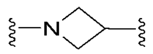, 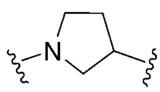, 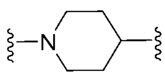,

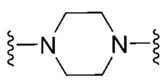, 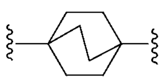, 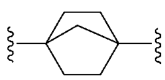, 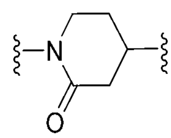, 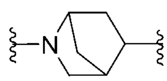, 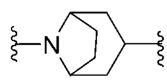,

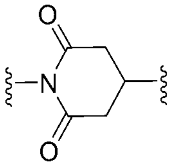, 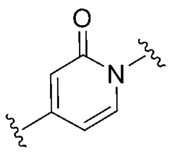, 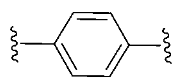, 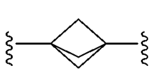, and 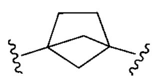;

preferably

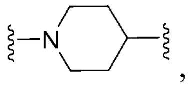, 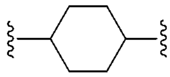, or 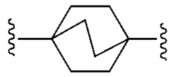;

preferably, $B^2$ is selected from the group consisting of the following groups:

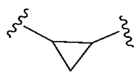, 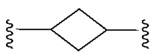, 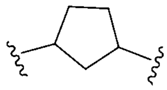, 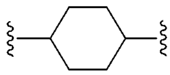,

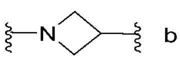, 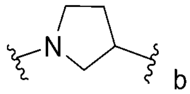, 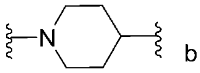, 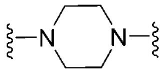, 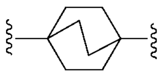,

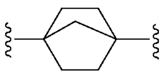, 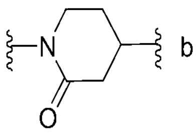, 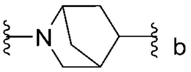, 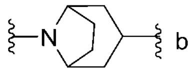, 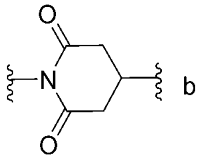,

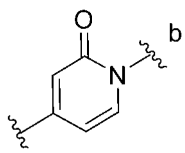, 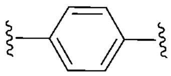, 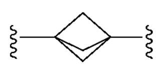, and 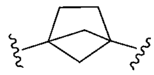,

wherein the b terminus is connected to $-(B^3)n_3-$; preferably

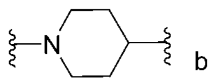, 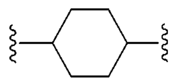, or 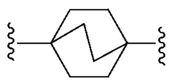.

**11.** The bifunctional compound, the isomer thereof, the deuterated compound thereof, or the pharmaceutically acceptable salt thereof as claimed in any one of claims 1 to 10, wherein $B^1$ is a $C_{1-3}$ alkylene chain, wherein 1 to 2 methylene units are optionally replaced by -O-, - C(O)-, $-C(R^a)(R^b)-$, or $-N(R^c)-$;

$B^3$ is an unsaturated $C_{2-6}$ hydrocarbylene chain, wherein 1 to 2 methylene units are optionally replaced by -O-, -C(O)-, or $-C(R^a)(R^b)-$; the hydrocarbylene chain is connected to the degron via the alkyne terminus;
$R^a$ and $R^b$ are each independently selected from the group consisting of H, deuterium, and $-CH_3$; and $R^a$ and $R^b$ are not simultaneously H; alternatively, $R^a$ and $R^b$ on the same carbon atom together with the carbon atom to which they are attached form a cyclopropyl ring or a cyclobutyl ring;
$R^c$ is selected from the group consisting of H and $-CH_3$;
$B^2$ is selected from the group consisting of the following groups:

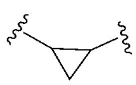,

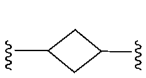, 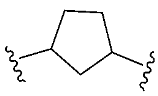, 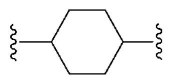, 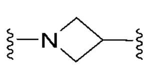, 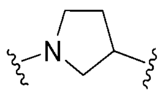, 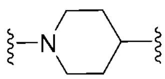,

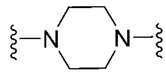, 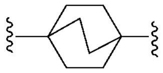, 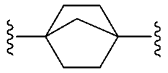, 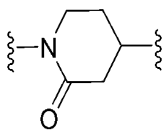, 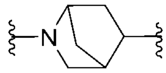, 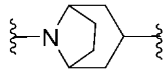,

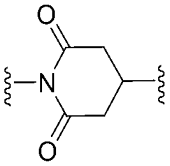, 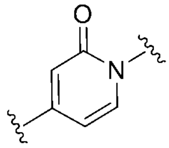, 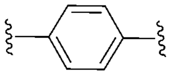, 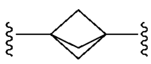, and 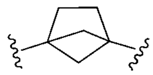.

**12.** The bifunctional compound, the isomer thereof, the deuterated compound thereof, or the pharmaceutically acceptable salt thereof as claimed in any one of claims 1 to 11, wherein $B^3$ is a $C_{2-6}$ alkynylene chain, wherein 1 to 2 methylene units are optionally replaced by -O- or -C(O)-; the alkynylene chain is connected to the degron via the alkyne terminus; preferably, $B^3$ is a $C_{2-6}$ alkynylene chain containing only one triple bond at the terminus; more preferably, $B^3$ is a $C_{2-4}$ alkynylene chain containing only one triple bond at the terminus.

**13.** The bifunctional compound, the isomer thereof, the deuterated compound thereof, or the pharmaceutically acceptable salt thereof as claimed in any one of claims 1 to 12, wherein $B^1$ is selected from the group consisting of $-CH_2-$, $-CD_2-$, and -C(O)-;

$B^2$ is selected from the group consisting of the following divalent rings: $C_{5-6}$ cycloalkylene, $C_{6-8}$ bridged cycloalkylene, 6-membered heterocyclylene, and 6- to 8-membered bridged heterocyclylene;
$B^3$ is selected from the group consisting of

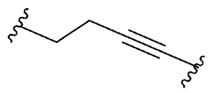, 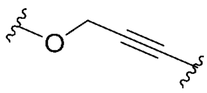,

and

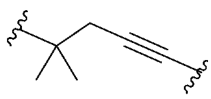.

**14.** The bifunctional compound, the isomer thereof, the deuterated compound thereof, or the pharmaceutically acceptable salt thereof as claimed in any one of claims 1 to 13, wherein L is selected from the group consisting of

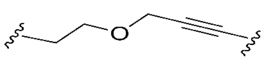, 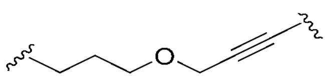,

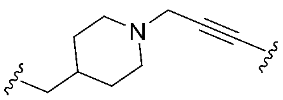, 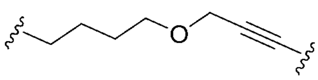, 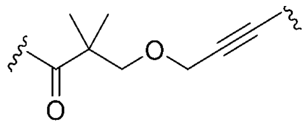,

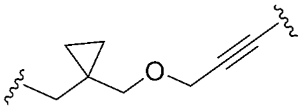, 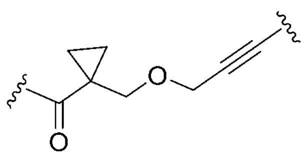, 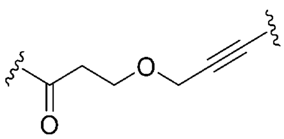,

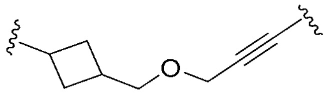, 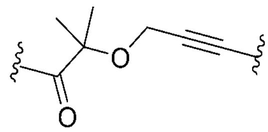, 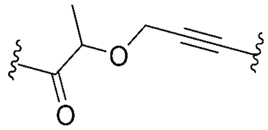,

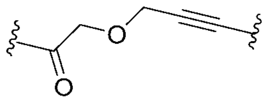, 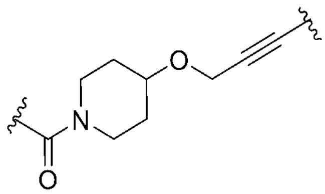, 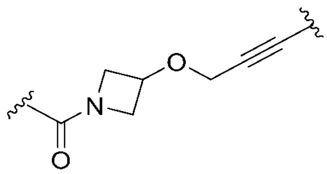,

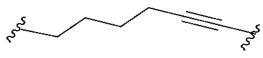, 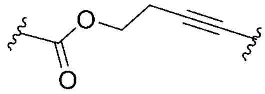, 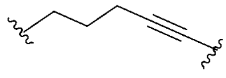, 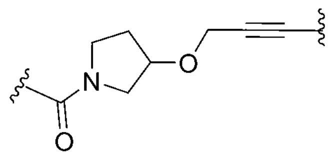,

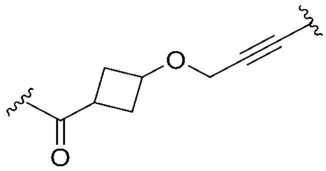, 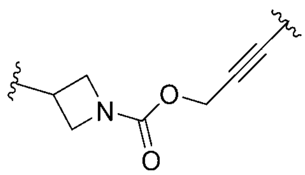, 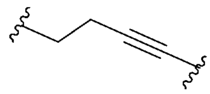, 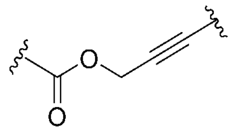,

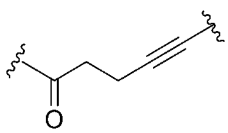, 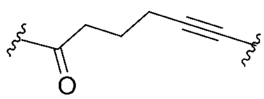, 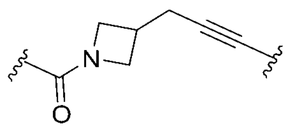, 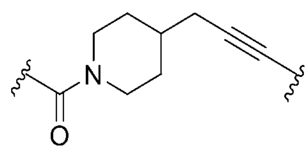,

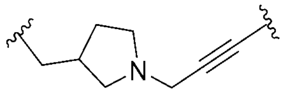, 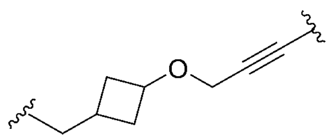, 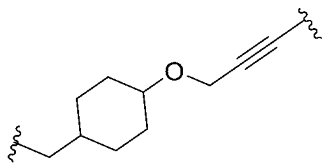,

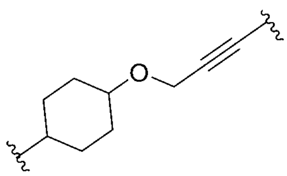, 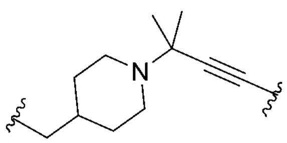, 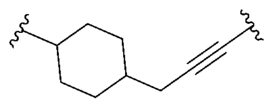,

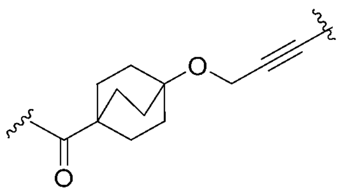, 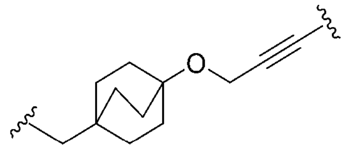, 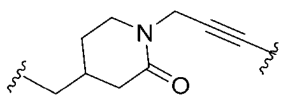,

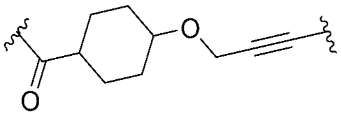 , 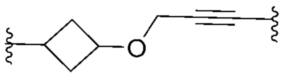 , 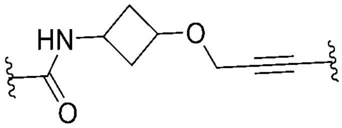 ,

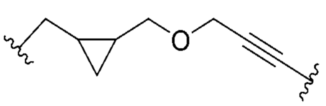 , 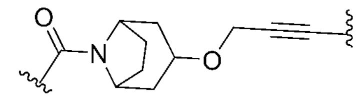 , 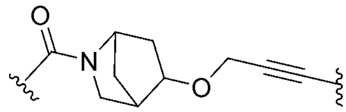 ,

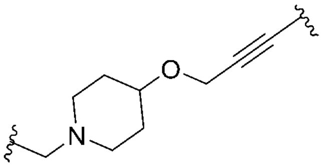 , 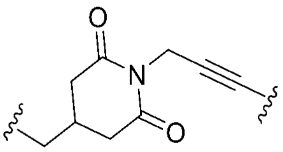 , 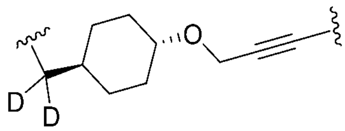 ,

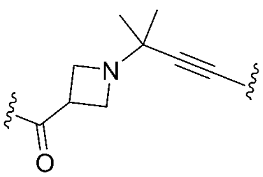 , 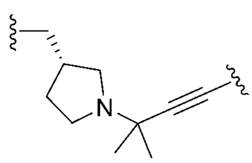 , 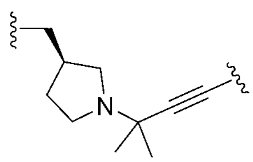 , 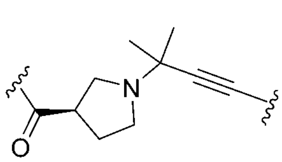 ,

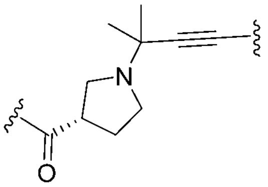 , 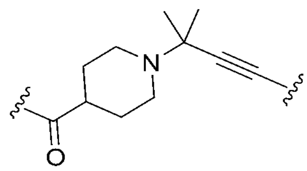 , 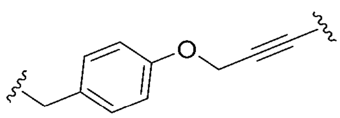 ,

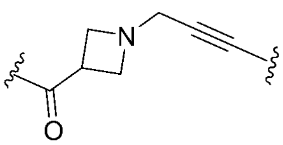 , 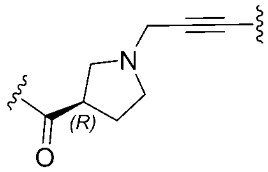 , 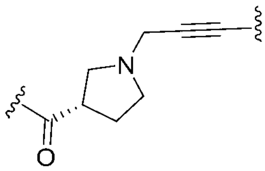 , 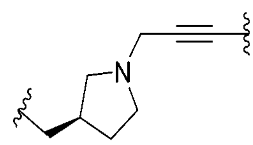 ,

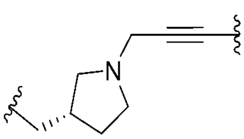 , 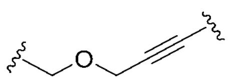 , 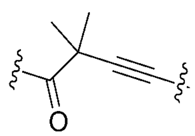 , 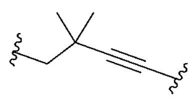 ,

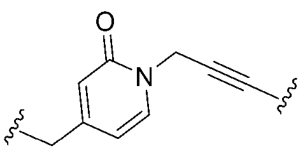 , 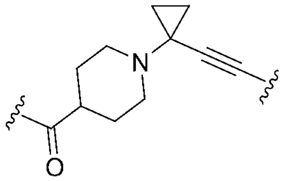 , 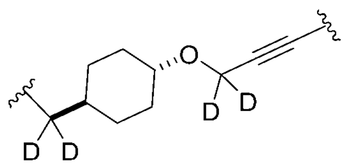 ,

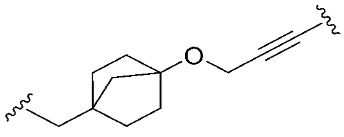, 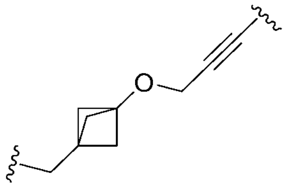, and 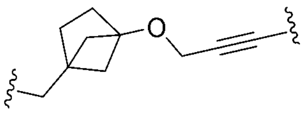;

preferably, L is selected from the group consisting of

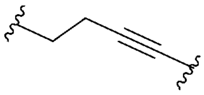,

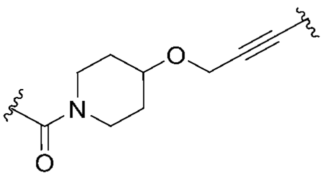, 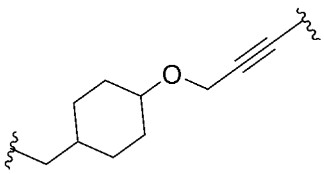, and 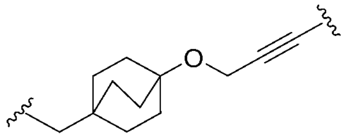;

more preferably, the alkyne terminus of L is connected to the degron.

**15.** The bifunctional compound, the isomer thereof, the deuterated compound thereof, or the pharmaceutically acceptable salt thereof as claimed in any one of claims 1 to 14, wherein the bifunctional compound, the isomer thereof, the deuterated compound thereof, or the pharmaceutically acceptable salt thereof satisfies one or more of the following conditions:

(1) each 9- to 10-membered fused heteroaryl is pyrazolopyrimidinyl, for example,

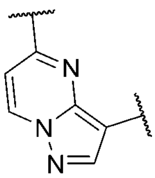;

(2) each halogen is fluorine, chlorine, bromine, or iodine, for example, fluorine;
(3) each $C_{1-6}$ alkyl is $C_{1-4}$ alkyl, for example, methyl;
(4) each $C_{1-6}$ alkoxy is $C_{1-4}$ alkoxy, for example, methoxy;
(5) the heteroatom in each 3- to 6-membered heterocyclyl is independently selected from the group consisting of one, two, or three types of N, O, and S, and the number of heteroatoms is independently 1 or 2, for example,

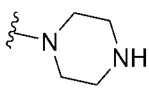;

(6) the heteroatom in each 7- to 9-membered bridged heterocyclyl is independently selected from the group consisting of one, two, or three types of N, O, and S, and the number of heteroatoms is independently 1 or 2, for example,

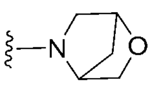, 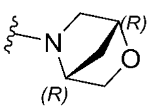, or 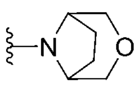;

(7) the heteroatom in each 6- to 10-membered fused heterocyclyl is independently selected from the group

consisting of one, two, or three types of N, O, and S, and the number of heteroatoms is independently 1 or 2, for example,

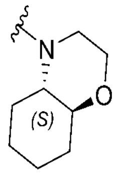;

(8) each 3- to 6-membered cycloalkyl is cyclopropyl, cyclobutyl, cyclopentyl, or cyclohexyl, for example, cyclohexyl;
(9) each 5- to 6-membered heteroaryl is pyrazolyl, for example,

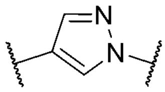;

(10) each 7- to 11-membered spirocycloalkylene is

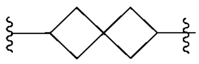, 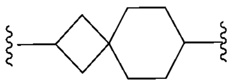,

or

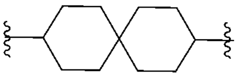;

(11) each 7- to 11-membered spiroheterocyclylene is

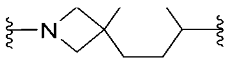,

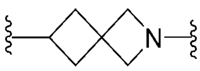, 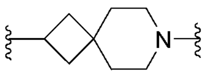, 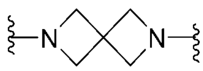, 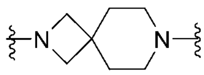,

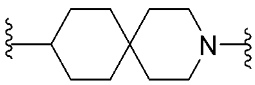, 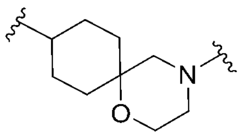, 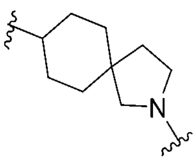, or 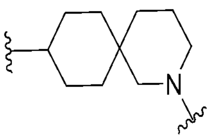;

(12) each 7- to 9-membered bridged cycloalkylene is

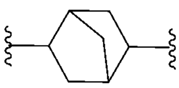, 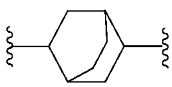,

or

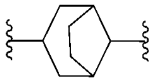;

(13) each 7- to 9-membered bridged heterocyclylene is

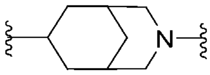;

(14) each $C_{1-6}$ alkylene chain is a $C_{1-4}$ alkylene chain, for example, methylene or ethylene;
(15) each $C_{3-6}$ cycloalkylene is

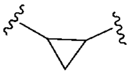, 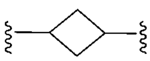, 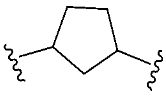, or 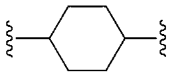;

(16) each $C_{5-8}$ bridged cycloalkylene is

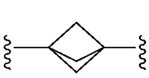, 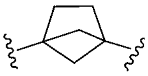, 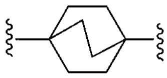,

or

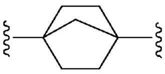;

(17) each 4- to 6-membered heterocyclylene is

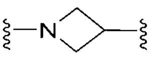, 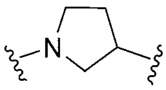,

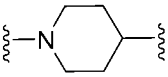, or 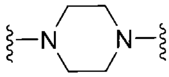;

(18) each 6- to 8-membered bridged heterocyclylene is

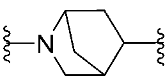 or 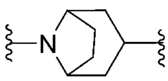;

(19) each 6- to 8-membered heterocycloalkenylene is

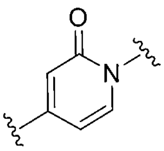;

(20) each saturated or unsaturated $C_{1-6}$ hydrocarbylene chain is an unsaturated $C_{3-6}$ alkynylene chain, preferably

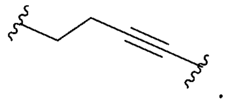

16. The bifunctional compound, the isomer thereof, the deuterated compound thereof, or the pharmaceutically acceptable salt thereof as claimed in any one of claims 1 to 15, wherein the bifunctional compound has a structure represented by the following general formula:

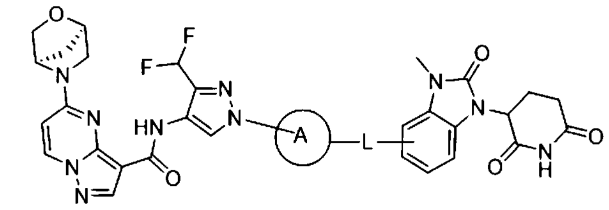

Formula (IA-1)

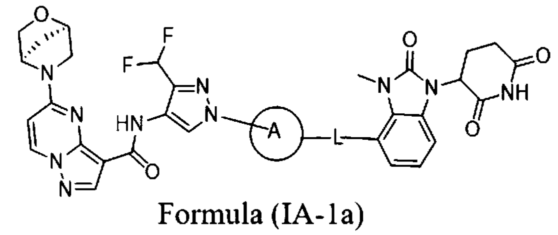

Formula (IA-1a)

or

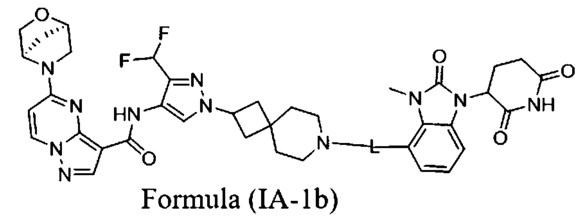

Formula (IA-1b)

ring A and L are as defined in any one of claims 1 to 15.

17. A bifunctional compound as shown below, an isomer thereof, a deuterated compound thereof, or a pharmaceutically acceptable salt thereof:

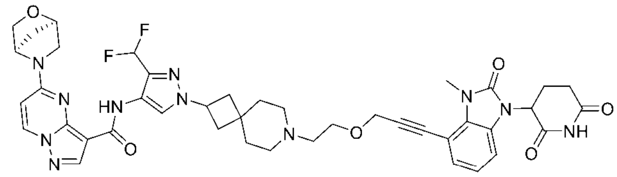

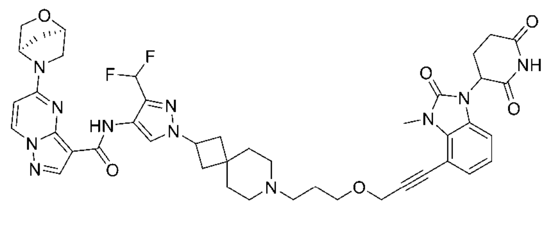

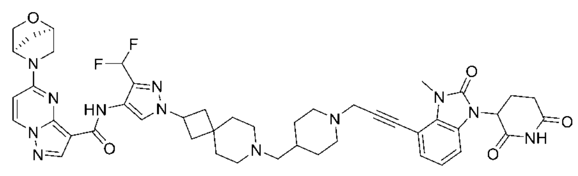

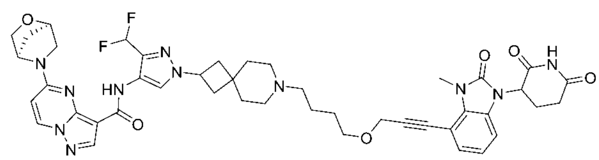

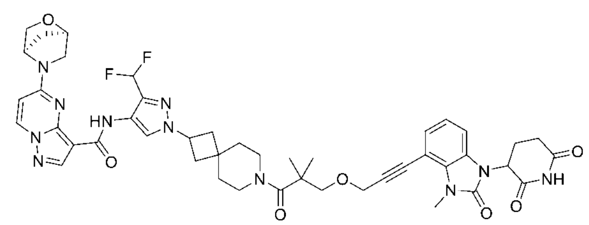

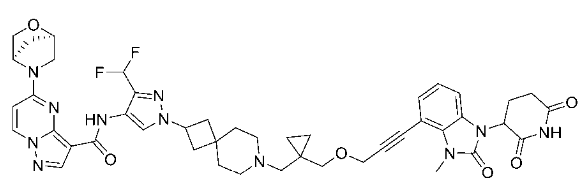

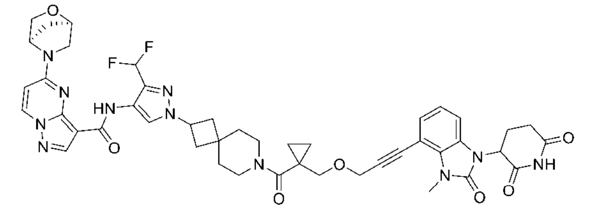

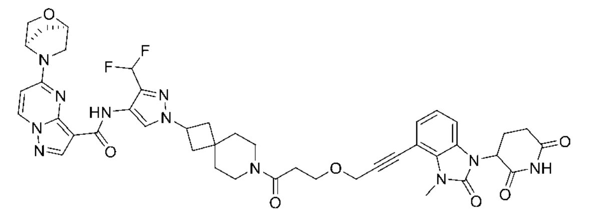

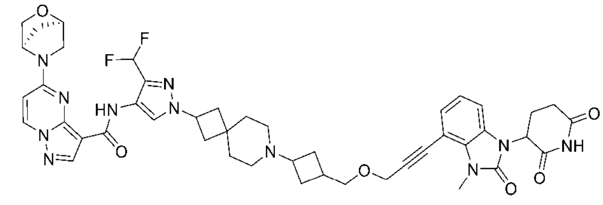

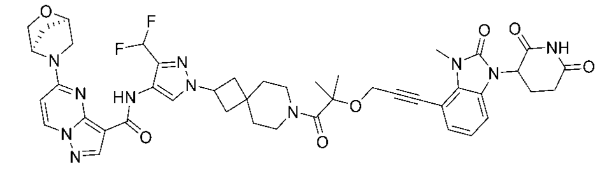

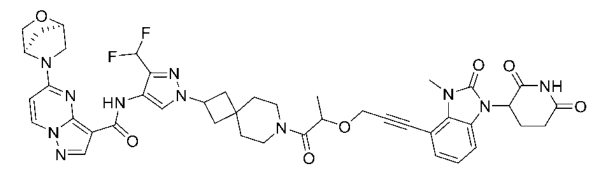

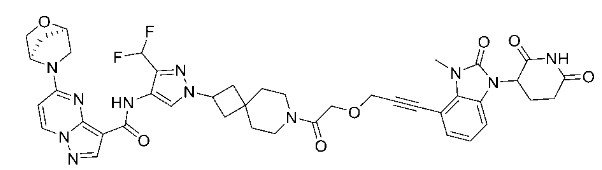

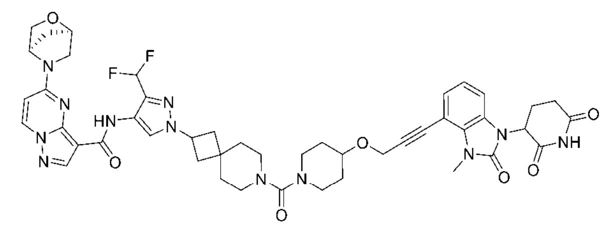

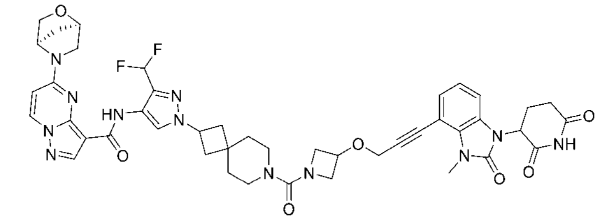

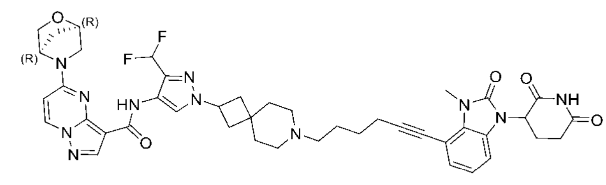

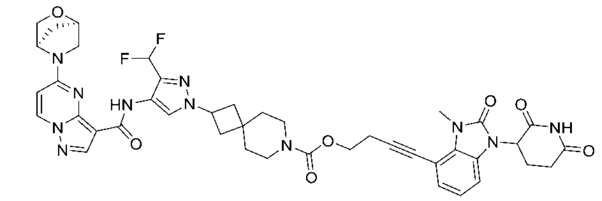

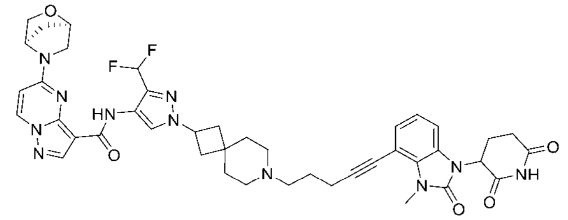

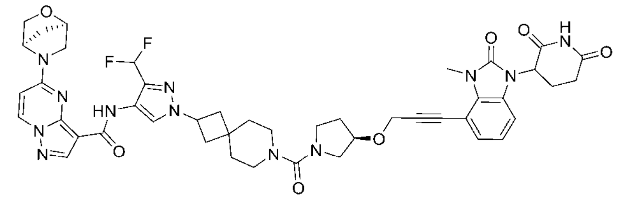

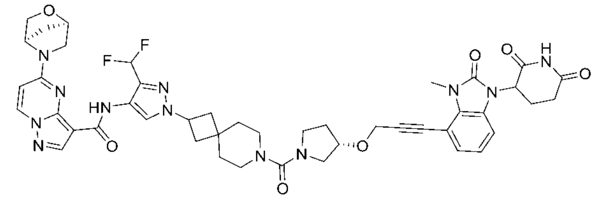

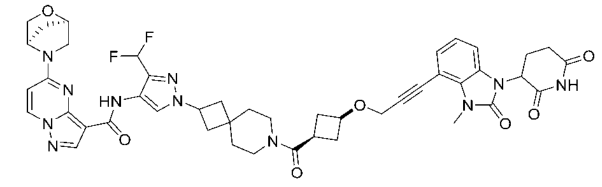

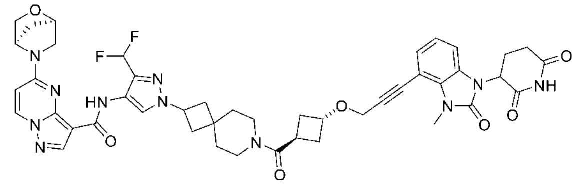

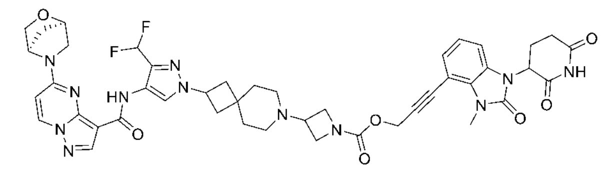

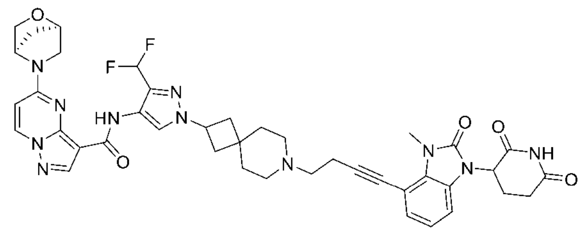

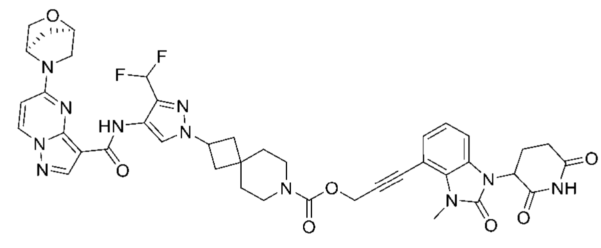

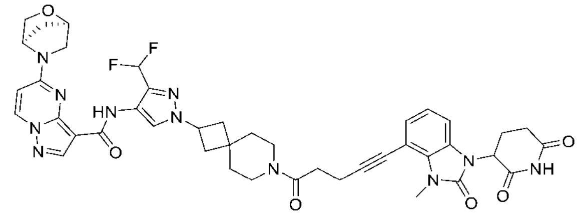

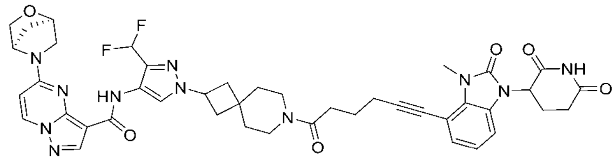

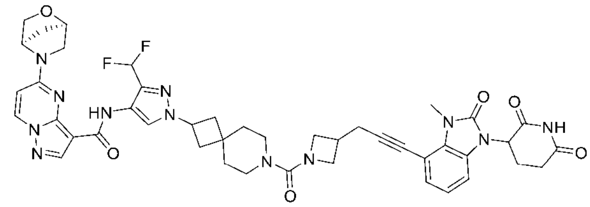

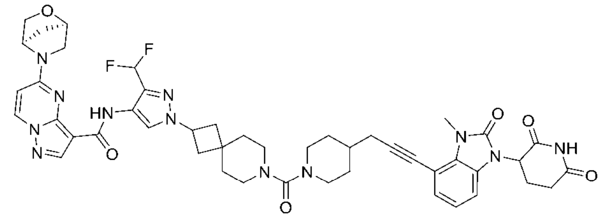

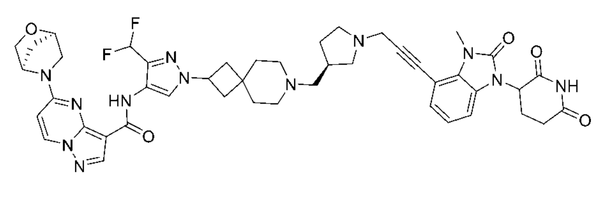

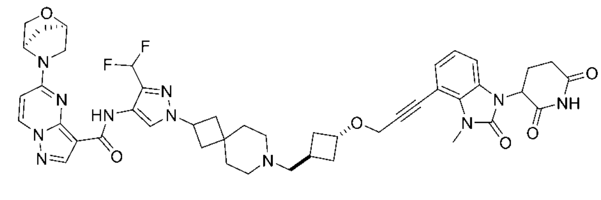

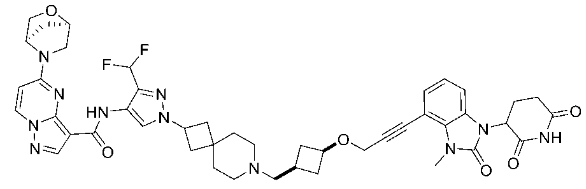

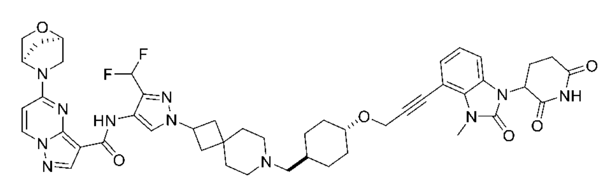

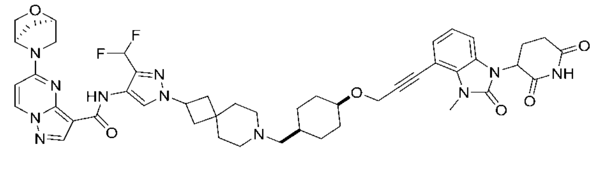

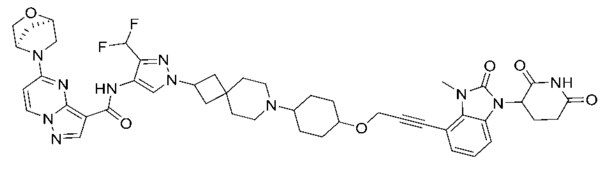

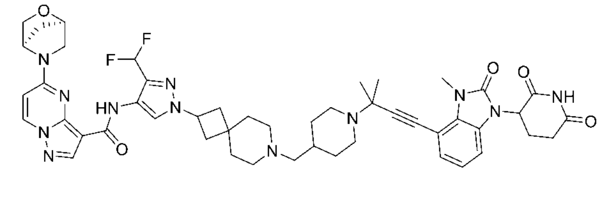

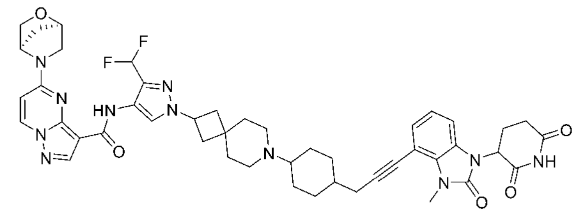

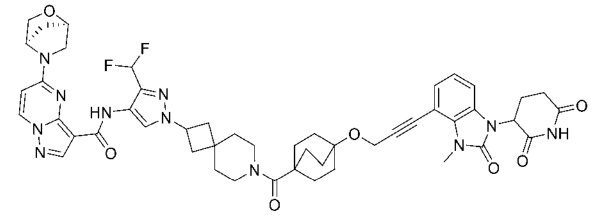

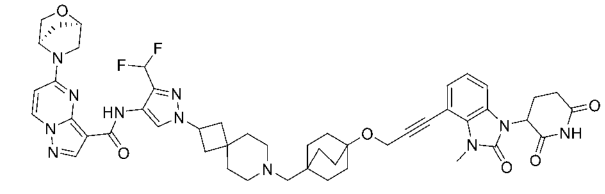

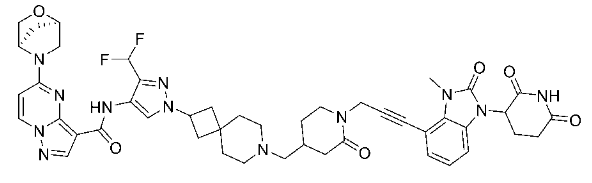

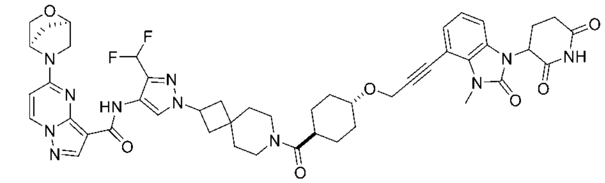

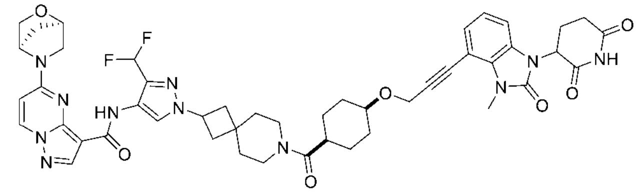

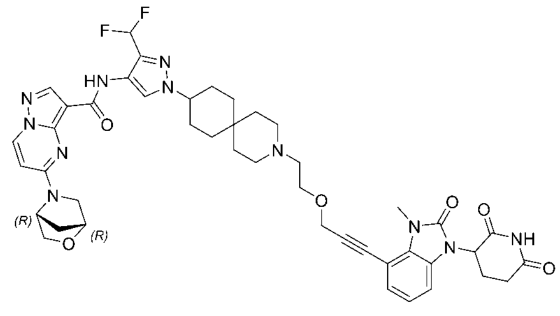

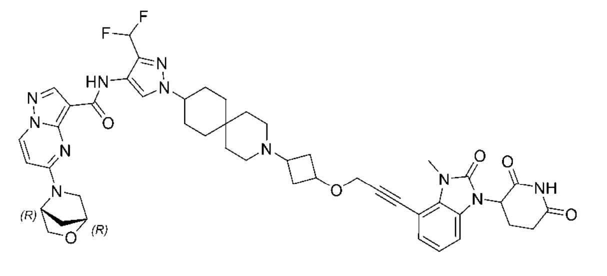

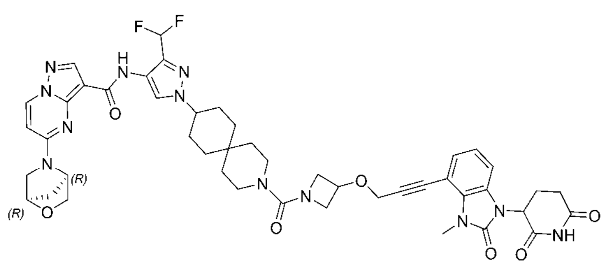

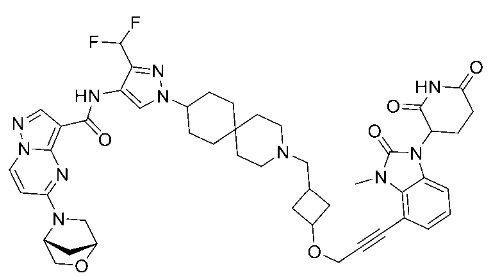

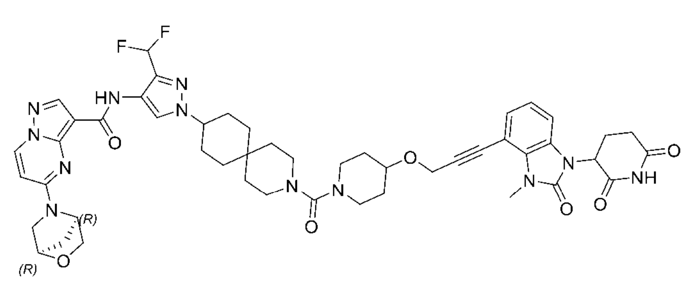

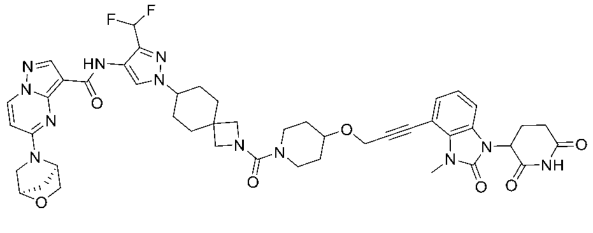

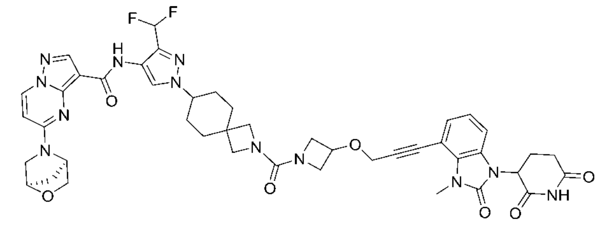

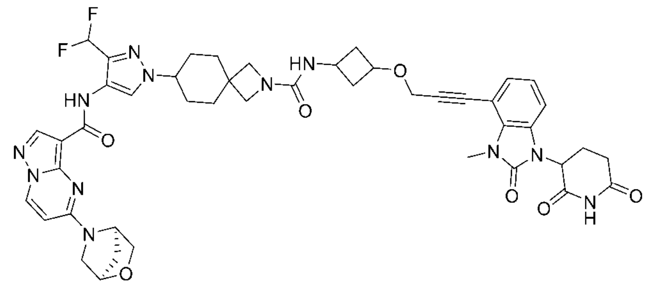

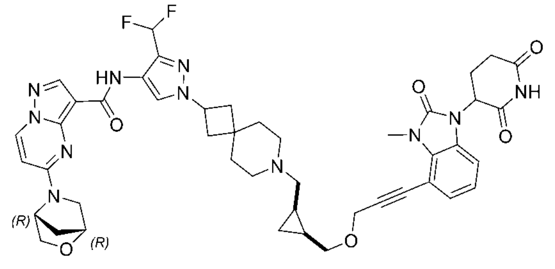

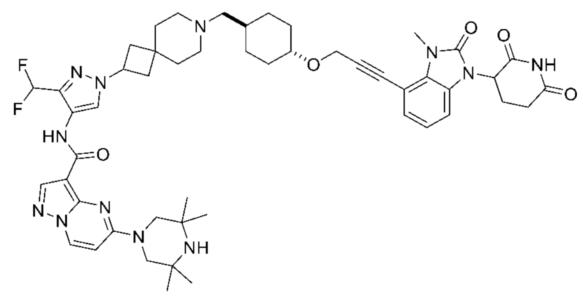

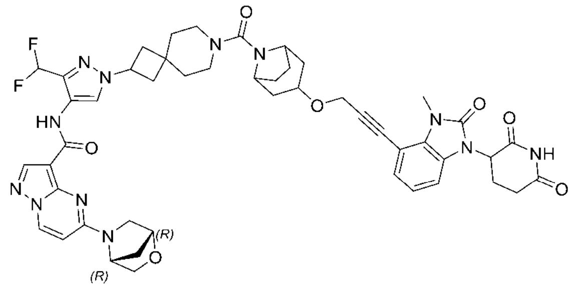

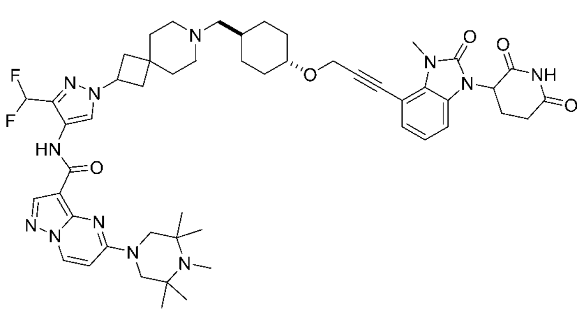

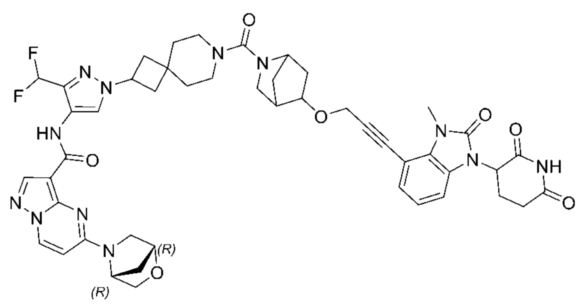

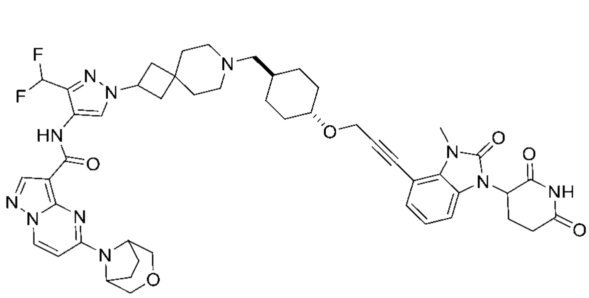

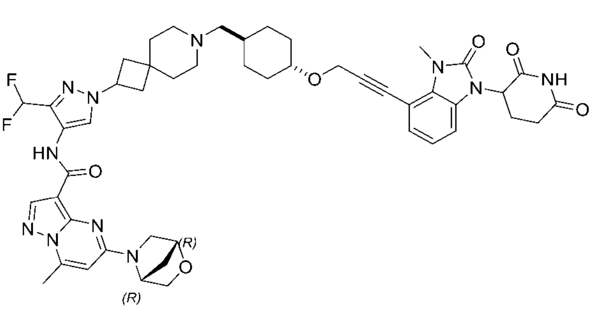

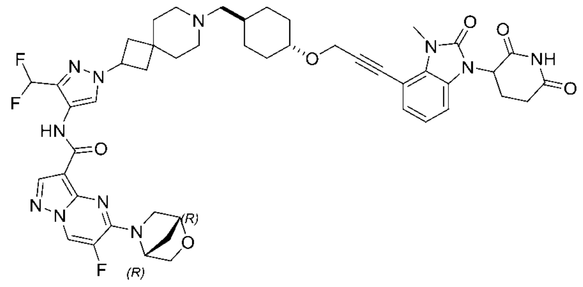

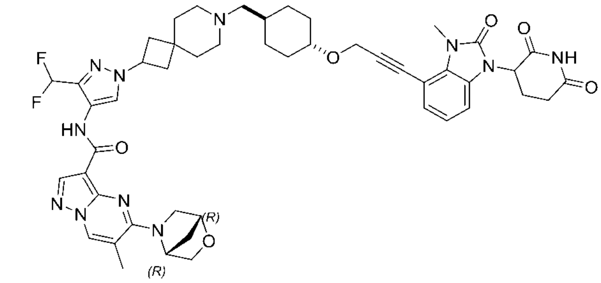

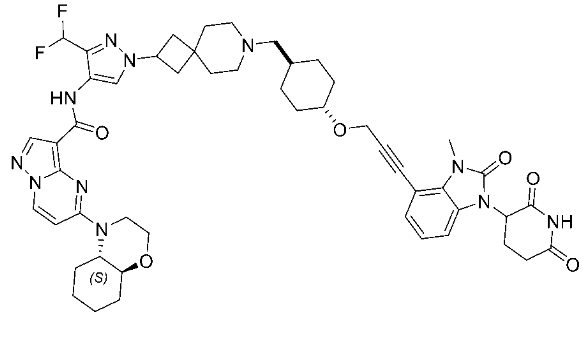

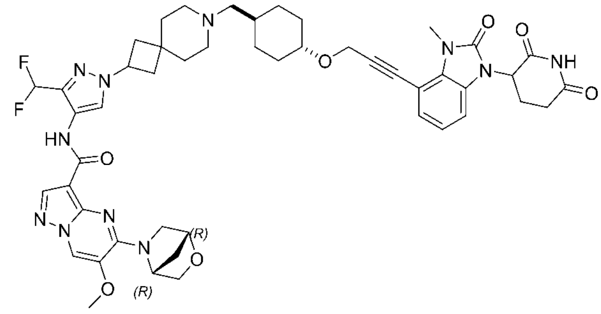

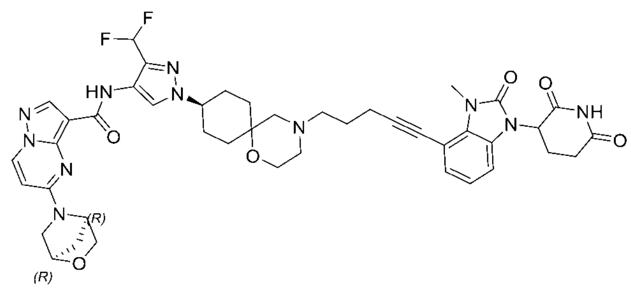

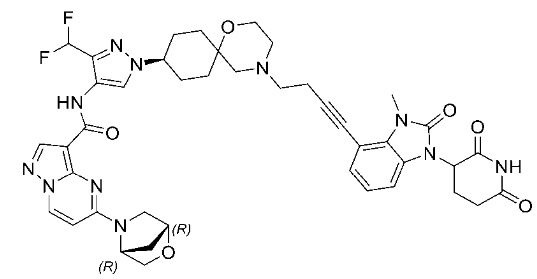

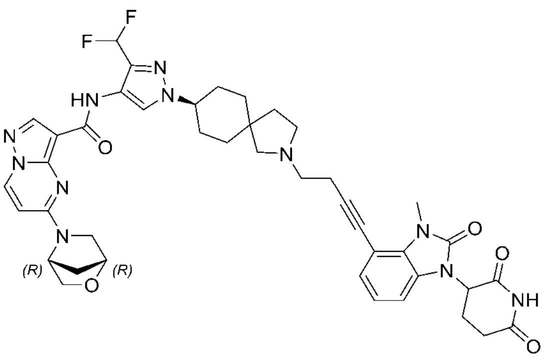

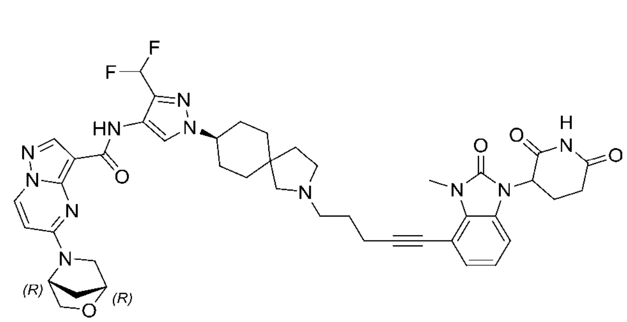

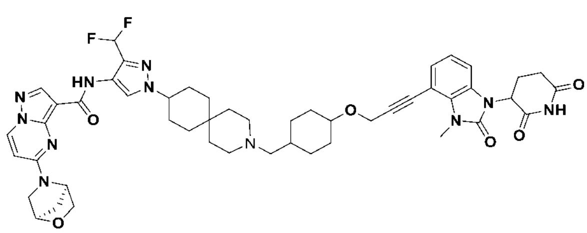

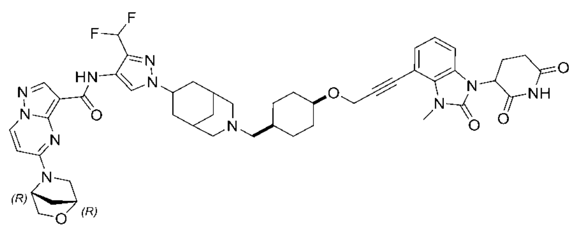

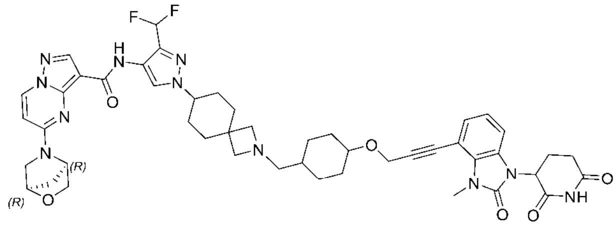

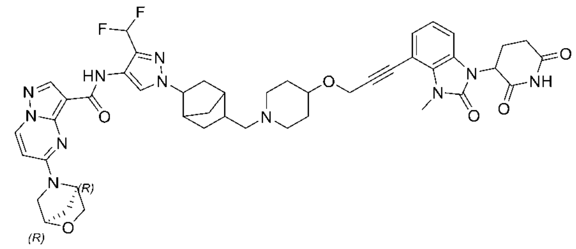

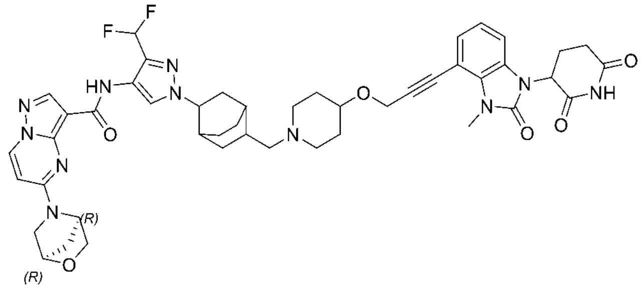

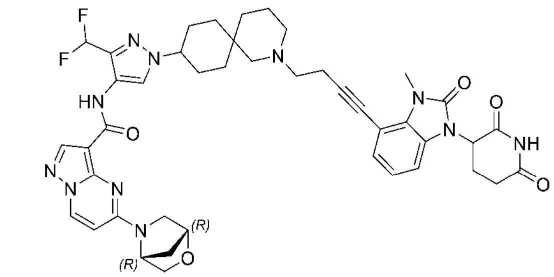

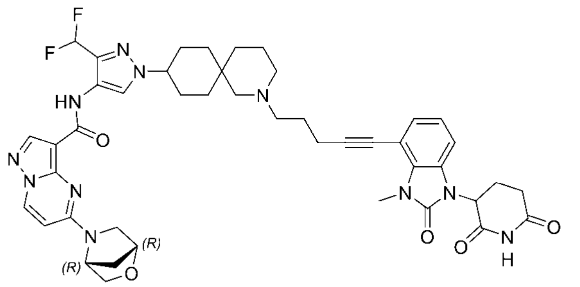

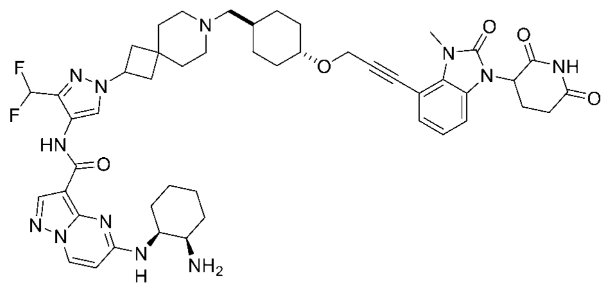

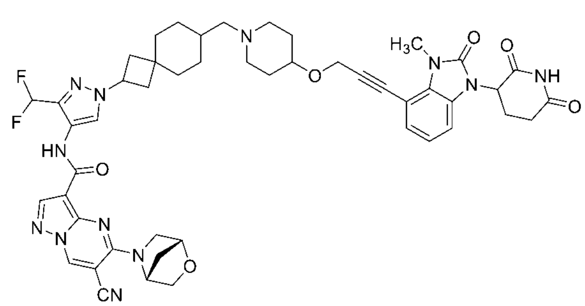

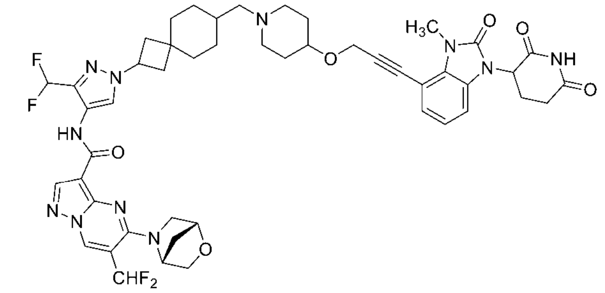

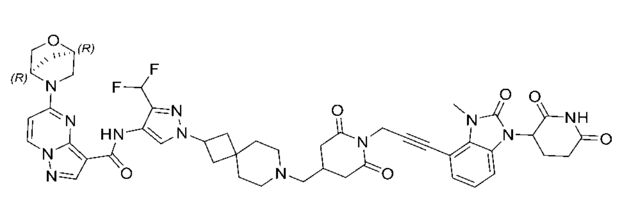

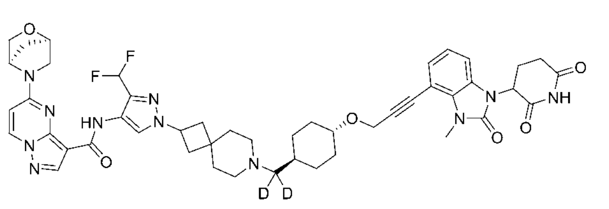

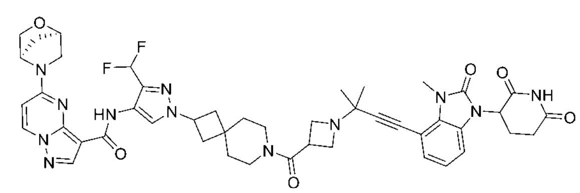

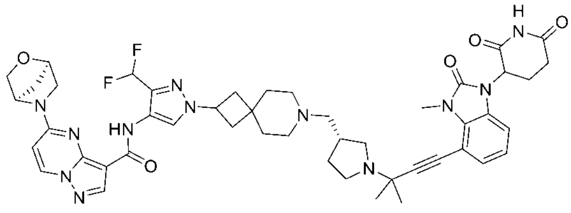

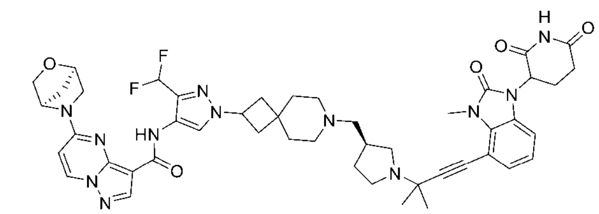

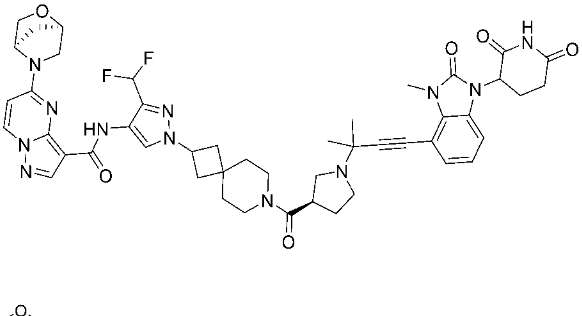

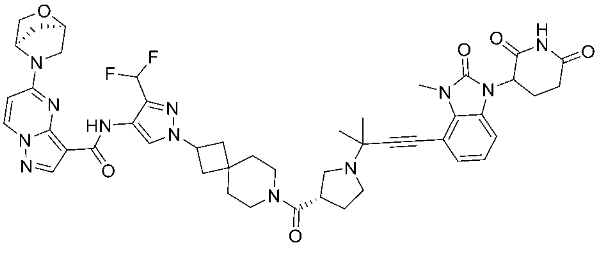

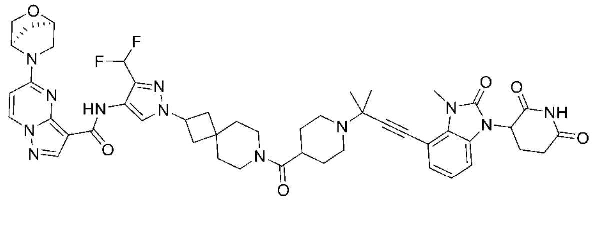

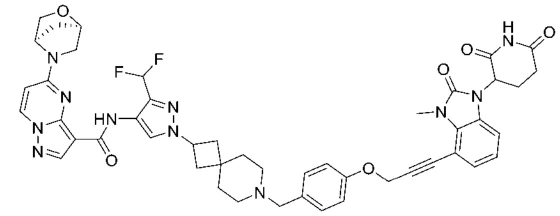

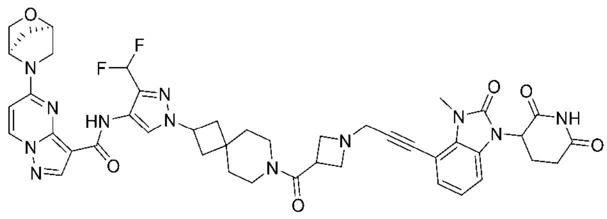

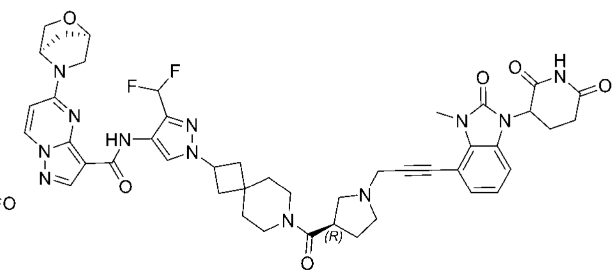

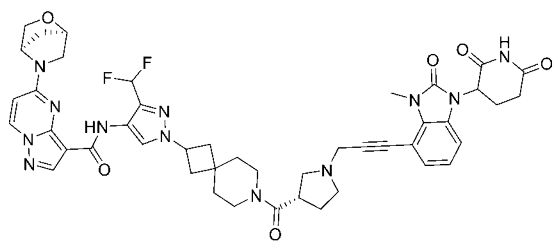

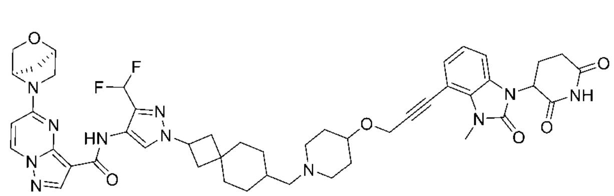

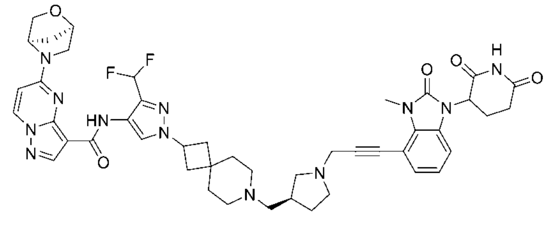

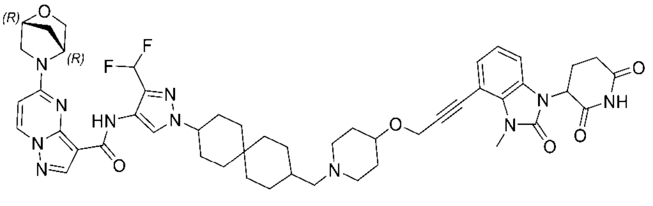

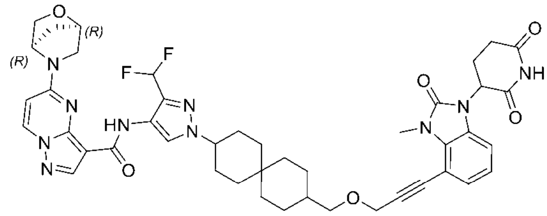 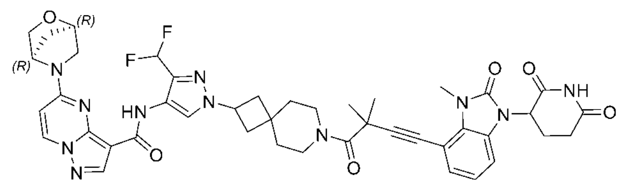

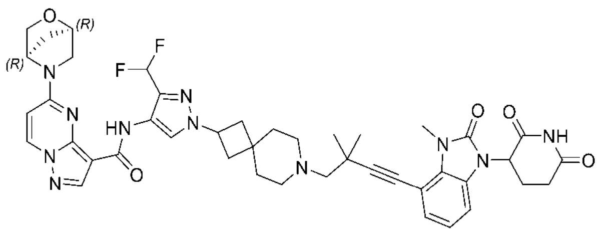 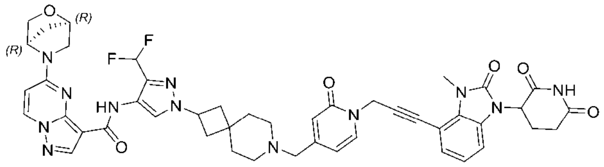

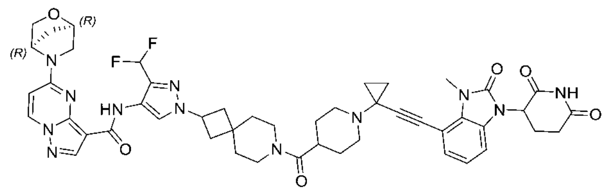 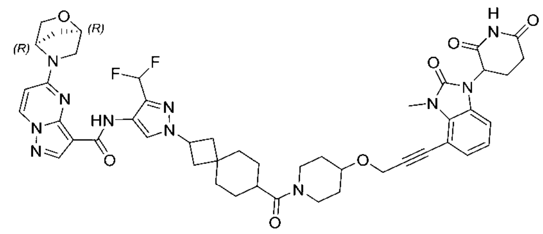

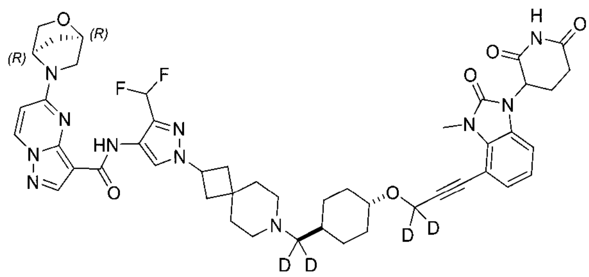 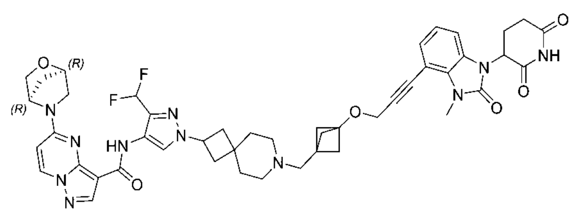

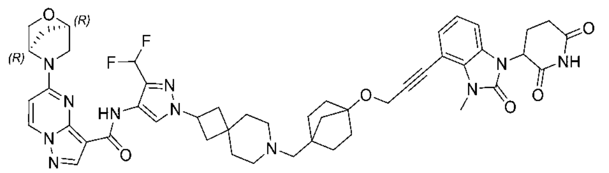

or

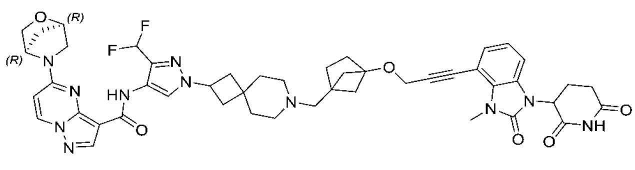.

**18.** A bifunctional compound represented by the following formula (IB), an isomer thereof, a deuterated compound thereof, or a pharmaceutically acceptable salt thereof,

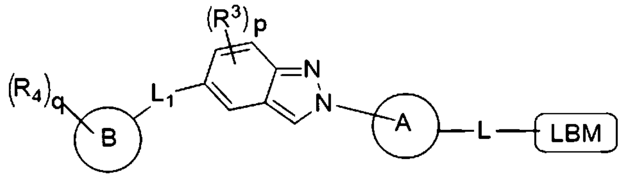

Formula (IB)

wherein ring A and L are as defined in any one of claims 1 to 15;
ring B is 8- to 10-membered fused heteroaryl;
$L_1$ is selected from the group consisting of -N($R^d$)-C(O)- and -C(O)-N($R^d$)-;
$R^d$ is selected from the group consisting of H and $C_{1-4}$ alkyl;
$R^3$ is selected from the group consisting of $C_{1-4}$ alkyl, $C_{1-4}$ alkoxy, and halo-$C_{1-4}$ alkyl;
p is selected from the group consisting of 0, 1, 2, and 3;
$R^4$ is selected from the group consisting of $C_{1-4}$ alkyl, halo-$C_{1-4}$ alkyl, and 7- to 9-membered bridged heterocyclyl;
q is selected from the group consisting of 0, 1, 2, 3, and 4;
LBM is selected from the group consisting of

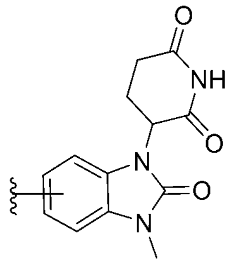

and

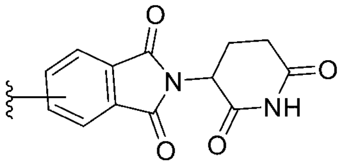

.

**19.** The bifunctional compound, the isomer thereof, the deuterated compound thereof, or the pharmaceutically acceptable salt thereof as claimed in claim 18, wherein ring B is selected from the group consisting of

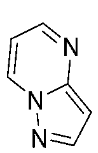 and 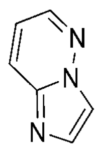 .

**20.** The bifunctional compound, the isomer thereof, the deuterated compound thereof, or the pharmaceutically acceptable salt thereof as claimed in claim 18 or 19, wherein $R^3$ is selected from the group consisting of $-CH_3$, $-CH_2CH_3$, $-OCH_3$, and $-OCH_2CH_3$; and p is selected from the group consisting of 0, 1, and 2.

**21.** The bifunctional compound, the isomer thereof, the deuterated compound thereof, or the pharmaceutically acceptable salt thereof as claimed in any one of claims 18 to 20, wherein the bifunctional compound has a structure shown below:

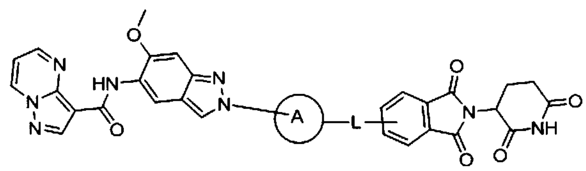

Formula (IB-1)

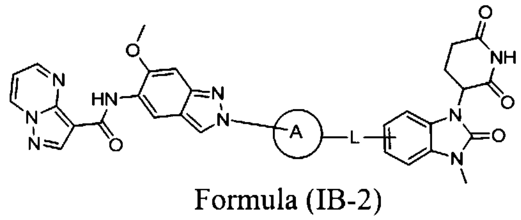

Formula (IB-2)

wherein ring A and L are as defined in any one of claims 1 to 15.

**22.** The bifunctional compound, the isomer thereof, the deuterated compound thereof, or the pharmaceutically acceptable salt thereof as claimed in any one of claims 18 to 21, wherein the bifunctional compound has a structure shown below:

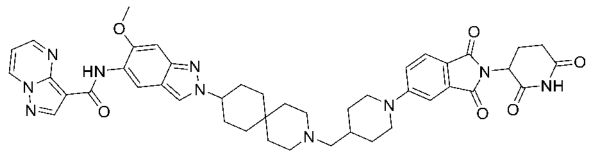

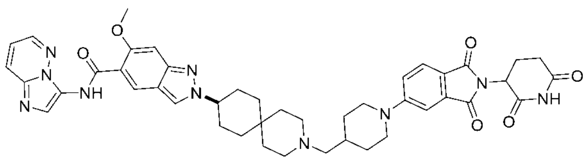

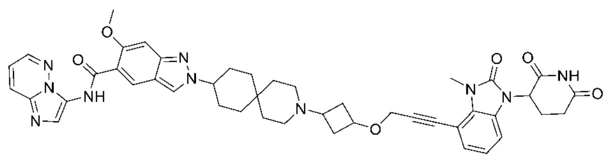

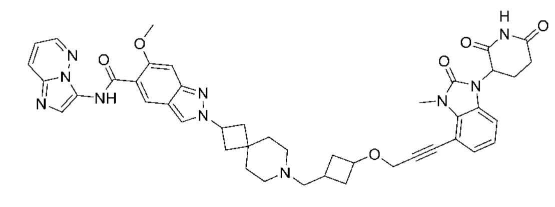

or

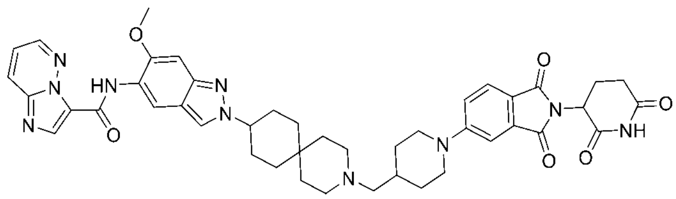

.

**23.** A pharmaceutical composition comprising the bifunctional compound, the isomer thereof, the deuterated compound thereof, or the pharmaceutically acceptable salt thereof as defined in any one of claims 1 to 22, and one or more pharmaceutically acceptable excipients;

preferably, in the pharmaceutical composition, the content of the compound, the isomer thereof, the deuterated compound thereof, or the pharmaceutically acceptable salt thereof is from 1% to 95%;
preferably, in the pharmaceutical composition, the pharmaceutically acceptable excipient comprises one or more of a filler, a disintegrant, a binder, a glidant, or a lubricant.

**24.** Use of the bifunctional compound, the isomer thereof, the deuterated compound thereof, or the pharmaceutically acceptable salt thereof as defined in any one of claims 1 to 22, or the composition as defined in claim 23 in the manufacture of a medicament for preventing and/or treating an IRAK4-mediated related disease;

preferably, the IRAK4-mediated related disease is selected from the group consisting of immune inflammatory diseases;
preferably, the IRAK4-mediated related disease is selected from the group consisting of hidradenitis suppurativa, rheumatoid arthritis, atopic dermatitis, lupus erythematosus, gouty arthritis, psoriasis, asthma, chronic obstructive pulmonary disease, polypoid sinusitis, and inflammatory bowel disease.

**25.** A compound represented by formula (Z), an isomer thereof, a deuterated compound thereof, or a pharmaceutically

acceptable salt thereof:

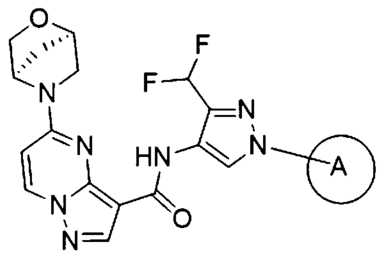

Formula (Z)

wherein ring A is as defined in any one of claims 1 to 15.

**26.** A compound represented by formula (X), an isomer thereof, a deuterated compound thereof, or a pharmaceutically acceptable salt thereof:

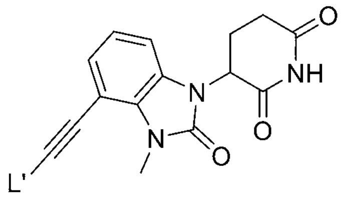

(X)

wherein L is $B^{1-1}$-$B^2$-$B^{3-1}$-;
$B^{1-1}$ is hydroxyl, amino, carboxyl, or hydroxy-substituted $C_{1-3}$ alkyl;
$B^{3-1}$ is a $C_{1-3}$ alkylene chain, any one methylene unit of which is optionally replaced by - O-, -C(O)-, -C($R^a$)($R^b$)-, -N($R^c$)-, or -S-; $R^a$ and $R^b$ are each independently selected from the group consisting of H, deuterium, halogen, $C_{1-6}$ alkyl, and halo-$C_{1-6}$ alkyl, and $R^a$ and $R^b$ are not simultaneously H;
$B^2$ is as defined in any one of claims 1 to 15.

**27.** A compound as shown below, an isomer thereof, a deuterated compound thereof, or a pharmaceutically acceptable salt thereof:

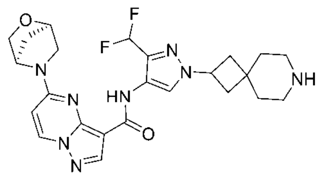 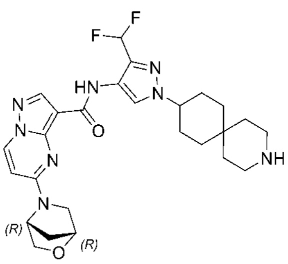 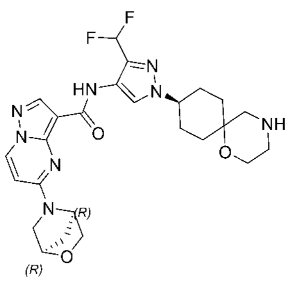 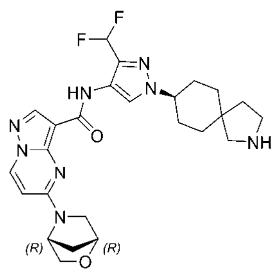

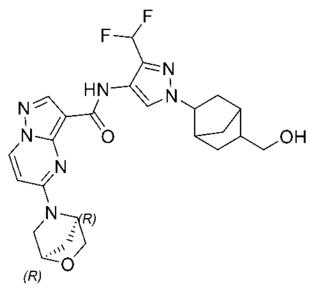 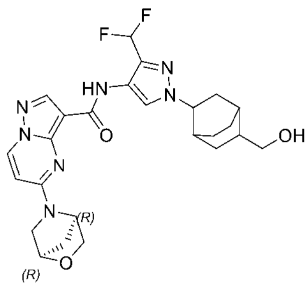 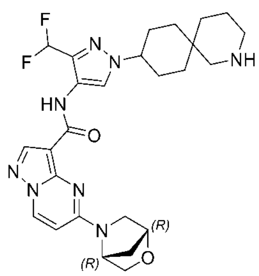 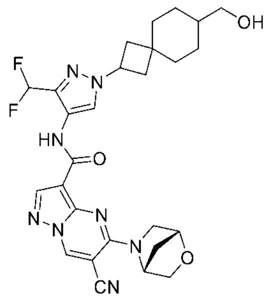

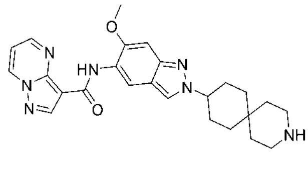
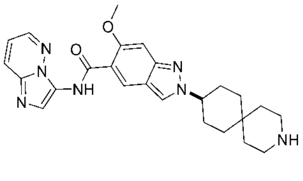
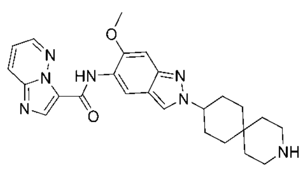
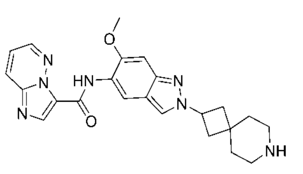

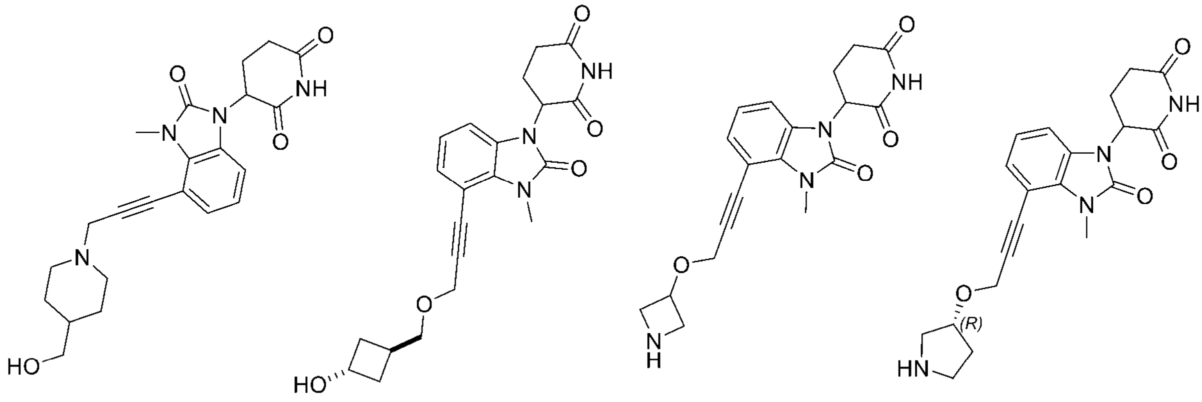

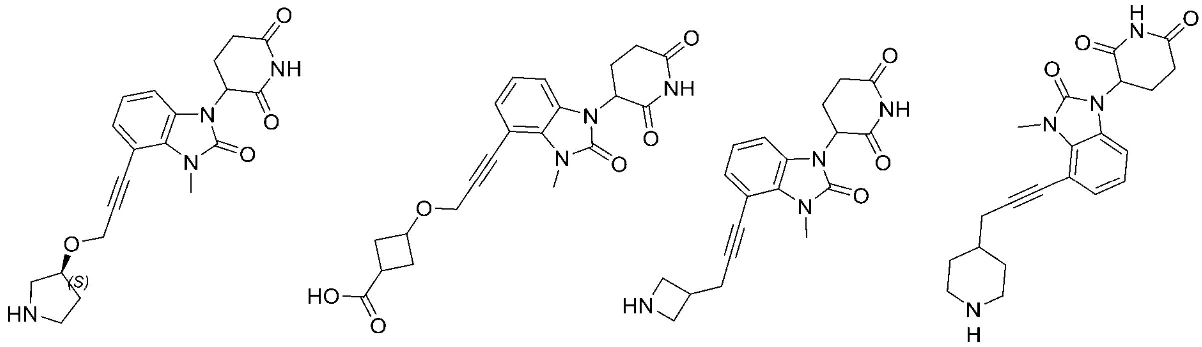

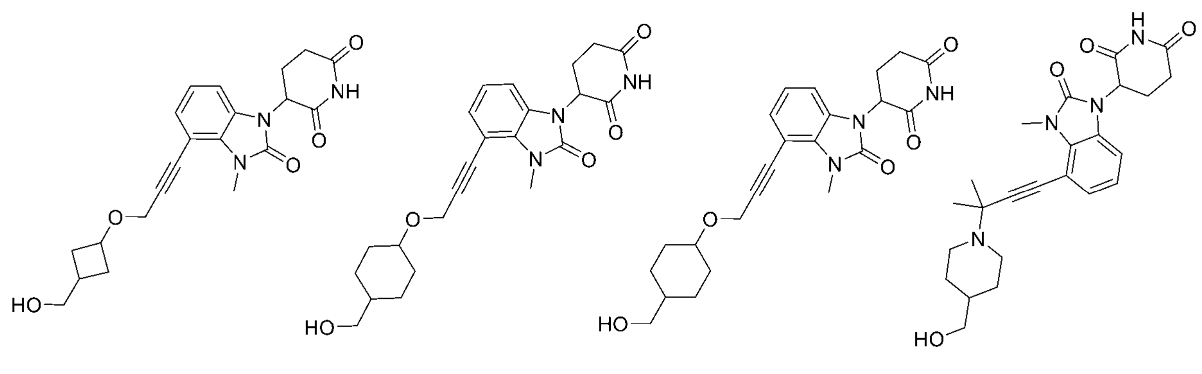

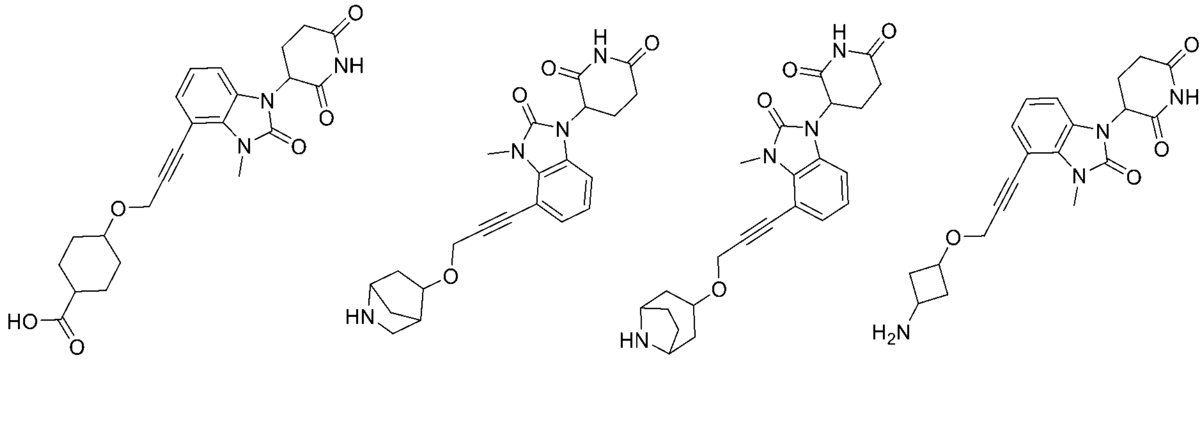

or

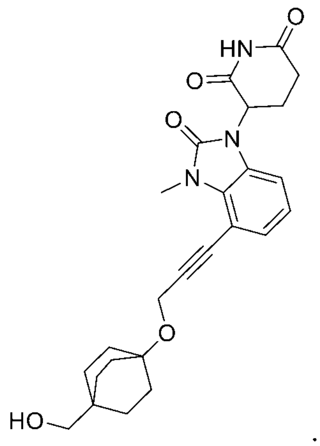

**28.** Use of the compound, the isomer thereof, the deuterated compound thereof, or the pharmaceutically acceptable salt thereof as defined in claim 25 or 26 in the manufacture of an IRAK4 degrader; preferably, the IRAK4 degrader is the bifunctional compound, the isomer thereof, the deuterated compound thereof, or the pharmaceutically acceptable salt thereof as defined in claim 1.

**29.** Use of the compound, the isomer thereof, the deuterated compound thereof, or the pharmaceutically acceptable salt thereof as defined in claim 27 in the manufacture of an IRAK4 degrader.

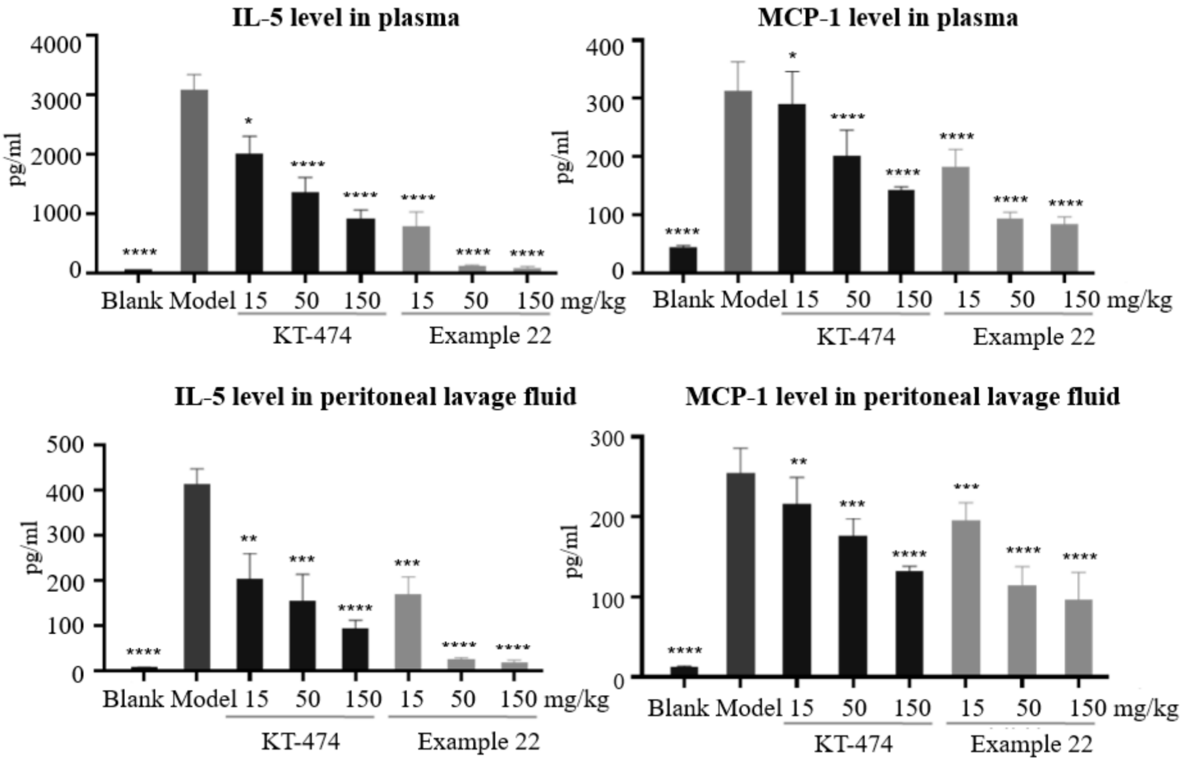
IL-5 level in plasma
pg/ml
4000
3000
2000
1000
0
*
****
Blank Model 15 50 150 15 50 150 mg/kg
KT-474
Example 22
MCP-1 level in plasma
pg/ml
400
300
200
100
0
IL-5 level in peritoneal lavage fluid
500
400
300
200
100
**
***
MCP-1 level in peritoneal lavage fluid


FIG. 1

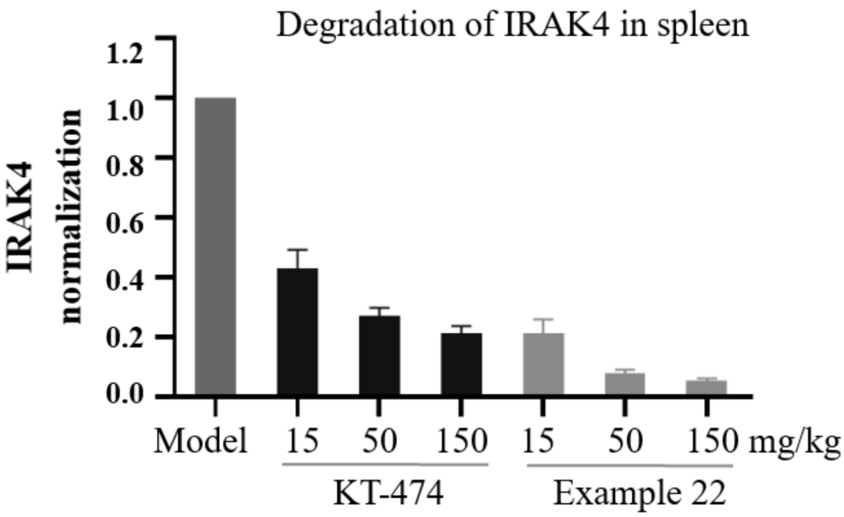
Degradation of IRAK4 in spleen
IRAK4
normalization
1.2
1.0
0.8
0.6
0.4
0.2
0.0
Model 15 50 150 15 50 150 mg/kg
KT-474
Example 22


FIG. 2

**INTERNATIONAL SEARCH REPORT**

International application No.
**PCT/CN2024/079646**

**A. CLASSIFICATION OF SUBJECT MATTER**

C07D487/04(2006.01)i; C07D401/14(2006.01)i; C07D471/04(2006.01)i; C07D471/08(2006.01)i; C07D471/10(2006.01)i; A61K31/513(2006.01)i; A61K31/496(2006.01)i; A61K31/40(2006.01)i; A61P35/00(2006.01)i; A61P37/00(2006.01)i; A61P25/00(2006.01)i; A61P29/00(2006.01)i

According to International Patent Classification (IPC) or to both national classification and IPC

**B. FIELDS SEARCHED**

Minimum documentation searched (classification system followed by classification symbols)

IPC: C07D A61K A61P

Documentation searched other than minimum documentation to the extent that such documents are included in the fields searched

Electronic data base consulted during the international search (name of data base and, where practicable, search terms used)

CNABS, CNTXT, CNKI, 万方数据库, WANFANG DATABASE, 百度学术, BAIDU SCHOLAR, VEN, ENTXT, STN: 齐鲁制药研究中心, 白细胞介素-1, IRAK, 3-甲基-2-氧代-苯并咪唑, 1, 3-二氧代异吲哚啉, 2, 6-二氧代哌啶, 二氟甲基吡唑, 吡唑并[1, 5-a]嘧啶, 氮杂螺[3.5]壬烷, 咪唑并[1, 2-b]哒嗪, 吲唑, 甲酰胺, interleukin-1, 3-methyl-2-oxo-benzo[d]imidazol, 1, 3-dioxoisoindolin, 2, 6-dioxopiperidin, difluoromethyl pyrazol, pyrazolo[1, 5-a]pyrimidine, azaspiro[3.5]nonane, imidazo[1, 2-b]pyridazine, indazol, carboxamide.

**C. DOCUMENTS CONSIDERED TO BE RELEVANT**

| Category* | Citation of document, with indication, where appropriate, of the relevant passages | Relevant to claim No. |
|---|---|---|
| PX | WO 2023241644 A1 (HANGZHOU POLYMED BIOPHARMACEUTICALS, INC.) 21 December 2023 (2023-12-21)<br>pages 2-6, 59, 65, and 66-67 | 18-24 |
| PX | WO 2023237049 A1 (BEIGENE, LTD.) 14 December 2023 (2023-12-14)<br>claims 1-53 | 18-24 |
| X | WO 2021158634 A1 (KYMERA THERAPEUTICS, INC.) 12 August 2021 (2021-08-12)<br>pages 4-8, 107, and 118-124 | 1-17, 23-29 |
| X | WO 2022161414 A1 (INCRELAND) 04 August 2022 (2022-08-04)<br>pages 3-13 and 151 | 25, 27-29 |
| X | WO 2022266258 A1 (ARVINAS OPERATIONS INC.) 22 December 2022 (2022-12-22)<br>pages 4-8, 189-201, and 104-109 | 18-24 |

☑ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **15 May 2024** | **25 May 2024** |

| Name and mailing address of the ISA/CN | Authorized officer |
|---|---|
| **China National Intellectual Property Administration (ISA/CN)**<br>**China No. 6, Xitucheng Road, Jimenqiao, Haidian District, Beijing 100088** | <br>Telephone No. |

Form PCT/ISA/210 (second sheet) (July 2022)

INTERNATIONAL SEARCH REPORT

International application No.
**PCT/CN2024/079646**

**C. DOCUMENTS CONSIDERED TO BE RELEVANT**

| Category* | Citation of document, with indication, where appropriate, of the relevant passages | Relevant to claim No. |
|---|---|---|
| X | CN 114502158 A (KYMERA THERAPEUTICS, INC.) 13 May 2022 (2022-05-13) description, pages 296-369, claim 1, and description, pages 372-374 | 18-24 |
| X | WO 2022147465 A1 (KYMERA THERAPEUTICS INC) 07 July 2022 (2022-07-07) claims 1-27 | 18-24 |
| X | WO 2022088551 A1 (SHANGHAI LEADINGTAC PHARMACEUTICAL CO., LTD.) 05 May 2022 (2022-05-05) claims 1-19 | 18-24 |
| X | WO 2023283610 A1 (BIOGEN MA INC. et al.) 12 January 2023 (2023-01-12) claims 1-62 | 18-24 |
| A | CN 114437035 A (HAISCO PHARMACEUTICAL GROUP CO., LTD.) 06 May 2022 (2022-05-06) entire document | 1-29 |
| A | CN 115710274 A (SHANGHAI LINGTAI BIOMEDICAL TECHNOLOGY CO., LTD.) 24 February 2023 (2023-02-24) entire document | 1-29 |
| A | WO 2021247897 A1 (KYMERA THERAPEUTICS, INC.) 09 December 2021 (2021-12-09) entire document | 1-29 |

Form PCT/ISA/210 (second sheet) (July 2022)

**INTERNATIONAL SEARCH REPORT**
**Information on patent family members**

International application No.
**PCT/CN2024/079646**

| Patent document cited in search report | Publication date (day/month/year) | Patent family member(s) | Publication date (day/month/year) |
|---|---|---|---|
| WO 2023241644 A1 | 21 December 2023 | None | |
| WO 2023237049 A1 | 14 December 2023 | None | |
| WO 2021158634 A1 | 12 August 2021 | EP 4100004 A1 | 14 December 2022 |
| | | EP 4100004 A4 | 28 February 2024 |
| | | US 2023122219 A1 | 20 April 2023 |
| WO 2022161414 A1 | 04 August 2022 | CN 116648248 A | 25 August 2023 |
| WO 2022266258 A1 | 22 December 2022 | US 2023099344 A1 | 30 March 2023 |
| CN 114502158 A | 13 May 2022 | EP 3989966 A1 | 04 May 2022 |
| | | EP 3989966 A4 | 27 September 2023 |
| | | JP 2022538192 A | 31 August 2022 |
| | | WO 2020264499 A1 | 30 December 2020 |
| | | US 2023069104 A1 | 02 March 2023 |
| | | BR 112021026517 A2 | 10 May 2022 |
| | | CA 3144805 A1 | 30 December 2020 |
| | | KR 20220032063 A | 15 March 2022 |
| | | IL 289267 A | 01 February 2022 |
| | | MX 2021015995 A | 11 March 2022 |
| | | AU 2020302118 A1 | 24 February 2022 |
| | | US 2023303526 A1 | 28 September 2023 |
| | | US 2024059674 A1 | 22 February 2024 |
| | | RU 2021137823 A | 28 July 2023 |
| WO 2022147465 A1 | 07 July 2022 | AU 2021413371 A1 | 13 July 2023 |
| | | CA 3202360 A1 | 07 July 2022 |
| | | JP 2024503300 A | 25 January 2024 |
| | | EP 4271664 A1 | 08 November 2023 |
| | | US 2023101353 A1 | 30 March 2023 |
| | | KR 20230143632 A | 12 October 2023 |
| | | CN 116867758 A | 10 October 2023 |
| WO 2022088551 A1 | 05 May 2022 | US 2023192655 A1 | 22 June 2023 |
| | | EP 4238968 A1 | 06 September 2023 |
| | | JP 2023529108 A | 07 July 2023 |
| | | JP 7474527 B2 | 25 April 2024 |
| WO 2023283610 A1 | 12 January 2023 | IL 309941 A | 01 March 2024 |
| | | KR 20240035526 A | 15 March 2024 |
| | | TW 202321236 A | 01 June 2023 |
| | | UY 39844 A | 31 January 2023 |
| | | AU 2022308734 A1 | 22 February 2024 |
| | | CA 3224732 A1 | 12 January 2023 |
| CN 114437035 A | 06 May 2022 | None | |
| CN 115710274 A | 24 February 2023 | WO 2023025159 A1 | 02 March 2023 |
| | | AU 2022334530 A1 | 04 April 2024 |
| | | TW 202315606 A | 16 April 2023 |
| | | CA 3229646 A1 | 02 March 2023 |
| WO 2021247897 A1 | 09 December 2021 | EP 4161521 A1 | 12 April 2023 |
| | | US 2023250110 A1 | 10 August 2023 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- CN 2023102043068
- CN 2023105328859
- CN 2023107154349
- CN 2023110041666
- CN 2024101967699
- WO 2020113233 A1


### Non-patent literature cited in the description


- **JAESEOK LEE et al.** Discovery of E3 Ligase Ligands for Target Protein Degradation. *Molecules*, 2022, vol. 27 (19), 6515